# EUROPEAN PATENT APPLICATION

(11) **EP 1 548 131 A2**
(43) Date of publication of application: **29.06.2005**
(21) Application number: 04029642.8
(22) Date of filing: 15.12.2004
(51) Int. Cl.: C12Q 1/68

(54) **Novel targets for obesity from skeletal muscle**

(30) Priority: 22.12.2003 EP 03104899
(71) Applicant: F. Hoffmann-La Roche AG, 4070 Basel (CH); Claes-Goran Ostenson, 17164 Solna (SE)
(72) Inventor: Clerc, Roger G., 4102 Binningen (CH); Duchateau-Nguyen, Guillemette, 68400 Riedisheim (FR); Gardes, Christophe, 68870 Bartenheim (FR); Mizrahi, Jacques, 4103 Bottmingen (CH); Ostenson, Claes-Goran, c/o Karolinska Hospital, 171 76 Stockholm (SE)
(74) Representative: Klostermeyer, Doerte

(57) **Abstract**

The present invention relates to novel targets identified in skeletal muscle for screening of compounds that may be useful for the prevention and treatment of obesity.

## Description

Multifactorial diseases such as obesity are caused by mutations in more than one gene with a large contribution from environmental factors. There has been spectacular success in identifying the genes responsible for Mendelian disorders, whereas finding the susceptibility genes involved in multifactorial diseases has so far been difficult. The evidence suggests that humans inherit a genetic predisposition to gain weight on a high fat diet. Therefore optimizing patient sampling for the collection of tissues on the bases of clinical and physiological parameters is critical.

There is clearly an unmet medical need for novel therapeutic solutions to this health problem, in particular in the light of the fact that current medication that promote weight loss are transient as the lost excess of weight is gained back within 1 to 5 years. Therefore, there is a need to identify new targets for the development of new treatments.

The invention provides methods (also referred to herein as "screening assays") for identifying compounds which can be used for the modulation of body weight, e.g., for the treatment of a body weight disorder.

A set of 8000 patients, enrolled in a Diabetes/Obesity Prevention Program with the Stockholm Prevention Program, was monitored for a number of clinical parameters and vital signs. From this large pool of patients, a clinically well annotated series of tissue biopsies from 10 obese, non diabetic, and 10 matched control patients were analyzed for gene expression profiling. The following matched clinical parameters and vital signs were, among others, used to recruit these patients: BMI (control mean=22.2 sd+/-1.3 and case mean=32.8 sd+/-2.1), age (control mean=54.6 years and case mean=56.3 years), male gender, VO2 ratio, total fat versus truncal fat, waist-hip ratio, energy expediture, blood pressure, FA oxidation; CHO oxydation, OGTT negative, birth weight, no diabetes in the family, no smoking, sedentary and no alcohol habits.

**Table 1**

| **Obese** | **Control** |
|---|---|
| BMI 30-35 | BMI 20-23 |
| without impaired OGTT | without impaired OGTT |
| without type 2 diabetes | without type 2 diabetes |

Other parameters used for patient selection:
* family history of diabetes
* birth weight
* blood pressure
* medication (if any)
* food intake
* physical activity education
* bodyweight history
* chronic illness
* tobacco and alcohol use
* housing conditions
* socio-economical factors

The methods provided by this invention entail identifying candidate or test compounds or agents (e.g., peptides, peptidomimetics, small molecules or other drugs) which bind a polypeptide selected from the group consisting of the polypeptides of Seq ID No. 9 to 16, and/or have a stimulatory or inhibitory effect on the activity or the expression of a polypeptide selected from the group consisting of the polypeptides of Seq ID No. 9 to 16, and then determining which of the compounds that bind a polypeptide selected from the group consisting of the polypeptides of Seq ID No. 9 to 16 or have a stimulatory or inhibitory effect on the activity or the expression of a polypeptide selected from the group consisting of the polypeptides of Seq ID No. 9 to 16 have an effect on the feeding behavior, body weight, or metabolic rate of a mammal (e.g., a mouse or a rat) in an in vivo assay.

The present invention pertains to a method of screening for compounds that reduce and/or prevent obesity comprising: a) contacting a cell expressing a gene listed in table 2 with a compound; and b) measuring the expression of said gene, or a polypeptide encoded by said gene; wherein a compound which up-regulates expression is a compound which causes an increase of expression of said gene or of the polypeptide encoded by said gene.

The term "up-regulation of expression" as used herein refers to an increase in expression of mRNA levels of a nucleic acid, or to an increase in expression of polypeptide levels. This term may also relate to increased post-translational modifications that are necessary for the activity and/or function of a polypeptide, e.g. addition of sugar moieties, phosphorylation etc.

A cell used in the method hereinbefore described, or in any of the methods hereinafter described may be a skeletal muscle cell, or a host or host cell as defined hereinafter.

Preferably, said gene is Seq ID. No. 1. In another preferred embodiment, said gene is Seq ID No. 2. In another preferred embodiment, said gene is Seq ID No. 3. In another preferred embodiment, said gene is Seq ID No. 4. In another preferred embodiment, said gene is Seq ID No. 5. In another preferred embodiment, said gene is Seq ID No. 6.

Preferably, said polypeptide is Seq ID No. 9. In another preferred embodiment, said polypeptide is Seq ID No. 10. In another preferred embodiment, said polypeptide is Seq ID No. 11. In another preferred embodiment, said polypeptide is Seq ID No. 12. In another preferred embodiment, said polypeptide is Seq ID No. 13. In another preferred embodiment, said polypeptide is Seq ID No. 14.

The present invention also pertains to a method of screening for compounds that reduce and/or prevent obesity comprising a) contacting a cell expressing a gene listed in table 3 with a compound; and b) measuring the expression of said gene, or a polypeptide encoded by said gene;
wherein a compound which down-regulates gene expression is a compound which causes a decrease of said gene or a polypeptide encoded by said gene.

The term "down-regulation of expression" as used herein refers to a decrease in expression of mRNA levels of a nucleic acid, or to a decrease in expression of polypeptide levels. This term may also relate to decreased post-translational modifications that are necessary for the activity and/or function of a polypeptide, e.g. addition of sugar moieties, phosphorylation etc.

Preferably, said gene is Seq ID. No. 7. In another preferred embodiment, said gene is Seq ID No. 8.

Preferably, said polypeptide is Seq ID No. 15. In another preferred embodiment, said polypeptide is Seq ID No. 16.

The present invention provides a method of screening for compounds that reduce and/or prevent obesity comprising: a) contacting a cell expressing a gene selected from the group consisting of Seq ID No. 17 to 27 with a compound; and b) measuring the expression of said gene, or a polypeptide encoded by said gene; wherein a compound which up-regulates gene expression is a compound which causes an increase of expression of said gene or of the polypeptide encoded by said gene.

The present invention further provides a method of screening for compounds that reduce and/or prevent obesity comprising: a) contacting a cell expressing a gene selected from the group consisting of Seq ID No. 28 to 43 with a compound; and b) measuring the expression of said gene, or a polypeptide encoded by said gene; wherein a compound which down-regulates gene expression is a compound which causes a decrease of said gene or a polypeptide encoded by said gene.

The present invention provides a method of screening for compounds that reduce and/or prevent obesity comprising: a) contacting a polypeptide selected from the group consisting of Seq ID No. 9 to 14 with a compound; and b) determining and/or measuring the activity and/or function of said polypeptide; wherein a compound which reduces and/or prevents obesity by agonizing said polypeptide is a compound which causes an increase in activity and/or function of said polypeptide.

The present invention also pertains to a method for screening of compounds that reduce and/or prevent obesity comprising: a) contacting a cell expressing a nucleic acid encoding a polypeptide selected from the group consisting of Seq ID No. 9 to 14 with a compound; and b) determining and/or measuring the activity and/or function of said polypeptide; wherein a compound which reduces and/or prevents obesity by agonizing said polypeptide is a compound which causes an increase in activity and/or function of said polypeptide.

Preferably, said polypeptide is Seq ID No. 9. In another preferred embodiment, said polypeptide is Seq ID No. 10. In another preferred embodiment, said polypeptide is Seq ID No. 11. In another preferred embodiment, said polypeptide is Seq ID No. 12. In another preferred embodiment, said polypeptide is Seq ID No. 13. In another preferred embodiment, said polypeptide is Seq ID No. 14.

The present invention also provides for a method of screening for compounds that reduce and/or prevent obesity comprising: a) contacting a polypeptide selected from the group consisting of Seq ID No. 44 to 54 with a compound; and b) determining and/or measuring the activity and/or function of said polypeptide; wherein a compound which reduces and/or prevents obesity by agonizing said polypeptide is a compound which causes an increase in activity and/or function of said polypeptide.

The present invention further provides a method of screening for compounds that reduce and/or prevent obesity comprising: a) contacting a polypeptide selected from the group consisting of Seq ID No. 55 to 70 with a compound; and b) determining and/or measuring the activity and/or function of said polypeptide; wherein a compound which reduces and/or prevents obesity by antagonizing said polypeptide is a compound which causes a decrease in activity and/or function of said polypeptide.

The terms "activity and/or function" as used with respect to the polypeptide encoded by Seq ID No. 1, phospholamban, refers to, as an example, phosphorylation of phospholamban, or phospholamban-dependent Calcium pump activity. Assays to determine these activities are well known in the art and are e.g. described in Tada et al., J. Mol. Cell Cardiol. 1983, 15, 335-346; Koller et al., Biochem. Biophys. Res. Comm. 2003, 300, 155-160; Cornwell et al., Mol. Pharmacol. 1991,40,923-931; Cantilina et al., J. Biol. Chem. 1993, 268, 17018-17025; Suzuki et al., 1986, J. Biol. Chem. 261, 7018-7023.

The terms "activity and/or function" as used with respect to the polypeptide encoded by Seq ID No. 2, sterol carrier protein 2 (SCP2), refers to sterol carrier activity and/or cholesterol exchange. Assays to measure the activity and/or function of SCP2 are well known in the art, and are, for example, described in Seedorf et al., J. Biol. Chem. 1994, 269, 2613-2618; Schroeder et al., Lipids 1990, 25, 669-674.

The terms "activity and/or function" as used with respect to the polypeptide encoded by Seq ID No. 3, beta 1,4-galactosyltransferase 5, refers to the galactosyl transferase activity of beta 1,4-galactosyltransferase. Assays to determine these activities are well known in the art and are e.g. described in Malissard et al., Eur. J. Biochem. 1996, 239, 340-348; Taki et al., 1994, Anal Biochem. 219, 104-108; Keusch et al., 1995, Glycobiol. 5, 365-700.

The terms "activity and/or function" as used with respect to the polypeptide encoded by Seq ID No. 4, lipoprotein lipase, refers to LPL activity. Assays to determine these activities are well known in the art and are e.g. found in Dugi et al., 2002, Atherosclerosis 163, 127-134; Ruge et al., 2001, Eur. J. Clin. Invest. 31, 1040-1047.

The terms "activity and/or function" as used with respect to the polypeptide encoded by Seq ID No. 5, low density lipoprotein-related protein 1b, refers to binding and internalization of single chain urokinase and PAI-1, to binding to urokinase plasminogen activator receptor, and to effects on cell migration. Assays to determine these activities are well known in the art and are found, e.g. in Li et al., 2002, J. Biol. Chem. 277, 42366-42371; Liu et al., 2001, J. Biol. Chem. 276, 28889-28896.

The terms "activity and/or function" as used with respect to the polypeptide encoded by Seq ID No.7, fatty acid desaturase 2 (FADS2), refers to desaturase activity of FADS2. Assays to determine this activitiy are well known in the art and are described, e.g. in Ge et al., J. Invest. Dermatol. 2003, 120, 707-714.

The terms "activity and/or function" as used with respect to the polypeptide encoded by Seq ID No.8, retinoic acid receptor-related orphan receptor a (RORA), refers to the activation of transcription by RORA. Assays to determine this activity are well known in the art and are found e.g. in Chauvet et al., Biochem. J. 2002, 264, 449-456.

The present invention pertains to a method for screening of compounds that reduce and/or prevent obesity comprising: a) contacting a cell expressing a polypeptide selected from the group consisting of Seq ID No. 15 and 16 with a compound; and b) determining and/or measuring the activity and/or function of said gene, or a polypeptide encoded by said gene; wherein a compound which reduces and/or prevents obesity by antagonizing said polypeptide is a compound which causes a decrease in activity and/or function of said polypeptide.

The present invention provides a method for screening of compounds that reduce and/or prevent obesity comprising: a) contacting a cell expressing a nucleic acid encoding a polypeptide selected from the group consisting of Seq ID No. 15 and 16 with a compound; and b) determining and/or measuring the activity and/or function of said polypeptide; wherein a compound which reduces and/or prevents obesity by antagonizing said polypeptide is a compound which causes a decrease in activity and/or function of said polypeptide.

Preferably, said polypeptide is Seq ID No. 15. In another preferred embodiment, said polypeptide is Seq ID No. 16.

The present invention also pertains to a method for screening of compounds that reduce and/or prevent obesity comprising: a) contacting a cell expressing a nucleic acid encoding a polypeptide selected from the group consisting of Seq ID No. 44 to 54 with a compound; and b) determining and/or measuring the activity and/or function of said polypeptide; wherein a compound which reduces and/or prevents obesity by agonizing said polypeptide is a compound which causes an increase in activity and/or function of said polypeptide.

The present invention further pertains to a method for screening of compounds that reduce and/or prevent obesity comprising: a) contacting a cell expressing a nucleic acid encoding a polypeptide selected from the group consisting of Seq ID No. 55 to 70 with a compound; and b) determining and/or measuring the activity and/or function of said polypeptide; wherein a compound which reduces and/or prevents obesity by antagonizing said polypeptide is a compound which causes a decrease in activity and/or function of said polypeptide.

The present invention also provides a method of screening for compounds that bind to a polypeptide selected from the group consisting of the polypeptides of Seq ID No. 9 to 16, comprising the steps of a) contacting a compound with said polypeptide; and b) determining the ability of said compound to bind to said polypeptide.

The present invention pertains to a method of screening for compounds that bind to a polypeptide selected from the group consisting of the polypeptides of Seq ID No. 44 to 70, comprising the steps of: a) contacting a compound with said polypeptide; and b) determining the ability of said compound to bind to said polypeptide.

Candidate or test compounds or agents which bind a polypeptide selected from the group consisting of Seq ID No 9 to 16, or the group consisting of Seq ID No. 44 to 70 and/or have a stimulatory or inhibitory effect on the activity or the expression of said polypeptide are identified in assays that employ either cells which express a form of said polypeptide (cell-based assays) or isolated polypeptide (cell-free assays). The various assays can employ a variety of forms of said polypeptide (e.g., full-length polypeptide, a biologically active fragment of a polypeptide, or a fusion protein which includes all or a portion of said polypeptide). Moreover, the polypeptide can be derived from any suitable mammalian species. The assay can be a binding assay entailing direct or indirect measurement of the binding of a test compound or the polypeptide to a known ligand or receptor, as defined above. The assay can also be an activity assay entailing direct or indirect measurement of the activity of said polypeptide. The assay can also be an expression assay entailing direct or indirect measurement of the expression of said polypeptide (e.g., polypeptide- encoding mRNA or the polypeptide). The various screening assays are combined with an in vivo assay entailing measuring the effect of the test compound on the feeding behavior, body weight, or metabolic rate of a mammal (e.g., a mouse or a rat).

In another embodiment, the assay is a cell-based assay comprising contacting a cell expressing a polypeptide (e.g., full-length polypeptide, a biologically active fragment of said polypeptide, or a fusion protein which includes all or a portion of said polypeptide) with a test compound and determining the ability of the test compound to modulate (e.g., stimulate or inhibit) the activity of the polypeptide. Determining the ability of the test compound to modulate the activity of said polypeptide can be accomplished by any method suitable for measuring the activity of said polypeptide.

The present invention also includes cell-free assays. Such assays involve contacting a form of a polypeptide selected from the group consisting of Seq ID No 9 to 16, or the group consisting of Seq ID No. 44 to 70 (e.g., full-length polypeptide, a biologically active fragment of said polypeptide, or a fusion protein comprising all or a portion of said polypeptide) with a test compound and determining the ability of the test compound to bind to said polypeptide. Binding of the test compound to said polypeptide can be determined either directly or indirectly as described above. In one embodiment, the assay includes contacting the said polypeptide with a known compound which binds said polypeptide to form an assay mixture, contacting the assay mixture with a test compound, and determining the ability of the test compound to interact with said polypeptide, wherein determining the ability of the test compound to interact with said polypeptide comprises determining the ability of the test compound to preferentially bind to the said polypeptide as compared to the known compound.

The cell-free assays of the present invention are amenable to use of either a membrane-bound form of a polypeptide or a soluble fragment thereof. In the case of cell-free assays comprising the membrane-bound form of the polypeptide, it may be desirable to utilize a solubilizing agent such that the membrane-bound form of the polypeptide is maintained in solution. Examples of such solubilizing agents include nonionic detergents such as n-octylglucoside, n-dodecylglucoside, n-dodecylmaltoside, octanoyl-N-mcthylglucamidc, decanoyl-Nmethylglucamide, Triton X-100, Triton X-114, Thesit, Isotridecypoly(ethylene glycol ether)n, 3-[(3-cholamidopropyl)dimethylamminio]-1-propane sulfonate (CHAPS), 3- [ (3-cholamidopropyl)dimethylamminio]-2-hydroxy-1-propane sulfonate (CHAPSO), or N-dodecyl-N, N-dimethyl-3-ammonio-1 -propane sulfonate.

In various embodiments of the above assay methods of the present invention, it may be desirable to immobilize a polypeptide to facilitate separation of complexed from uncomplexed forms of the polypeptide with a binding molecule, as well as to accommodate automation of the assay. Binding of a test compound to a polypeptide, or interaction of a polypeptide with a binding molecule in the presence and absence of a candidate compound, can be accomplished in any vessel suitable for containing the reactants. Examples of such vessels include microtitre plates, test tubes, and microcentrifuge tubes. In one embodiment, a fusion protein can be provided which adds a domain that allows one or both of the proteins to be bound to a matrix. For example, glutathione-S-transferase fusion proteins can be adsorbed onto glutathione sepharose beads (Sigma Chemical; St. Louis, Mo.) or glutathione derivatized microtitre plates, which are then combined with the test compound or the test compound and either the non-adsorbed binding protein or polypeptide, and the mixture incubated under conditions conducive to complex formation (e.g., at physiological conditions for salt and pH). Following incubation, the beads or microtitre plate wells are washed to remove any unbound components and complex formation is measured either directly or indirectly, for example, as described above. Alternatively, the complexes can be dissociated from the matrix, and the level of binding or activity of a polypeptide hereinbefore described can be determined using standard techniques.

Other techniques for immobilizing proteins on matrices can also be used in the screening assays of the invention. For example, either a polypeptide hereinbefore described or its binding molecule can be immobilized utilizing conjugation of biotin and streptavidin. Biotinylated polypeptide of the invention or target molecules can be prepared from biotin-NHS (N-hydroxy-succinimide) using techniques well known in the art (e.g., biotinylation kit, Pierce Chemicals; Rockford, Ill.), and immobilized in the wells of streptavidin-coated 96 well plates (Pierce Chemical). Alternatively, antibodies reactive with a polypeptide or binding molecules, but which do not interfere with binding of the polypeptide of the invention to its binding molecule, can be derivatized to the wells of the plate. Unbound binding protein or polypeptide of the invention is trapped in the wells by antibody conjugation. Methods for detecting such complexes, in addition to those described above for the GST-immobilized complexes, include immunodetection of complexes using antibodies reactive with a polypeptide hereinbefore described or binding molecule, as well as enzyme-linked assays which rely on detecting an enzymatic activity associated with a polypeptide or binding molecule.

### II. Test Compounds

Suitable test compounds for use in the screening assays of the invention can be obtained from any suitable source, e.g., conventional compound libraries. The test compounds can also be obtained using any of the numerous approaches in combinatorial library methods known in the art, including biological libraries; spatially addressable parallel solid phase or solution phase libraries; synthetic library methods requiring deconvolution; the "one-bead one-compound" library method; and synthetic library methods using affinity chromatography selection. The biological library approach is limited to peptide libraries, while the other four approaches are applicable to peptide, non-peptide oligomer or small molecule libraries of compounds (Lam (1997) *Anticancer Drug Des. 12:145).*

Examples of methods for the synthesis of molecular libraries can be found in the art, for example in: DeWitt et al. (1993) *Proc. Natl. Acad. Sci. USA* 90:6909; Erb Ct al. (1994) *Proc. Natl.Acad. Sci. USA* 91:11422; Zuckermann et al. (1994). *J. Med. Chem.* 37:2678; Cho et al. (1993) *Science* 261:1303; Carrell et al. *(1994) Angew Chem. Int. Ed.* *Engl*. 33:2059; Carell et al. (1994) *Angew Chem*. *Int*. *Ed. Engi*.33:2061; and Gallop et al. (1994) J. *Med. Chem*. 37:1233.

Libraries of compounds may be presented in solution (e.g., Houghten (1992) *BioTechniques* 13.412-421), or on beads (Lam (1991) *Nature* 354:82-84), chips (Fodor (1993) *Nature 364:555-556),* bacteria (U.S. Pat. No.5,223,409), spores (U.S. Pat. Nos. *5,571,698;* 5,403,484; and 5,223, 409), plasmids (Cull et al. (1992) *Proc. Natl. Acad. Sci*. USA 89.1865-1869) or phage (Scott and Smith (1990) Science249:386-390; Devlin (1990) *Science* 249:404-406; Cwirla et al. (1990) *Proc. Natl*. *Acad. Sci. USA* 87:6378-6382; and Felici (1991) *J. Mol. Biol*. 222:301-310).

The present invention provides a compound identified by any of the methods hereinbefore described.

### III. Isolated Nucleic Acid Molecules

One aspect of the invention pertains to isolated nucleic acid molecules that encode a polypeptide selected from the group consisting of Seq ID No. 9 to 16, or the group consisting of 44 to 70 or a biologically active portion thereof, as well as nucleic acid molecules sufficient for use as hybridization probes to identify nucleic acid molecules encoding a gene selected from the group consisting of Seq ID No. 1 to 8 or the group consisting of Seq ID No. 17 to 43 and fragments of such nucleic acid molecules suitable for use as PCR primers for the amplification or mutation of nucleic acid molecules. As used herein, the term "nucleic acid molecule" is intended to include DNA molecules (e.g., cDNA or genomic DNA) and RNA molecules (e.g., mRNA) and analogs of the DNA or RNA generated using nucleotide analogs. The nucleic acid molecule can be single-stranded or double-stranded, but preferably is double-stranded DNA. This section describes the nucleic acids hereinbefore described and methods for making and using such nucleic acids.

An "isolated" nucleic acid molecule is one which is separated from other nucleic acid molecules which are present in the natural source of the nucleic acid molecule. Preferably, an "isolated" nucleic acid molecule is free of sequences (preferably protein encoding sequences) which naturally flank the nucleic acid (i.e., sequences located at the 5' and 3' ends of the nucleic acid) in the genomic DNA of the organism from which the nucleic acid is derived. For example, in various embodiments, the isolated nucleic acid molecule can contain less than about 5 kb, 4 kb, 3 kb, 2 kb, 1 kb, 0.5 kb or 0.1 kb of nucleotide sequences which naturally flank the nucleic acid molecule in genomic DNA of the cell from which the nucleic acid is derived. Moreover, an "isolated" nucleic acid molecule, such as a cDNA molecule, can be substantially free of other cellular material, or culture medium when produced by recombinant techniques, or substantially free of chemical precursors or other chemicals when chemically synthesized.

A nucleic acid molecule of the present invention can be isolated using standard molecular biology techniques and the sequence information provided herein. Using all or a portion of the nucleic acid sequences selected from the group consisting of Seq ID No. 1 to 8 or from the group consisting of Seq ID No. 17 to 43 as a hybridization probe, nucleic acid molecules of the invention can be isolated using standard hybridization and cloning techniques (e.g., as described in Sambrook et al., eds., *Molecular Cloning: A Laboratory Manual, 2nd ed.,* Cold Spring Harbor Laboratory, Cold Spring Harbor Laboratory Press, Cold Spring Harbor, N.Y, 1989).

A nucleic acid molecule of the invention can be amplified using cDNA, mRNA or genomic DNA as a template and appropriate oligonucleotide primers according to standard PCR amplification techniques. The nucleic acid so amplified can be cloned into an appropriate vector and characterized by DNA sequence analysis. Furthermore, oligonucleotides corresponding to all or a portion of a nucleic acid molecule of the invention can be prepared by standard synthetic techniques, e.g., using an automated DNA synthesizer.

Moreover, a nucleic acid molecule of the invention can comprise only a portion of a nucleic acid sequence encoding a polypeptide selected from the group consisting of Seq ID No. 9 to 16, or the group consisting of Seq ID No. 44 to 70, for example, a fragment which can be used as a probe or primer or a fragment encoding a biologically active portion of a polypeptide selected from the group consisting of Seq ID No.9 to 16, or the group consisting of Seq ID No. 44 to 70. The nucleotide sequence determined from the cloning of any one of the genes listed in tables 2 and 3, or 4 and 5 for the generation of probes and primers designed for use in identifying and/or cloning allelic variants and other variants of any one of the genes listed in tables 2 and 3, or 4 and 5. The probe/primer typically comprises substantially purified oligonucleotide. The oligonucleotide typically comprises a region of nucleotide sequence that hybridizes under stringent conditions to at least about 12, preferably about 25, more preferably about 50, 75, 100, 125, 150, 175, 200, 250, 300, 350 or 400 consecutive nucleotides of the sense or antisense sequence of any one of the genes listed in tables 2 and 3, or 4 and 5 or naturally occurring mutant or allelic variant thereof.

Probes based on the sequence of a nucleic acid molecule of the invention can be used to detect transcripts or genomic sequences encoding the same protein molecule encoded by a selected nucleic acid molecule. The probe comprises a label group attached thereto, e.g., a radioisotope, a fluorescent compound, an enzyme, or an enzyme cofactor. Such probes can be used as part of a diagnostic test kit for identifying cells or tissues which miss-express the protein, such as by measuring levels of a nucleic acid molecule encoding the protein in a sample of cells from a subject, e.g., detecting mRNA levels or determining whether a gene encoding the protein has been mutated or deleted.

A nucleic acid fragment encoding a "biologically active portion" of a polypeptide selected from the group consisting of Seq ID No. 9 to 16, or the group consisting of Seq ID No. 44 to 70 can be prepared by isolating a portion of nucleic acid which encodes a polypeptide having a biological activity, expressing the encoded portion of the polypeptide protein (e.g., by recombinant expression in vitro) and assessing the activity of the encoded portion of the polypeptide.

The invention further encompasses nucleic acid molecules that differ from the nucleotide sequence of a nucleic acid selected from the group consisting of Seq ID No. 1 to 8 or the group consisting of Seq ID No. 17 to 43 due to degeneracy of the genetic code and thus encode the same protein as that encoded by the said nucleotide sequence.

In addition to the nucleotide sequence of any one of Seq ID No. 1 to 8 or Seq ID No. 17 to 43, it will be appreciated by those skilled in the art that DNA sequence polymorphisms that lead to changes in the amino acid sequence may exist within a population. Such genetic polymorphisms may exist among individuals within a population due to natural allelic variation. An allele is one of a group of genes which occur alternatively at a given genetic locus. As used herein, the phrase "allelic variant" refers to a nucleotide sequence which occurs at a given locus or to a polypeptide encoded by the nucleotide sequence. Such natural allelic variations can typically result in *1-5%* variance in the nucleotide sequence of a given gene. Alternative alleles can be identified by sequencing the gene of interest in a number of different individuals. This can be readily carried out by using hybridization probes to identify the same genetic locus in a variety of individuals. Any and all such nucleotide variations and resulting amino acid polymorphisms or variations that are the result of natural allelic variation and that do not alter the functional activity are intended to be within the scope of the invention.

Accordingly, in another embodiment, an isolated nucleic acid molecule of the invention is at least 300 (325, 350, 375, 400, 425, 450, 500, 550, 600, 650, 700, 800, 900, 1000, or 1290) nucleotides in length and hybridizes under stringent conditions to the nucleic acid molecule comprising the nucleotide sequence, preferably the coding sequence of any one of the genes listed in table 2 and/or 3 and/or 4 and/or 5 and encodes an allelic variant or mutant of said gene.

As used herein, the term "hybridizes under stringent conditions" is intended to describe conditions for hybridization and washing under which nucleotide sequences at least 60% (65%, 70%, preferably 75%) identical to each other typically remain hybridized to each other. Such stringent conditions are known to those skilled in the art and can be found in *Current Protocols in Molecular Biology,* John Wiley & Sons, N.Y (1989), 6.3.1-6.3.6. A preferred, non-limiting example of stringent hybridization conditions are hybridization in 6xsodium chloride/sodium citrate (SSC) at about 45 degrees C., followed by one or more washes in 0.2xSSC, 0.1% SDS at 50-65 degrees C. Preferably, an isolated nucleic acid molecule of the invention that hybridizes under stringent conditions to the sequence selected from the group consisting of Seq ID No. 1 to 8 or the group consisting of Seq ID No. 17 to 43, corresponds to a naturally-occurring nucleic acid molecule. As used herein, a "naturally-occurring" nucleic acid molecule refers to an RNA or DNA molecule having a nucleotide sequence that occurs in nature (e.g., encodes a natural protein).

In addition to naturally occurring allelic variants of any one of the genes listed in tables 2 and 3, 4 and 5 the skilled artisan will further appreciate that changes can be introduced by mutation thereby leading to changes in the amino acid sequence of the encoded protein, without altering the biological activity of the protein. For example, one can make nucleotide substitutions leading to amino acid substitutions at "non-essential" amino acid residues. A "non-essential" amino acid residue is a residue that can be altered from the wild-type sequence without altering the biological activity, whereas an "essential" amino acid residue is required for biological activity. For example, amino acid residues that are not conserved or only semi-conserved among homologues of various species may be non-essential for activity and thus would be likely targets for alteration. Alternatively, amino acid residues that are conserved among the homologues of various species (e.g., murine and human) may be essential for activity and thus would not be likely targets for alteration.

Accordingly, another aspect of the invention pertains to nucleic acid molecules encoding a polypeptide selected from the group consisting of Seq ID No. 9 to 16, or the group consisting of Seq ID No. 44 to 70, that contain changes in amino acid residues that are not essential for activity. Such polypeptides differ in amino acid sequence from said polypeptide yet retain biological activity. In one embodiment, the isolated nucleic acid molecule includes a nucleotide sequence encoding a protein that includes an amino acid sequence that is at least about 85%, 95%, 96%, 97%, 98%, or 99% identical to the amino acid sequence of any one of Seq ID No. 9 to 16 or 44 to 70.

An isolated nucleic acid molecule encoding a variant protein can be created by introducing one or more nucleotide substitutions, additions or deletions into the nucleotide sequence of any one of the genes listed in tables 2 and 3, 4 and 5 such that one or more amino acid substitutions, additions or deletions are introduced into the encoded protein. Mutations can be introduced by standard techniques, such as site-directed mutagenesis and PCR-mediated mutagenesis. Preferably, conservative amino acid substitutions are made at one or more predicted non-essential amino acid residues. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). Alternatively, mutations can be introduced randomly along all or part of the coding sequence, such as by saturation mutagenesis, and the resultant mutants can be screened for biological activity to identify mutants that retain activity. Following mutagenesis, the encoded protein can be expressed recombinantly and the activity of the protein can be determined.

In one embodiment, a mutant polypeptide that is a variant of a polypeptide selected from the group consisting of Seq ID No. 9 to 16, or the group consisting of 44 to 70 can be assayed for (1) the ability to form protein-protein interactions with proteins in a signaling pathway; (2) the ability to bind a ligand of said polypeptide; or (3) the ability to bind to an intracellular binding protein for said polypeptide. In another embodiment, the mutant polypeptide can be assayed for the ability to mediate changes in feeding behavior, body weight, or metabolism.

The present invention encompasses antisense nucleic acid molecules, i.e., molecules which are complementary to a sense nucleic acid encoding a polypeptide selected from the group consisting of Seq ID No.15 to 16 or the group consisting of Seq ID No. 55 to 70, e.g., complementary to the coding strand of a double-stranded cDNA molecule or complementary to an mRNA sequence. Accordingly, an antisense nucleic acid can hydrogen bond to a sense nucleic acid. The antisense nucleic acid can be complementary to an entire coding strand, or to only a portion thereof, e.g., all or part of the protein coding region (or open reading frame). An antisense nucleic acid molecule can be antisense to all or part of a noncoding region of the coding strand of a nucleotide sequence encoding a polypeptide selected from the group consisting of Seq ID No. 15 to 16 or the group consisting of Seq ID No. 55 to 70. The noncoding regions ("5' and 3' untranslated regions") are the 5' and 3' sequences which flank the coding region and are not translated into amino acids.

An antisense oligonucleotide can be, for example, about 5, 10, 15, 20, 25, 30, 35, 40, 45 or 50 nucleotides in length. An antisense nucleic acid of the invention can be constructed using chemical synthesis and enzymatic ligation reactions using procedures known in the art. For example, an antisense nucleic acid (e.g., an antisense oligonucleotide) can be chemically synthesized using naturally occurring nucleotides or variously modified nucleotides designed to increase the biological stability of the molecules or to increase the physical stability of the duplex formed between the antisense and sense nucleic acids, e.g., phosphorothioate derivatives and acridine substituted nucleotides can be used. Examples of modified nucleotides which can be used to generate the antisense nucleic acid include 5-fluorouracil, 5 -bromouracil, 5-chlorouracil, 5 -iodouracil, hypoxanthine xanthine, 4-acetylcytosine, 5-(carboxyhydroxylmethyl) uracil, 5 -carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine 2-methyladenine, 2-methylguanine, 3-methylcytosine, 5-methylcytosine, N6-adenine, 7-methylguanine, 5 -methylaminomethyluracil, 5-methoxyaminomethyl-2-thiouracil, beta-D-mannosylqueosine, 5'-methoxycarboxymethyluracil, 5-methoxyuracil, uracil-5-oxyacetic acid (v), wybutoxosine, pseudouracil, queosine, 2-thiocytosine, 5 -methyl-2-thiouracil, 2-thiouracil, 4-thiouracil, 5-methyluracil, uracil-5-oxyacetic acid methylester, uracil-5-oxyacetic acid (v), 5-methyl-2-thiotiracil, 3-(3-amino-3-N-2-carboxypropyl) tiracil, (acp3) w, and 2,6-diaminopurine. Alternatively, the antisense nucleic acid can be produced biologically using an expression vector into which a nucleic acid has been subcloned in an antisense orientation (i.e., RNA transcribed from the inserted nucleic acid will be of an antisense orientation to a target nucleic acid of interest, described further in the following subsection).

The antisense nucleic acid molecules of the invention are typically administered to a subject or generated in situ such that they hybridize with or bind to cellular mRNA and/or genomic DNA encoding uracil to thereby inhibit expression, e.g., by inhibiting transcription and/or translation. The hybridization can be by conventional nucleotide complementarity to form a stable duplex, or, for example, in the case of an antisense nucleic acid molecule which binds to DNA duplexes, through specific interactions in the major groove of the double helix. An example of a route of administration of antisense nucleic acid molecules of the invention includes direct injection at a tissue site. Alternatively, antisense nucleic acid molecules can be modified to target selected cells and then administered systemically. For example, for systemic administration, antisense molecules can be modified such that they specifically bind to receptors or antigens expressed on a selected cell surface, e.g., by linking the antisense nucleic acid molecules to peptides or antibodies which bind to cell surface receptors or antigens. The antisense nucleic acid molecules can also be delivered to cells using the vectors described herein. To achieve sufficient intracellular concentrations of the anti-sense molecules, vector constructs in which the antisense nucleic acid molecule is placed under the control of a strong pol II or pol III promoter are preferred.

An antisense nucleic acid molecule of the invention can be an α-anomeric nucleic acid molecule. An α-anomeric nucleic acid molecule forms specific double-stranded hybrids with complementary RNA in which, contrary to the usual α-units, the strands run parallel to each other (Gaultier et al. (1987) *Nucleic Acids Res.* 15:6625-6641). The anti-sense nucleic acid molecule can also comprise a 2'-o-methylribonucleotide (Inolle C et al. (1987) *Nucleic Acids Res.* 15:6131-6148) or a chimeric RNA-DNA analogue (Inoue et al. (1987) *FEBS Lett*. 215:327-330).

The invention also encompasses ribozymes. Ribozymes are catalytic RNA molecules with ribonuclease activity which are capable of cleaving a single-stranded nucleic acid, such as an mRNA, to which they have a complementary region. Thus, ribozymes (e.g., hammerhead ribozymes (described in Haselhoff and Gerlach (1988) *Nature* 334:585-591)) can be used to catalytically cleave mRNA transcripts to thereby inhibit translation of the protein encoded by the mRNA. A ribozyme having specificity for a nucleic acid molecule encoding a polypeptide selected from the group consisting of Seq ID No. 13 to 24 can be designed based upon the nucleotide sequence of a cDNA disclosed herein. For example, a derivative of a Tetrahymena L-19 JVS RNA can be constructed in which the nucleotide sequence of the active site is complementary to the nudeotide sequence to be cleaved. Cech et al., U.S. Pat. No.4,987,071; and Cech et al., U.S. Pat. No. 5,116,742. Alternatively, an mRNA encoding any one of the genes listed in tables 1 and 2 can be used to select a catalytic RNA having a specific ribonuclease activity from a pool of RNA molecules. See, e.g., Bartel and Szostak (1993), *Science* 261.1411-1418.

The invention also encompasses nucleic acid molecules which form triple helical structures. For example, expression of a polypeptide selected from the group consisting of Seq ID No. 15 to 16 or the group consisting of Seq ID No. 55 to 70 can be inhibited by targeting nucleotide sequences complementary to the regulatory region of the gene encoding the polypeptide (e.g., the promoter and/or enhancer) to form triple helical structures that prevent transcription of the gene in target cells. See generally Helene (1991) *Anticancer Drug Des. 6(6):569-84;* Helene (1992) *Ann. N. Y Acad. Sci.* 660:27-36; and Maher (1992) *Bioassays* 14(12):807-15.

In certain embodiments, the nucleic acid molecules of the invention can be modified at the base moiety, sugar moiety or phosphate backbone to improve, e.g., the stability, hybridization, or solubility of the molecule. For example, the deoxyribose phosphate backbone of the nucleic acids can be modified to generate peptide nucleic acids (see Hyrup et al. (1996) *Bioorganic & Medicinal Chemistry* 4(1): *5-23).* As used herein, the terms "peptide nucleic acids" or "PNAs" refer to nucleic acid mimics, e.g., DNA mimics, in which the deoxyribose phosphate backbone is replaced by a pseudopeptide backbone and only the four natural nucleobases are retained. The neutral backbone of PNAs has been shown to allow for specific hybridization to DNA and RNA under conditions of low ionic strength. The synthesis of PNA oligomers can be performed using standard solid phase peptide synthesis protocols as described in Hyrup et al. (1996), supra; Perry-O'Keefe et al. (1996) *Proc. Natl*. *Acad. Sci*. *USA* 93: 14670-675. PNAs can be used in therapeutic and diagnostic applications. For example, PNAs can be used as antisense or antigene agents for sequence-specific modulation of gene expression by, e.g., inducing transcription or translation arrest or inhibiting replication. PNAs can also be used, e.g., in the analysis of single base pair mutations in a gene by, e.g., PNA directed PCR clamping; as artificial restriction enzymes when used in combination with other enzymes, e.g., 51 nucleases (Hyrup (1996), supra; or as probes or primers for DNA sequence and hybridization (Hyrup (1996), supra; Perry-O'Keefe et al. (1996) *Proc. Natl*. *Acad. Sci. USA 93: 1467∼675)*.

In another embodiment, PNAs can be modified, e.g., to enhance their stability or cellular uptake, by attaching lipophilic or other helper groups to PNA, by the formation of PNA-DNA chimeras, or by the use of liposomes or other techniques of drug delivery known in the art. For example, PNA-DNA chimeras can be generated which may combine the advantageous properties of PNA and DNA. Such chimeras allow DNA recognition enzymes, e.g., RNAse H and DNA polymerases, to interact with the DNA portion while the PNA portion would provide high binding affinity and specificity. PNA-DNA chimeras can be linked using linkers of appropriate lengths selected in terms of base stacking, number of bonds between the nucleobases, and orientation (Hyrup (1996), supra). The synthesis of PNA-DNA chimeras can be performed as described in Hyrup (1996), supra, and Finn et al. (1996) *Nucleic Acids Res. 24(17):3357-63.* For example, a DNA chain can be synthesized on a solid support using standard phosphoramidite coupling chemistry and modified nucleoside analogs. Compounds such as 5'-(4-methoxytrityl)amino-5'-deoxy-thymidine phosphoramidite can be used as a link between the PNA and the 5' end of DNA (Mag et al. (1989) *Nucleic Acids Res.* 17:5973-88). PNA monomers are then coupled in a stepwise manner to produce a chimeric molecule with a 5' PNA segment and a 3' DNA segment (Finn et al. (1996) *Nucleic Acids Res. 24(17):3357-63).* Alternatively, chimeric molecules can be synthesized with a 5' DNA segment and a 3' PNA segment (Petersen et al. (1975) *Bioorganic Med. Chem*. *Lett*. 5:1119-11124).

In other embodiments, the oligonucleotide may include other appended groups such as peptides (e.g., for targeting host cell receptors in vivo), or agents facilitating transport across the cell membrane (see, e.g., Letsinger et al. (1989) *Proc. Natl*. *Acad. Sci*. *USA 86:6553-6556;* Lemaitre et al. (1987) *Proc. Natl*. *Acad. Sci. USA* 84:648-652; PCT Publication No. WO 88/09810) or the blood-brain barrier (see, e.g., PCT Publication No. WO 89110134). In addition, oligonucleotides can be modified with hybridization-triggered cleavage agents (see, e.g., Krol et al. (1988) *BiolTechniques 6:958-976)* or intercalating agents (see, e.g., Zon (1988) *Pharm. Res.* 5:539-549). To this end, the oligonucleotide may be conjugated to another molecule, e.g., a peptide, hybridization triggered cross-linking agent, transport agent, hybridization-triggered cleavage agent, etc.

The present invention provides a use of a gene or a polypeptide encoded by a gene listed in tables 2 and/or 3 and/or 4 and/or 5 as a target for screening of compounds that reduce and/or prevent obesity.

The present invention also provides a use of a nucleic acid encoding a polypeptide selected from the group consisting of Seq ID No. 9-16, or of mutants or fragments thereof as a target for screening of compounds that reduce and/or prevent obesity.

Furthermore, the use of a gene or a polypeptide encoded by a gene selected from the group consisting of Seq ID No. 17 to 70 as a target for screening of compounds that reduce and/or prevent obesity is provided.

In addition, the use of a nucleic acid encoding a polypeptide selected from the group consisting of Seq ID No 44 to 70, or of mutants or fragments thereof as a target for screening of compounds that reduce and/or prevent obesity is provided.

### V. Isolated Proteins and Antibodies

One aspect of the invention pertains to isolated proteins, and biologically active portions thereof, as well as polypeptide fragments suitable for use as immunogen to raise antibodies directed against a polypeptide selected from the group consisting of Seq ID No. 9 to 16, or the group selected from Seq ID No. 44 to 70. In one embodiment, native polypeptide can be isolated from cells or tissue sources by an appropriate purification scheme using standard protein purification techniques. In another embodiment, polypeptides of the invention are produced by recombinant DNA techniques. Alternative to recombinant expression, polypeptides can be synthesized chemically using standard peptide synthesis techniques. This section describes polypeptides of any one of Seq ID No. 9 to 16, or Seq ID No. 44 to 70, antibodies directed against said polypeptides, and methods for making and using such polypeptides and antibodies.

An "isolated" or "purified" protein or biologically active portion thereof is substantially free of cellular material or other contaminating proteins from the cell or tissue source from which the protein is derived, or substantially free of chemical precursors or other chemicals when chemically synthesized. The language "substantially free of cellular material" includes preparations of protein in which the protein is separated from cellular components of the cells from which it is isolated or recombinantly produced. Thus, protein that is substantially free of cellular material includes preparations of protein having less than about 30%, 20%, 10%, or 5% (by dry weight) of heterologous protein (also referred to herein as a "contaminating protein"). When the protein or biologically active portion thereof is recombinantly produced, it is also preferably substantially free of culture medium, i.e., culture medium represents less than about 20%, 10%, or 5% of the volume of the protein preparation. When the protein is produced by chemical synthesis, it is preferably substantially free of chemical precursors or other chemicals, i.e., it is separated from chemical precursors or other chemicals which are involved in the synthesis of the protein. Accordingly such preparations of the protein have less than about 30%, 20%, 10%, 5% (by dry weight) of chemical precursors or unrelated chemicals.

Biologically active portions of a polypeptide selected from the group consisting of Seq ID No. 9 to 16, or the group consisting of Seq ID No. 44 to 70 include polypeptides comprising amino acid sequences sufficiently identical to or derived from the amino acid sequence of the protein which include fewer amino acids than the full length protein, and exhibit at least one activity of the corresponding full-length protein. Typically, biologically active portions comprise a domain or motif with at least one activity of the corresponding portion. A biologically active portion of the invention can be a polypeptide which is, for example, 10, 25, 50, 100 or more amino acids in length. Moreover, other biologically active portions, in which other regions of the protein are deleted, can be prepared by recombinant techniques and evaluated for one or more of the functional activities of the native form of said polypeptide.

Among the useful polypeptides are those having the amino acid sequence of a polypeptide selected from the group consisting of Seq. ID No. 9 to 16, or the group consisting of Seq ID No. 44 to 70. Other useful proteins are substantially identical (e.g., at least about 96%, 97%, 98%, 99%, or 99.5%) to any of said polypeptides and retain the functional activity of the protein of the corresponding naturally-occurring protein yet differ in amino acid sequence due to natural allelic variation or mutagenesis. To determine the percent identity of two amino acid sequences or of two nucleic acid sequences, the sequences are aligned for optimal comparison purposes (e.g., gaps can be introduced in the sequence of a first amino acid or nucleic acid sequence for optimal alignment with a second amino or nucleic acid sequence). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences (i.e., % identity=# of identical positions/total # of positions (e.g., overlapping positions)x100). Preferably, the two sequences are the same length.

The invention also provides chimeric or fusion proteins. As used herein, a "chimeric protein" or "fusion protein" comprises all or part (e.g., biologically active fragment) of a polypeptide selected from the group consisting of one Seq ID No. 9 to 16, or the group consisting of Seq ID No. 44 to 70 operably linked to a heterologous polypeptide (i.e., a polypeptide other than the same polypeptide of the invention). Within the fusion protein, the term "operably linked" is intended to indicate that the polypeptide of the invention and the heterologous polypeptide are fused in-frame to each other. The heterologous polypeptide can be fused to the N-terminus or C-terminus of said polypeptide.

One useful fusion protein is a GST fusion protein in which all or a portion of a polypeptide selected from the group consisting of Seq ID No. 9 to 16, or the group consisting of Seq ID No. 44 to 70 is fused to the C-terminus of GST sequences. Such fusion proteins can facilitate the purification of a recombinant polypeptide. Other useful fusion proteins include fusions to FLAGTM, a portion lacZ, GST, calmodulin-binding peptide, His⁶, or HA. Vectors for preparing such fusions proteins are available from Clontech, Inc. (Palo Alto, Calif.) and Stratagene, Inc. (La Jolla, Calif.).

In another embodiment, the fusion protein contains a heterologous signal sequence at its N-terminus. For example, the native signal sequence of any one of the polypeptides of Seq ID No. 9 to 16, or 44 to 70 can be removed and replaced with a signal sequence from another protein. For example, the gp67 secretory sequence of the baculovirus envelope protein can be used as a heterologous signal sequence *(Current Protocol in Molecular Biology,* Ausubel et al., eds., John Wiley & Sons, 1992). Other examples of eukaryotic heterologous signal sequences include the secretory sequences of melittin and human placental alkaline phosphatase (Stratagene; La Jolla, Calif.). In yet another example, useful prokaryotic heterologous signal sequences include the phoA secretory signal (Sambrook Ct al., supra) and the protein A secretory signal (Pharmacia Biotech; Piscataway, N.J.).

In yet another embodiment, the fusion protein is an immunoglobulin fusion protein in which all or part of the sequence of a polypeptide of Seq ID No. 9 to 16, or 44 to 70 is fused to sequences derived from a member of the immunoglobulin protein family. The immunoglobulin fusion proteins of the invention can be incorporated into pharmaceutical compositions and administered to a subject to inhibit an interaction between a ligand (soluble or membrane-bound) and a protein on the surface of a cell (receptor), to thereby suppress signal transduction in vivo. The immunoglobulin fusion protein can be used to affect the bioavailability of a cognate ligand of a polypeptide of Seq ID No. 9 to 16, or 44 to 70. Inhibition of ligand/receptor interaction may be useful therapeutically for modulating feeding behavior, body weight, and/or metabolic rate. Moreover, the immunoglobulin fusion proteins of the invention can be used as immunogen to produce antibodies directed against a polypeptide hereinbefore described in a subject, to purify ligands and in screening assays to identify molecules which inhibit the interaction of receptors with ligands.

Chimeric and fusion protein of the invention can be produced by standard recombinant DNA techniques. In another embodiment, the fusion gene can be synthesized by conventional techniques including automated DNA synthesizers. Alternatively, PCR amplification of gene fragments can be carried out using anchor primers which give rise to complementary overhangs between two consecutive gene fragments which can subsequently be annealed and reamplified to generate a chimeric gene sequence (see, e.g., Ausubel et al., supra). Moreover, many expression vectors are commercially available that already encode a fusion moiety (e.g., a GSJ polypeptide). A nucleic acid encoding any one of the polypeptides of Seq ID No. 9 to 16, or 44 to 70 can be cloned into such an expression vector such that the fusion moiety is linked in-frame to the polypeptide of the invention.

The present invention also pertains to variants of any one of the polypeptides of Seq ID No. 9 to 16, or 44 to 70. Such variants have an altered amino acid sequence which can function as either agonists (mimetics) or as antagonist. Variants can be generated by mutagenesis, e.g., discrete point mutation or truncation. An agonist can retain substantially the same, or a subset, of the biological activities of the naturally occurring form of the protein. An antagonist of a protein can inhibit one or more of the activities of the naturally occurring form of the protein by, for example, competitively binding to a downstream or upstream member of a cellular signaling cascade which includes the protein of interest. Thus, specific biological effects can be elicited by treatment with a variant of limited function. Treatment of a subject with a variant having a subset of the biological activities of the naturally occurring form of the protein can have fewer side effects in a subject relative to treatment with the naturally occurring form of the protein.

Variants of a protein of the invention which function as either agonists (mimetics) or as antagonists can be identified by screening combinatorial libraries of mutants, e.g., truncation mutants, of the protein of the invention for agonist or antagonist activity. In one embodiment, a variegated library of variants is generated by combinatorial mutagenesis at the nucleic acid level and is encoded by a variegated gene library. A variegated library of variants can be produced by, for example, enzymatically ligating a mixture of synthetic oligonucleotides into gene sequences such that a degenerate set of potential protein sequences is expressible as individual polypeptides, or alternatively, as a set of larger fusion proteins (e.g., for phage display). There are a variety of methods which can be used to produce libraries of potential variants of the polyepeptides of the invention from a degenerate oligonucleotide sequence. Methods for synthesizing degenerate oligonucleotides are known in the art (see, e.g., Narang (1983) *Tetrahedron 39:3;* Itakura et al. (1984) Annu. *Rev. Biochem*. *53:323;* Itakura et al. (1984) *Science* 198:1056; Ike et al. (1983) *NucleicAcid Res. 11:477).*

In addition, libraries of fragments of a polypeptide selected from the group consisting of Seq ID No. 9 to 16, or the group consisting of 44 to 70 can be used to generate a variegated population of polypeptides for screening and subsequent selection of variants. For example, a library of coding sequence fragments can be generated by treating a double stranded PCR fragment of the coding sequence of interest with a nuclease under conditions wherein nicking occurs only about once per molecule, denaturing the double stranded DNA, renaturing the DNA to form double stranded DNA which can include sense/antisense pairs from different nicked products, removing single stranded portions from reformed duplexes by treatment with SI nuclease, and ligating the resulting fragment library into an expression vector. By this method, an expression library can be derived which encodes N-terminal and internal fragments of various sizes of the protein of interest.

Several techniques are known in the art for screening gene products of combinatorial libraries made by point mutations or truncation, and for screening cDNA libraries for gene products having a selected property. The most widely used techniques, which are amenable to high through-put analysis, for screening large gene libraries typically include cloning the gene library into replicable expression vectors, transforming appropriate cells with the resulting library of vectors, and expressing the combinatorial genes under conditions in which detection of a desired activity facilitates isolation of the vector encoding the gene whose product was detected.

Recursive ensemble mutagenesis (REM), a technique which enhances the frequency of functional mutants in the libraries, can be used in combination with the screening assays to identify variants of a protein of the invention (Arkin and YollrvaIl (1992) *Proc. Natl Acad USA* 89:7811-7815; Delgrave et al. (1993) *Protein Engineering* 6(3):327-331).

The present invention provides for a kit for screening for compounds that reduce and/or prevent obesity comprising a polypeptide selected from the group consisting of Seq ID No. 9 to 16.

Additionally it provides a kit for screening for compounds that reduce and/or prevent obesity comprising a polypeptide selected from the group consisting of Seq ID No. 44 to 70.

### VI. Recombinant Expression Vectors and Host Cells

Another aspect of the invention pertains to vectors (e.g., expression vectors) containing a nucleic acid encoding a polypeptide selected from the group consisting of Seq ID No. 9 to 16, or the group consisting of 44 to 70 (or a portion thereof). As used herein, the "vector" refers to a nucleic acid molecule capable of transporting another nucleic acid to which it has been linked. One type of vector is a "plasmid," which refers to a circular double stranded DNA loop into which additional DNA segments can be ligated. Another type of vector is a viral vector, wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) are integrated into the genome of a host cell upon introduction into the host cell, and thereby are replicated along with the host genome. Moreover, certain vectors, expression vectors, are capable of directing the expression of genes to which they are operably linked. In general, expression vectors of utility in recombinant DNA techniques are often in the form of plasmids (vectors). However, the invention is intended to include such other forms of expression vectors, such as viral vectors (e.g., replication defective retroviruses, adenoviruses and adeno-associated viruses), which serve equivalent functions. This section describes vectors and host cells harboring nucleic acids selected from the group consisting of Seq ID No. 1 to 8 or the group consisting of Seq ID No. 17 to 43 and variants thereof and methods for their production and use.

The recombinant expression vectors of the invention comprise a nucleic acid of the invention in a form suitable for expression of the nucleic acid in a host cell. This means that the recombinant expression vectors include one or more regulatory sequences, selected on the basis of the host cells to be used for expression, which is operably linked to the nucleic acid sequence to be expressed. Within a recombinant expression vector, "operably linked" is intended to mean that the nucleotide sequence of interest is linked to the regulatory sequence(s) in a manner which allows for expression of the nucleotide sequence (e.g., in an in vitro transcription/translation system or in a host cell when the vector is introduced into the host cell). The term "regulatory sequence" is intended to include promoters, enhancers and other expression control elements (e.g., polyadenylation signals). Such regulatory sequences are described, for example, in Goeddel, *Gene Expression Technology: Methods in Enzymology* 185, Academic Press, San Diego, Calif. (1990). Regulatory sequences include those which direct constitutive expression of a nucleotide sequence in many types of host cell and those which direct expression of the nucleotide sequence only in certain host cells (e.g., tissue-specific regulatory sequences). It will be appreciated by those skilled in the art that the design of the expression vector can depend on such factors as the choice of the host cell to be transformed, the level of expression of protein desired, etc. The expression vectors of the invention can be introduced into host cells to thereby produce proteins or peptides, including fusion proteins or peptides, encoded by nucleic acids as described herein.

The recombinant expression vectors of the present invention can be designed for expression of a gene listed in tables 2 or 3 or 4 or 5 in prokaryotic or eukaryotic cells, e.g., bacterial cells such as *E. coli,* insect cells (using baculovirus expression vectors), yeast cells or mammalian cells. Suitable host cells are discussed further in Goeddel, supra. Alternatively, the recombinant expression vector can be transcribed and translated in vitro, for example using T7 promoter regulatory sequences and T7 polymerase.
Expression of proteins in prokaryotes is most often carried out in *E*. *coli* with vectors containing constitutive or inducible promoters directing the expression of either fusion or non-fusion proteins. Fusion vectors add a number of amino acids to a protein encoded therein, usually to the amino terminus of the recombinant protein. Such fusion vectors typically serve three purposes: 1) to increase expression of recombinant protein; 2) to increase the solubility of the recombinant protein; and 3) to aid in the purification of the recombinant protein by acting as a ligand in affinity purification. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase. Typical fusion expression vectors include PGEX (Pharmacia Biotech Inc; Smith and Johnson (1988) *Gene* 67:31-40), pMAL (New England Biolabs, Beverly, Mass.) and pRIT5 (Pharmacia Piscataway, N.J.) which fuse glutathione 5-transferase (GST), maltose E binding protein, or protein A, respectively, to the target recombinant protein.

Examples of suitable inducible non-fusion *E. coli* expression vectors include pTrc (Amann et al., (1988) *Gene* 69:301-315) and pET lid (Studier et al., *Gene Expression Technology: Methods in Enzymology* 185, Academic Press San Diego, Calif. (1990) 60∼9). Target gene expression from the pTrc vector relies on host RNA polymerase transcription from a hybrid trp-lac fusion promoter. Target gene expression from the pET lid vector relies on transcription from a T7 gnl0-lac fusion promoter mediated by a coexpressed viral RNA polymerase (T7 gn1). The viral polymerase is supplied by host strains BL21(DE3) or HM5174 (DE3) from a resident prophage harboring a T7 gn1 gene under the transcriptional control of the lacUV 5 promoter.

One strategy to maximize recombinant protein expression in *E. coli* is to express the protein in a host bacteria with an impaired capacity to proteolytically cleave the recombinant protein (Gottesman, *Gene Expression Technology: Methods in Enzymology* 185, Academic Press, San Diego, Calif. (1990) 119-128). Another strategy is to alter the nucleic acid sequence of the nucleic acid to be inserted into an expression vector so that the individual codons for each amino acid are those preferentially utilized in *E*. *coli* (Wada et al. (1992) *Nucleic Acids Res.* 20:2111-2118). Such alteration of nucleic acid sequences of the invention can be carried out by standard DNA synthesis techniques.

In another embodiment, the expression vector is a yeast expression vector. Examples of vectors for expression in yeast S. *cerivisae* include pYepSecl (Baldari et al. (1987) m*EMBO J*. 6:229-234), pMFa (Kurjan and Herskowitz, (1982) *Cell* 30:933∼943), pJRY88 (Schultz et al. (1987) *Gene 54:113-123)*, pYES2 (Invitrogen Corporation, SanDiego, Calif.), and pPicZ (InVitrogen Corp, San Diego, 15 Calif.).

Alternatively, the expression vector is a baculovirus expression vector. Baculovirus vectors available for expression of proteins in cultured insect cells (e.g., Sf 9 cells) include the pAc series (Smith et al. (1983) *Mol. Cell Biol*.20 *3:2156-2165)* and the pVL series (Lucklow and Summers (1989) *virology* 170:31-39).

In yet another embodiment, a nucleic acid of the invention is expressed in mammalian cells using a mammalian expression vector. Examples of mammalian expression vectors include pCDM8 (Seed (1987) *Nature* 329:840) and pMT2PC (Kaufman et al. (1987) *EMBO J*. *6:187-195)*. When used in mammalian cells, the expression vector's control functions are often provided by viral regulatory elements. For example, commonly used promoters are derived from polyoma, Adenovirus 2, cytomegalovirus and Simian Virus 40. For other suitable expression systems for both prokaryotic and eukaryotic cells see chapters 16 and 17 of Sambrook et al., supra.

In another embodiment, the recombinant mammalian expression vector is capable of directing expression of the nucleic acid preferentially in a particular cell type (e.g., tissue-specific regulatory elements are used to express the nucleic acid). Tissue-specific regulatory elements are known in the art. Non-limiting examples of suitable tissue-specific promoters include the albumin promoter (liver-specific; Pinkert et al. (1987) *Genes Dev.* 1:268-277), lymphoid-specific promoters (Calame and Eaton *(1988)Adv Immunol. 43:235-275),* in particular promoters of T cell receptors (Winoto and Baltimore (1989) *EMBO J*. 8:729-733) and immunoglobulins (Banerji et al. (1983) *Cell* 33:729-740; Queen and Baltimore (1983) *Cell* 33:741-748), neuron-specific promoters (e.g., the neurofilament promoter; Byrne and Ruddle (1989) *Proc. Natl*. *Acad. Sci*. *USA 86:5473-5477)*, pancreas-specific promoters (Edlund et al. *(1985) Science* 230:912-916), and mammary gland-specific promoters (e.g., milk whey promoter; U.S. Pat. No.4,873, 316 and European Application Publication No.264,166). Developmentally-regulated promoters are also encompassed, for example the murine hox promoters (Kessel and Gruss (1990) *Science* 249:374-379) and the CL-fetoprotein promoter (Campes and Tilghman (1989) *Genes Dev. 3:537-546).*

The invention further provides a recombinant expression vector comprising a DNA molecule of the invention cloned into the expression vector in an antisense orientation. That is, the DNA molecule is operably linked to a regulatory sequence in a manner which allows for expression (by transcription of the DNA molecule) of an RNA molecule which is antisense to the mRNA encoding a polypeptide selected from the group consisting of Seq ID No. 9 to 16, or 44 to 70. Regulatory sequences operably linked to a nucleic acid cloned in the antisense orientation can be chosen which direct the continuous expression of the antisense RNA molecule in a variety of cell types, for instance viral promoters and/or enhancers, or regulatory sequences can be chosen which direct constitutive, tissue specific or cell type specific expression of antisense RNA. The antisense expression vector can be in the form of a recombinant plasmid, phagemid or attenuated virus in which antisense nucleic acids are produced under the control of a high efficiency regulatory region, the activitvity of which can be determined by the cell type into which the vector is introduced. For a discussion of the regulation of gene expression using anti-sense genes see Weintraub et al. (*Reviews-Trends in Genetics*, Vol.1(1) 1986).

Another aspect of the invention pertains to host cells into which a recombinant expression vector of the invention has been introduced. It is understood that such a term refers not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein.

A host cell can be any prokaryotic or eukaryotic cell (e.g., *E*. *coli*, insect cells, yeast or mammalian cells). Vector DNA can be introduced into prokaryotic or eukaryotic cells via conventional transformation or transfection techniques. As used herein, the terms "transformation" and "transfection" are intended to refer to a variety of art-recognized techniques for introducing foreign nucleic acid into a host cell, including calcium phosphate or calcium chloride co-precipitation, DEAE-dextran-mediated transfection, lipofection, or electroporation. Suitable methods for transforming or transfecting host cells can be found in Sambrook, et al. (supra), and other laboratory manuals.

For stable transfection of mammalian cells, it is known that, depending upon the expression vector and transfection technique used, only a small fraction of cells may integrate the foreign DNA into their genome. In order to identify and select these integrants, a gene that encodes a selectable marker (e.g., for resistance to antibiotics) is generally introduced into the host cells along with the gene of interest. Useful selectable markers include those which confer resistance to drugs, such as G418, hygromycin and methotrexate. Cells stably transfected with the introduced nucleic acid can be identified by drug selection (e.g., cells that have incorporated the selectable marker gene will survive, while the other cells die).

A host cell of the invention, such as a prokaryotic or eukaryotic host cell in culture, can be used to produce a polypeptide selected from the group consisting of Seq ID No. 9 to 16, or 44 to 70. Accordingly, the invention further provides methods for producing a polypeptide selected from the group consisting of Seq ID No. 9 to 16, orthe group consisting of Seq ID No. 44 to 70 using the host cells of the invention. In one embodiment, the method comprises culturing the host cell of invention (into which a recombinant expression vector encoding a polypeptide selected from the group consisting of Seq ID No. 9 to 16, or the group consisting of Seq ID No. 44 to 70 has been introduced) in a suitable medium such that the polypeptide is produced. In another embodiment, the method further comprises isolating the polypeptide from the medium or the host cell.

### VII. Methods of Treatment

The present invention provides for both prophylactic and therapeutic methods for modulating body weight, e.g., by altering feeding behavior or metabolic rate.

In one aspect, the present invention provides a method for modulating body weight by administering an agent which modulates an activity of any one of the polypeptides of Seq ID No. 9 to 16, or 44 to 70. Such methods are useful for modulating body weight both in patients having aberrant expression or activity of said polypeptide or other patients which would benefit from administration of an agent which modulates activity of said polypeptide. Depending on the needs of the patient a polypeptide agonist or antagonist can be used for treating the subject.

Agonists of the activity of any one of the polypeptides of Seq ID No. 9-14, or Seq ID No. 44 to 54, or compounds which increase expression of said polypeptide are useful for treatment of high body weight, e.g., obesity, because they can be used to reduce body weight. Similarly, compounds which increase the activity or expression of a protein in the signalling pathway of said polypeptide are useful for treatment of high body weight. Conversely, antagonists of the activity of said polypeptide or compounds which reduce the expression of said polypeptide are useful for treatment of low body weight, e.g., cachexia, because they can be used to increase body weight. Compounds which reduce the activity or expression of a protein in the signalling pathway of a polypeptide of any one of Seq ID No. 9 to 14 or Seq ID No. 44 to 54 are useful for treatment of low body weight.

Antagonists of the activity of any one of the polypeptides of Seq ID No. 15 or 16, or Seq ID No. 55 to 70 or compounds which decrease expression of said polypeptide are useful for treatment of high body weight, e.g., obesity, because they can be used to reduce body weight. Similarly, compounds which decrease the activity or expression of a protein in the signalling pathway of said polypeptide are useful for treatment of high body weight. Conversely, agonists of the activity of said polypeptide or compounds which increase the expression of said polypeptide are useful for treatment of low body weight, e.g., cachexia, because they can be used to increase body weight. Compounds which reduce the activity or expression of a protein in the signalling pathway of a polypeptide of Seq ID No. 15 or 16 or Seq ID No. 55 to 70 are useful for treatment of low body weight.

The present invention also provides a use of an agonist, or a compound which increases the expression of a polypeptide selected from the group consisting of Seq ID No. 9 to 16, or the group consisting of Seq ID No. 44 to 70 for the preparation of a medicament for the treatment of obesity. Futhermore, the present invention provides a use of an antagonist, or a compound that decreases the expression of a polypeptide selected from the group consisting of Seq ID No. 9 to 16, or the group consisting of Seq ID No. 44 to 70 for the preparation of a medicament for the treatment of obesity.

### VIII. Pharmaceutical Compositions

The present invention further pertains to novel agents identified by the above-described screening assays and uses thereof for treatments as described herein. The nucleic acid molecules, polypeptides, and antibodies (also referred to herein as "active compounds") of the invention can be incorporated into pharmaceutical compositions suitable for administration. Such compositions typically comprise the nucleic acid molecule, protein, or anti-body and a pharmaceutically acceptable carrier. As used herein the language "pharmaceutically acceptable carrier" is intended to include any and all solvents, dispersion media, coatings, antibacterial and antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary active compounds can also be incorporated into the compositions.

The invention includes pharmaceutical compositions comprising a modulator of expression or activity of any one of the polypeptides of Seq ID No. 9 to 16, or Seq ID No. 44 to 70 (and/or a modulator of the activity or expression of a protein in the signalling pathway of said polypeptide) as a well as methods for preparing such compositions by combining one or more such modulators and a pharmaceutically acceptable carrier. Also within the scope of the present invention are pharmaceutical compositions comprising a modulator identified using the screening assays of the invention packaged with instructions for use. For modulators that are antagonists of the activity of any one of the polypeptides of Seq ID No. 9 to 16, or 44 to 70 or which reduce the expression of said polypeptide, the instructions would specify use of the pharmaceutical composition for treatment of low body weight (e.g., increase of body weight). For modulators that are agonists of the activity of said polypeptide or increase the expression of said polypeptide, the instructions would specify use of the pharmaceutical composition for treatment of high body weight (i.e., reduction of body weight).

The present invention also provides a pharmaceutical formulation for the modulation of body weight, comprising a compound that modulates the activity of a polypeptide selected from the group consisting of Seq ID No. 44 to 70, mixed with a pharmaceutically acceptable carrier.

A pharmaceutical composition of the invention is formulated to be compatible with its intended route of administration. Examples of routes of administration include parenteral, e.g., intravenous, intradermal, subcutaneous, oral (e. g., inhalation), transdermal (topical), transmucosal, and rectal administration. Solutions or suspensions used for parenteral, intradermal, or subcutaneous application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

Pharmaceutical compositions suitable for injectable use include sterile aqueous solutions (where water soluble) or dispersions and sterile powders for the extemporaneous preparation of sterile injectable solutions or dispersions are also provided. For intravenous administration, suitable carriers include physiological saline, bacteriostatic water, Cremophor ELTM (BASF; Parsippany, N.J.) or phosphate buffered saline (PBS). In all cases, the composition must be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyetheylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, for example, sugars, polyalcohols such as mannitol, sorbitol, sodium chloride in the composition. Prolonged absorption of the injectable compositions can be brought about by including in the composition an agent which delays absorption, for example, aluminum monostearate and gelatin.

Sterile injectable solutions can be prepared by incorporating the active compound (e.g., a polypeptide or antibody) in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

Oral compositions generally include an inert diluent or an edible carrier. They can be enclosed in gelatin capsules or compressed into tablets. For the purpose of oral therapeutic administration, the active compound can be incorporated with excipients and used in the form of tablets, troches, or capsules. Oral compositions can also be prepared using a fluid carrier for use as a mouthwash, wherein the compound in the fluid carrier is applied orally and swished and expectorated or swallowed. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

For administration by inhalation, the compounds are delivered in the form of an aerosol spray from a pressurized container or dispenser which contains a suitable propellant, e.g., a gas such as carbon dioxide, or a nebulizer.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art.

The compounds can also be prepared in the form of suppositories (e.g., with conventional suppository bases such as cocoa butter and other glycerides) or retention enemas for rectal delivery.

In one embodiment, the active compounds are prepared with carriers that will protect the compound against rapid elimination from the body, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art. The materials can also be obtained commercially from Alza Corporation and Nova Pharmaceuticals, Inc. Liposomal suspensions (including liposomes targeted to infected cells with monoclonal antibodies to viral antigens) can also be used as pharmaceutically acceptable carriers. These can be prepared according to methods known to those skilled in the art, for example, as described in U.S. Pat. No.4,522,811.

It is especially advantageous to formulate oral or parenteral compositions in dosage unit form for ease of administration and uniformity of dosage. Dosage unit form as used herein refers to physically discrete units suited as unitary dosages for the subject to be treated; each unit containing a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to t)e achieved, and the limitations inherent in the art of compounding such an active compound for the treatment of individuals.

The nucleic acid molecules of the invention can be inserted into vectors and used as gene therapy vectors. Gene therapy vectors can be delivered to a subject by, for example, intravenous injection, local administration (U.S. Pat. No. 5,328,470) or by stereotactic injection (see, e.g., Chen et al. (1994) *Proc. Natl Acad. Sci. USA 91:3054-3057).* The pharmaceutical preparation of the gene therapy vector can include the gene therapy vector in an acceptable diluent, or can comprise a slow release matrix in which the gene delivery vehicle is imbedded. Alternatively, where the complete gene delivery vector can be produced intact from recombinant cells, e.g. retroviral vectors, the pharmaceutical preparation can include one or more cells which produce the gene delivery system.

The pharmaceutical compositions can be included in a container, pack, or dispenser together with instructions for administration.

The present invention provides a pharmaceutical formulation for the modulation of body weight, comprising a compound that modulates the activity of a polypeptide selected from the group consisting of Seq ID No. 9 to 16, or the group consisting of Seq ID No. 44 to 70, mixed with a pharmaceutically acceptable carrier.

The present invention also refers to a package comprising the pharmaceutical formulation hereinbefore described and instructions for administering the pharmaceutical formulation for the purpose of modulating body weight.

The present invention pertains to a method for preparing a pharmaceutical composition useful for modulating body weight, the method comprising: a) contacting a test compound with a polypeptide selected from the group consisting of Seq ID No. 9 to 16, or the group consisting of Seq ID No. 44 to 70; b) determining whether the test compound binds to the polypeptide selected from the group consisting of Seq ID No. 9 to 16 or the group consisting of Seq ID No. 44 to 70; and c) combining the test compound that binds to the polypeptide selected from the group consisting of Seq ID No. 9 to 16 or the group consisting of Seq ID No. 44 to 70 with a pharmaceutically acceptable carrier to create a pharmaceutical composition useful for modulating body weight.

The present invention provides a method for preparing a pharmaceutical composition useful for modulating body weight, the method comprising: a) contacting a ligand of a polypeptide selected from the group consisting of Seq ID No. 9 to 16 or the group consisting of Seq ID No. 44 to 70 with a polypeptide selected from the group consisting of Seq ID No. 9 to 16 or the group consisting of Seq ID No. 44 to 70 in the presence and absence of a test compound; b) determining whether the test compound alters the binding of the ligand of the polypeptide selected from the group consisting of Seq ID No. 9 to 16 or the group consisting of Seq ID No. 44 to 70 to the polypeptide selected from the group consisting of Seq ID No. 9 to 16 or the group consisting of Seq ID No. 44 to 70; and c) combining the test compound that alters the binding of said ligand to said polypeptide with a pharmaceutically acceptable carrier to create a pharmaceutical composition useful for modulating body weight.

The present invention also provides a use of a gene selected from the group consisting of Seq ID No. 1 to 8 or the group consisting of Seq ID No. 17 to 43, or of a polypeptide selected from the group consisting of Seq ID No 9 to 16 or the group consisting of Seq ID No. 44 to 70 as a target for screening of compounds that reduce and/or prevent obesity,

Further to this, the present invention also pertains to a use of a nucleic acid encoding a polypeptide selected from the group consisting of Seq ID No. 9 to 16 or the group consisting of Seq ID No. 44 to 70, or of mutants or fragments thereof, as a target for screening of compounds that reduce and/or prevent obesity.

### Examples:

### Example 1. RNA preparation

Total RNA from 300 mg skeletal muscle was isolated using the TriZol reagent (Life Technologies) and the Fast RNA green (BIO101) kit according to the manufacturer's protocols. Total DNA was purified from contaminating DNA the RNeasy kit (Qiagen).

### Example 2. Gene expression measurement by DNA chips

Synthesis of first and second strand cDNA were performed using the SuperScript Choice Gene Chip Kit (Life Technologies) and reagents from Gibco. The double stranded cDNA, containing an incorporated T7 RNA polymerase binding site, was purified by extraction with a mix of phenol:chloroform:isoamylalcohol (Life Technologies). The organic and aqueous phases were separated by Phase Lock Gel (Eppendorf) and double-stranded cDNA was recovered by precipitation according to the manufacturer's protocol and then resuspended in water.

The double-stranded cDNA was converted to biotin-labeled cRNA by *in vitro* transcription (IVT) using a T7 kit (Ambion) and biotin-containing ribonucleotides (Enzo - LOXO GmbH). The IVT-material was purified from unincorporated ribonucleotides using RNeasy spin columns (Qiagen). Following cleanup, the single-stranded biotin-labeled cRNA were chemically hydrolyzed to smaller fragments in 500 mM calcium acetate, 150 mM magnesium acetate, pH 8.1 for 35 min at 95°C. The reaction was terminated by chilling samples on ice.

Probes were hybridized to the U133 A GeneChip Microarray (Affymetrix) which contains features representing ∼22,000 genes. All washing, hybridization, detection, and signal amplification steps were performed using a GeneChip Fluidics Station (Affymetrix). Fluorescence intensity data was collected from the hybridized GeneArrays using a GeneArray scanner (Affymetrix). The raw files containing the fluorescence intensity information were transformed into data files using the Affymetrix Microarray Suite (MAS) software. Differentially expressed genes were identified using the Roche Affymetrix Chip Experiment Analysis (RACE-A) software. Differences between control patients (n=10) vs. obese case patients (n=10) were evaluated by several statistical filters as change factor vs. control.

**Table 2:**

| Genes down-regulated in skeletal muscle in obesity | | | | | | |
|---|---|---|---|---|---|---|
| Seq ID No. DNA (protein) | Description | CHCF | P value | Lean mean | Obese mean | Unigene No Accession No |
| 1 (9) | Phospholamban (PLN) | -1.4 | 0.002 | 387.24 | 178.73 | Hs.85050 NM_702185 |
| 2 (10) | Sterol carrier protein 2 (SCP2) | -1.02 | 0.0067 | 71.59 | 43.16 | Hs.75760 NM_002979 |
| 3(11) | Udp-gal:betaglcnac beta 1,4- galactosyltransferase | -1.03 | 0.0074 | 126.8 | 85.43 | Hs.107526 NM_004776 |
| 4 (12) | Lipoprotein lipase (LPL) | -0.6 | 0.0067 | 154.76 | 119.44 | Hs.180878 NM_000237 |
| 5 (13) | Low density lipoprotein-related protein 1b | -0.71 | 0.0416 | 21.95 | 12.84 | Hs.47005 NM_018557 |
| 6 (14) | Hypothetical protein mgc10940 | -1.11 | 0.0082 | 82.21 | 45.46 | Hs.47986 AL833735 |

**Table 3**

| Genes up-regulated in skeletal muscle in obesity | | | | | | |
|---|---|---|---|---|---|---|
| Seq ID No. DNA (protein) | Description | CHCF | P value | Lean mean | Obese mean | Unigene No Accession No |
| 7 ( 15) | Fatty acid desaturase 2 (FADS2) | 0.58 | 0.0004 | 29.97 | 40.69 | Hs.184641 AL520270 |
| 8 (16) | Rar-related orphan receptor a (RORA) | 0.45 | 0.0173 | 45.08 | 70.28 | Hs.2156 BC008831 |

**Table 4**

| Genes down-regulated in skeletal muscle in obesity | | | | | |
|---|---|---|---|---|---|
| Seq ID No. DNA (protein) | Description | CHCF | P value | LocusID (GeneID) | Accession No. |
| 17 (44) | Fatty-acid-coenzyme a ligase, long chain 3 (ACSL3) | -0.34 | 0.02067 | 2181 | NM 004457 (variant1) |
| 18 (45) | | | | | NM 203372 (variant2) |
| 19 (46) | Solute carrier family 25 (mitochondrial carrier; adenine nucleotide translocator), member 4 (SLC25A4) | -0.33 | 0.00528 | 291 | NM 001151 |
| 20 (47) | Tek tyrosine kinase, endothelial (TEK) | -0.31 | 0.17824 | 7010 | NM 000459 |
| 21 (48) | Atp synthase, h+ transporting, mitochondrial f0 complex, subunit g (ATP5L) | -0.68 | 0.00009 | 10632 | NM 006476 |
| 22 (49) 23(50) 24 (51) | Atp synthase, h+ transporting, mitochondrial f0 complex, subunit b, isoform 1 | -0.35 | 0.00042 | 515 | NM 001002014 Isoform 2 NM 001002015 Isoform 3 NM 001688 Isoform 1 |
| 25 (52) | Thyroid hormone receptor interactor 3 | -0.21 | 0.00963 | 9326 | NM 004773 |
| 26 (53) | Lactate dehydrogenase b | -1.15 | 0.00912 | 3945 | NM 02300 |
| 27 (54) | Fatty acid binding protein 3, muscle and heart | -0.46 | 0.03249 | 2170 | NM 004102 |

**Table 5**

| Genes up-regulated in skeletal muscle in obesity | | | | | |
|---|---|---|---|---|---|
| Seq ID No. DNA (protein) | Description | CHCF | P value | LocusID (GeneID) | Accession No. |
| 28 (55) | glycogenin | 0.29 | 0.02090 | 2992 | NM 004130 |
| 29 (56) | Fatty acid desaturase 2 | 0.58 | 0.00043 | 9415 | NM 004265 |
| 30 (57) | Rar-related orphan receptor a | 0.45 | 0.01727 | 6095 | NM 002943 |
| 31 (58) 32 (59) 33 (60) | | | | | Isoform c NM 134260 Isoform b NM 134261 Isoform a NM 134262 Isoform d |
| 34 (61) | Phosphofructokinase, muscle | 0.17 | 0.04255 | 5213 | NM 000289 |
| 35 (62) 36 (63) 37 (64) 38 (65) 39 (66) 40 (67) 41 (68) 42 (69) 43 (70) | Calpain 3 (p94) | 0.44 | 0.01741 | 825 | NM 000070 Isoform a NM 024344 Isoform b NM 173087 Isoform c NM 173088 Isoform d NM 173089 Isoform e NM 173090 Isoform e (variant 6) NM 212464 Isoform g NM 212465 isoform f NM 212467 Isoform h |

## Claims

1. A method of screening for compounds that reduce and/or prevent obesity comprising
a) contacting a cell expressing a gene listed in table 2 with a compound; and
b) measuring the expression of said gene, or a polypeptide encoded by said gene;
wherein a compound which up-regulates gene expression is a compound which causes an increase of expression of said gene or of the polypeptide encoded by said gene.

2. The method of claim 1, wherein the gene is Seq. ID No. 1.

3. The method of claim 1, wherein the gene is Seq. ID No. 2.

4. The method of claim 1, wherein the gene is Seq. ID No. 3.

5. The method of claim 1, wherein the gene is Seq. ID No. 4.

6. The method of claim 1, wherein the gene is Seq. ID No. 5.

7. The method of claim 1, wherein the gene is Seq. ID No. 6.

8. A method of screening for compounds that reduce and/or prevent obesity comprising
a) contacting a cell expressing a gene listed in table 3 with a compound; and
b) measuring the expression of said gene, or a polypeptide encoded by said gene;
wherein a compound which down-regulates gene expression is a compound which causes a decrease of said gene or a polypeptide encoded by said gene.

9. The method of claim 8, wherein the gene is Seq. ID No. 7.

10. The method of claim 8, wherein the gene is Seq. ID No. 8.

11. A method of screening for compounds that reduce and/or prevent obesity comprising
a) contacting a cell expressing a gene selected from the group consisting of Seq ID No. 17 to 27 with a compound; and
b) measuring the expression of said gene, or a polypeptide encoded by said gene;
wherein a compound which up-regulates gene expression is a compound which causes an increase of expression of said gene or of the polypeptide encoded by said gene.

12. A method of screening for compounds that reduce and/or prevent obesity comprising
a) contacting a cell expressing a gene selected from the group consisting of Seq ID No. 28 to 43 with a compound; and
b) measuring the expression of said gene, or a polypeptide encoded by said gene;
wherein a compound which down-regulates gene expression is a compound which causes a decrease of said gene or a polypeptide encoded by said gene.

13. A method of screening for compounds that reduce and/or prevent obesity comprising:
a) contacting a polypeptide selected from the group consisting of Seq ID No. 9 to 14 with a compound; and
b) determining and/or measuring the activity and/or function of said polypeptide;
wherein a compound which reduces and/or prevents obesity by agonizing said polypeptide is a compound which causes an increase in activity and/or function of said polypeptide.

14. A method of screening for compounds that reduce and/or prevent obesity comprising:
a) contacting a polypeptide selected from the group consisting of Seq ID No. 15 to 16 with a compound; and
b) determining and/or measuring the activity and/or function of said polypeptide;
wherein a compound which reduces and/or prevents obesity by antagonizing said polypeptide is a compound which causes a decrease in activity and/or function of said polypeptide.

15. A method of screening for compounds that reduce and/or prevent obesity comprising:
a) contacting a polypeptide selected from the group consisting of Seq ID No. 44 to 54 with a compound; and
b) determining and/or measuring the activity and/or function of said polypeptide;
wherein a compound which reduces and/or prevents obesity by agonizing said polypeptide is a compound which causes an increase in activity and/or function of said polypeptide.

16. A method of screening for compounds that reduce and/or prevent obesity comprising:
a) contacting a polypeptide selected from the group consisting of Seq ID No. 55 to 70 with a compound; and
b) determining and/or measuring the activity and/or function of said polypeptide;
wherein a compound which reduces and/or prevents obesity by antagonizing said polypeptide is a compound which causes a decrease in activity and/or function of said polypeptide.

17. A method for screening of compounds that reduce and/or prevent obesity comprising:
a) contacting a cell expressing a nucleic acid encoding a polypeptide selected from the group consisting of Seq ID No. 9 to 14 with a compound; and
b) determining and/or measuring the activity and/or function of said polypeptide;
wherein a compound which reduces and/or prevents obesity by agonizing said polypeptide is a compound which causes an increase in activity and/or function of said polypeptide.

18. A method for screening of compounds that reduce and/or prevent obesity comprising:
a) contacting a cell expressing a nucleic acid encoding a polypeptide selected from the group consisting of Seq ID No. 15 and/or 16 with a compound; and
b) determining and/or measuring the activity and/or function of said polypeptide;
wherein a compound which reduces and/or prevents obesity by antagonizing said polypeptide is a compound which causes a decrease in activity and/or function of said polypeptide.

19. A method for screening of compounds that reduce and/or prevent obesity comprising:
a) contacting a cell expressing a nucleic acid encoding a polypeptide selected from the group consisting of Seq ID No. 44 to 54 with a compound; and
b) determining and/or measuring the activity and/or function of said polypeptide;
wherein a compound which reduces and/or prevents obesity by agonizing said polypeptide is a compound which causes an increase in activity and/or function of said polypeptide.

20. A method for screening of compounds that reduce and/or prevent obesity comprising:
a) contacting a cell expressing a nucleic acid encoding a polypeptide selected from the group consisting of Seq ID No. 55 to 70 with a compound; and
b) determining and/or measuring the activity and/or function of said polypeptide;
wherein a compound which reduces and/or prevents obesity by antagonizing said polypeptide is a compound which causes a decrease in activity and/or function of said polypeptide.

21. A method of screening for compounds that bind to a polypeptide selected from the group consisting of the polypeptides of Seq ID No. 9 to 16, comprising the steps of
a) contacting a compound with said polypeptide; and
b) determining the ability of said compound to bind to said polypeptide.

22. A method of screening for compounds that bind to a polypeptide selected from the group consisting of the polypeptides of Seq ID No. 44 to 70, comprising the steps of
a) contacting a compound with said polypeptide; and
b) determining the ability of said compound to bind to said polypeptide.

23. Use of a gene or a polypeptide encoded by a gene listed in tables 2 and/or 3 as a target for screening of compounds that reduce and/or prevent obesity.

24. Use of a nucleic acid encoding a polypeptide selected from the group consisting of Seq ID No. 9-16, or of mutants or fragments thereof as a target for screening of compounds that reduce and/or prevent obesity.

25. Use of a gene or a polypeptide encoded by a gene selected from the group consisting of Seq ID No. 17 to 70 as a target for screening of compounds that reduce and/or prevent obesity.

26. Use of a nucleic acid encoding a polypeptide selected from the group consisting of Seq ID No 44 to 70, or of mutants or fragments thereof as a target for screening of compounds that reduce and/or prevent obesity.

27. A kit for screening for compounds that reduce and/or prevent obesity comprising a polypeptide selected from the group consisting of Seq ID No. 9 to 16.

28. A kit for screening for compounds that reduce and/or prevent obesity comprising a polypeptide selected from the group consisting of Seq ID No. 44 to 70.

29. A compound identified by the method of any one of claims 1 to 22.

30. A pharmaceutical formulation for the modulation of body weight, comprising a compound that modulates the activity of a polypeptide selected from the group consisting of Seq ID No. 9 to 16, mixed with a pharmaceutically acceptable carrier.

31. A pharmaceutical formulation for the modulation of body weight, comprising a compound that modulates the activity of a polypeptide selected from the group consisting of Seq ID No 44 to 70, mixed with a pharmaceutically acceptable carrier.

32. Use of a compound of claim 29 for the preparation of a medicament for the treatment of obesity.

33. Use of a compound of claim 29 for the preparation of a medicament for the treatment of cachexia.

34. The methods, compound, formulation and uses substantially as hereinbefore described, especially with reference to the foregoing examples.


SEQUENCE LISTING

<110>  F. Hoffmann-La Roche AG

<120>  novel targets for obesity from skeletal muscle

<130>  case 22314

<160>  70

<170>  PatentIn version 3.2

<210>  1

<211>  1712

<212>  DNA

<213>  Homo sapiens

<220>

<221>  phospholamban (PLN)

<222>  (1)..(1712)

<223>  accession No.s: NM002667.2; Hs.85050; LocusID: 5350

<400>  1

cagagtcaga aaactcccca gctaaacacc cgtaagactt catacaacac aatactctat    60

actgtgatga tcacagctgc caaggctacc taaaagaaga cagttatctc atatttggct   120

gccagctttt tatctttctc tcgaccactt aaaacttcag acttcctgtc ctgctggtat   180

catggagaaa gtccaatacc tcactcgctc agctataaga agagcctcaa ccattgaaat   240

gcctcaacaa gcacgtcaaa agctacagaa tctatttatc aatttctgtc tcatcttaat   300

atgtctcttg ctgatctgta tcatcgtgat gcttctctga agttctgcta caacctctag   360

atctgcagct tgccacatca gcttaaaatc tgtcatccca tgcagacagg aaaacaatat   420

tgtataacag accacttcct gagtagaaga gtttctttgt gaaaaggtca agattaagac   480

taaaacttat tgttaccata tgtattcatc tgttggatct tgtaaacatg aaaagggctt   540

tattttcaaa aattaacttc aaaataagtg tataaaatgc aactgttgat ttcctcaaca 600

tggctcacaa atttctatcc caaatctttt ctgaagatga agagtttagt tttaaaactg 660

cactgccaac aagttcactt catatataaa gcattatttt tactcttttg aggtgaatat 720

aatttatatt acaatgtaaa agcttcttta atactaagta tttttcaggt cttcaccaag 780

tatcaaagta ataacacaaa tgaagtgtca ttattcaaaa tagtccactg actcctcaca 840

tctgttatct tattataaag aactatttgt agtaactatc agaatctaca ttctaaaaca 900

gaaattgtat tttttctatg ccacattaac atcttttaaa gttgatgaga atcaagtatg 960

gaaaagtaag gccatactct tacataataa aattcctttt aagtaatttt ttcaaagaat 1020

cacagaattc tagtacatgt aggtaaatca taaatctgtt ctaagacata tgatcaacag 1080

atgagaactg gtggttaata tgtgacagtg agattagtca tatcactaat atactaacaa 1140

cagaatctaa tcttcattta aggcactgta gtgaattatc tgagctagag ttacctagct 1200

taccatacta tatctttgga atcatgaaac cttaagactt cagaatgatt ttgcaggttg 1260

tcttccattc cagcctaaca tccaatgcag gcaaggaaaa taaaagattt ccagtgacag 1320

aaaaatatat tatctcaagt atttttaaaa aatatatgaa ttctctctcc aaatattaac 1380

taattattag attatatttt gaaatgaact tgttggccca tctattacat ctacagctga 1440

cccttgaaca tgggggttag gggagctgac aattcgtggg tccgcaaaat cttaactacc 1500

taatagccta ctattgacca taaaccttac tgataacata aacagtaaat taacacatat 1560

tttgcgtgtt atatgtatta tacactatat tcctacaata aagtaagcta gagaaaatgt 1620

tatttagaaa atcataagaa agagaaaata tatttactat tcattaaatg gaagtgggtc 1680

aacaaaaaaa aaaaaaaaaa aaaaaaaaaa aa 1712

<210> 2

<211> 2572

<212> DNA

<213>  Homo sapiens

<220>

<221>  sterol carrier protein 2 (SCP2)

<222>  (1)..(2572)

<223>  accession No.s: NM002979; Hs.75760; LocusID: 6342

<400>  2

cggtcccgca ctggtgcagc catgtcctct tccccgtggg agcctgcgac cctgcgccgg    60

gtgttcgtgg tgggggttgg catgaccaag tttgtgaagc ctggagctga gaattcaaga   120

gactaccctg acttggcaga agaagcaggc aagaaggctt tagctgatgc acagatccct   180

tattcagcag tggaccaggc atgtgttggc tatgtttttg gtgactctac ctgtgggcag   240

agggctatct atcacagttt gggaatgact ggaattccta taatcaatgt caacaataac   300

tgtgctactg gttctactgc tttgtttatg gcccgccagc tgattcaggg tggtgtggca   360

gaatgtgtct tggctcttgg gtttgagaag atgagtaagg gaagccttgg aataaaattt   420

tcagatagaa ccattcccac tgataagcat gttgacctcc tgatcaataa gtatggattg   480

tctgctcacc cagttgctcc tcagatgttt gggtatgctg gaaaagaaca tatggaaaaa   540

tatggaacaa aaattgaaca ctttgcaaaa attggatgga aaaatcataa acattcagtt   600

aataacccgt attcccagtt ccaagatgaa tacagtttag atgaagtgat ggcatctaaa   660

gaagtttttg attttttgac tatcttacaa tgttgtccca cttcagatgg tgctgcagca   720

gcaattttgg ccagtgaagc atttgtacag aagtatggcc tgcaatccaa agctgtggaa   780

attttggcac aagaaatgat gactgatttg ccaagctcgt ttgaagaaaa aagcattatt   840

aaaatggttg gctttgatat gagtaaagaa gctgcaagaa aatgctatga gaaatctggc   900

ctgacaccaa atgatattga cgtaatagaa cttcacgatt gcttttctac caacgaactc   960

ctgacttatg aagcactcgg actctgtcca gaaggacaag gtgcaacgct ggttgataga  1020

ggagataata catatggagg aaagtgggtc ataaatccta gtggtggact gatttcaaag  1080

ggacacccac taggcgctac aggtcttgct cagtgtgcag aactctgctg gcagctgaga 1140

ggggaagccg gaaagaggca agttcctggt gcaaaggtgg ctctgcagca taatttaggc 1200

attggaggag ctgtggttgt aacactctac aagatgggtt ttccggaagc cgccagttct 1260

tttagaactc atcaaattga agctgttcca accagctctg caagtgatgg atttaaggca 1320

aatcttgttt ttaaggagat tgagaagaaa cttgaagagg aaggggaaca gtttgtgaag 1380

aaaatcggtg gtatttttgc cttcaaggtg aaagatggcc ctgggggtaa agaggccacc 1440

tgggtggtgg atgtgaagaa tggcaaagga tcagtgcttc ctaactcaga taagaaggct 1500

gactgcacaa tcacaatggc tgactcagac ttcctggctt taatgactgg taaaatgaat 1560

cctcagtcgg ccttctttca aggcaaattg aaaatcactg gcaacatggg tctcgctatg 1620

aagttacaaa atcttcagct tcagccaggc aacgctaagc tctgaagaac tccctttggc 1680

tacttttgaa aatcaagatg agatatatag atatatatcc atacatttta ttgtcagaat 1740

ttagactgaa actacacatt ggcaaatagc gtggatagga tttgtttctt aatgggtgtg 1800

accaatcctg tttttcctat gctctgggtg aatagagcct gatggtatac tactgctttg 1860

cggaattgca tacaactgtg cattacaaag ttaatatggt aattatggtc tggggtaaaa 1920

ttgagtttca gaataaaatt aggaacagta aaatccaaag aactatgtaa acaaaaaagc 1980

ttttgttttg cttacaaagt atatttaagg attattctgc tgaagattca gtttaagagt 2040

tttccttggg agaactaagt aagaaacaca atgccaacag ctggccagta attagtgttg 2100

tgcacttcat gtcattaatc aatttctcaa tagttcttaa aattagtgag attaaaaatc 2160

taaaaatttt gcatttcatg ctatcagaaa cagtattttc ttcccaaatc aaaataaaag 2220

aaatatgatc agagcttgaa cacaggctta tttttaaaat aaaaatattt ttaacatggg 2280

tttccttatt gaaaaatcag tgtattagtc ataaaacacc atcattaaga ataattgaac 2340

aataaagttt gctttcagat gcagttttca aattataatc tcatttcaat ttataacgtt 2400

ctcagtcctt tgttataatt ttcctttttc atgtaagttt aattatctgc atttatcttt 2460

tttcctagtt tttctaatac taatgttatt tcttaaaatt cagtgagata taggataaaa 2520

taatgctttg agaagaatgt ttaatagaaa attaaaataa ctttttctgg ca          2572

<210>  3

<211>  4646

<212>  DNA

<213>  Homo sapiens

<220>

<221>  UDP-Gal:betaGlcNAc beta 1,4- galactosyltransferase polypeptide 5

       (B4GALT5)

<222>  (1)..(4646)

<223>  accession No.s NM004776.2; Hs.107526; LocusID:6342

<400>  3

gggaggcggt ggccgaggcc caggcggtgg cggcggcggc ccaggaggcg gcggacgggg    60

agctgcggga gcaggcccgg gcctggctct ctagcggccg cctggctgca gcatgcgcgc   120

ccgccggggg ctgctgcggc tgccgcgccg ctcgctgctc gccgcgctct tcttcttttc   180

tctctcgtcc tcgctgctgt acttcgtcta tgtggcgccc ggcatagtga acacctacct   240

cttcatgatg caagcccaag gcattctgat ccgggacaac gtgagaacaa tcggtgctca   300

ggtttatgag caggtgcttc ggagtgctta tgccaagagg aacagcagtg taaatgactc   360

agattatcct cttgacttga accacagtga aaccttcctg caaactacaa catttcttcc   420

tgaagacttc acctactttg caaaccatac ctgccctgaa agactccctt ccatgaaggg   480

cccaatagac ataaacatga gtgaaattgg aatggattac attcatgaac tcttctccaa   540

agacccaacc atcaagctcg gaggtcactg gaagccttct gattgcatgc ctcggtggaa   600

ggtggcgatc cttatcccct tccggaaccg ccacgagcac ctcccagtcc tgttcagaca   660

cctgcttccc atgctccagc gccagcgctt gcagtttgca ttttatgtgg ttgaacaagt   720

tggtacccaa ccctttaatc gagccatgct tttcaacgtt ggctttcaag aggcaatgaa 780

agacttggat tgggactgtt tgatttttca tgatgtagat cacataccgg aaagtgatcg 840

caactattat ggatgtggac agatgccgag gcattttgca accaaattgg ataagtatat 900

gtatctgctt ccttataccg agttctttgg cggagtgagt ggcttaacag tggaacaatt 960

tcggaaaatc aatggctttc ctaatgcttt ctggggttgg ggtggagaag atgacgacct 1020

ctggaacaga gtacagaatg caggctattc tgtgagccgg ccagagggtg acacaggaaa 1080

gtacaagtcc attcctcatc accatcgagg agaagtccag tttcttggaa ggtatgctct 1140

gctgaggaag tcaaaagaac ggcaagggct ggatggcctc aacaacctga actactttgc 1200

aaacatcaca tacgacgcct tgtataaaaa cataactgtc aacctgacac ccgagctggc 1260

tcaggtgaac gagtactgag aggagagaat gtacgtttgc tttacccacc gccaccaaga 1320

aagcagtccg atgagatttt tttttggagg ggggagggtc tacacagcaa gagaacagaa 1380

atactgtgtc tcatgaagga tcacagagtt cagggggaaa atgtgacagc acacgcacaa 1440

acgccttcac tggatcagcc gctggaactg agggagtgag cttggggact tccttcgtca 1500

gcactggctt tctgttttca caagacagac gtctgtcccg ctgctctctc cccatctcct 1560

accccacatc ctgtcttagc cgcagtctcc agaacccatg atgaactgtg atctgccgtg 1620

gtcctgccgt ggtcctgccg tggagcctgt ccctacacat gaccttggag cctcttggcc 1680

ttcagagcag aggcaaaccc accacagggc agctgcgttt taggaagagc aaatgaaact 1740

ccacaccatt cttctagatc tctggtgttc tctttggttt cattttttta aaaaattacc 1800

ttctttgggt ggggattgag ggtggagggg agggtgtttg ggaaagataa atagacataa 1860

atatataaca atcacttctt gaagaagtat aattgtaaat aagccatgta aaatgccttt 1920

ttaaaattta attttctagc tggctccaat tcaaattgag gatttatgta ttaggccact 1980

tacttggttg gcaagtgcag gaactcagtt aaaatgcagt tgaagaatgt catctcccga 2040

attgctgtca ctttggcgag ggagtggata tagggcatgt cacaaaagaa caaaataacc 2100

cgacctttat tgctgggagc tggcttctgt ccctttcttc ccccccccac gagtcttgcc 2160

cttgacttct gctctggatt cactcttccc tgtcggccgc gcatgtgctc atcccactct 2220

ccgctaagcg ggaggctgct gttagagcag gctgcttcct gcctaaagca ggcccttcgg 2280

ggctcgctgc acacacatct ctggctctcc aggcttcgtg ttctgtcttt tcatcagcat 2340

ggcggggcgg ggggcggggg gcgggggtgt gtatgggaat ccctccccct cttacttttt 2400

ctcttgtgga acttggccac agtttctgaa caatgtgcct acattaccag ctggcttcag 2460

tgattcctct gtgtcccttt ttggtttctg gaaagattct ttgtcaacat tagtaactga 2520

tacatagaac caaggagcac tcaaataggg agccaggagc cagggagctg gtgacacttg 2580

tgtgctgtgg ggcagctggg atccaggtaa gaccggattg aagctttgaa attagactaa 2640

caaagctcca gacagcaaga gcccaggtgc actgctcaca cccccacctg cattttgaag 2700

tcatattatt ttttgttttg ttttttaaga cggtctggct ctgtcgccta agctggagtg 2760

tggtggcacg atcacagctc actgcagcct ccatctccta ggctcaagcc attttcccac 2820

ctcagcctcc cgagtagctg ggactacagg tgcacaccac cacacctggc taattttttg 2880

tatttttagt agagacaggg gtttcttcca tgttgcccag gctggtctcg aactcctgga 2940

ctcaagcaat ccgcccacct tgacttccca aagtgctggg attatgggcg ggtgtgagcc 3000

attgcgccca gccttgaagt catgttctaa attgtatttg aatttgtgcc tctttgtttt 3060

tccccaaacc aaagccctca aattgtagtc tctgtcggct tctgcagaat tctggaaaat 3120

gccagttttc ctcccccgcc cttgttttcc ataaaacata tttatatatt gtgatgagga 3180

gtactttctg aagagtactt cgtatttttt tttaattgcc ttgtttgcct tcaacttcct 3240

tgattttcat agtttacatg ggtgtgtgta ggggtgtgtg tgtgtatgtg tgtgggttag 3300

ggcttttttc gttgcatgtg atggttctgt ggacatatga tccccacaaa ctgtgggagt 3360

gattggccag gccttgtttt gtttgtttgt ttgtttgtgt tttgttcttt ttgaagaata 3420

gagtggtatt tagaaaataa attgcattgc aaagctctta tcggctcata tgagagagca 3480

ggttcctgcc cttgaaaatg ccggtaagct atagcatatg ttttttaaga cttaagcatt 3540

tcatgcttta aaataccttc acaagtgaac attacacaca gaagttcatt tggttttcct 3600

ttgttttatg gtgcatatag caataaagac ccccctccac cctgcaaccc ccatccccca 3660

ccgggccttt gtccctgcct tggcttttct ccccttctca ttctcctctc ccctttcctc 3720

actgaaggct gtgagttgct ttcaatgtga caacactatg atgtcatttg gaaggatttg 3780

ccaggacaga ctgattctga gtcctgggtg ccgtatgtgt atgcggcagt gttgtcaggc 3840

gatcttgttt gaagctctat gttgccataa ttaccatcaa gtacacactg ttggcaaaag 3900

gctaacacct gactttagaa aatgctgatt tgagaacaaa aggaaaggtc ttttttcact 3960

gcttaaagtg gggtcacttt gatacctttg cggtcatgtc tgtgtctgat gagtgtagaa 4020

tctctggatg tgcactgtca gtcatgtgtc caccaggcct cgaatatcat atgggaaatg 4080

tcatagttaa aaacgtacag ccaggcccgt gtgctgttaa tagtgtgaaa ttgtcatgtt 4140

aaaaaaaaaa acaggaacca aatgtgacct tgtgcatata ttggtagctg aaaatcttca 4200

aggctactga tgggtggccc cttaatcttg tctttgattg ctgtgtgcag ggaaaggtgt 4260

ccccgtttgt tcatgctgtt ttggggggtg ggggggtatt tgcaagaata ctcattttga 4320

cataataggt cctcttgtca gagatcctct accacagaca ttaatagctg agcaggagcc 4380

acatggattg attgtatcca ctcaccattg acgatggcat tgagcgtagc tagcttattt 4440

ccatcactac gtgtttttga gcttgctctt acgttttaag aggtgccagg ggtacatttt 4500

tgcactgaaa tctaaagatg ttttaaaaaa cacttttcac aaaaatagtc ctttgtcatt 4560

acattattta ctcatgtgtt tgtacatttt tgtatgttaa tttatgaatg attttttcag 4620

taaaaaatac atattcaaga accaaa 4646

<210> 4

<211> 3549

<212> DNA

<213> Homo sapiens

<220>

<221>  lipoprotein lipase (LPL)

<222>  (1)..(3549)

<223>  accession No.s NM000237.1; Hs.180878; LocusID:4023

<400>  4

cccctcttcc tcctcctcaa gggaaagctg cccacttcta gctgccctgc catccccttt    60

aaagggcgac ttgctcagcg ccaaaccgcg gctccagccc tctccagcct ccggctcagc   120

cggctcatca gtcggtccgc gccttgcagc tcctccagag ggacgcgccc cgagatggag   180

agcaaagccc tgctcgtgct gactctggcc gtgtggctcc agagtctgac cgcctcccgc   240

ggaggggtgg ccgccgccga ccaaagaaga gattttatcg acatcgaaag taaatttgcc   300

ctaaggaccc ctgaagacac agctgaggac acttgccacc tcattcccgg agtagcagag   360

tccgtggcta cctgtcattt caatcacagc agcaaaacct tcatggtgat ccatggctgg   420

acggtaacag gaatgtatga gagttgggtg ccaaaacttg tggccgccct gtacaagaga   480

gaaccagact ccaatgtcat tgtggtggac tggctgtcac gggctcagga gcattaccca   540

gtgtccgcgg gctacaccaa actggtggga caggatgtgg cccggtttat caactggatg   600

gaggaggagt ttaactaccc tctggacaat gtccatctct tgggatacag ccttggagcc   660

catgctgctg gcattgcagg aagtctgacc aataagaaag tcaacagaat tactggcctc   720

gatccagctg gacctaactt tgagtatgca gaagccccga gtcgtctttc tcctgatgat   780

gcagattttg tagacgtctt acacacattc accagagggt cccctggtcg aagcattgga   840

atccagaaac cagttgggca tgttgacatt tacccgaatg gaggtacttt tcagccagga   900

tgtaacattg gagaagctat ccgcgtgatt gcagagagag gacttggaga tgtggaccag   960

ctagtgaagt gctcccacga gcgctccatt catctcttca tcgactctct gttgaatgaa  1020

gaaaatccaa gtaaggccta caggtgcagt tccaaggaag cctttgagaa agggctctgc  1080

ttgagttgta gaaagaaccg ctgcaacaat ctgggctatg agatcaataa agtcagagcc  1140

aaaagaagca gcaaaatgta cctgaagact cgttctcaga tgccctacaa agtcttccat  1200

taccaagtaa agattcattt ttctgggact gagagtgaaa cccataccaa tcaggccttt 1260

gagatttctc tgtatggcac cgtggccgag agtgagaaca tcccattcac tctgcctgaa 1320

gtttccacaa ataagaccta ctccttccta atttacacag aggtagatat tggagaacta 1380

ctcatgttga agctcaaatg gaagagtgat tcatacttta gctggtcaga ctggtggagc 1440

agtcccggct tcgccattca gaagatcaga gtaaaagcag gagagactca gaaaaaggtg 1500

atcttctgtt ctagggagaa agtgtctcat ttgcagaaag gaaaggcacc tgcggtattt 1560

gtgaaatgcc atgacaagtc tctgaataag aagtcaggct gaaactgggc gaatctacag 1620

aacaaagaac ggcatgtgaa ttctgtgaag aatgaagtgg aggaagtaac ttttacaaaa 1680

catacccagt gtttggggtg tttcaaaagt ggattttcct gaatattaat cccagcccta 1740

cccttgttag ttattttagg agacagtctc aagcactaaa aagtggctaa ttcaatttat 1800

ggggtatagt ggccaaatag cacatcctcc aacgttaaaa gacagtggat catgaaaagt 1860

gctgttttgt cctttgagaa agaaataatt gtttgagcgc agagtaaaat aaggctcctt 1920

catgtggcgt attgggccat agcctataat tggttagaac ctcctatttt aattggaatt 1980

ctggatcttt cggactgagg ccttctcaaa ctttactcta agtctccaag aatacagaaa 2040

atgcttttcc gcggcacgaa tcagactcat ctacacagca gtatgaatga tgttttagaa 2100

tgattccctc ttgctattgg aatgtggtcc agacgtcaac caggaacatg taacttggag 2160

agggacgaag aaagggtctg ataaacacag aggttttaaa cagtccctac cattggcctg 2220

catcatgaca aagttacaaa ttcaaggaga tataaaatct agatcaatta attcttaata 2280

ggctttatcg tttattgctt aatccctctc tcccccttct tttttgtctc aagattatat 2340

tataataatg ttctctgggt aggtgttgaa aatgagcctg taatcctcag ctgacacata 2400

atttgaatgg tgcagaaaaa aaaaagatac cgtaatttta ttattagatt ctccaaatga 2460

ttttcatcaa tttaaaatca ttcaatatct gacagttact cttcagtttt aggcttacct 2520

tggtcatgct tcagttgtac ttccagtgcg tctcttttgt tcctggcttt gacatgaaaa 2580

gataggtttg agttcaaatt ttgcattgtg tgagcttcta cagattttag acaaggaccg 2640

tttttactaa gtaaaagggt ggagaggttc ctggggtgga ttcctaagca gtgcttgtaa 2700

accatcgcgt gcaatgagcc agatggagta ccatgagggt tgttatttgt tgtttttaac 2760

aactaatcaa gagtgagtga acaactattt ataaactaga tctcctattt ttcagaatgc 2820

tcttctacgt ataaatatga aatgataaag atgtcaaata tctcagaggc tatagctggg 2880

aacccgactg tgaaagtatg tgatatctga acacatacta gaaagctctg catgtgtgtt 2940

gtccttcagc ataattcgga agggaaaaca gtcgatcaag ggatgtattg gaacatgtcg 3000

gagtagaaat tgttcctgat gtgccagaac ttcgaccctt tctctgagag agatgatcgt 3060

gcctataaat agtaggacca atgttgtgat taacatcatc aggcttggaa tgaattctct 3120

ctaaaaataa aatgatgtat gatttgttgt tggcatcccc tttattaatt cattaaattt 3180

ctggatttgg gttgtgaccc agggtgcatt aacttaaaag attcactaaa gcagcacata 3240

gcactgggaa ctctggctcc gaaaaacttt gttatatata tcaaggatgt tctggcttta 3300

cattttattt attagctgta aatacatgtg tggatgtgta aatggagctt gtacatattg 3360

gaaaggtcat tgtggctatc tgcatttata aatgtgtggt gctaactgta tgtgtcttta 3420

tcagtgatgg tctcacagag ccaactcact cttatgaaat gggctttaac aaaacaagaa 3480

agaaacgtac ttaactgtgt gaagaaatgg aatcagcttt taataaaatt gacaacattt 3540

tattaccac 3549

<210> 5

<211> 16556

<212> DNA

<213> Homo sapiens

<220>

<221> low density lipoprotein-related protein 1B

<222> (1)..(16556)

<223>  accession No.s NM018557; Hs.417952; LocusID:53353

<220>

<221>  misc_feature

<222>  (13999)..(13999)

<223>  n is a, c, g, or t

<220>

<221>  misc_feature

<222>  (15128)..(15128)

<223>  n is a, c, g, or t

<400>  5

aaagacagaa ccccagagaa aaacgctgcc aattcgttgc tttattgttc cctgcctggg    60

gacctcaata gccttttcca ttaaccttcc cttcttacgc aacggttaat gacttttggg   120

gttgttttgc tttctgtttc tgctgagtca ctaaattttg cctctttgtc cccaggtgct   180

gctcagcata aaagttaaaa gtgcaattca ggaagtactg ggattctgtg tagagccgag   240

gaaaccattt ccctaagaga agctctgttc cttggcttgt ccttccttcc cgggaaggaa   300

gcttccgagg aacgaaggga gaagctttgt tttgcctgca gaagcagccc tgtgctcggc   360

tgagggttct cagctggctg tgaactgcgg agcattgtag gcgcctggct ggctcaggcc   420

aatgcagaag tctctccctt ctccaaagac ccaaatcccc acagaaccag cttcgagtta   480

ctttcccttc aaggggatta aaataattgt gatttgtggc gctctccgtt cgcggtggta   540

ttttcctgtt gtgttaaatg cctcttatta agtaatagat gtgatttatg tgaacgacga   600

aggggtgtgt ggtggattcg gtgattaatc agtgaattcc catccgctgg catctctcac   660

tgcccctctt gcgtgatgta agatcagacg taccctgcat tgaaaagtca agacacacgg   720

gcgtctcgct cgcgctcaca cacgctctgc ctcctctctc ccgcacgcgc gcatccctcc   780

accttccaca tcctgctcca ggcaggagaa ggctgactgg ctggactcat tgagctgaag 840

aatttccagt gacatttgta aatgacgccg ctcgattcca ggctccaagc ggccgctgcc 900

gccgccgccg ccgccgggcc gaaggtgccg ccgagcagtc tccagcgcag gcttccttac 960

cgggcgacca caatgtccga gtttctcctc gccttactca ctctctcggg attattgccg 1020

attgccaggg tgctgaccgt gggagccgac cgagatcagc agttgtgtga tcctggtgaa 1080

tttctttgcc acgatcacgt gacttgtgtc tcccagagct ggctgtgtga tggggaccct 1140

gactgccctg atgattcaga cgagtcttta gatacctgtc ccgaggaggt agaaatcaag 1200

tgccccttga atcacattgc ttgccttggt accaacaaat gtgttcattt atcccagctg 1260

tgcaatggtg tcttggactg cccagatggg tatgacgaag gagtacattg tcaggaactg 1320

ttatccaatt gccaacagct gaattgtcag tataaatgta caatggtcag aaatagtaca 1380

agatgttact gtgaggatgg attcgaaata acagaagatg ggagaagctg taaagatcaa 1440

gatgaatgtg ctgtttatgg tacatgcagc cagacctgca gaaacacaca tggatcctac 1500

acttgcagtt gtgtggaagg ctacctaatg cagccagaca acagatcttg caaggctaaa 1560

attgaaccta cagatagacc acctatacta ttaattgcaa attttgaaac aattgaggtt 1620

ttctatctta atggaagtaa aatggcaact ctaagctcag tcaatggaaa tgaaattcat 1680

actctggatt ttatttataa tgaagatatg atttgttgga ttgaatcaag agaatcttca 1740

aatcaactca aatgtatcca gataacaaaa gcaggaggat taacagatga atggacaatc 1800

aatattcttc aatccttcca caatgtgcaa caaatggcga ttgactggct cactcgaaat 1860

ctctattttg tggaccatgt cggtgaccgg atctttgttt gtaattccaa cggttctgta 1920

tgtgtcaccc tgattgatct ggagcttcac aatcctaaag caatagcagt agatccaata 1980

gcaggaaaac ttttctttac tgactacggg aatgtcgcca aagtggagag atgtgacatg 2040

gatgggatga accgaacaag gataattgat tcaaagacag agcagccagc tgcactggca 2100

ctagacctag tcaacaaatt ggtttactgg gtagatcttt acttggacta tgtgggagta 2160

gtggactatc aaggaaaaaa tagacacact gtcattcaag gcagacaagt cagacatctt 2220

tatggtataa ctgtgtttga agattatttg tatgcaacca attctgataa ctacaatatc 2280

gtaaggataa accgatttaa tgggactgat attcactcat taattaaaat tgagaatgct 2340

tggggaatcc gaatttatca aaaaagaact caaccaacag tcagaagcca tgcatgtgaa 2400

gtcgatccat atggaatgcc agggggctgt tcacacatct gtctactcag cagcagttac 2460

aaaactcgga cttgtcgctg caggactggc ttcaacttgg gaagtgatgg caggtcatgc 2520

aaaagaccaa agaatgagtt gttcctcttt tatgggaaag gacgcccagg aattgttaga 2580

ggaatggact tgaataccaa gatagctgat gaatacatga tccccataga aaatctggta 2640

aaccctcgtg ctttagactt tcacgcagaa accaattaca tctactttgc tgacaccacc 2700

agtttcctaa ttggccggca gaagatagat ggcacagaga gagaaaccat cctgaaagat 2760

gatctggata atgtagaggg cattgctgtg gactggattg gaaataatct ttactggacc 2820

aatgatggcc ataggaaaac cattaatgtg gccaggctgg aaaaagcttc tcagagtcgg 2880

aagactcttt tagagggtga aatgtctcat cccagaggaa ttgtggtgga tccagttaat 2940

ggttggatgt attggacaga ctgggaggaa gatgaaatag atgacagcgt gggaaggatt 3000

gagaaggcct ggatggatgg attcaatcgg cagatttttg tgacttcaaa gatgctgtgg 3060

ccaaacggtt taactctgga ctttcacacc aacacattat actggtgtga tgcctattac 3120

gatcatattg aaaaagtatt tttgaatggg actcacagga agattgttta cagtgggaga 3180

gagttgaacc accctttcgg actgtcgcat catggaaatt atgtgttctg gactgattat 3240

atgaatggtt ccatttttca actagatttg ataacaagtg aggtgacatt gctgaggcat 3300

gaaagaccac ccctatttgg gcttcagatt tatgatccac gaaagcaaca aggtgacaat 3360

atgtgccgag taaataatgg gggctgtagt acactttgct tggctatccc aggaggccgg 3420

gtgtgtgctt gtgccgataa tcaacttttg gatgaaaatg ggacaacttg cacatttaat 3480

cctggagaag cactacctca catatgtaaa gctggagagt ttcgctgcaa aaacagacac 3540

tgtatccaag ctcggtggaa atgtgatggc gacgatgact gcctagacgg aagcgatgag 3600

gattcagtaa actgcttcaa tcatagctgt cctgatgatc agtttaaatg ccagaataat 3660

cgctgcatcc ccaagagatg gctttgtgat ggagctaatg actgtgggag caatgaagat 3720

gaatccaatc aaacttgcac agccagaaca tgccaggtag accagttttc ttgcggaaat 3780

gggcgttgca ttcccagagc atggctgtgt gacagggaag acgactgtgg tgaccagaca 3840

gatgaaatgg catcttgtga attcccaact tgtgagccac taacccaatt cgtatgcaaa 3900

agtggaagat gcattagcag caaatggcac tgcgactctg atgacgactg tggggacggg 3960

agtgatgagg tgggctgtgt tcactcttgc tttgataatc agttcagatg ttccagtggc 4020

agatgcatcc caggccactg ggcctgtgat ggtgacaatg actgtgggga cttcagtgat 4080

gaagcccaga tcaattgtac taaagaagag attcattctc ctgctggttg taacggaaat 4140

gaatttcagt gccaccctga tggtaattgc gttcctgatt tgtggcgctg tgatggagaa 4200

aaagactgtg aagatggtag tgatgaaaaa ggttgcaatg gtaccatacg attgtgtgac 4260

cacaaaacca agttttcctg ttggagtaca gggagatgca tcaacaaagc atgggtgtgt 4320

gatggagata ttgattgcga agatcagtca gatgaagatg actgtgacag tttcttgtgt 4380

ggaccaccca agcatccttg tgctaatgac acctcagtct gcctgcagcc agagaaactc 4440

tgcaatggga aaaaggattg tcctgatggc tctgatgaag gctatctctg tgatgagtgt 4500

tcgctgaaca atggaggctg tagcaaccac tgttctgttg ttcctggaag aggaattgtc 4560

tgttcctgcc ctgaaggact tcaactcaac aaagacaata aaacatgtga aattgtggat 4620

tattgtagca atcatctaaa gtgcagccaa gtatgtgagc agcacaagca cacagtcaag 4680

tgctcatgtt atgaaggttg gaagctggat gtagacggtg aaagttgtac aagtgttgat 4740

ccttttgaag cattcatcat cttttctatt cgtcatgaga tcagaaggat tgatcttcac 4800

aaaagagact atagtctact tgttcctgga ttgagaaaca caatagcact tgattttcac 4860

ttcaatcaaa gtttacttta ttggacagat gttgtagaag acagaatata ccggggaaag 4920

ctttctgaaa gtggaggtgt cagtgccatt gaagtggttg tggagcatgg cctggctact 4980

ccagaaggcc tgacagtcga ctggatagca ggaaacatat actggataga cagcaatctg 5040

gaccaaatcg aagtggccaa actagatggc tccctaagaa ctacactaat agcaggagcc 5100

atggaacacc ccagggccat tgctttggac ccaagatatg gaattctttt ctggacagac 5160

tgggatgcaa attttcctcg cattgaatct gcctctatga gtggtgctgg gagaaaaacc 5220

atctataaag acatgaaaac tggggcttgg cctaatggac taactgtgga ccactttgag 5280

aaaaggatag tgtggacaga cgccaggtca gatgctattt attcagccct ctatgatgga 5340

acaaacatga tagaaatcat ccgaggtcat gaataccttt cccatccctt tgctgtgtct 5400

ctatatggga gtgaagtcta ctggacagac tggaggacca acacattgtc caaagccaat 5460

aagtggacag ggcagaatgt cagtgtgatt cagaaaacca gtgcacagcc atttgacctt 5520

cagatatacc atcccagtcg ccagccacag gctcccaatc cttgtgcagc taatgatggc 5580

aaaggcccct gctctcacat gtgtctaatc aatcacaata ggagtgctgc ctgtgcgtgc 5640

ccccacttga tgaagctttc ttcagacaag aagacctgct atgaaatgaa aaaatttctt 5700

ctttatgcaa gacgttctga aatcagagga gtggatattg acaatccata ctttaacttc 5760

atcacggcat ttacagtccc tgatattgat gacgttactg tgatagactt cgatgcatct 5820

gaggaacgtt tatactggac agatattaaa acacaaacca ttaaacgagc ttttattaac 5880

ggaactgggt tagaaactgt tatttcaaga gatattcaga gtatcagagg gctagcagtg 5940

gattgggtgt cacgtaattt atactggatt agctcagaat ttgatgaaac gcaaattaat 6000

gtggcaaggc tagatggctc tttgaaaacc tcaattatcc atggaatcga taagccacag 6060

tgtcttgcag ctcacccagt caggggaaaa ctctactgga ccgatggaaa cacaattaac 6120

atggcaaata tggatggcag taatagcaag attctgtttc agaatcagaa ggagccagtt 6180

ggtctatcga tagactatgt ggaaaacaag ctttattgga tcagttcggg gaatggaacc 6240

ataaatagat gcaacctgga tggtggtaat ttagaagtaa tcgagtcaat gaaagaagaa 6300

ttaacaaaag ctacagccct aaccatcatg gataagaaac tgtggtgggc agaccaaaac 6360

ttagcccagc taggaacctg cagcaaaaga gacggaagaa accccaccat cctacggaat 6420

aagacttctg gggtagttca tatgaaagtc tatgataaag aagcacagca aggcagcaat 6480

tcctgccaac taaacaatgg tggatgctct caactttgtt taccaacatc tgaaactaca 6540

aggacttgta tgtgtacagt gggatattat ctccaaaaga accgtatgtc atgtcaaggt 6600

atagaatcat ttcttatgta ctctgttcat gaaggaatca ggggaatacc tcttgaacca 6660

agtgacaaaa tggatgcttt gatgcctata tcaggaactt catttgccgt gggaatagat 6720

ttccatgcag aaaatgatac catctactgg acagacatgg gcttcaataa aattagcaga 6780

gctaaaagag atcagacttg gaaagaagat atcattacca atggcttggg aagagtggaa 6840

gggatagctg ttgactggat tgctggtaac atatattgga cagatcatgg tttcaactta 6900

attgaagttg caagactcaa tggttctttc cgttatgtaa ttatttccca aggcctggat 6960

caaccaagat ctatagctgt gcacccagag aaaggcctct tgttctggac tgaatgggga 7020

caaatgccct gtattggaaa ggctcgcttg gatggctcag agaaggttgt ccttgtaagc 7080

atgggaatag catggccgaa tggcatctcc atcgactatg aggaaaataa attgtactgg 7140

tgtgatgctc gcacagacaa gatagagaga atcgaccttg agactggagg gaatcgcgag 7200

atggtgctgt caggaagcaa tgtggatatg ttttcagttg cagtctttgg ggcttacatc 7260

tactggtctg acagagcaca tgcaaacggg tctgtcagaa ggggccacaa gaatgatgcc 7320

acagaaacga taaccatgag aaccggcctt ggagtcaacc tgaaggaggt taaaatattt 7380

aaccgagtaa gagagaaagg gaccaatgtt tgtgccaggg acaatggtgg ctgtaagcaa 7440

ctctgtcttt atcgaggaaa ttcccggaga acttgtgctt gtgcccatgg atatttggca 7500

gaagatggag ttacttgcct gaggcatgaa ggctatttac tgtattcagg aagaacaata 7560

ttaaaaagta tacatctttc tgatgaaacc aatttaaatt ccccaataag gccatatgag 7620

aatccacgtt atttcaagaa tgtcatagcc ttggcttttg actataatca aagaagaaaa 7680

ggtaccaacc gaatctttta cagtgatgca cactttggaa atatacagct tattaaagac 7740

aactgggaag acagacaagt aattgttgaa aatgtgggtt ctgtggaagg acttgcctat 7800

cacagagcct gggatacact gtactggaca agctctacca cctcatccat caccagacac 7860

actgtggacc agactcggcc tggagcattt gacagggaag ctgtcatcac catgtcagaa 7920

gatgaccatc cacatgtgct agccttggat gaatgtcaaa atttaatgtt ttggaccaac 7980

tggaatgaac aacatccaag tatcatgaga tctactctga ctgggaaaaa tgctcaagtg 8040

gtggtcagta cagacatact cactccaaat ggacttacta tcgactaccg tgcagagaag 8100

ctgtatttct cagatggcag tctaggaaaa attgaaaggt gtgaatacga tggatcccag 8160

agacatgtga tagttaaatc tgggccaggg actttcctca gtttggctgt ttatgacaat 8220

tatatattct ggtcggactg gggaagaaga gctatactgc ggtccaacaa gtacacagga 8280

ggagatacaa aaattcttcg ttccgatatt ccacatcagc caatgggaat catagctgtt 8340

gccaatgaca ccaatagctg tgaactttct ccatgtgcat tattgaatgg aggctgccat 8400

gacttgtgcc ttttaactcc caatggagaa gtgaattgtt cctgcagagg ggaccgaata 8460

ttgctagagg acaacagatg tgtgactaaa aattcctcct gcaacgctta ttcggagttt 8520

gaatgtggaa atggtgagtg cattgactac cagctcacct gtgatggcat tcctcactgt 8580

aaagataaat cagatgaaaa actgctctac tgtgaaaaca gaagctgtcg aagaggcttc 8640

aagccatgct ataatcgccg ctgcattcct catggcaagt tatgtgatgg agaaaatgac 8700

tgcggagaca actctgatga attagattgt aaagtttcaa cctgtgccac ggttgagttc 8760

cgctgtgcag atgggacttg tattccaaga tcagcacgat gcaaccagaa catagattgt 8820

gcagatgctt cagatgaaaa gaactgcaat aacacagact gcacacattt ctataagctt 8880

ggagtgaaaa ccacagggtt cataagatgt aattctacct cactgtgtgt tctgccaacc 8940

tggatatgcg acgggtctaa tgactgtgga gactattcag atgaattaaa gtgcccagtt 9000

cagaacaaac acaaatgtga agaaaattat tttagttgtc ctagtggaag atgcattttg 9060

aatacctgga tatgcgatgg tcagaaagat tgtgaggatg gacgtgatga attccactgt 9120

gattcttctt gctcttggaa ccaatttgct tgttccgcac aaaaatgtat ttctaagcat 9180

tggatttgtg atggagaaga tgactgtggg gatgggttag atgaaagtga cagcatttgt 9240

ggtgccataa cctgtgctgc tgacatgttc agctgccagg gctctcgtgc ctgcgtgccc 9300

cgacattggc tttgtgatgg tgaaagggac tgtccagatg gaagcgatga gctttccaca 9360

gcaggctgcg ctcccaataa tacatgtgat gaaaatgctt tcatgtgcca taataaagta 9420

tgcattccca agcaatttgt ttgtgaccat gatgacgact gtggagatgg ctctgatgag 9480

tcaccgcagt gtggataccg acagtgtggt acagaagaat ttagttgtgc tgatgggcgg 9540

tgtcttctaa atactcaatg gcagtgtgat ggagactttg actgtcctga ccattctgat 9600

gaagcacctt taaacccaaa gtgtaaaagt gcagaacagt catgcaacag ttcatttttt 9660

atgtgcaaaa atggcaggtg cattcccagt ggaggtcttt gtgacaataa ggatgactgt 9720

ggcgatggtt cagatgagag aaactgccat ataaatgaat gtttgagtaa gaaagtcagt 9780

ggatgttctc aagattgtca agaccttccg gtcagttata agtgcaaatg ctggcctgga 9840

ttccaactga aggatgacgg caaaacatgt gtagacattg atgaatgctc ttcaggcttt 9900

ccctgtagcc agcaatgcat caatacatac gggacttaca agtgcctctg tacagatggg 9960

tatgaaatac aacctgataa cccaaatggc tgcaaatcgc tctcagatga agaacctttt 10020

ttaattcttg ctgatcatca tgagataagg aaaattagca ctgatggctc caactacaca 10080

cttttaaaac agggattaaa caatgttatt gctatagact ttgattacag agaagaattc 10140

atctattgga tcgattctag ccgacccaat ggcagtcgca taaatagaat gtgtttaaat 10200

ggaagtgaca ttaaggtagt tcataacaca gcggtcccca atgcacttgc tgtcgattgg 10260

attggaaaaa acctctattg gtctgacaca gaaaaaagaa tcattgaagt atccaaactc 10320

aatggcttgt accctactat actcgttagc aaaaggctga agtttcccag agacttgtct 10380

ttagatcctc aagctggata tttgtattgg attgactgct gcgagtatcc tcatattggc 10440

cgtgttggaa tggatggaac caatcagagt gttgtcatag aaaccaagat ttctagacct 10500

atggcactaa caatagatta tgttaatcgt agactctact gggccgatga aaatcacatt 10560

gaatttagca acatggatgg atctcataga cacaaagtcc ctaatcaaga tattccaggg 10620

gtgattgcac taacattgtt tgaagactac atctactgga ctgatgggaa aaccaagtca 10680

ctcagccgtg cccataaaac atcgggagca gacagactct cactgattta ctcatggcat 10740

gccatcacag atatccaggt gtatcattct tatagacaac ctgatgtctc caaacatctc 10800

tgcatgataa ataatggtgg ttgcagtcat ttgtgccttt tagcccctgg aaaaacccac 10860

acttgtgcat gtcccactaa cttctatctg gcagctgata ataggacttg cttatccaac 10920

tgcacagcca gccagtttcg ttgcaaaact gacaaatgta ttccattctg gtggaaatgt 10980

gacaccgtgg atgactgtgg tgatggatct gatgaacctg atgactgtcc tgaatttaga 11040

tgtcagccag gccgatttca gtgtgggact ggactctgtg ctctaccagc tttcatctgt 11100

gatggagaga atgattgtgg agacaattct gatgaactca actgtgacac acatgtctgc 11160

ctgtcaggtc aattcaaatg taccaagaac cagaaatgta tcccagtaaa cttaagatgt 11220

aatgggcaag atgactgtgg tgatgaggaa gatgaaagag actgtcctga aaacagctgt 11280

tctccagact atttccagtg taagactacg aagcattgca tttccaagct gtgggtttgt 11340

gacgaggatc cagactgtgc agatgcatca gacgaggcca actgcgataa aaagacttgt 11400

ggacctcatg aattccagtg taaaaacaac aactgtattc ccgatcactg gcggtgtgat 11460

agccaaaatg actgcagtga taattcagat gaagaaaact gtaagccaca gacatgtaca 11520

ttgaaagatt tcctctgtgc caatggggac tgtgtttctt caaggttttg gtgtgatgga 11580

gattttgact gtgcagatgg ctctgatgag agaaattgtg agacaagttg ttccaaagat 11640

cagttccggt gttccaatgg tcagtgtata ccagcaaaat ggaaatgtga tggccatgaa 11700

gactgcaaat atggggaaga tgagaaaagc tgtgagccag cttctcctac ttgctcatca 11760

cgtgaatata tatgtgccag tgatggatgt atttcagcat ctttgaaatg taatggagaa 11820

tatgattgtg ctgatggttc agatgagatg gactgtgtga ctgaatgtaa ggaagatcag 11880

tttcggtgca aaaataaagc ccactgtatt ccaattagat ggctgtgtga tggaattcat 11940

gactgtgtgg atggcagtga tgaagagaac tgtgaaagag gaggaaatat atgtagagct 12000

gatgagttcc tttgcaataa ttctctctgc aaactacatt tctgggtgtg tgatggagag 12060

gacgactgtg gagacaactc tgatgaagcc cctgatatgt gtgtcaaatt tctttgtcca 12120

tccacgagac ctcacagatg cagaaataac agaatatgcc tacagtcgga gcaaatgtgc 12180

aatggaattg atgaatgcgg tgacaattca gatgaagatc actgtggtgg taagctgaca 12240

tataaagcaa ggccttgtaa aaaggatgag tttgcttgta gtaataaaaa atgcatccct 12300

atggatctcc agtgtgatcg acttgatgac tgcggagatg gttcagatga gcaaggatgc 12360

agaatagctc ctactgaata tacctgtgaa gataatgtga atccatgtgg agatgatgca 12420

tattgtaatc aaataaaaac atctgttttc tgtcgctgta agcctggatt tcagagaaac 12480

atgaaaaaca gacaatgtga agaccttaat gaatgtttgg tgtttggcac atgttcccat 12540

caatgtataa atgtggaagg atcatataaa tgtgtgtgtg accagaattt tcaagaaaga 12600

aataacacct gcatagcaga aggctctgaa gatcaagttc tctacattgc taatgacact 12660

gatatcctgg gttttatata tccattcaac tacagtggcg atcatcaaca aatttctcat 12720

attgaacata attcaagaat aacagggatg gatgtatatt atcaaagaga tatgattatt 12780

tggagtactc agtttaatcc aggcggaatt ttctacaaaa ggatccatgg cagagaaaaa 12840

aggcaagcaa acagtggctt gatttgtcct gaatttaaaa ggcccaggga cattgcagtt 12900

gactgggtgg ctggaaacat ttactggact gatcattcta gaatgcattg gttcagttac 12960

tacactactc actggaccag tctgaggtac tctatcaacg tagggcagct gaatggcccc 13020

aactgcacca gactcttaac aaatatggct ggagaaccct atgctattgc agtaaatcct 13080

aaaagaggga tgatgtactg gactgttgtt ggggatcatt cccatataga agaagcagcc 13140

atggatggta cactgagaag gattttagta caaaagaact tacagagacc cacaggtttg 13200

gctgtggatt attttagtga acgcatatat tgggctgact ttgagctctc catcattggc 13260

agtgttctgt atgatggctc taattcagta gtctctgtca gcagcaaaca aggtttatta 13320

catccacata ggatcgatat ctttgaagat tatatatatg gagcaggacc taaaaatggt 13380

gtatttcgag ttcaaaaatt tggccatggt tcagtagagt acttagcttt aaatattgat 13440

aaaacaaaag gtgttttgat atctcatcgt tataaacaac tagatttacc caatccatgc 13500

ttggatttag catgcgaatt tctttgcttg ctaaatcctt ctggggccac ttgtgtgtgt 13560

ccagaaggaa aatatttgat taatggcacc tgcaatgatg acagcctgtt agatgattca 13620

tgtaagttaa cttgtgaaaa tggaggaaga tgcattttaa atgagaaagg tgatttgagg 13680

tgtcactgtt ggcccagtta ttcaggagaa agatgtgaag tcaaccactg tagcaactac 13740

tgccagaatg gaggaacttg cgtaccatca gttctaggga gacccacctg cagctgtgca 13800

ctgggtttca ctgggccaaa ctgtggtaag acagtctgtg aggatttttg tcaaaatgga 13860

ggaacctgca ttgtgactgc tggaaaccag ccttactgcc actgccagcc ggaatacacc 13920

ggagacagat gtcagtacta cgtgtgccac cactattgtg tgaattctga atcatgtacc 13980

attggggatg atggaagtnt tgaatgtgtc tgtccaacgc gctatgaagg accaaaatgt 14040

gaggttgaca agtgtgtaag gtgccatggg gggcactgca ttataaataa agacagtgaa 14100

gatatatttt gcaactgcac taatggaaag attgcctcta gctgtcagtt atgtgatggc 14160

tactgttaca atggtggcac atgccagctg gaccccgaga caaatgtacc tgtgtgtcta 14220

tgctccacca actggtcagg cacacagtgt gaaaggccag ccccaaagag cagcaagtct 14280

gatcatatca gcacaagaag cattgccatc attgtgcctc tcgtcctctt ggtgactttg 14340

ataaccacct tagtaattgg tttagtgctt tgtaaaagaa aaagaaggac aaaaacaatt 14400

agaagacaac ctattatcaa tggaggaata aatgtagaaa ttggcaatcc atcttataac 14460

atgtatgagg tagatcatga tcacaacgat ggaggtcttt tagatcctgg ctttatgata 14520

gacccaacaa aggccaggta cataggggga ggacccagtg ctttcaagct tccacacaca 14580

gcgccgccca tctacctaaa ctctgatttg aaaggaccac taactgctgg gccaacaaat 14640

tactccaatc cggtatatgc aaaattatat atggatgggc aaaactgtcg aaactcctta 14700

ggaagtgttg atgaaaggaa agaactgctt ccaaagaaaa tagaaattgg tataagagag 14760

acagtggcat aatcagtgat atcttttata tgctgtataa atgtataaaa tataaggatt 14820

acttttgtat gttccaacag tattatactt gttttggcat cagcattacc tctttcttta 14880

tctttttcct ggttaattgt tttctgagtt ttttgggttt tattttttgc tgatgactat 14940

tgattgacca tttgtatggt atttttatga aaaagaactg cactacagta caatttacaa 15000

caatgctgct gatatgacac acctttgaat ttgttaaaat taaaaacaac gtattccttt 15060

gtagtgtgaa tatgagcaat ctattttata tgaacttttt tggttgtact taatcaacga 15120

ggagaatntc tgcacttttc cattatacgg tttgaaggct gtaatacagt gtcattttat 15180

ttttctgttt aaattgatgg aaaaatgatt gaatggtcaa ctctcttctt tgtgcccata 15240

aagatcgatt cagactctgc tgaaaatata tagctctcac aagttcagca tcacctgctt 15300

tgaaattagc cttagattgc caaccaatag atgagaattt tgaggaaaaa aattaaaaat 15360

atgtaaaatt aataatttgc atgaacacag atgactacat tttccaaaac ttagtggact 15420

ctatgtgatg tactaaatgt atacaccttg taagcaatag ttatatttag gtggtagaac 15480

atagcaaaaa tataaccgaa agttggccga ctgcacttgc tatggaataa gaccttttat 15540

tctccctcag tctcgagata aatagccagc ctagagcaca acagggcatt gggtacttgc 15600

atcttaggta tttcttccca gtcacatcca ttttgtggaa gattaaccca accccttaca 15660

ctacactgaa cactaaagaa taacatataa gcacacaaat tggtgacaga atttcaatta 15720

cgtgaacgca tcctctttgc taggtcaaaa acaaagggca aagcagacat tttagtatac 15780

agagtgattg gcaaatattt tcaagattta atatgagcaa cccattattt gccctatcca 15840

aaatatattc aagggccttc caagttgtag aagaacaatg atcttcccat aatcaaaagt 15900

ggagagtcga aatgctgtgc cagttgctct ggtattcagg tttctctggg ttttacagaa 15960

cgcatggacc ccattcacgt ttggtttgtt tatcttcaaa tttgagttga aacgagtgcg 16020

atttatttaa gttgtatata aaaataaaag gatagcattt ttatacaaat atctttaaag 16080

gcacaaaaga tttattcaca agttttggag ggctttttgt tcctctgata gacatgactg 16140

acttttagct gtcataatgt attaacctaa cagatgaaat atgttaaata tgtggttgct 16200

ctttatccct ttgtacaagc attaaaaaaa ctgctgtttt ataagaagac tttttgttgt 16260

actatgtgca tgcatactac ctatttctaa actttgccat attgaggcct ttataaacta 16320

ttgatttatg taatactagt gcaattttgc ttgaacaatg ttatgcatat cataaacttt 16380

ttcaggttct tgtttaagta cattttttaa attgaacagt atttttcatt ttggttataa 16440

tatagtcatt ttgcctatgt ttctacaatg aagtgttaaa tactttataa aaaattgttg 16500

actgacttat ttaaatgaaa ttctacatat aaaaaaaaaa aaaaaaaaaa aaaaaa 16556

<210>  6

<211>  3003

<212>  DNA

<213>  Homo sapiens

<220>

<221>  hypothetical protein MGC10940

<222>  (1)..(2906)

<223>  accession No.s: BC004331.1; Hs.388160; LocusID:84263

<400>  6

gataaatgcg gagggacggt ccagctttag ctctctgctc gccgccgccg ctgtcgccgc    60

cacctcctct gatctacgaa agtcatgtta cccaacaccg ggaggctggc aggatgtaca   120

gtttttatca caggtgcaag ccgtggcatt ggcaaagcta ttgcattgaa agcagcaaag   180

gatggagcaa atattgttat tgctgcaaag accgcccagc cacatccaaa acttctaggc   240

acaatctata ctgctgctga agaaattgaa gcagttggag gaaaggcctt gccatgtatt   300

gttgatgtga gagatgaaca gcagatcagt gctgcagtgg agaaagccat caagaaattt   360

ggagcttata ccattgctaa gtatggtatg tctatgtatg tgcttggaat ggcagaagaa   420

tttaaaggtg aaattgcagt caatgcatta tggcctaaaa cagccataca cactgctgct   480

atggatatgc tgggaggacc tggtatcgaa agccagtgta gaaaagttga tatcattgca   540

gatgcagcat attccatttt ccaaaagcca aaaagtttta ctggcaactt tgtcattgat   600

gaaaatatct taaaagaaga aggaatagaa aattttgacg tttatgcaat taaaccaggt   660

catcctttgc aaccagattt cttcttagat gaatacccag aagcagttag caagaaagtg   720

gaatcaactg gtgctgttcc agaattcaaa gaagagaaac tgcagctgca accaaaacca   780

cgttctggag ctgtggaaga aacatttaga attgttaagg actctctcag tgatgatgtt   840

gttaaagcca ctcaagcaat ctatctgttt gaactctccg gtgaagatgg tggcacgtgg 900

tttcttgatc tgaaaagcaa gggtgggaat gtcggatatg gagagccttc tgatcaggca 960

gatgtggtga tgagtatgac tactgatgac tttgtaaaaa tgttttcagg gaaactaaaa 1020

ccaacaatgg cattcatgtc agggaaattg aagattaaag gtaacatggc cctagcaatc 1080

aaattggaga agctaatgaa tcagatgaat gccagactgt gaaggaaaat ataaaaaaaa 1140

agtcgactgc tatgctcaaa aagtaaaaaa agctcaacag ttaaaatcta atgtttgttt 1200

tctttcctgt tatattataa ggatatgcac gtttgttctg gaaaagatag aatttgtctc 1260

taaaagactt gaaattgtaa ttaaaatggc aagctaatca aacataagct tcattaagtg 1320

ggattctaag acagtctgtg tttttatatt tcaagggttt aaccctttga gccttacatc 1380

tcattcactg tctttctcca agaaaagtat tttgggcgga cagtcagatc aagcagtaaa 1440

attagctctt tcaaatcttc ttgtcatgta aaatgaagct agtctgtttt aaaattttta 1500

gttttggatt gtatactaat gaaaatctta atgatgtttt tgatttttat atacttattt 1560

taaagaaaat cttatatagt acattttaca aaaattataa aaaatgaatt agtactggcg 1620

aggactaaat gaaacaataa tttttcattt tgataactag ctttccaggt ggacttagcc 1680

ataggaaaat attactaatg taatttaaca aattgctgca tgtattccat ttaaaaatat 1740

gtttaaattg tcctaaaaca aaataatttt ctccctagga gtatgcattt ggctacagtg 1800

ttttgaaaca gaaaccttag aataggtcat tggtatgggc tgaactgtgt atcccccaat 1860

tcatttgttg aggtcctaac tcccatttct tttgaatgtg actgttcgga gatgaggcct 1920

ttaaagaggt gacttaagtt caaaggaggc tgttagtcta atccaacatg gtgtcctttg 1980

gacataagag ataccagcaa tgtgtgcaca gaacaaagac caggagagga cacagtgaga 2040

aggcagttat ctgcaagcaa agagagaggc ttcagaagaa acaaaatcac cagcaccttg 2100

atctttgact tctaatctcc agaatagtga gaaataaatt tctgttgtta agccgtccac 2160

tgtgggaggc cgacgcagga ggattgcttg aggccaggag ttcaaggcca gcctggacaa 2220

catagtaaga ccctatctct acccccctaa taaattaatt taaaaagccc cccaatctgt 2280

ggtattttat tatggcagcc ctagcaagct aatacagtgg tttgagaggc tgggagggtt 2340

gaggggaaga taaactttta aaaagctctt atctttcatt tcaatcagtt aaaaatactt 2400

gctcagtgta acaattttgc ttctcagctt ccactctaat attgttgtgc cattaagcaa 2460

tttagctaat cctgacattt cttagattca taatgttagg agcatttaat ctgtatttta 2520

caagttagga agcagaggat cagagatggg aaaggactag cccaaggcca acattaacaa 2580

gccctctaac aaaaacttta caatacattt atgttgaatg gaactccaag atctcacctc 2640

tccatccagg aatggagtcc atgtaatcaa agtgaactta aaaataggac agtttcaaca 2700

agtcaggaga ttcacagcaa ctgatcaaag ggagtccagt caacgtgagc aagcgtgatt 2760

atgatgagga agccccctct gctttaatcc acacaaggaa cgtaacctga agtaacctga 2820

tgttaaccaa tctgctgtgt ctactatgct gtttccttgt tcctgctagt gctgctttac 2880

aaatgcagac cattctatca tacctggcag ggcttctgtt ttattttgta ggctggatgc 2940

tacccagttc atgaatcgct aataaaagcc aattagatct ttaaaaaaaa aaaaaaaaaa 3000

aaa 3003

<210> 7

<211> 3149

<212> DNA

<213> Homo sapiens

<220>

<221> fatty acid desaturase 2 (FADS2)

<222> (1)..(3149)

<223> accession No.s: NM_004265; Hs.184641; LocusID: 9415

<400> 7

agggggcgcg gtgggaggag taggagaaga caaaagccga aagcgaagag ggcccgggct 60

gcacacaccg gctgggaggc agccgtctgt gcagcgagca gccggcgcgg ggaggccgca 120

gtgcacgggg cgtcacagtc ggcaggcagc atggggaagg gagggaacca gggcgagggg 180

gccgccgagc gcgaggtgtc ggtgcccacc ttcagctggg aggagattca gaagcataac 240

ctgcgcaccg acaggtggct ggtcattgac cgcaaggttt acaacatcac caaatggtcc 300

atccagcacc cggggggcca gcgggtcatc gggcactacg ctggagaaga tgcaacggat 360

gccttccgcg ccttccaccc tgacctggaa ttcgtgggca agttcttgaa acccctgctg 420

attggtgaac tggccccgga ggagcccagc caggaccacg gcaagaactc aaagatcact 480

gaggacttcc gggccctgag gaagacggct gaggacatga acctgttcaa gaccaaccac 540

gtgttcttcc tcctcctcct ggcccacatc atcgccctgg agagcattgc atggttcact 600

gtcttttact ttggcaatgg ctggattcct accctcatca cggcctttgt ccttgctacc 660

tctcaggccc aagctggatg gctgcaacat gattatggcc acctgtctgt ctacagaaaa 720

cccaagtgga accaccttgt ccacaaattc gtcattggcc acttaaaggg tgcctctgcc 780

aactggtgga atcatcgcca cttccagcac cacgccaagc ctaacatctt ccacaaggat 840

cccgatgtga acatgctgca cgtgtttgtt ctgggcgaat ggcagcccat cgagtacggc 900

aagaagaagc tgaaatacct gccctacaat caccagcacg aatacttctt cctgattggg 960

ccgccgctgc tcatccccat gtatttccag taccagatca tcatgaccat gatcgtccat 1020

aagaactggg tggacctggc ctgggccgtc agctactaca tccggttctt catcacctac 1080

atccctttct acggcatcct gggagccctc cttttcctca acttcatcag gttcctggag 1140

agccactggt ttgtgtgggt cacacagatg aatcacatcg tcatggagat tgaccaggag 1200

gcctaccgtg actggttcag tagccagctg acagccacct gcaacgtgga gcagtccttc 1260

ttcaacgact ggttcagtgg acaccttaac ttccagattg agcaccacct cttccccacc 1320

atgccccggc acaacttaca caagatcgcc ccgctggtga agtctctatg tgccaagcat 1380

ggcattgaat accaggagaa gccgctactg agggccctgc tggacatcat caggtccctg 1440

aagaagtctg ggaagctgtg gctggacgcc taccttcaca aatgaagcca cagcccccgg 1500

gacaccgtgg ggaaggggtg caggtggggt gatggccaga ggaatgatgg gcttttgttc 1560

tgaggggtgt ccgagaggct ggtgtatgca ctgctcacgg accccatgtt ggatctttct 1620

ccctttctcc tctccttttt ctcttcacat ctcccccata gcaccctgcc ctcatgggac 1680

ctgccctccc tcagccgtca gccatcagcc atggccctcc cagtgcctcc tagccccttc 1740

ttccaaggag cagagaggtg gccaccgggg gtggctctgt cctacctcca ctctctgccc 1800

ctaaagatgg gaggagacca gcggtccatg ggtctggcct gtgagtctcc ccttgcagcc 1860

tggtcactag gcatcacccc cgctttggtt cttcagatgc tcttggggtt cataggggca 1920

ggtcctagtc gggcagggcc cctgaccctc ccggcctggc ttcactctcc ctgacggctg 1980

ccattggtcc accctttcat agagaggcct gctttgttac aaagctcggg tctccctcct 2040

gcagctcggt taagtacccg aggcctctct taagatgtcc agggccccag gcccgcgggc 2100

acagccagcc caaaccttgg gccctggaag agtcctccac cccatcacta gagtgctctg 2160

accctgggct ttcacgggcc ccattccacc gcctccccaa cttgagcctg tgaccttggg 2220

accaaagggg gagtccctcg tctcttgtga ctcagcagag gcagtggcca cgttcaggga 2280

ggggccggct ggcctggagg ctcagcccac cctccagctt ttcctcaggg tgtcctgagg 2340

tccaagattc tggagcaatc tgacccttct ccaaaggctc tgttatcagc tgggcagtgc 2400

cagccaatcc ctggccattt ggccccaggg gacgtgggcc ctgcaggctg caggagggca 2460

ctggagctgg gaggtctcgt cccagccctc cccatctcgg ggctgctgtg tggacggcgc 2520

tgcctcaggc actctcctgt ctgaacctgc ccttactgtg tttaacctgt tgctccagga 2580

tgcattctga taggaggggg cggcagggct gggccttgtg acaatctgcc tttcaccaca 2640

tggccttgcc tcggtggccc tgactgtcag ggagggccag ggaggcagag cgggagggag 2700

tctcaggagg aggctgccct gaggggctgg ggagggggta cctcatgagg accagggtgg 2760

agctgagaag aggaggaggt gggggctgga ggtgctggta gctgagggga cgggcaagtg 2820

agaggggagg gagggaagtc ctgggaggat cctgagctgc tgttgcagtc taacccacta 2880

atcagttctt agattcaggg gaagggcagg caccaacaac tcagaatggg ggctttcggg 2940

gagggcgcct agtcccccca gctctaagca gccaggaggg acctgcatct aagcatctgg 3000

gttgccatgg caatggcatg ccccccagct actgtatgcc cccgaccccc gcagaggcag 3060

aatgaaccca tagggagctg atcgtaatgt ttatcatgtt acttccccac ccctacattt 3120

tttgaaataa aataaggaat tttattctc 3149

<210> 8

<211> 1816

<212> DNA

<213> Homo sapiens

<220>

<221> RAR-related orphan receptor A

<222> (1)..(1816)

<223> accession No.s: BC008831.1; Hs.388617; LocusID: 6095

<400> 8

ggcacgaggg aaaaaacatg gagtcagctc cggcagcccc cgaccccgcc gccagcgagc 60

caggcagcag cggcgcggac gcggccgccg gctccaggga gaccccgctg aaccaggaat 120

ccgcccgcaa gagcgagccg cctgccccgg tgcgcagaca gagctattcc agcaccagca 180

gaggtatctc agtaacgaag aagacacata catctcaaat tgaaattatt ccatgcaaga 240

tctgtggaga caaatcatca ggaatccatt atggtgtcat tacatgtgaa ggctgcaagg 300

gctttttcag gagaagtcag caaagcaatg ccacctactc ctgtcctcgt cagaagaact 360

gtttgattga tcgaaccagt agaaaccgct gccaacactg tcgattacag aaatgccttg 420

ccgtagggat gtctcgagat gctgtaaaat ttggccgaat gtcaaaaaag cagagagaca 480

gcttgtatgc agaagtacag aaacaccgga tgcagcagca gcagcgcgac caccagcagc 540

agcctggaga ggctgagccg ctgacgccca cctacaacat ctcggccaac gggctgacgg 600

aacttcacga cgacctcagt aactacattg acgggcacac ccctgagggg agtaaggcag 660

actccgccgt cagcagcttc tacctggaca tacagccttc cccagaccag tcaggtcttg 720

atatcaatgg aatcaaacca gaaccaatat gtgactacac accagcatca ggcttctttc 780

cctactgttc gttcaccaac ggcgagactt ccccaactgt gtccatggca gaattagaac 840

accttgcaca gaatatatct aaatcgcatc tggaaacctg ccaatacttg agagaagagc 900

tccagcagat aacgtggcag acctttttac aggaagaaat tgagaactat caaaacaagc 960

agcgggaggt gatgtggcaa ttgtgtgcca tcaaaattac agaagctata cagtatgtgg 1020

tggagtttgc caaacgcatt gatggattta tggaactgtg tcaaaatgat caaattgtgc 1080

ttctaaaagc aggttctcta gaggtggtgt ttatcagaat gtgccgtgcc tttgactctc 1140

agaacaacac cgtgtacttt gatgggaagt atgccagccc cgacgtcttc aaatccttag 1200

gttgtgaaga ctttattagc tttgtgtttg aatttggaaa gagtttatgt tctatgcacc 1260

tgactgaaga tgaaattgca ttattttctg catttgtact gatgtcagca gatcgctcat 1320

ggctgcaaga aaaggtaaaa attgaaaaac tgcaacagaa aattcagcta gctcttcaac 1380

acgtcctaca gaagaatcac cgagaagatg gaatactaac aaagttaata tgcaaggtgt 1440

ctacattaag agccttatgt ggacgacata cagaaaagct aatggcattt aaagcaatat 1500

acccagacat tgtgcgactt cattttcctc cattatacaa ggagttgttc acttcagaat 1560

ttgagccagc aatgcaaatt gatgggtaaa tgttatcacc taagcacttc tagaatgtct 1620

gaagtacaaa catgaaaaac aaacaaaaaa attaaccgag acactttata tggccctgca 1680

cagacctgga gcgccacaca ctgcacatct tttggtgatc ggggtcaggc aaaggagggg 1740

aaacaatgaa aacaaataaa agttgaactt gtttttctca tgaaaaaaaa aaaaaaaaaa 1800

aaaaaaaaaa aaaaaa                                                    1816

<210>  9

<211>  52

<212>  PRT

<213>  Homo sapiens

<220>

<221>  phospholamban (PLN)

<222>  (1)..(52)

<223>  accession No. NM_002667.2

<400>  9

Met Glu Lys Val Gln Tyr Leu Thr Arg Ser Ala Ile Arg Arg Ala Ser
1               5                   10                  15

Thr Ile Glu Met Pro Gln Gln Ala Arg Gln Lys Leu Gln Asn Leu Phe
            20                  25                  30

Ile Asn Phe Cys Leu Ile Leu Ile Cys Leu Leu Leu Ile Cys Ile Ile
        35                  40                  45

Val Met Leu Leu
    50

<210>  10

<211>  547

<212>  PRT

<213>  Homo sapiens

<220>

<221>  sterol carrier protein 2 (SCP2)

<222>  (1)..(547)

<223>  accession No. NM_002979

<400>  10

Met Ser Ser Ser Pro Trp Glu Pro Ala Thr Leu Arg Arg Val Phe Val
1               5                   10                  15

Val Gly Val Gly Met Thr Lys Phe Val Lys Pro Gly Ala Glu Asn Ser
            20                  25                  30

Arg Asp Tyr Pro Asp Leu Ala Glu Glu Ala Gly Lys Lys Ala Leu Ala
        35                  40                  45

Asp Ala Gln Ile Pro Tyr Ser Ala Val Asp Gln Ala Cys Val Gly Tyr
    50                  55                  60

Val Phe Gly Asp Ser Thr Cys Gly Gln Arg Ala Ile Tyr His Ser Leu
65                  70                  75                  80

Gly Met Thr Gly Ile Pro Ile Ile Asn Val Asn Asn Asn Cys Ala Thr
                85                  90                  95

Gly Ser Thr Ala Leu Phe Met Ala Arg Gln Leu Ile Gln Gly Gly Val

            100                 105                 110

Ala Glu Cys Val Leu Ala Leu Gly Phe Glu Lys Met Ser Lys Gly Ser

        115                 120                 125

Leu Gly Ile Lys Phe Ser Asp Arg Thr Ile Pro Thr Asp Lys His Val

    130                 135                 140

Asp Leu Leu Ile Asn Lys Tyr Gly Leu Ser Ala His Pro Val Ala Pro

145                 150                 155                 160

Gln Met Phe Gly Tyr Ala Gly Lys Glu His Met Glu Lys Tyr Gly Thr

                165                 170                 175

Lys Ile Glu His Phe Ala Lys Ile Gly Trp Lys Asn His Lys His Ser

            180                 185                 190

Val Asn Asn Pro Tyr Ser Gln Phe Gln Asp Glu Tyr Ser Leu Asp Glu

        195                 200                 205

Val Met Ala Ser Lys Glu Val Phe Asp Phe Leu Thr Ile Leu Gln Cys

    210                 215                 220

Cys Pro Thr Ser Asp Gly Ala Ala Ala Ala Ile Leu Ala Ser Glu Ala

225                 230                 235                 240

Phe Val Gln Lys Tyr Gly Leu Gln Ser Lys Ala Val Glu Ile Leu Ala

                245                 250                 255

Gln Glu Met Met Thr Asp Leu Pro Ser Ser Phe Glu Glu Lys Ser Ile

            260                 265                 270

Ile Lys Met Val Gly Phe Asp Met Ser Lys Glu Ala Ala Arg Lys Cys

        275                 280                 285

Tyr Glu Lys Ser Gly Leu Thr Pro Asn Asp Ile Asp Val Ile Glu Leu

290                 295                 300

His Asp Cys Phe Ser Thr Asn Glu Leu Leu Thr Tyr Glu Ala Leu Gly

305                 310                 315                 320

Leu Cys Pro Glu Gly Gln Gly Ala Thr Leu Val Asp Arg Gly Asp Asn

                325                 330                 335

Thr Tyr Gly Gly Lys Trp Val Ile Asn Pro Ser Gly Gly Leu Ile Ser

            340                 345                 350

Lys Gly His Pro Leu Gly Ala Thr Gly Leu Ala Gln Cys Ala Glu Leu

        355                 360                 365

Cys Trp Gln Leu Arg Gly Glu Ala Gly Lys Arg Gln Val Pro Gly Ala

    370                 375                 380

Lys Val Ala Leu Gln His Asn Leu Gly Ile Gly Gly Ala Val Val Val

385                 390                 395                 400

Thr Leu Tyr Lys Met Gly Phe Pro Glu Ala Ala Ser Ser Phe Arg Thr

                405                 410                 415

His Gln Ile Glu Ala Val Pro Thr Ser Ser Ala Ser Asp Gly Phe Lys

            420                 425                 430

Ala Asn Leu Val Phe Lys Glu Ile Glu Lys Lys Leu Glu Glu Glu Gly

        435                 440                 445

Glu Gln Phe Val Lys Lys Ile Gly Gly Ile Phe Ala Phe Lys Val Lys

    450                 455                 460

Asp Gly Pro Gly Gly Lys Glu Ala Thr Trp Val Val Asp Val Lys Asn

465                 470                 475                 480

Gly Lys Gly Ser Val Leu Pro Asn Ser Asp Lys Lys Ala Asp Cys Thr

                485                 490                 495

Ile Thr Met Ala Asp Ser Asp Phe Leu Ala Leu Met Thr Gly Lys Met

            500                 505                 510

Asn Pro Gln Ser Ala Phe Phe Gln Gly Lys Leu Lys Ile Thr Gly Asn

        515                 520                 525

Met Gly Leu Ala Met Lys Leu Gln Asn Leu Gln Leu Gln Pro Gly Asn

    530                 535                 540

Ala Lys Leu

545

<210> 11

<211> 388

<212> PRT

<213> Homo sapiens

<220>

<221> UDP-Gal:betaGlcNAc beta 1,4- galactosyltransferase polypeptide 5

(B4GALT5),

<222> (1)..(388)

<223> accession No. NM_004776.2

<400> 11

Met Arg Ala Arg Arg Gly Leu Leu Arg Leu Pro Arg Arg Ser Leu Leu

1               5                   10                  15

Ala Ala Leu Phe Phe Phe Ser Leu Ser Ser Ser Leu Leu Tyr Phe Val

            20                  25                  30

Tyr Val Ala Pro Gly Ile Val Asn Thr Tyr Leu Phe Met Met Gln Ala

        35                  40                  45

Gln Gly Ile Leu Ile Arg Asp Asn Val Arg Thr Ile Gly Ala Gln Val

    50                  55                  60

Tyr Glu Gln Val Leu Arg Ser Ala Tyr Ala Lys Arg Asn Ser Ser Val

65                  70                  75                  80

Asn Asp Ser Asp Tyr Pro Leu Asp Leu Asn His Ser Glu Thr Phe Leu

                85                  90                  95

Gln Thr Thr Thr Phe Leu Pro Glu Asp Phe Thr Tyr Phe Ala Asn His

            100                 105                 110

Thr Cys Pro Glu Arg Leu Pro Ser Met Lys Gly Pro Ile Asp Ile Asn

        115                 120                 125

Met Ser Glu Ile Gly Met Asp Tyr Ile His Glu Leu Phe Ser Lys Asp

    130                 135                 140

Pro Thr Ile Lys Leu Gly Gly His Trp Lys Pro Ser Asp Cys Met Pro

145                 150                 155                 160

Arg Trp Lys Val Ala Ile Leu Ile Pro Phe Arg Asn Arg His Glu His

                165                 170                 175

Leu Pro Val Leu Phe Arg His Leu Leu Pro Met Leu Gln Arg Gln Arg

            180                 185                 190

Leu Gln Phe Ala Phe Tyr Val Val Glu Gln Val Gly Thr Gln Pro Phe

        195                 200                 205

Asn Arg Ala Met Leu Phe Asn Val Gly Phe Gln Glu Ala Met Lys Asp

    210                 215                 220

Leu Asp Trp Asp Cys Leu Ile Phe His Asp Val Asp His Ile Pro Glu

225                 230                 235                 240

Ser Asp Arg Asn Tyr Tyr Gly Cys Gly Gln Met Pro Arg His Phe Ala

                245                 250                 255

Thr Lys Leu Asp Lys Tyr Met Tyr Leu Leu Pro Tyr Thr Glu Phe Phe

            260                 265                 270

Gly Gly Val Ser Gly Leu Thr Val Glu Gln Phe Arg Lys Ile Asn Gly

        275                 280                 285

Phe Pro Asn Ala Phe Trp Gly Trp Gly Gly Glu Asp Asp Asp Leu Trp

    290                 295                 300

Asn Arg Val Gln Asn Ala Gly Tyr Ser Val Ser Arg Pro Glu Gly Asp

305                 310                 315                 320

Thr Gly Lys Tyr Lys Ser Ile Pro His His His Arg Gly Glu Val Gln

                325                 330                 335

Phe Leu Gly Arg Tyr Ala Leu Leu Arg Lys Ser Lys Glu Arg Gln Gly

            340                 345                 350

Leu Asp Gly Leu Asn Asn Leu Asn Tyr Phe Ala Asn Ile Thr Tyr Asp

        355                 360                 365

Ala Leu Tyr Lys Asn Ile Thr Val Asn Leu Thr Pro Glu Leu Ala Gln

    370                 375                 380

Val Asn Glu Tyr

385

<210>  12

<211>  475

<212>  PRT

<213>  Homo sapiens

<220>

<221>  lipoprotein lipase (LPL)

<222>  (1)..(475)

<223>  accession No. NM_000237.1

<400>  12

<210>  13

<211>  4599

<212>  PRT

<213>  Homo sapiens

<220>

<221>  low density lipoprotein-related protein 1B

<222>  (1)..(4599)

<223>  accession No. NM_018557

<220>

<221>  misc_feature

<222>  (4343)..(4343)

<223>  Xaa can be any naturally occurring amino acid

<400>  13

Met Ser Glu Phe Leu Leu Ala Leu Leu Thr Leu Ser Gly Leu Leu Pro
1               5                   10                  15

Ile Ala Arg Val Leu Thr Val Gly Ala Asp Arg Asp Gln Gln Leu Cys
            20                  25                  30

Asp Pro Gly Glu Phe Leu Cys His Asp His Val Thr Cys Val Ser Gln
        35                  40                  45
Ser Trp Leu Cys Asp Gly Asp Pro Asp Cys Pro Asp Asp Ser Asp Glu
    50                  55                  60
Ser Leu Asp Thr Cys Pro Glu Glu Val Glu Ile Lys Cys Pro Leu Asn
65                  70                  75                  80
His Ile Ala Cys Leu Gly Thr Asn Lys Cys Val His Leu Ser Gln Leu
                85                  90                  95
Cys Asn Gly Val Leu Asp Cys Pro Asp Gly Tyr Asp Glu Gly Val His
            100                 105                 110
Cys Gln Glu Leu Leu Ser Asn Cys Gln Gln Leu Asn Cys Gln Tyr Lys
        115                 120                 125
Cys Thr Met Val Arg Asn Ser Thr Arg Cys Tyr Cys Glu Asp Gly Phe
    130                 135                 140
Glu Ile Thr Glu Asp Gly Arg Ser Cys Lys Asp Gln Asp Glu Cys Ala
145                 150                 155                 160
Val Tyr Gly Thr Cys Ser Gln Thr Cys Arg Asn Thr His Gly Ser Tyr
                165                 170                 175
Thr Cys Ser Cys Val Glu Gly Tyr Leu Met Gln Pro Asp Asn Arg Ser
            180                 185                 190
Cys Lys Ala Lys Ile Glu Pro Thr Asp Arg Pro Pro Ile Leu Leu Ile
        195                 200                 205
Ala Asn Phe Glu Thr Ile Glu Val Phe Tyr Leu Asn Gly Ser Lys Met
    210                 215                 220

Ala Thr Leu Ser Ser Val Asn Gly Asn Glu Ile His Thr Leu Asp Phe
225                 230                 235                 240
Ile Tyr Asn Glu Asp Met Ile Cys Trp Ile Glu Ser Arg Glu Ser Ser
                245                 250                 255
Asn Gln Leu Lys Cys Ile Gln Ile Thr Lys Ala Gly Gly Leu Thr Asp
            260                 265                 270
Glu Trp Thr Ile Asn Ile Leu Gln Ser Phe His Asn Val Gln Gln Met
        275                 280                 285
Ala Ile Asp Trp Leu Thr Arg Asn Leu Tyr Phe Val Asp His Val Gly
    290                 295                 300
Asp Arg Ile Phe Val Cys Asn Ser Asn Gly Ser Val Cys Val Thr Leu
305                 310                 315                 320
Ile Asp Leu Glu Leu His Asn Pro Lys Ala Ile Ala Val Asp Pro Ile
                325                 330                 335
Ala Gly Lys Leu Phe Phe Thr Asp Tyr Gly Asn Val Ala Lys Val Glu
            340                 345                 350
Arg Cys Asp Met Asp Gly Met Asn Arg Thr Arg Ile Ile Asp Ser Lys
        355                 360                 365
Thr Glu Gln Pro Ala Ala Leu Ala Leu Asp Leu Val Asn Lys Leu Val
    370                 375                 380
Tyr Trp Val Asp Leu Tyr Leu Asp Tyr Val Gly Val Val Asp Tyr Gln
385                 390                 395                 400
Gly Lys Asn Arg His Thr Val Ile Gln Gly Arg Gln Val Arg His Leu
                405                 410                 415

Tyr Gly Ile Thr Val Phe Glu Asp Tyr Leu Tyr Ala Thr Asn Ser Asp
            420                 425                 430
Asn Tyr Asn Ile Val Arg Ile Asn Arg Phe Asn Gly Thr Asp Ile His
        435                 440                 445
Ser Leu Ile Lys Ile Glu Asn Ala Trp Gly Ile Arg Ile Tyr Gln Lys
    450                 455                 460
Arg Thr Gln Pro Thr Val Arg Ser His Ala Cys Glu Val Asp Pro Tyr
465                 470                 475                 480
Gly Met Pro Gly Gly Cys Ser His Ile Cys Leu Leu Ser Ser Ser Tyr
                485                 490                 495
Lys Thr Arg Thr Cys Arg Cys Arg Thr Gly Phe Asn Leu Gly Ser Asp
            500                 505                 510
Gly Arg Ser Cys Lys Arg Pro Lys Asn Glu Leu Phe Leu Phe Tyr Gly
        515                 520                 525
Lys Gly Arg Pro Gly Ile Val Arg Gly Met Asp Leu Asn Thr Lys Ile
    530                 535                 540
Ala Asp Glu Tyr Met Ile Pro Ile Glu Asn Leu Val Asn Pro Arg Ala
545                 550                 555                 560
Leu Asp Phe His Ala Glu Thr Asn Tyr Ile Tyr Phe Ala Asp Thr Thr
                565                 570                 575
Ser Phe Leu Ile Gly Arg Gln Lys Ile Asp Gly Thr Glu Arg Glu Thr
            580                 585                 590
Ile Leu Lys Asp Asp Leu Asp Asn Val Glu Gly Ile Ala Val Asp Trp
        595                 600                 605

Ile Gly Asn Asn Leu Tyr Trp Thr Asn Asp Gly His Arg Lys Thr Ile

    610                 615                 620

Asn Val Ala Arg Leu Glu Lys Ala Ser Gln Ser Arg Lys Thr Leu Leu

625                 630                 635                 640

Glu Gly Glu Met Ser His Pro Arg Gly Ile Val Val Asp Pro Val Asn

                645                 650                 655

Gly Trp Met Tyr Trp Thr Asp Trp Glu Glu Asp Glu Ile Asp Asp Ser

            660                 665                 670

Val Gly Arg Ile Glu Lys Ala Trp Met Asp Gly Phe Asn Arg Gln Ile

        675                 680                 685

Phe Val Thr Ser Lys Met Leu Trp Pro Asn Gly Leu Thr Leu Asp Phe

    690                 695                 700

His Thr Asn Thr Leu Tyr Trp Cys Asp Ala Tyr Tyr Asp His Ile Glu

705                 710                 715                 720

Lys Val Phe Leu Asn Gly Thr His Arg Lys Ile Val Tyr Ser Gly Arg

                725                 730                 735

Glu Leu Asn His Pro Phe Gly Leu Ser His His Gly Asn Tyr Val Phe

            740                 745                 750

Trp Thr Asp Tyr Met Asn Gly Ser Ile Phe Gln Leu Asp Leu Ile Thr

        755                 760                 765

Ser Glu Val Thr Leu Leu Arg His Glu Arg Pro Pro Leu Phe Gly Leu

    770                 775                 780

Gln Ile Tyr Asp Pro Arg Lys Gln Gln Gly Asp Asn Met Cys Arg Val

785                 790                 795                 800

Asn Asn Gly Gly Cys Ser Thr Leu Cys Leu Ala Ile Pro Gly Gly Arg

                805                 810                 815

Val Cys Ala Cys Ala Asp Asn Gln Leu Leu Asp Glu Asn Gly Thr Thr

            820                 825                 830

Cys Thr Phe Asn Pro Gly Glu Ala Leu Pro His Ile Cys Lys Ala Gly

        835                 840                 845

Glu Phe Arg Cys Lys Asn Arg His Cys Ile Gln Ala Arg Trp Lys Cys

    850                 855                 860

Asp Gly Asp Asp Asp Cys Leu Asp Gly Ser Asp Glu Asp Ser Val Asn

865                 870                 875                 880

Cys Phe Asn His Ser Cys Pro Asp Asp Gln Phe Lys Cys Gln Asn Asn

                885                 890                 895

Arg Cys Ile Pro Lys Arg Trp Leu Cys Asp Gly Ala Asn Asp Cys Gly

            900                 905                 910

Ser Asn Glu Asp Glu Ser Asn Gln Thr Cys Thr Ala Arg Thr Cys Gln

        915                 920                 925

Val Asp Gln Phe Ser Cys Gly Asn Gly Arg Cys Ile Pro Arg Ala Trp

    930                 935                 940

Leu Cys Asp Arg Glu Asp Asp Cys Gly Asp Gln Thr Asp Glu Met Ala

945                 950                 955                 960

Ser Cys Glu Phe Pro Thr Cys Glu Pro Leu Thr Gln Phe Val Cys Lys

                965                 970                 975

Ser Gly Arg Cys Ile Ser Ser Lys Trp His Cys Asp Ser Asp Asp Asp

            980                 985                 990

Cys Gly Asp Gly Ser Asp Glu Val Gly Cys Val His Ser Cys Phe Asp

        995                 1000                1005

Asn Gln Phe Arg Cys Ser Ser Gly Arg Cys Ile Pro Gly His Trp

    1010                1015                1020

Ala Cys Asp Gly Asp Asn Asp Cys Gly Asp Phe Ser Asp Glu Ala

    1025                1030                1035

Gln Ile Asn Cys Thr Lys Glu Glu Ile His Ser Pro Ala Gly Cys

    1040                1045                1050

Asn Gly Asn Glu Phe Gln Cys His Pro Asp Gly Asn Cys Val Pro

    1055                1060                1065

Asp Leu Trp Arg Cys Asp Gly Glu Lys Asp Cys Glu Asp Gly Ser

    1070                1075                1080

Asp Glu Lys Gly Cys Asn Gly Thr Ile Arg Leu Cys Asp His Lys

    1085                1090                1095

Thr Lys Phe Ser Cys Trp Ser Thr Gly Arg Cys Ile Asn Lys Ala

    1100                1105                1110

Trp Val Cys Asp Gly Asp Ile Asp Cys Glu Asp Gln Ser Asp Glu

    1115                1120                1125

Asp Asp Cys Asp Ser Phe Leu Cys Gly Pro Pro Lys His Pro Cys

    1130                1135                1140

Ala Asn Asp Thr Ser Val Cys Leu Gln Pro Glu Lys Leu Cys Asn

    1145                1150                1155

Gly Lys Lys Asp Cys Pro Asp Gly Ser Asp Glu Gly Tyr Leu Cys

    1160                1165                1170

Asp Glu Cys Ser Leu Asn Asn Gly Gly Cys Ser Asn His Cys Ser
    1175                1180                1185
Val Val Pro Gly Arg Gly Ile Val Cys Ser Cys Pro Glu Gly Leu
    1190                1195                1200
Gln Leu Asn Lys Asp Asn Lys Thr Cys Glu Ile Val Asp Tyr Cys
    1205                1210                1215
Ser Asn His Leu Lys Cys Ser Gln Val Cys Glu Gln His Lys His
    1220                1225                1230
Thr Val Lys Cys Ser Cys Tyr Glu Gly Trp Lys Leu Asp Val Asp
    1235                1240                1245
Gly Glu Ser Cys Thr Ser Val Asp Pro Phe Glu Ala Phe Ile Ile
    1250                1255                1260
Phe Ser Ile Arg His Glu Ile Arg Arg Ile Asp Leu His Lys Arg
    1265                1270                1275
Asp Tyr Ser Leu Leu Val Pro Gly Leu Arg Asn Thr Ile Ala Leu
    1280                1285                1290
Asp Phe His Phe Asn Gln Ser Leu Leu Tyr Trp Thr Asp Val Val
    1295                1300                1305
Glu Asp Arg Ile Tyr Arg Gly Lys Leu Ser Glu Ser Gly Gly Val
    1310                1315                1320
Ser Ala Ile Glu Val Val Val Glu His Gly Leu Ala Thr Pro Glu
    1325                1330                1335
Gly Leu Thr Val Asp Trp Ile Ala Gly Asn Ile Tyr Trp Ile Asp
    1340                1345                1350

Ser Asn Leu Asp Gln Ile Glu Val Ala Lys Leu Asp Gly Ser Leu

    1355                1360                1365

Arg Thr Thr Leu Ile Ala Gly Ala Met Glu His Pro Arg Ala Ile

    1370                1375                1380

Ala Leu Asp Pro Arg Tyr Gly Ile Leu Phe Trp Thr Asp Trp Asp

    1385                1390                1395

Ala Asn Phe Pro Arg Ile Glu Ser Ala Ser Met Ser Gly Ala Gly

    1400                1405                1410

Arg Lys Thr Ile Tyr Lys Asp Met Lys Thr Gly Ala Trp Pro Asn

    1415                1420                1425

Gly Leu Thr Val Asp His Phe Glu Lys Arg Ile Val Trp Thr Asp

    1430                1435                1440

Ala Arg Ser Asp Ala Ile Tyr Ser Ala Leu Tyr Asp Gly Thr Asn

    1445                1450                1455

Met Ile Glu Ile Ile Arg Gly His Glu Tyr Leu Ser His Pro Phe

    1460                1465                1470

Ala Val Ser Leu Tyr Gly Ser Glu Val Tyr Trp Thr Asp Trp Arg

    1475                1480                1485

Thr Asn Thr Leu Ser Lys Ala Asn Lys Trp Thr Gly Gln Asn Val

    1490                1495                1500

Ser Val Ile Gln Lys Thr Ser Ala Gln Pro Phe Asp Leu Gln Ile

    1505                1510                1515

Tyr His Pro Ser Arg Gln Pro Gln Ala Pro Asn Pro Cys Ala Ala

    1520                1525                1530

Asn Asp Gly Lys Gly Pro Cys Ser His Met Cys Leu Ile Asn His
    1535                1540                1545
Asn Arg Ser Ala Ala Cys Ala Cys Pro His Leu Met Lys Leu Ser
    1550                1555                1560
Ser Asp Lys Lys Thr Cys Tyr Glu Met Lys Lys Phe Leu Leu Tyr
    1565                1570                1575
Ala Arg Arg Ser Glu Ile Arg Gly Val Asp Ile Asp Asn Pro Tyr
    1580                1585                1590
Phe Asn Phe Ile Thr Ala Phe Thr Val Pro Asp Ile Asp Asp Val
    1595                1600                1605
Thr Val Ile Asp Phe Asp Ala Ser Glu Glu Arg Leu Tyr Trp Thr
    1610                1615                1620
Asp Ile Lys Thr Gln Thr Ile Lys Arg Ala Phe Ile Asn Gly Thr
    1625                1630                1635
Gly Leu Glu Thr Val Ile Ser Arg Asp Ile Gln Ser Ile Arg Gly
    1640                1645                1650
Leu Ala Val Asp Trp Val Ser Arg Asn Leu Tyr Trp Ile Ser Ser
    1655                1660                1665
Glu Phe Asp Glu Thr Gln Ile Asn Val Ala Arg Leu Asp Gly Ser
    1670                1675                1680
Leu Lys Thr Ser Ile Ile His Gly Ile Asp Lys Pro Gln Cys Leu
    1685                1690                1695
Ala Ala His Pro Val Arg Gly Lys Leu Tyr Trp Thr Asp Gly Asn
    1700                1705                1710

Thr Ile Asn Met Ala Asn Met Asp Gly Ser Asn Ser Lys Ile Leu
    1715                1720                1725
Phe Gln Asn Gln Lys Glu Pro Val Gly Leu Ser Ile Asp Tyr Val
    1730                1735                1740
Glu Asn Lys Leu Tyr Trp Ile Ser Ser Gly Asn Gly Thr Ile Asn
    1745                1750                1755
Arg Cys Asn Leu Asp Gly Gly Asn Leu Glu Val Ile Glu Ser Met
    1760                1765                1770
Lys Glu Glu Leu Thr Lys Ala Thr Ala Leu Thr Ile Met Asp Lys
    1775                1780                1785
Lys Leu Trp Trp Ala Asp Gln Asn Leu Ala Gln Leu Gly Thr Cys
    1790                1795                1800
Ser Lys Arg Asp Gly Arg Asn Pro Thr Ile Leu Arg Asn Lys Thr
    1805                1810                1815
Ser Gly Val Val His Met Lys Val Tyr Asp Lys Glu Ala Gln Gln
    1820                1825                1830
Gly Ser Asn Ser Cys Gln Leu Asn Asn Gly Gly Cys Ser Gln Leu
    1835                1840                1845
Cys Leu Pro Thr Ser Glu Thr Thr Arg Thr Cys Met Cys Thr Val
    1850                1855                1860
Gly Tyr Tyr Leu Gln Lys Asn Arg Met Ser Cys Gln Gly Ile Glu
    1865                1870                1875
Ser Phe Leu Met Tyr Ser Val His Glu Gly Ile Arg Gly Ile Pro
    1880                1885                1890

Leu Glu Pro Ser Asp Lys Met Asp Ala Leu Met Pro Ile Ser Gly

    1895                1900                1905

Thr Ser Phe Ala Val Gly Ile Asp Phe His Ala Glu Asn Asp Thr

    1910                1915                1920

Ile Tyr Trp Thr Asp Met Gly Phe Asn Lys Ile Ser Arg Ala Lys

    1925                1930                1935

Arg Asp Gln Thr Trp Lys Glu Asp Ile Ile Thr Asn Gly Leu Gly

    1940                1945                1950

Arg Val Glu Gly Ile Ala Val Asp Trp Ile Ala Gly Asn Ile Tyr

    1955                1960                1965

Trp Thr Asp His Gly Phe Asn Leu Ile Glu Val Ala Arg Leu Asn

    1970                1975                1980

Gly Ser Phe Arg Tyr Val Ile Ile Ser Gln Gly Leu Asp Gln Pro

    1985                1990                1995

Arg Ser Ile Ala Val His Pro Glu Lys Gly Leu Leu Phe Trp Thr

    2000                2005                2010

Glu Trp Gly Gln Met Pro Cys Ile Gly Lys Ala Arg Leu Asp Gly

    2015                2020                2025

Ser Glu Lys Val Val Leu Val Ser Met Gly Ile Ala Trp Pro Asn

    2030                2035                2040

Gly Ile Ser Ile Asp Tyr Glu Glu Asn Lys Leu Tyr Trp Cys Asp

    2045                2050                2055

Ala Arg Thr Asp Lys Ile Glu Arg Ile Asp Leu Glu Thr Gly Gly

    2060                2065                2070

Asn Arg Glu Met Val Leu Ser Gly Ser Asn Val Asp Met Phe Ser

    2075                2080                2085

Val Ala Val Phe Gly Ala Tyr Ile Tyr Trp Ser Asp Arg Ala His

    2090                2095                2100

Ala Asn Gly Ser Val Arg Arg Gly His Lys Asn Asp Ala Thr Glu

    2105                2110                2115

Thr Ile Thr Met Arg Thr Gly Leu Gly Val Asn Leu Lys Glu Val

    2120                2125                2130

Lys Ile Phe Asn Arg Val Arg Glu Lys Gly Thr Asn Val Cys Ala

    2135                2140                2145

Arg Asp Asn Gly Gly Cys Lys Gln Leu Cys Leu Tyr Arg Gly Asn

    2150                2155                2160

Ser Arg Arg Thr Cys Ala Cys Ala His Gly Tyr Leu Ala Glu Asp

    2165                2170                2175

Gly Val Thr Cys Leu Arg His Glu Gly Tyr Leu Leu Tyr Ser Gly

    2180                2185                2190

Arg Thr Ile Leu Lys Ser Ile His Leu Ser Asp Glu Thr Asn Leu

    2195                2200                2205

Asn Ser Pro Ile Arg Pro Tyr Glu Asn Pro Arg Tyr Phe Lys Asn

    2210                2215                2220

Val Ile Ala Leu Ala Phe Asp Tyr Asn Gln Arg Arg Lys Gly Thr

    2225                2230                2235

Asn Arg Ile Phe Tyr Ser Asp Ala His Phe Gly Asn Ile Gln Leu

    2240                2245                2250

Ile Lys Asp Asn Trp Glu Asp Arg Gln Val Ile Val Glu Asn Val
    2255                2260                2265
Gly Ser Val Glu Gly Leu Ala Tyr His Arg Ala Trp Asp Thr Leu
    2270                2275                2280
Tyr Trp Thr Ser Ser Thr Thr Ser Ser Ile Thr Arg His Thr Val
    2285                2290                2295
Asp Gln Thr Arg Pro Gly Ala Phe Asp Arg Glu Ala Val Ile Thr
    2300                2305                2310
Met Ser Glu Asp Asp His Pro His Val Leu Ala Leu Asp Glu Cys
    2315                2320                2325
Gln Asn Leu Met Phe Trp Thr Asn Trp Asn Glu Gln His Pro Ser
    2330                2335                2340
Ile Met Arg Ser Thr Leu Thr Gly Lys Asn Ala Gln Val Val Val
    2345                2350                2355
Ser Thr Asp Ile Leu Thr Pro Asn Gly Leu Thr Ile Asp Tyr Arg
    2360                2365                2370
Ala Glu Lys Leu Tyr Phe Ser Asp Gly Ser Leu Gly Lys Ile Glu
    2375                2380                2385
Arg Cys Glu Tyr Asp Gly Ser Gln Arg His Val Ile Val Lys Ser
    2390                2395                2400
Gly Pro Gly Thr Phe Leu Ser Leu Ala Val Tyr Asp Asn Tyr Ile
    2405                2410                2415
Phe Trp Ser Asp Trp Gly Arg Arg Ala Ile Leu Arg Ser Asn Lys
    2420                2425                2430

Tyr Thr Gly Gly Asp Thr Lys Ile Leu Arg Ser Asp Ile Pro His

    2435                2440                2445

Gln Pro Met Gly Ile Ile Ala Val Ala Asn Asp Thr Asn Ser Cys

    2450                2455                2460

Glu Leu Ser Pro Cys Ala Leu Leu Asn Gly Gly Cys His Asp Leu

    2465                2470                2475

Cys Leu Leu Thr Pro Asn Gly Arg Val Asn Cys Ser Cys Arg Gly

    2480                2485                2490

Asp Arg Ile Leu Leu Glu Asp Asn Arg Cys Val Thr Lys Asn Ser

    2495                2500                2505

Ser Cys Asn Ala Tyr Ser Glu Phe Glu Cys Gly Asn Gly Glu Cys

    2510                2515                2520

Ile Asp Tyr Gln Leu Thr Cys Asp Gly Ile Pro His Cys Lys Asp

    2525                2530                2535

Lys Ser Asp Glu Lys Leu Leu Tyr Cys Glu Asn Arg Ser Cys Arg

    2540                2545                2550

Arg Gly Phe Lys Pro Cys Tyr Asn Arg Arg Cys Ile Pro His Gly

    2555                2560                2565

Lys Leu Cys Asp Gly Glu Asn Asp Cys Gly Asp Asn Ser Asp Glu

    2570                2575                2580

Leu Asp Cys Lys Val Ser Thr Cys Ala Thr Val Glu Phe Arg Cys

    2585                2590                2595

Ala Asp Gly Thr Cys Ile Pro Arg Ser Ala Arg Cys Asn Gln Asn

    2600                2605                2610

Ile Asp Cys Ala Asp Ala Ser Asp Glu Lys Asn Cys Asn Asn Thr

    2615                2620                2625

Asp Cys Thr His Phe Tyr Lys Leu Gly Val Lys Thr Thr Gly Phe

    2630                2635                2640

Ile Arg Cys Asn Ser Thr Ser Leu Cys Val Leu Pro Thr Trp Ile

    2645                2650                2655

Cys Asp Gly Ser Asn Asp Cys Gly Asp Tyr Ser Asp Glu Leu Lys

    2660                2665                2670

Cys Pro Val Gln Asn Lys His Lys Cys Glu Glu Asn Tyr Phe Ser

    2675                2680                2685

Cys Pro Ser Gly Arg Cys Ile Leu Asn Thr Trp Ile Cys Asp Gly

    2690                2695                2700

Gln Lys Asp Cys Glu Asp Gly Arg Asp Glu Phe His Cys Asp Ser

    2705                2710                2715

Ser Cys Ser Trp Asn Gln Phe Ala Cys Ser Ala Gln Lys Cys Ile

    2720                2725                2730

Ser Lys His Trp Ile Cys Asp Gly Glu Asp Asp Cys Gly Asp Gly

    2735                2740                2745

Leu Asp Glu Ser Asp Ser Ile Cys Gly Ala Ile Thr Cys Ala Ala

    2750                2755                2760

Asp Met Phe Ser Cys Gln Gly Ser Arg Ala Cys Val Pro Arg His

    2765                2770                2775

Trp Leu Cys Asp Gly Glu Arg Asp Cys Pro Asp Gly Ser Asp Glu

    2780                2785                2790

Leu Ser Thr Ala Gly Cys Ala Pro Asn Asn Thr Cys Asp Glu Asn
    2795                2800                2805
Ala Phe Met Cys His Asn Lys Val Cys Ile Pro Lys Gln Phe Val
    2810                2815                2820
Cys Asp His Asp Asp Asp Cys Gly Asp Gly Ser Asp Glu Ser Pro
    2825                2830                2835
Gln Cys Gly Tyr Arg Gln Cys Gly Thr Glu Glu Phe Ser Cys Ala
    2840                2845                2850
Asp Gly Arg Cys Leu Leu Asn Thr Gln Trp Gln Cys Asp Gly Asp
    2855                2860                2865
Phe Asp Cys Pro Asp His Ser Asp Glu Ala Pro Leu Asn Pro Lys
    2870                2875                2880
Cys Lys Ser Ala Glu Gln Ser Cys Asn Ser Ser Phe Phe Met Cys
    2885                2890                2895
Lys Asn Gly Arg Cys Ile Pro Ser Gly Gly Leu Cys Asp Asn Lys
    2900                2905                2910
Asp Asp Cys Gly Asp Gly Ser Asp Glu Arg Asn Cys His Ile Asn
    2915                2920                2925
Glu Cys Leu Ser Lys Lys Val Ser Gly Cys Ser Gln Asp Cys Gln
    2930                2935                2940
Asp Leu Pro Val Ser Tyr Lys Cys Lys Cys Trp Pro Gly Phe Gln
    2945                2950                2955
Leu Lys Asp Asp Gly Lys Thr Cys Val Asp Ile Asp Glu Cys Ser
    2960                2965                2970

Ser Gly Phe Pro Cys Ser Gln Gln Cys Ile Asn Thr Tyr Gly Thr
    2975                2980                2985
Tyr Lys Cys Leu Cys Thr Asp Gly Tyr Glu Ile Gln Pro Asp Asn
    2990                2995                3000
Pro Asn Gly Cys Lys Ser Leu Ser Asp Glu Glu Pro Phe Leu Ile
    3005                3010                3015
Leu Ala Asp His His Glu Ile Arg Lys Ile Ser Thr Asp Gly Ser
    3020                3025                3030
Asn Tyr Thr Leu Leu Lys Gln Gly Leu Asn Asn Val Ile Ala Ile
    3035                3040                3045
Asp Phe Asp Tyr Arg Glu Glu Phe Ile Tyr Trp Ile Asp Ser Ser
    3050                3055                3060
Arg Pro Asn Gly Ser Arg Ile Asn Arg Met Cys Leu Asn Gly Ser
    3065                3070                3075
Asp Ile Lys Val Val His Asn Thr Ala Val Pro Asn Ala Leu Ala
    3080                3085                3090
Val Asp Trp Ile Gly Lys Asn Leu Tyr Trp Ser Asp Thr Glu Lys
    3095                3100                3105
Arg Ile Ile Glu Val Ser Lys Leu Asn Gly Leu Tyr Pro Thr Ile
    3110                3115                3120
Leu Val Ser Lys Arg Leu Lys Phe Pro Arg Asp Leu Ser Leu Asp
    3125                3130                3135
Pro Gln Ala Gly Tyr Leu Tyr Trp Ile Asp Cys Cys Glu Tyr Pro
    3140                3145                3150

His Ile Gly Arg Val Gly Met Asp Gly Thr Asn Gln Ser Val Val

    3155                3160                3165

Ile Glu Thr Lys Ile Ser Arg Pro Met Ala Leu Thr Ile Asp Tyr

    3170                3175                3180

Val Asn Arg Arg Leu Tyr Trp Ala Asp Glu Asn His Ile Glu Phe

    3185                3190                3195

Ser Asn Met Asp Gly Ser His Arg His Lys Val Pro Asn Gln Asp

    3200                3205                3210

Ile Pro Gly Val Ile Ala Leu Thr Leu Phe Glu Asp Tyr Ile Tyr

    3215                3220                3225

Trp Thr Asp Gly Lys Thr Lys Ser Leu Ser Arg Ala His Lys Thr

    3230                3235                3240

Ser Gly Ala Asp Arg Leu Ser Leu Ile Tyr Ser Trp His Ala Ile

    3245                3250                3255

Thr Asp Ile Gln Val Tyr His Ser Tyr Arg Gln Pro Asp Val Ser

    3260                3265                3270

Lys His Leu Cys Met Ile Asn Asn Gly Gly Cys Ser His Leu Cys

    3275                3280                3285

Leu Leu Ala Pro Gly Lys Thr His Thr Cys Ala Cys Pro Thr Asn

    3290                3295                3300

Phe Tyr Leu Ala Ala Asp Asn Arg Thr Cys Leu Ser Asn Cys Thr

    3305                3310                3315

Ala Ser Gln Phe Arg Cys Lys Thr Asp Lys Cys Ile Pro Phe Trp

    3320                3325                3330

Trp Lys Cys Asp Thr Val Asp Asp Cys Gly Asp Gly Ser Asp Glu

    3335                3340                3345

Pro Asp Asp Cys Pro Glu Phe Arg Cys Gln Pro Gly Arg Phe Gln

    3350                3355                3360

Cys Gly Thr Gly Leu Cys Ala Leu Pro Ala Phe Ile Cys Asp Gly

    3365                3370                3375

Glu Asn Asp Cys Gly Asp Asn Ser Asp Glu Leu Asn Cys Asp Thr

    3380                3385                3390

His Val Cys Leu Ser Gly Gln Phe Lys Cys Thr Lys Asn Gln Lys

    3395                3400                3405

Cys Ile Pro Val Asn Leu Arg Cys Asn Gly Gln Asp Asp Cys Gly

    3410                3415                3420

Asp Glu Glu Asp Glu Arg Asp Cys Pro Glu Asn Ser Cys Ser Pro

3425 3430 .3435

Asp Tyr Phe Gln Cys Lys Thr Thr Lys His Cys Ile Ser Lys Leu

3440 3445 3450

Trp Val Cys Asp Glu Asp Pro Asp Cys Ala Asp Ala Ser Asp Glu

3455 3460 3465

Ala Asn Cys Asp Lys Lys Thr Cys Gly Pro His Glu Phe Gln Cys

3470 3475 3480

Lys Asn Asn Asn Cys Ile Pro Asp His Trp Arg Cys Asp Ser Gln

3485 3490 3495

Asn Asp Cys Ser Asp Asn Ser Asp Glu Glu Asn Cys Lys Pro Gln

3500 3505 3510

Thr Cys Thr Leu Lys Asp Phe Leu Cys Ala Asn Gly Asp Cys Val

3515 3520 3525

Ser Ser Arg Phe Trp Cys Asp Gly Asp Phe Asp Cys Ala Asp Gly

3530 3535 3540

Ser Asp Glu Arg Asn Cys Glu Thr Ser Cys Ser Lys Asp Gln Phe

3545 3550 3555

Arg Cys Ser Asn Gly Gln Cys Ile Pro Ala Lys Trp Lys Cys Asp

3560 3565 3570

Gly His Glu Asp Cys Lys Tyr Gly Glu Asp Glu Lys Ser Cys Glu

3575 3580 3585

Pro Ala Ser Pro Thr Cys Ser Ser Arg Glu Tyr Ile Cys Ala Ser

3590 3595 3600

Asp Gly Cys Ile Ser Ala Ser Leu Lys Cys Asn Gly Glu Tyr Asp

3605 3610 3615

Cys Ala Asp Gly Ser Asp Glu Met Asp Cys Val Thr Glu Cys Lys

3620 3625 3630

Glu Asp Gln Phe Arg Cys Lys Asn Lys Ala His Cys Ile Pro Ile

3635 3640 3645

Arg Trp Leu Cys Asp Gly Ile His Asp Cys Val Asp Gly Ser Asp

3650 3655 3660

Glu Glu Asn Cys Glu Arg Gly Gly Asn Ile Cys Arg Ala Asp Glu

3665 3670 3675

Phe Leu Cys Asn Asn Ser Leu Cys Lys Leu His Phe Trp Val Cys

3680 3685 3690

Asp Gly Glu Asp Asp Cys Gly Asp Asn Ser Asp Glu Ala Pro Asp

3695 3700 3705

Met Cys Val Lys Phe Leu Cys Pro Ser Thr Arg Pro His Arg Cys

3710 3715 3720

Arg Asn Asn Arg Ile Cys Leu Gln Ser Glu Gln Met Cys Asn Gly

3725 3730 3735

Ile Asp Glu Cys Gly Asp Asn Ser Asp Glu Asp His Cys Gly Gly

3740 3745 3750

Lys Leu Thr Tyr Lys Ala Arg Pro Cys Lys Lys Asp Glu Phe Ala

3755 3760 3765

Cys Ser Asn Lys Lys Cys Ile Pro Met Asp Leu Gln Cys Asp Arg

3770 3775 3780

Leu Asp Asp Cys Gly Asp Gly Ser Asp Glu Gln Gly Cys Arg Ile

3785 3790 3795

Ala Pro Thr Glu Tyr Thr Cys Glu Asp Asn Val Asn Pro Cys Gly

3800 3805 3810

Asp Asp Ala Tyr Cys Asn Gln Ile Lys Thr Ser Val Phe Cys Arg

3815 3820 3825

Cys Lys Pro Gly Phe Gln Arg Asn Met Lys Asn Arg Gln Cys Glu

3830 3835 3840

Asp Leu Asn Glu Cys Leu Val Phe Gly Thr Cys Ser His Gln Cys

3845 3850 3855

Ile Asn Val Glu Gly Ser Tyr Lys Cys Val Cys Asp Gln Asn Phe

3860 3865 3870

Gln Glu Arg Asn Asn Thr Cys Ile Ala Glu Gly Ser Glu Asp Gln
3875 3880 3885
Val Leu Tyr Ile Ala Asn Asp Thr Asp Ile Leu Gly Phe Ile Tyr
3890 3895 3900
Pro Phe Asn Tyr Ser Gly Asp His Gln Gln Ile Ser His Ile Glu
3905 3910 3915
His Asn Ser Arg Ile Thr Gly Met Asp Val Tyr Tyr Gln Arg Asp
3920 3925 3930
Met Ile Ile Trp Ser Thr Gln Phe Asn Pro Gly Gly Ile Phe Tyr
3935 3940 3945
Lys Arg Ile His Gly Arg Glu Lys Arg Gln Ala Asn Ser Gly Leu
3950 3955 3960
Ile Cys Pro Glu Phe Lys Arg Pro Arg Asp Ile Ala Val Asp Trp
3965 3970 3975
Val Ala Gly Asn Ile Tyr Trp Thr Asp His Ser Arg Met His Trp
3980 3985 3990
Phe Ser Tyr Tyr Thr Thr His Trp Thr Ser Leu Arg Tyr Ser Ile
3995 4000 4005
Asn Val Gly Gln Leu Asn Gly Pro Asn Cys Thr Arg Leu Leu Thr
4010 4015 4020
Asn Met Ala Gly Glu Pro Tyr Ala Ile Ala Val Asn Pro Lys Arg
4025 4030 4035
Gly Met Met Tyr Trp Thr Val Val Gly Asp His Ser His Ile Glu
4040 4045 4050

Glu Ala  Ala Met Asp Gly Thr  Leu Arg Arg Ile Leu  Val Gln Lys

     4055                 4060                 4065

Asn Leu  Gln Arg Pro Thr Gly  Leu Ala Val Asp Tyr  Phe Ser Glu

     4070                 4075                 4080

Arg Ile  Tyr Trp Ala Asp Phe  Glu Leu Ser Ile Ile  Gly Ser Val

     4085                 4090                 4095

Leu Tyr  Asp Gly Ser Asn Ser  Val Val Ser Val Ser  Ser Lys Gln

     4100                 4105                 4110

Gly Leu  Leu His Pro His Arg  Ile Asp Ile Phe Glu  Asp Tyr Ile

     4115                 4120                 4125

Tyr Gly  Ala Gly Pro Lys Asn  Gly Val Phe Arg Val  Gln Lys Phe

     4130                 4135                 4140

Gly His  Gly Ser Val Glu Tyr  Leu Ala Leu Asn Ile  Asp Lys Thr

     4145                 4150                 4155

Lys Gly  Val Leu Ile Ser His  Arg Tyr Lys Gln Leu  Asp Leu Pro

     4160                 4165                 4170

Asn Pro  Cys Leu Asp Leu Ala  Cys Glu Phe Leu Cys  Leu Leu Asn

     4175                 4180                 4185

Pro Ser  Gly Ala Thr Cys Val  Cys Pro Glu Gly Lys  Tyr Leu Ile

     4190                 4195                 4200

Asn Gly  Thr Cys Asn Asp Asp  Ser Leu Leu Asp Asp  Ser Cys Lys

     4205                 4210                 4215

Leu Thr  Cys Glu Asn Gly Gly  Arg Cys Ile Leu Asn  Glu Lys Gly

     4220                 4225                 4230

Asp Leu Arg Cys His Cys Trp Pro Ser Tyr Ser Gly Glu Arg Cys
4235 4240 4245
Glu Val Asn His Cys Ser Asn Tyr Cys Gln Asn Gly Gly Thr Cys
4250 4255 4260
Val Pro Ser Val Leu Gly Arg Pro Thr Cys Ser Cys Ala Leu Gly
4265 4270 4275
Phe Thr Gly Pro Asn Cys Gly Lys Thr Val Cys Glu Asp Phe Cys
4280 4285 4290
Gln Asn Gly Gly Thr Cys Ile Val Thr Ala Gly Asn Gln Pro Tyr
4295 4300 4305
Cys His Cys Gln Pro Glu Tyr Thr Gly Asp Arg Cys Gln Tyr Tyr
4310 4315 4320
Val Cys His His Tyr Cys Val Asn Ser Glu Ser Cys Thr Ile Gly
4325 4330 4335
Asp Asp Gly Ser Xaa Glu Cys Val Cys Pro Thr Arg Tyr Glu Gly
4340 4345 4350
Pro Lys Cys Glu Val Asp Lys Cys Val Arg Cys His Gly Gly His
4355 4360 4365
Cys Ile Ile Asn Lys Asp Ser Glu Asp Ile Phe Cys Asn Cys Thr
4370 4375 4380
Asn Gly Lys Ile Ala Ser Ser Cys Gln Leu Cys Asp Gly Tyr Cys
4385 4390 4395
Tyr Asn Gly Gly Thr Cys Gln Leu Asp Pro Glu Thr Asn Val Pro
4400 4405 4410

Val Cys Leu Cys Ser Thr Asn Trp Ser Gly Thr Gln Cys Glu Arg

4415 4420 4425

Pro Ala Pro Lys Ser Ser Lys Ser Asp His Ile Ser Thr Arg Ser

4430 4435 4440

Ile Ala Ile Ile Val Pro Leu Val Leu Leu Val Thr Leu Ile Thr

4445 4450 4455

Thr Leu Val Ile Gly Leu Val Leu Cys Lys Arg Lys Arg Arg Thr

4460 4465 4470

Lys Thr Ile Arg Arg Gln Pro Ile Ile Asn Gly Gly Ile Asn Val

4475 4480 4485

Glu Ile Gly Asn Pro Ser Tyr Asn Met Tyr Glu Val Asp His Asp

4490 4495 4500

His Asn Asp Gly Gly Leu Leu Asp Pro Gly Phe Met Ile Asp Pro

4505 4510 4515

Thr Lys Ala Arg Tyr Ile Gly Gly Gly Pro Ser Ala Phe Lys Leu

4520 4525 4530

Pro His Thr Ala Pro Pro Ile Tyr Leu Asn Ser Asp Leu Lys Gly

4535 4540 4545

Pro Leu Thr Ala Gly Pro Thr Asn Tyr Ser Asn Pro Val Tyr Ala

4550 4555 4560

Lys Leu Tyr Met Asp Gly Gln Asn Cys Arg Asn Ser Leu Gly Ser

4565 4570 4575

Val Asp Glu Arg Lys Glu Leu Leu Pro Lys Lys Ile Glu Ile Gly

4580 4585 4590

Ile Arg  Glu Thr Val Ala

    4595

<210>  14

<211>  345

<212>  PRT

<213>  Homo sapiens

<220>

<221>  hypothetical protein MGC10940

<222>  (1)..(345)

<223>  accession No. BC004331.1

<400>  14

Met Leu Pro Asn Thr Gly Arg Leu Ala Gly Cys Thr Val Phe Ile Thr

1               5                   10                  15

Gly Ala Ser Arg Gly Ile Gly Lys Ala Ile Ala Leu Lys Ala Ala Lys

            20                  25                  30

Asp Gly Ala Asn Ile Val Ile Ala Ala Lys Thr Ala Gln Pro His Pro

        35                  40                  45

Lys Leu Leu Gly Thr Ile Tyr Thr Ala Ala Glu Glu Ile Glu Ala Val

    50                  55                  60

Gly Gly Lys Ala Leu Pro Cys Ile Val Asp Val Arg Asp Glu Gln Gln

65                  70                  75                  80

Ile Ser Ala Ala Val Glu Lys Ala Ile Lys Lys Phe Gly Ala Tyr Thr

                85                  90                  95

Ile Ala Lys Tyr Gly Met Ser Met Tyr Val Leu Gly Met Ala Glu Glu

            100                 105                 110

Phe Lys Gly Glu Ile Ala Val Asn Ala Leu Trp Pro Lys Thr Ala Ile

        115                 120                 125

His Thr Ala Ala Met Asp Met Leu Gly Gly Pro Gly Ile Glu Ser Gln

    130                 135                 140

Cys Arg Lys Val Asp Ile Ile Ala Asp Ala Ala Tyr Ser Ile Phe Gln

145                 150                 155                 160

Lys Pro Lys Ser Phe Thr Gly Asn Phe Val Ile Asp Glu Asn Ile Leu

                165                 170                 175

Lys Glu Glu Gly Ile Glu Asn Phe Asp Val Tyr Ala Ile Lys Pro Gly

            180                 185                 190

His Pro Leu Gln Pro Asp Phe Phe Leu Asp Glu Tyr Pro Glu Ala Val

        195                 200                 205

Ser Lys Lys Val Glu Ser Thr Gly Ala Val Pro Glu Phe Lys Glu Glu

    210                 215                 220

Lys Leu Gln Leu Gln Pro Lys Pro Arg Ser Gly Ala Val Glu Glu Thr

225                 230                 235                 240

Phe Arg Ile Val Lys Asp Ser Leu Ser Asp Asp Val Val Lys Ala Thr

                245                 250                 255

Gln Ala Ile Tyr Leu Phe Glu Leu Ser Gly Glu Asp Gly Gly Thr Trp

260                 265                 270

Phe Leu Asp Leu Lys Ser Lys Gly Gly Asn Val Gly Tyr Gly Glu Pro

        275                 280                 285

Ser Asp Gln Ala Asp Val Val Met Ser Met Thr Thr Asp Asp Phe Val

    290                 295                 300

Lys Met Phe Ser Gly Lys Leu Lys Pro Thr Met Ala Phe Met Ser Gly

305                 310                 315                 320

Lys Leu Lys Ile Lys Gly Asn Met Ala Leu Ala Ile Lys Leu Glu Lys

                325                 330                 335

Leu Met Asn Gln Met Asn Ala Arg Leu

            340                 345

<210>  15

<211>  444

<212>  PRT

<213>  Homo sapiens

<220>

<221>  fatty acid desaturase 2 (FADS2)

<222>  (1)..(444)

<223>  accession No. NM_004265

<400>  15

Met Gly Lys Gly Gly Asn Gln Gly Glu Gly Ala Ala Glu Arg Glu Val
1               5                   10                  15
Ser Val Pro Thr Phe Ser Trp Glu Glu Ile Gln Lys His Asn Leu Arg
            20                  25                  30
Thr Asp Arg Trp Leu Val Ile Asp Arg Lys Val Tyr Asn Ile Thr Lys
        35                  40                  45
Trp Ser Ile Gln His Pro Gly Gly Gln Arg Val Ile Gly His Tyr Ala
    50                  55                  60
Gly Glu Asp Ala Thr Asp Ala Phe Arg Ala Phe His Pro Asp Leu Glu
65                  70                  75                  80
Phe Val Gly Lys Phe Leu Lys Pro Leu Leu Ile Gly Glu Leu Ala Pro
                85                  90                  95
Glu Glu Pro Ser Gln Asp His Gly Lys Asn Ser Lys Ile Thr Glu Asp
            100                 105                 110
Phe Arg Ala Leu Arg Lys Thr Ala Glu Asp Met Asn Leu Phe Lys Thr
        115                 120                 125
Asn His Val Phe Phe Leu Leu Leu Leu Ala His Ile Ile Ala Leu Glu
    130                 135                 140
Ser Ile Ala Trp Phe Thr Val Phe Tyr Phe Gly Asn Gly Trp Ile Pro
145                 150                 155                 160
Thr Leu Ile Thr Ala Phe Val Leu Ala Thr Ser Gln Ala Gln Ala Gly
                165                 170                 175
Trp Leu Gln His Asp Tyr Gly His Leu Ser Val Tyr Arg Lys Pro Lys
            180                 185                 190

Trp Asn His Leu Val His Lys Phe Val Ile Gly His Leu Lys Gly Ala
        195                 200                 205
Ser Ala Asn Trp Trp Asn His Arg His Phe Gln His His Ala Lys Pro
    210                 215                 220
Asn Ile Phe His Lys Asp Pro Asp Val Asn Met Leu His Val Phe Val
225                 230                 235                 240
Leu Gly Glu Trp Gln Pro Ile Glu Tyr Gly Lys Lys Lys Leu Lys Tyr
                245                 250                 255
Leu Pro Tyr Asn His Gln His Glu Tyr Phe Phe Leu Ile Gly Pro Pro
            260                 265                 270
Leu Leu Ile Pro Met Tyr Phe Gln Tyr Gln Ile Ile Met Thr Met Ile
        275                 280                 285
Val His Lys Asn Trp Val Asp Leu Ala Trp Ala Val Ser Tyr Tyr Ile
    290                 295                 300
Arg Phe Phe Ile Thr Tyr Ile Pro Phe Tyr Gly Ile Leu Gly Ala Leu
305                 310                 315                 320
Leu Phe Leu Asn Phe Ile Arg Phe Leu Glu Ser His Trp Phe Val Trp
                325                 330                 335
Val Thr Gln Met Asn His Ile Val Met Glu Ile Asp Gln Glu Ala Tyr
            340                 345                 350
Arg Asp Trp Phe Ser Ser Gln Leu Thr Ala Thr Cys Asn Val Glu Gln
        355                 360                 365
Ser Phe Phe Asn Asp Trp Phe Ser Gly His Leu Asn Phe Gln Ile Glu
    370                 375                 380

His His Leu Phe Pro Thr Met Pro Arg His Asn Leu His Lys Ile Ala
385                 390                 395                 400
Pro Leu Val Lys Ser Leu Cys Ala Lys His Gly Ile Glu Tyr Gln Glu
                405                 410                 415
Lys Pro Leu Leu Arg Ala Leu Leu Asp Ile Ile Arg Ser Leu Lys Lys
            420                 425                 430
Ser Gly Lys Leu Trp Leu Asp Ala Tyr Leu His Lys
        435                 440

<210>  16
<211>  523
<212>  PRT
<213>  Homo sapiens
<220>
<221>  RAR-related orphan receptor A
<222>  (1)..(523)
<223>  accession No. BC008831.1

<400>  16

<210>  17

<211>  4369

<212>  DNA

<213>  Homo sapiens

<220>

<221>  acyl-CoA synthetase long-chain family member 3 variant 1

<222>  (1)..(4369)

<223>  LocusID: 2181

NM_004457

<400>  17

gtcccaggcg gttccgctca acagacgctg ctgtggctgc gccgggctgc gacactgcag    60

ttgtctacgc ggccggggcc gggacgagga ggcgttggac ggggtcgcat acgttcgtcc   120

cctcgcattg cggccccgac agctgcgcca ggatccccgg gcggcggcgc ggggcgtgaa   180

cgctctgggg ctcagccagg cctgcgcggg cccgaggccg gaggaacccg gactccggcg   240

tagcggtttt gacacaaggg cgcatatctt caaagcacct agtacctcct accattgtca   300

actgatacag aattcgttgt tgggaaggac tggggaaaca gctgtaacat ttgccaccct   360

cagaagctgc tggtcctgtg tcacaccacc ttagcctctt gatcgaggaa gattctcgct   420

gaagtctgtt aattctactt tttgagtact tatgaataac cacgtgtctt caaaaccatc   480

taccatgaag ctaaaacata ccatcaaccc tattctttta tattttatac attttctaat   540

atcactttat actattttaa catacattcc gttttatttt ttctccgagt caagacaaga   600

aaaatcaaac cgaattaaag caaagcctgt aaattcaaaa cctgattctg catacagatc   660

tgttaatagt ttggatggtt tggcttcagt attataccct ggatgtgata ctttagataa   720

agtttttaca tatgcaaaaa acaaatttaa gaacaaaaga ctcttgggaa cacgtgaagt   780

tttaaatgag gaagatgaag tacaaccaaa tggaaaaatt tttaaaaagg ttattcttgg   840

acagtataat tggctttcct atgaagatgt ctttgttcga gcctttaatt ttggaaatgg   900

attacagatg ttgggtcaga aaccaaagac caacatcgcc atcttctgtg agaccagggc   960

cgagtggatg atagctgcac aggcgtgttt tatgtataat tttcagcttg ttacattata  1020

tgccactcta ggaggtccag ccattgttca tgcattaaat gaaacagagg tgaccaacat  1080

cattactagt aaagaactct tacaaacaaa gttgaaggat atagtttctt tggtcccacg  1140

cctgcggcac atcatcactg ttgatggaaa gccaccgacc tggtccgagt tccccaaggg 1200

catcattgtg cataccatgg ctgcagtgga ggccctggga gccaaggcca gcatggaaaa 1260

ccaacctcat agcaaaccat tgccctcaga tattgcagta atcatgtaca caagtggatc 1320

cacaggactt ccaaagggag tcatgatctc acatagtaac attattgctg gtataactgg 1380

gatggcagaa aggattccag aactaggaga ggaagatgtc tacattggat atttgcctct 1440

ggcccatgtt ctagaattaa gtgctgagct tgtctgtctt tctcacggat gccgcattgg 1500

ttactcttca ccacagactt tagcagatca gtcttcaaaa attaaaaaag gaagcaaagg 1560

ggatacatcc atgttgaaac caacactgat ggcagcagtt ccggaaatca tggatcggat 1620

ctacaaaaat gtcatgaata aagtcagtga aatgagtagt tttcaacgta atctgtttat 1680

tctggcctat aattacaaaa tggaacagat ttcaaaagga cgtaatactc cactgtgcga 1740

cagctttgtt ttccggaaag ttcgaagctt gctaggggga aatattcgtc tcctgttgtg 1800

tggtggcgct ccactttctg caaccacgca gcgattcatg aacatctgtt tctgctgtcc 1860

tgttggtcag ggatacgggc tcactgaatc tgctggggct ggaacaattt ccgaagtgtg 1920

ggactacaat actggcagag tgggagcacc attagtttgc tgtgaaatca aattaaaaaa 1980

ctgggaggaa ggtggatact ttaatactga taagccacac cccaggggtg aaattcttat 2040

tgggggccaa agtgtgacaa tggggtacta caaaaatgaa gcaaaaacaa aagctgattt 2100

ctttgaagat gaaaatggac aaaggtggct ctgtactggg gatattggag agtttgaacc 2160

cgatggatgc ttaaagatta ttgatcgtaa aaaggacctt gtaaaactac aggcagggga 2220

atatgtttct cttgggaaag tagaggcagc tttgaagaat cttccactag tagataacat 2280

ttgtgcatat gcaaacagtt atcattctta tgtcattgga tttgttgtgc caaatcaaaa 2340

ggaactaact gaactagctc gaaagaaagg acttaaaggg acttgggagg agctgtgtaa 2400

cagttgtgaa atggaaaatg aggtacttaa agtgctttcc gaagctgcta tttcagcaag 2460

tctggaaaag tttgaaattc cagtaaaaat tcgtttgagt cctgaaccgt ggacccctga 2520

aactggtctg gtgacagatg ccttcaagct gaaacgcaaa gagcttaaaa cacattacca 2580

ggcggacatt gagcgaatgt atggaagaaa ataattattc tcttctggca tcagtttgct 2640

acagtgagct cagatcaaat aggaaaatac ttgaaatgca tgtctcaagc tgcaaggcaa 2700

actccattcc tcatattaaa ctattacttc tcatgacgtc accattttta actgacagga 2760

ttagtaaaac attaagacag caaacttgtg tctgtctctt ctttcatttt ccccgccacc 2820

aacttacttt accacctatg actgtacttg tcagtatgag aatttttctg aatcatattg 2880

gggaagcagt gattttaaaa cctcaagttt ttaaacatga tttatatgtt ctgtataatg 2940

ttcagtttgt aactttttaa aagtttggat gtatagaggg ataaatagga aatataagaa 3000

ttggttattt gggggctttt ttacttactg tatttaaaaa tacaagggta ttgatatgaa 3060

attatgtaaa tttcaaatgc ttatgaatca aatcattgtt gaacaaaaga tttgttgctg 3120

tgtaattatt gtcttgtatg catttgagag aaataaatat acccatactt atgttttaag 3180

aagttgagat cttgtgaata tatgcctgtc agtgtcttct ttatatattt attttttatt 3240

agaaaaaatg aagtttggtt ggtgatgcat gaaacaaaat agcaagagag ggttatagtt 3300

taatagtaag ggagataaca cagcatgtgt agcaccagtt gataattggt ctctagtagc 3360

ttactgtcaa aatgttcaat gaagtcttct gttcatctgt tgaaactagg aaaataccca 3420

aacttaaatg gaagaattct gaaagagagg atagaattta aagaacaaga gtatataaag 3480

ttattctttg aatatttcgt tgactatatg tacattgagt tatctatatt tgtaaacaaa 3540

ttagtcatgg aaaattattc tatctcaaag tctcctttta gtctagataa tcattatttc 3600

attttaaaat tagtgttttt cctagtttgc actgatgcgt gtatggatgt gtgtgagtca 3660

gtggtagctt atttaaaaag caccttatcc tttctcccat aacctttgta cactaaaaaa 3720

tgaaagaatt tagaatgtat ttgatgatag cattctcact aagacacatg agaatttaac 3780

tttataaccg cgtgagttaa gatttaattc ataggttttg atgtcattgt tgaagttatt 3840

tgtaattcag aaaccttgct tgtgtgatac atagtctctt catttattac tgcttgtctg 3900

ttgttatatc tggattatca aaagcaatag tgcaccaatt aagatgtgct caaatcagga 3960

cttaaatcat aggcaccaca tttttcatgt cagactagtt actttgttga ttctcagtta 4020

ctgtaggcat caaaaggcaa aaatcaaaaa aaaaaaaaac aaaaacaaaa aaaaagatga 4080

acctaggtct gtgtaaagta aggggagtgt taggagcagc caggactgtg tagtgtgtgt 4140

ttggttgcat cacaaacatc gtatgtggag acattgcaat acagtgtttt ttgttttcaa 4200

cttttcttgt attgtatatt tgtattatgt tttgaatgct tttctctttt cataattaaa 4260

tattaatgtt tgggataact gccaagaaga agtaaaaata ttgaatggaa cttctatatg 4320

aggatgctgt gatctaaaaa ttaaatctca gtgggcggag aaaaaaaaa 4369

<210> 18

<211> 4262

<212> DNA

<213> Homo sapiens

<220>

<221> acyl-CoA synthetase long-chain family member 3 variant 2

<222> (1)..(4262)

<223> LocusID: 2181

NM_203372

<400> 18

gtcccaggcg gttccgctca acagacgctg ctgtggctgc gccgggctgc gacactgcag 60

ttgtctacgc ggccggggcc gggacgagga ggcgttggac ggggtcgcat acgttcgtcc 120

cctcgcattg cggccccgac agctgcgcca ggatccccgg gcggcggcgc ggggcgtgaa 180

cgctctgggg ctcagccagg cctgcgcggg cccgaggccg gaggaacccg gactccggcg 240

tagcggtttt gacacaaggg cgcatatctt caaagcacct agtacctcct accattgtca 300

actgattctc gctgaagtct gttaattcta ctttttgagt acttatgaat aaccacgtgt 360

cttcaaaacc atctaccatg aagctaaaac ataccatcaa ccctattctt ttatatttta 420

tacattttct aatatcactt tatactattt taacatacat tccgttttat tttttctccg 480

agtcaagaca agaaaaatca aaccgaatta aagcaaagcc tgtaaattca aaacctgatt 540

ctgcatacag atctgttaat agtttggatg gtttggcttc agtattatac cctggatgtg 600

atactttaga taaagttttt acatatgcaa aaaacaaatt taagaacaaa agactcttgg 660

gaacacgtga agttttaaat gaggaagatg aagtacaacc aaatggaaaa atttttaaaa 720

aggttattct tggacagtat aattggcttt cctatgaaga tgtctttgtt cgagccttta 780

attttggaaa tggattacag atgttgggtc agaaaccaaa gaccaacatc gccatcttct 840

gtgagaccag ggccgagtgg atgatagctg cacaggcgtg ttttatgtat aattttcagc 900

ttgttacatt atatgccact ctaggaggtc cagccattgt tcatgcatta aatgaaacag 960

aggtgaccaa catcattact agtaaagaac tcttacaaac aaagttgaag gatatagttt 1020

ctttggtccc acgcctgcgg cacatcatca ctgttgatgg aaagccaccg acctggtccg 1080

agttccccaa gggcatcatt gtgcatacca tggctgcagt ggaggccctg ggagccaagg 1140

ccagcatgga aaaccaacct catagcaaac cattgccctc agatattgca gtaatcatgt 1200

acacaagtgg atccacagga cttccaaagg gagtcatgat ctcacatagt aacattattg 1260

ctggtataac tgggatggca gaaaggattc cagaactagg agaggaagat gtctacattg 1320

gatatttgcc tctggcccat gttctagaat taagtgctga gcttgtctgt ctttctcacg 1380

gatgccgcat tggttactct tcaccacaga ctttagcaga tcagtcttca aaaattaaaa 1440

aaggaagcaa aggggataca tccatgttga aaccaacact gatggcagca gttccggaaa 1500

tcatggatcg gatctacaaa aatgtcatga ataaagtcag tgaaatgagt agttttcaac 1560

gtaatctgtt tattctggcc tataattaca aaatggaaca gatttcaaaa ggacgtaata 1620

ctccactgtg cgacagcttt gttttccgga aagttcgaag cttgctaggg ggaaatattc 1680

gtctcctgtt gtgtggtggc gctccacttt ctgcaaccac gcagcgattc atgaacatct 1740

gtttctgctg tcctgttggt cagggatacg ggctcactga atctgctggg gctggaacaa 1800

tttccgaagt gtgggactac aatactggca gagtgggagc accattagtt tgctgtgaaa 1860

tcaaattaaa aaactgggag gaaggtggat actttaatac tgataagcca caccccaggg 1920

gtgaaattct tattgggggc caaagtgtga caatggggta ctacaaaaat gaagcaaaaa 1980

caaaagctga tttctttgaa gatgaaaatg gacaaaggtg gctctgtact ggggatattg 2040

gagagtttga acccgatgga tgcttaaaga ttattgatcg taaaaaggac cttgtaaaac 2100

tacaggcagg ggaatatgtt tctcttggga aagtagaggc agctttgaag aatcttccac 2160

tagtagataa catttgtgca tatgcaaaca gttatcattc ttatgtcatt ggatttgttg 2220

tgccaaatca aaaggaacta actgaactag ctcgaaagaa aggacttaaa gggacttggg 2280

aggagctgtg taacagttgt gaaatggaaa atgaggtact taaagtgctt tccgaagctg 2340

ctatttcagc aagtctggaa aagtttgaaa ttccagtaaa aattcgtttg agtcctgaac 2400

cgtggacccc tgaaactggt ctggtgacag atgccttcaa gctgaaacgc aaagagctta 2460

aaacacatta ccaggcggac attgagcgaa tgtatggaag aaaataatta ttctcttctg 2520

gcatcagttt gctacagtga gctcagatca aataggaaaa tacttgaaat gcatgtctca 2580

agctgcaagg caaactccat tcctcatatt aaactattac ttctcatgac gtcaccattt 2640

ttaactgaca ggattagtaa aacattaaga cagcaaactt gtgtctgtct cttctttcat 2700

tttccccgcc accaacttac tttaccacct atgactgtac ttgtcagtat gagaattttt 2760

ctgaatcata ttggggaagc agtgatttta aaacctcaag tttttaaaca tgatttatat 2820

gttctgtata atgttcagtt tgtaactttt taaaagtttg gatgtataga gggataaata 2880

ggaaatataa gaattggtta tttgggggct tttttactta ctgtatttaa aaatacaagg 2940

gtattgatat gaaattatgt aaatttcaaa tgcttatgaa tcaaatcatt gttgaacaaa 3000

agatttgttg ctgtgtaatt attgtcttgt atgcatttga gagaaataaa tatacccata 3060

cttatgtttt aagaagttga gatcttgtga atatatgcct gtcagtgtct tctttatata 3120

tttatttttt attagaaaaa atgaagtttg gttggtgatg catgaaacaa aatagcaaga 3180

gagggttata gtttaatagt aagggagata acacagcatg tgtagcacca gttgataatt 3240

ggtctctagt agcttactgt caaaatgttc aatgaagtct tctgttcatc tgttgaaact 3300

aggaaaatac ccaaacttaa atggaagaat tctgaaagag aggatagaat ttaaagaaca 3360

agagtatata aagttattct ttgaatattt cgttgactat atgtacattg agttatctat 3420

atttgtaaac aaattagtca tggaaaatta ttctatctca aagtctcctt ttagtctaga 3480

taatcattat ttcattttaa aattagtgtt tttcctagtt tgcactgatg cgtgtatgga 3540

tgtgtgtgag tcagtggtag cttatttaaa aagcacctta tcctttctcc cataaccttt 3600

gtacactaaa aaatgaaaga atttagaatg tatttgatga tagcattctc actaagacac 3660

atgagaattt aactttataa ccgcgtgagt taagatttaa ttcataggtt ttgatgtcat 3720

tgttgaagtt atttgtaatt cagaaacctt gcttgtgtga tacatagtct cttcatttat 3780

tactgcttgt ctgttgttat atctggatta tcaaaagcaa tagtgcacca attaagatgt 3840

gctcaaatca ggacttaaat cataggcacc acatttttca tgtcagacta gttactttgt 3900

tgattctcag ttactgtagg catcaaaagg caaaaatcaa aaaaaaaaaa aacaaaaaca 3960

aaaaaaaaga tgaacctagg tctgtgtaaa gtaaggggag tgttaggagc agccaggact 4020

gtgtagtgtg tgtttggttg catcacaaac atcgtatgtg gagacattgc aatacagtgt 4080

tttttgtttt caacttttct tgtattgtat atttgtatta tgttttgaat gcttttctct 4140

tttcataatt aaatattaat gtttgggata actgccaaga agaagtaaaa atattgaatg 4200

gaacttctat atgaggatgc tgtgatctaa aaattaaatc tcagtgggcg gagaaaaaaa 4260

aa 4262

<210> 19

<211> 1300

<212> DNA

<213>  Homo sapiens

<220>

<221>  solute carrier family 25 member 4

<222>  (1)..(1300)

<223>  LocusID: 291

NM_001151

<400>  19

ccccctagcg tcgcgcaggg tcggggactg cgcgcggtgc caggccgggc gtgggcgaga    60

gcacgaacgg gctgctgcgg gctgagagcg tcgagctgtc accatgggtg atcacgcttg   120

gagcttccta aaggacttcc tggccggggc ggtcgccgct gccgtctcca agaccgcggt   180

cgcccccatc gagagggtca aactgctgct gcaggtccag catgccagca aacagatcag   240

tgctgagaag cagtacaaag ggatcattga ttgtgtggtg agaatcccta aggagcaggg   300

cttcctctcc ttctggaggg gtaacctggc caacgtgatc cgttacttcc ccacccaagc   360

tctcaacttc gccttcaagg acaagtacaa gcagctcttc ttagggggtg tggatcggca   420

taagcagttc tggcgctact ttgctggtaa cctggcgtcc ggtggggccg ctggggccac   480

ctccctttgc tttgtctacc cgctggactt tgctaggacc aggttggctg ctgatgtggg   540

caggcgcgcc cagcgtgagt tccatggtct gggcgactgt atcatcaaga tcttcaagtc   600

tgatggcctg agggggctct accagggttt caacgtctct gtccaaggca tcattatcta   660

tagagctgcc tacttcggag tctatgatac tgccaagggg atgctgcctg accccaagaa   720

cgtgcacatt tttgtgagct ggatgattgc ccagagtgtg acggcagtcg cagggctgct   780

gtcctacccc tttgacactg ttcgtcgtag aatgatgatg cagtccggcc ggaaaggggc   840

cgatattatg tacacgggga cagttgactg ctggaggaag attgcaaaag acgaaggagc   900

caaggccttc ttcaaaggtg cctggtccaa tgtgctgaga ggcatgggcg gtgcttttgt 960

attggtgttg tatgatgaga tcaaaaaata tgtctaatgt aattaaaaca caagttcaca 1020

gatttacatg aacttgatct acaagttcac agatccattg tgtggtttaa tagactattc 1080

ctaggggaag taaaaagatc tgggataaaa ccagactgaa aggaatacct cagaagagat 1140

gcttcattga gtgttcatta aaccacacat gtattttgta tttattttac atttaaattc 1200

ccacagcaaa tagaaataat ttatcatact tgtacaatta actgaagaat tgataataac 1260

tgaatgtgaa acatcaataa agaccactta atgcacaaaa 1300

<210> 20

<211> 4130

<212> DNA

<213> Homo sapiens

<220>

<221> TEK tyrosine kinase

<222> (1)..(4130)

<223> LocusID: 7010

NM_000459

<400> 20

cttctgtgct gttccttctt gcctctaact tgtaaacaag acgtactagg acgatgctaa 60

tggaaagtca caaaccgctg ggtttttgaa aggatccttg ggacctcatg cacatttgtg 120

gaaactggat ggagagattt ggggaagcat ggactcttta gccagcttag ttctctgtgg 180

agtcagcttg ctcctttctg gaactgtgga aggtgccatg gacttgatct tgatcaattc 240

cctacctctt gtatctgatg ctgaaacatc tctcacctgc attgcctctg ggtggcgccc 300

ccatgagccc atcaccatag gaagggactt tgaagcctta atgaaccagc accaggatcc 360

gctggaagtt actcaagatg tgaccagaga atgggctaaa aaagttgttt ggaagagaga 420

aaaggctagt aagatcaatg gtgcttattt ctgtgaaggg cgagttcgag gagaggcaat 480

caggatacga accatgaaga tgcgtcaaca agcttccttc ctaccagcta ctttaactat 540

gactgtggac aagggagata acgtgaacat atctttcaaa aaggtattga ttaaagaaga 600

agatgcagtg atttacaaaa atggttcctt catccattca gtgccccggc atgaagtacc 660

tgatattcta gaagtacacc tgcctcatgc tcagccccag gatgctggag tgtactcggc 720

caggtatata ggaggaaacc tcttcacctc ggccttcacc aggctgatag tccggagatg 780

tgaagcccag aagtggggac ctgaatgcaa ccatctctgt actgcttgta tgaacaatgg 840

tgtctgccat gaagatactg gagaatgcat ttgccctcct gggtttatgg gaaggacgtg 900

tgagaaggct tgtgaactgc acacgtttgg cagaacttgt aaagaaaggt gcagtggaca 960

agagggatgc aagtcttatg tgttctgtct ccctgacccc tatgggtgtt cctgtgccac 1020

aggctggaag ggtctgcagt gcaatgaagc atgccaccct ggtttttacg ggccagattg 1080

taagcttagg tgcagctgca acaatgggga gatgtgtgat cgcttccaag gatgtctctg 1140

ctctccagga tggcaggggc tccagtgtga gagagaaggc ataccgagga tgaccccaaa 1200

gatagtggat ttgccagatc atatagaagt aaacagtggt aaatttaatc ccatttgcaa 1260

agcttctggc tggccgctac ctactaatga agaaatgacc ctggtgaagc cggatgggac 1320

agtgctccat ccaaaagact ttaaccatac ggatcatttc tcagtagcca tattcaccat 1380

ccaccggatc ctcccccctg actcaggagt ttgggtctgc agtgtgaaca cagtggctgg 1440

gatggtggaa aagcccttca acatttctgt taaagttctt ccaaagcccc tgaatgcccc 1500

aaacgtgatt gacactggac ataactttgc tgtcatcaac atcagctctg agccttactt 1560

tggggatgga ccaatcaaat ccaagaagct tctatacaaa cccgttaatc actatgaggc 1620

ttggcaacat attcaagtga caaatgagat tgttacactc aactatttgg aacctcggac 1680

agaatatgaa ctctgtgtgc aactggtccg tcgtggagag ggtggggaag ggcatcctgg 1740

acctgtgaga cgcttcacaa cagcttctat cggactccct cctccaagag gtctaaatct 1800

cctgcctaaa agtcagacca ctctaaattt gacctggcaa ccaatatttc caagctcgga 1860

agatgacttt tatgttgaag tggagagaag gtctgtgcaa aaaagtgatc agcagaatat 1920

taaagttcca ggcaacttga cttcggtgct acttaacaac ttacatccca gggagcagta 1980

cgtggtccga gctagagtca acaccaaggc ccagggggaa tggagtgaag atctcactgc 2040

ttggaccctt agtgacattc ttcctcctca accagaaaac atcaagattt ccaacattac 2100

acactcctcg gctgtgattt cttggacaat attggatggc tattctattt cttctattac 2160

tatccgttac aaggttcaag gcaagaatga agaccagcac gttgatgtga agataaagaa 2220

tgccaccatc attcagtatc agctcaaggg cctagagcct gaaacagcat accaggtgga 2280

catttttgca gagaacaaca tagggtcaag caacccagcc ttttctcatg aactggtgac 2340

cctcccagaa tctcaagcac cagcggacct cggagggggg aagatgctgc ttatagccat 2400

ccttggctct gctggaatga cctgcctgac tgtgctgttg gcctttctga tcatattgca 2460

attgaagagg gcaaatgtgc aaaggagaat ggcccaagcc ttccaaaacg tgagggaaga 2520

accagctgtg cagttcaact cagggactct ggccctaaac aggaaggtca aaaacaaccc 2580

agatcctaca atttatccag tgcttgactg gaatgacatc aaatttcaag atgtgattgg 2640

ggagggcaat tttggccaag ttcttaaggc gcgcatcaag aaggatgggt tacggatgga 2700

tgctgccatc aaaagaatga aagaatatgc ctccaaagat gatcacaggg actttgcagg 2760

agaactggaa gttctttgta aacttggaca ccatccaaac atcatcaatc tcttaggagc 2820

atgtgaacat cgaggctact tgtacctggc cattgagtac gcgccccatg gaaaccttct 2880

ggacttcctt cgcaagagcc gtgtgctgga gacggaccca gcatttgcca ttgccaatag 2940

caccgcgtcc acactgtcct cccagcagct ccttcacttc gctgccgacg tggcccgggg 3000

catggactac ttgagccaaa aacagtttat ccacagggat ctggctgcca gaaacatttt 3060

agttggtgaa aactatgtgg caaaaatagc agattttgga ttgtcccgag gtcaagaggt 3120

gtacgtgaaa aagacaatgg gaaggctccc agtgcgctgg atggccatcg agtcactgaa 3180

ttacagtgtg tacacaacca acagtgatgt atggtcctat ggtgtgttac tatgggagat 3240

tgttagctta ggaggcacac cctactgcgg gatgacttgt gcagaactct acgagaagct 3300

gccccagggc tacagactgg agaagcccct gaactgtgat gatgaggtgt atgatctaat 3360

gagacaatgc tggcgggaga agccttatga gaggccatca tttgcccaga tattggtgtc 3420

cttaaacaga atgttagagg agcgaaagac ctacgtgaat accacgcttt atgagaagtt 3480

tacttatgca ggaattgact gttctgctga agaagcggcc taggacagaa catctgtata 3540

ccctctgttt ccctttcact ggcatgggag acccttgaca actgctgaga aaacatgcct 3600

ctgccaaagg atgtgatata taagtgtaca tatgtgctgg aattctaaca agtcataggt 3660

taatatttaa gacactgaaa aatctaagtg atataaatca gattcttctc tctcatttta 3720

tccctcacct gtagcatgcc agtcccgttt catttagtca tgtgaccact ctgtcttgtg 3780

tttccacagc ctgcaagttc agtccaggat gctaacatct aaaaatagac ttaaatctca 3840

ttgcttacaa gcctaagaat ctttagagaa gtatacataa gtttaggata aaataatggg 3900

attttctttt cttttctctg gtaatattga cttgtatatt ttaagaaata acagaaagcc 3960

tgggtgacat ttgggagaca tgtgacattt atatattgaa ttaatatccc tacatgtatt 4020

gcacattgta aaaagtttta gttttgatga gttgtgagtt taccttgtat actgtaggca 4080

cactttgcac tgatatatca tgagtgaata aatgtcttgc ctactcaaaa 4130

<210> 21

<211> 1340

<212> DNA

<213>  Homo sapiens

<220>

<221>  ATP synthase, H+ transporting, mitochondrial F0 complex, subunit

       g

<222>  (1)..(1340)

<223>  LocusID: 10632

NM_006476

<400>  21

cgagtggctg ccccacaaaa tgcctgcttc tctgcggaat cctactgttc ttcacatgct    60

ctctaatacc atcttttcat atccactttc tcttccatgt tgaaaaatta aattgacagg   120

ctggattctg caaagatctt tggacattta agtatcttcg accggcgcga aaagaggcgg   180

cctgaccttg gaagtgggac ggggtcctgc agcgggtcct tccggcgggt gacattcagc   240

cggcggttcg gggcgacgga ctctccattc cagaaccatg gcccaatttg tccgtaacct   300

tgtggagaag accccggcgc tggtgaacgc tgctgtgact tactcgaagc ctcgattggc   360

cacattttgg tactacgcca aggttgagct ggttcctccc acccctgctg agatccctag   420

agctattcag agcctgaaaa aaatagtcaa tagtgctcag actggtagct tcaaacagct   480

cacagttaag gaagctgtgc tgaatggttt ggtggccact gaggtgttga tgtggtttta   540

tgtcggagag attataggca agcggggcat cattggctat gatgtttgaa gaccaatctt   600

taacatctga ttatatttga tttattattt gagtgttgtt ggaccatgtg tgatcagact   660

gctatctgaa taaaataaga tttgtcaaaa ctcagtgttt tctccatcag atactccatg   720

aaaggtcaca atttctcttg atattaagct gggttgtctt taaacaaccc taaatacacg   780

tctgtttagc ccgcaattgg aaaggatata tgtggcaata ttaacctggt acatgaatat   840

atggggataa cattttaatt tgaaggtttg gaatatatat atttaagctt tatttccaga   900

acagtgaggg ttaggtcttg ggaaaactat aacttgccaa agtagaagaa atagtagtac   960

catatgccaa agtgatagag atgaatcatg tcagtagtta gaataacatt tcaactgttt  1020

tctttgctaa aatcacagaa agaccctatt gacaacatct atgtctgtaa aaatgttaga  1080

gtacttgtca tcttgaatat agcctcccca agagagaaca gggtggtatt ctaagtatgt  1140

ttctttgtaa catctttagc agtaggacag agccatacat gtgaaatctg atttttatgt  1200

gtgttattcg tttgtctggt tttactacct ttgcaaaaac aaaatacccc aaagatattt  1260

aaacaaggtt ataatttagc atcttccctg gatctaaata gtatattata tcctgaaata  1320

aatgaaatga ttgctataaa                                              1340

<210>  22

<211>  1478

<212>  DNA

<213>  Homo sapiens

<220>

<221>  ATP synthase, H+ transporting, mitochondrial F0 complex, subunit

       b, isoform 1

<222>  (1)..(1478)

<223>  Locus ID:515

transcript variant 2

NM_001002014

<400> 22

agggggggtg gggtttcctt ccgcatctcc acggttccaa ctccaaccta gactcaaact 60

ggacgccggc cggagactcc gctccggcag caaaccccac gtggtgcacc tctgagcctc 120

cgcccctctc ccgagggaac cgcaactcta cttctcgcga gaattgcttc tatggctcca 180

tcctgctttc cggctgtcgc cctcatgcga taggctctca gcgttacttg actcttctcg 240

cgataatttt ttttaaaaat ctcccaagga aagttgaagg aagagtacaa aattttcatc 300

tcgcgagact tgtgagcggc catcttggtc ctgccctgac agattctcct atcggggtca 360

cagggacgct aagattgcta cctggacttt cgttgaccat gctgtcccgg gtggtacttt 420

ccgccgccgc cacagcggcc ccctctctga agaatgcagc cttcctaggt ccagggaccc 480

tatgtactcg gaactgggct tatcttgtac gctttatcca aagaaatata tgtgattagc 540

gcagagacct tcactgccct atcagtacta ggtgtaatgg tctatggaat taaaaaatat 600

ggtccctttg ttgcagactt tgctgataaa ctcaatgagc aaaaacttgc ccaactagaa 660

gaggcgaagc aggcttccat ccaacacatc cagaatgcaa ttgatacgga gaagtcacaa 720

caggcactgg ttcagaagcg ccattacctt tttgatgtgc aaaggaataa cattgctatg 780

gctttggaag ttacttaccg ggaacgactg tatagagtat ataaggaagt aaagaatcgc 840

ctggactatc atatatctgt gcagaacatg atgcgtcgaa aggaacaaga acacatgata 900

aattgggtgg agaagcacgt ggtgcaaagc atctccacac agcaggaaaa ggagacaatt 960

gccaagtgca ttgcggacct aaagctgctg gcaaagaagg ctcaagcaca gccagttatg 1020

taaatgtatc tatcccaatt gagacagcta gaaacagttg actgactaaa tggaaactag 1080

tctatttgac aaagtctttc tgtgttggtg tctactgaag ttatagttta cccttcctaa 1140

aaatgaaaag tttgtttcat atagtgagag aacgaaatct ctatcggcca gtcagatgtt 1200

tctcatcctt cttgctctgc ctttgagttg ttccgtgatc acttctgaat aagcagtttg 1260

cctttataaa aacttgctgc ctgactaaag attaacaggt tatagtttaa atttgtaatt 132

aattctacca tcttgcaata aagtgacaat tgaatgaaac agggtttttc aagttgtata 1380

attctctgaa atactcagct tttgtcatat gggtaaaaat taaagatgtc attgaactac 1440

tgtcttgttt atgagaccat tcagtggtga actgtttc 1478

<210> 23

<211> 1441

<212> DNA

<213> Homo sapiens

<220>

<221> ATP synthase, H+ transporting, mitochondrial F0 complex, subunit

b, isoform 1

<222> (1)..(1441)

<223> LocuID: 515

transcript variant 3

NM_001002015

<400> 23

agggggggtg gggtttcctt ccgcatctcc acggttccaa ctccaaccta gactcaaact 60

ggacgccggc cggagactcc gctccggcag caaaccccac gtggtgcacc tctgagcctc 120

cgcccctctc ccgagggaac cgcaactcta cttctcgcga gaattgcttc tatggctcca 180

tcctgctttc cggctgtcgc cctcatgcga taggctctca gcgttacttg actcttctcg 240

cgataatttt ttttaaaaat ctcccaagga aagttgaagg aagagtacaa aattttcatc 300

tcgcgagact tgtgagcggc catcttggtc ctgccctgac agattctcct atcggggtca 360

cagggacgct aagattgcta cctggacttt cgttgaccat gctgtcccgg gtggtacttt 420

ccgccgccgc cacagcggga ccctatgtac tcggaactgg gcttatcttg tacgctttat 480

ccaaagaaat atatgtgatt agcgcagaga ccttcactgc cctatcagta ctaggtgtaa 540

tggtctatgg aattaaaaaa tatggtccct ttgttgcaga ctttgctgat aaactcaatg 600

gcaaaaact tgcccaacta gaagaggcga agcaggcttc catccaacac atccagaatg 660

caattgatac ggagaagtca caacaggcac tggttcagaa gcgccattac ctttttgatg 720

tgcaaaggaa taacattgct atggctttgg aagttactta ccgggaacga ctgtatagag 780

tatataagga agtaaagaat cgcctggact atcatatatc tgtgcagaac atgatgcgtc 840

gaaaggaaca agaacacatg ataaattggg tggagaagca cgtggtgcaa agcatctcca 900

cacagcagga aaaggagaca attgccaagt gcattgcgga cctaaagctg ctggcaaaga 960

aggctcaagc acagccagtt atgtaaatgt atctatccca attgagacag ctagaaacag 1020

ttgactgact aaatggaaac tagtctattt gacaaagtct ttctgtgttg gtgtctactg 1080

aagttatagt ttacccttcc taaaaatgaa aagtttgttt catatagtga gagaacgaaa 1140

tctctatcgg ccagtcagat gtttctcatc cttcttgctc tgcctttgag ttgttccgtg 1200

atcacttctg aataagcagt ttgcctttat aaaaacttgc tgcctgacta aagattaaca 1260

ggttatagtt taaatttgta attaattcta ccatcttgca ataaagtgac aattgaatga 1320

aacagggttt ttcaagttgt ataattctct gaaatactca gcttttgtca tatgggtaaa 1380

aattaaagat gtcattgaac tactgtcttg tttatgagac cattcagtgg tgaactgttt 1440

c 1441

<210> 24

<211>  1624

<212>  DNA

<213>  Homo sapiens

<220>

<221>  ATP synthase, H+ transporting, mitochondrial F0 complex, subunit

       b, isoform 1

<222>  (1)..(1624)

<223>  LocusID: 515

transcript variant 1

NM_001688

<400>  24

agggggggtg gggtttcctt ccgcatctcc acggttccaa ctccaaccta gactcaaact    60

ggacgccggc cggagactcc gctccggcag caaaccccac gtggtgcacc tctgagcctc   120

cgcccctctc ccgagggaac cgcaactcta cttctcgcga gaattgcttc tatggctcca   180

tcctgctttc cggctgtcgc cctcatgcga taggctctca gcgttacttg actcttctcg   240

cgataatttt ttttaaaaat ctcccaagga aagttgaagg aagagtacaa aattttcatc   300

tcgcgagact tgtgagcggc catcttggtc ctgccctgac agattctcct atcggggtca   360

cagggacgct aagattgcta cctggacttt cgttgaccat gctgtcccgg gtggtacttt   420

ccgccgccgc cacagcggcc ccctctctga agaatgcagc cttcctaggt ccaggggtat   480

tgcaggcaac aaggaccttt catacagggc agccacacct tgtccctgta ccacctcttc   540

ctgaatacgg aggaaaagtt cgttatggac tgatccctga ggaattcttc cagtttcttt   600

atcctaaaac tggtgtaaca ggaccctatg tactcggaac tgggcttatc ttgtacgctt 660

tatccaaaga aatatatgtg attagcgcag agaccttcac tgccctatca gtactaggtg 720

taatggtcta tggaattaaa aaatatggtc cctttgttgc agactttgct gataaactca 780

atgagcaaaa acttgcccaa ctagaagagg cgaagcaggc ttccatccaa cacatccaga 840

atgcaattga tacggagaag tcacaacagg cactggttca gaagcgccat tacctttttg 900

atgtgcaaag gaataacatt gctatggctt tggaagttac ttaccgggaa cgactgtata 960

gagtatataa ggaagtaaag aatcgcctgg actatcatat atctgtgcag aacatgatgc 1020

gtcgaaagga acaagaacac atgataaatt gggtggagaa gcacgtggtg caaagcatct 1080

ccacacagca ggaaaaggag acaattgcca agtgcattgc ggacctaaag ctgctggcaa 1140

agaaggctca agcacagcca gttatgtaaa tgtatctatc ccaattgaga cagctagaaa 1200

cagttgactg actaaatgga aactagtcta tttgacaaag tctttctgtg ttggtgtcta 1260

ctgaagttat agtttaccct tcctaaaaat gaaaagtttg tttcatatag tgagagaacg 1320

aaatctctat cggccagtca gatgtttctc atccttcttg ctctgccttt gagttgttcc 1380

gtgatcactt ctgaataagc agtttgcctt tataaaaact tgctgcctga ctaaagatta 1440

acaggttata gtttaaattt gtaattaatt ctaccatctt gcaataaagt gacaattgaa 1500

tgaaacaggg tttttcaagt tgtataattc tctgaaatac tcagcttttg tcatatgggt 1560

aaaaattaaa gatgtcattg aactactgtc ttgtttatga gaccattcag tggtgaactg 1620

tttc 1624

<210> 25

<211> 930

<212> DNA

<213>  Homo sapiens

<220>

<221>  thyroid hormone receptor interactor 3

<222>  (1)..(930)

<223>  LocusID: 9326

NM_004773

<400>  25

gcgcggcggc gcagtaaaca gtctccttcc acaaaaccat ggcgtcgctc aaatgtagca    60

ccgtcgtctg cgtgatctgc ttggagaagc ccaaataccg ctgtccagcc tgccgcgtgc   120

cctactgctc ggtagtctgc ttccggaagc acaaagaaca gtgcaaccct gaaactcgtc   180

ctgttgagaa aaaaataaga tcagctcttc ctaccaaaac cgtaaagcct gtggaaaaca   240

aagatgatga tgactctata gctgattttc tcaatagtga tgaggaagaa gacagagttt   300

ctttgcagaa tttaaagaat ttaggggaat ctgcaacatt aagaagctta ttgctcaatc   360

cacacctcag gcagttgatg gtcaacctcg atcagggaga agacaaagca aagctcatga   420

gagcttacat gcaagagcct ttgtttgtgg agtttgcaga ctgctgttta ggaattgtgg   480

agccatccca gaatgaggag tcttaagatg gattattgtg ctgcttgctc aagcgtgtgc   540

ttgactcctg gaacctgcct gctccctctc ccagaccagc tagtttgggg ctggggagct   600

caggcaaaag aggtttccag gatgcagatt aggtcatgca ggcctttacc ggcattgatg   660

tggctcatgt ttcaggcaga cttggggtcc ttaaggtggc aagtccttta tggagagaaa   720

acttgacatt cagatgattg tttttaaatg ttttactttt ggtacagttg atagacatca   780

taaacgatat caagcttaca cttcatatgg agttaaactt ggtcagtgtt aataaaatca   840

aaacgtgatt ctactgtaca ttgcattatt cataatttaa ttgtttgaaa ttacattaaa   900

taaatcaact aattaaatac gaaaaaaaaa 930

<210> 26

<211> 1310

<212> DNA

<213> Homo sapiens

<220>

<221> lactate dehydrogenase B

<222> (1)..(1310)

<223> LocusID: 3945

NM_002300

<400> 26

ggggcttgca gagccggcgc cggaggagac gcacgcagct gactttgtct tctccgcacg 60

actgttacag aggtctccag agccttctct ctcctgtgca aaatggcaac tcttaaggaa 120

aaactcattg caccagttgc ggaagaagag gcaacagttc caaacaataa gatcactgta 180

gtgggtgttg gacaagttgg tatggcgtgt gctatcagca ttctgggaaa gtctctggct 240

gatgaacttg ctcttgtgga tgttttggaa gataagctta aaggagaaat gatggatctg 300

cagcatggga gcttatttct tcagacacct aaaattgtgg cagataaaga ttattctgtg 360

accgccaatt ctaagattgt agtggtaact gcaggagtcc gtcagcaaga aggggagagt 420

cggctcaatc tggtgcagag aaatgttaat gtcttcaaat tcattattcc tcagatcgtc 480

aagtacagtc ctgattgcat cataattgtg gtttccaacc cagtggacat tcttacgtat 540

gttacctgga aactaagtgg attacccaaa caccgcgtga ttggaagtgg atgtaatctg 600

gattctgcta gatttcgcta ccttatggct gaaaaacttg gcattcatcc cagcagctgc 660

catggatgga ttttggggga acatggcgac tcaagtgtgg ctgtgtggag tggtgtgaat 720

gtggcaggtg tttctctcca ggaattgaat ccagaaatgg gaactgacaa tgatagtgaa 780

aattggaagg aagtgcataa gatggtggtt gaaagtgcct atgaagtcat caagctaaaa 840

ggatatacca actgggctat tggattaagt gtggctgatc ttattgaatc catgttgaaa 900

aatctatcca ggattcatcc cgtgtcaaca atggtaaagg ggatgtatgg cattgagaat 960

gaagtcttcc tgagccttcc atgtatcctc aatgcccggg gattaaccag cgttatcaac 1020

cagaagctaa aggatgatga ggttgctcag ctcaagaaaa gtgcagatac cctgtgggac 1080

atccagaagg acctaaaaga cctgtgacta gtgagctcta ggctgtagaa atttaaaaac 1140

tacaatgtga ttaactcgag cctttagttt tcatccatgt acatggatca cagtttgctt 1200

tgatcttctt caatatgtga atttgggctc acagaatcaa agcctatgct tggtttaatg 1260

cttgcaatct gagctcttga acaaataaaa ttaactattg tagtgcgaaa 1310

<210> 27

<211> 679

<212> DNA

<213> Homo sapiens

<220>

<221> fatty acid binding protein 3

<222> (1)..(679)

<223> LocusID: 2170

fatty acid binding protein 3

<400> 27

gaattcggca cgaggtagct tctctcagcc tagcccagca tcactatggt ggacgctttc 60

ctgggcacct ggaagctagt ggacagcaag aatttcgatg actacatgaa gtcactcggt 120

gtgggttttg ctaccaggca ggtggccagc atgaccaagc ctaccacaat catcgaaaag 180

aatggggaca ttctcaccct aaaaacacac agcaccttca agaacacaga gatcagcttt 240

aagttggggg tggagttcga tgagacaaca gcagatgaca ggaaggtcaa gtccattgtg 300

acactggatg gagggaaact tgttcacctg cagaaatggg acgggcaaga gaccacactt 360

gtgcgggagc taattgatgg aaaactcatc ctgacactca cccacggcac tgcagtttgc 420

actcgcactt acgagaaaga ggcatgacct gactgcactg ttgctgacta ctactctgcc 480

aatcggctac ccctcgacta gcaccacatt gcctcatttc ttcctctgat tttgtacaaa 540

tccacgaatt cttctggggt caggtgccac tgaccgggat ccagttccag ttcccatggt 600

gtatgtggtt tttttttttt ttttttttaa ctgcactcat agggtgctct gaggtcaata 660

aagcagagcc aaggccacc 679

<210> 28

<211> 1807

<212> DNA

<213> Homo sapiens

<220>

<221> glycogenin

<222> (1)..(1807)

<223>  LocusID: 2992

NM_004130

<400>  28

ctctgagtca ccaacctgag gctgccccgg ccgcctgcgc acccggcagc accatgacag    60

atcaggcctt tgtgacacta accacaaacg atgcctacgc caaaggtgcc ctggtcctgg   120

gatcatctct gaaacagcac aggaccacca ggaggctggt cgtgctcgcc acccctcagg   180

tctcagactc catgagaaaa gttttagaga cagtctttga tgaagtcatc atggtagatg   240

tcttggacag tggcgattct gctcatctaa ccttaatgaa gaggccagag ttgggtgtca   300

cgctgacaaa gctccactgc tggtcgctta cacagtattc aaaatgtgta ttcatggatg   360

cagatactct ggtcctagca aatattgatg atctttttga cagagaagaa ttgtcagcag   42

caccagaccc agggtggcct gactgcttca attccggagt cttcgtttat cagccttcag   480

ttgaaacata caatcagctg ttgcatcttg cttctgagca aggtagtttt gatggtgggg   540

accaaggcat actgaacaca ttttttagca gctgggcaac aacagatatc agaaaacacc   600

tgccgtttat ttataaccta agcagcatct ctatatactc ctacctcccg gcatttaaag   660

tgtttggtgc aagtgccaaa gttgtgcatt tcctgggacg agtcaaacca tggaattata   720

cttatgatcc caaaacaaaa agtgtcaaaa gtgaggccca tgatcccaac atgactcatc   780

cagagtttct catcctgtgg tggaacatct ttaccaccaa cgttttacct ctgcttcaac   840

aatttggcct tgtcaaagac acctgctcat atgtaaatgt gctttcagac ttggtctata   900

cactggcttt ctcttgtggc ttctgtagaa aggaagatgt ctcaggagcc atatcacatc   960

tgtcccttgg ggagatccca gctatggcac agccgtttgt atcctcggaa gaacggaagg  1020

aacgatggga acagggccag gctgattata tgggagcaga ttcctttgac aacatcaaga  1080

ggaaacttga cacttacctc cagtagaaac actgcatttt tctgtgaaca catccacttc  1140

acaagccttg tttctgatac ttagtatcta gagctgggtt gagaaaagtc tgttacagtt 1200

gctagaggtt ttcattaaaa cttatcagat gagaggcttt tttaggataa gaggtgagaa 1260

ctgggcaaaa gttgtgaagc agcaattctg ttatatggac agtgttctgc tttttaatcc 1320

tatttagctt gtttcagaaa ttctcacttt tgttgactgc caacatacaa agtaagggaa 1380

actcaagata ttaagatggc tgtatcagtt cttaaaatct gcagagcctg gttcaaaatc 1440

agtcactccc ttcagaagca gacatggcat ctgttccttg cttgcttgtt ggttgtgtac 1500

ctttcacgag acctgaattt tagaattgcc cagtgctgcc agagtgagtg agtgtaattc 1560

tcctttcagg taaagatagg ctatctcaac actgctgagt gattcataaa catatcaacc 1620

aatagcatta acccatttta tttcctgtcc ttagtgtctg aagatgctca ccagttttct 1680

gtgtacagta aggcagcatg ctaaaatgct tttgttcagt tctggatatt tgaaaatagc 1740

agtgtgttct ctgatggtta cctgcagtgg caccctgtac aaaaaataaa agacttattg 1800

gtgtaaa 1807

<210> 29

<211> 3149

<212> DNA

<213> Homo sapiens

<220>

<221> fatty acid desaturase 2

<222> (1)..(3149)

<223> LocusID:9415

NM_004265

<400> 29

agggggcgcg gtgggaggag taggagaaga caaaagccga aagcgaagag ggcccgggct 60

gcacacaccg gctgggaggc agccgtctgt gcagcgagca gccggcgcgg ggaggccgca 120

gtgcacgggg cgtcacagtc ggcaggcagc atggggaagg gagggaacca gggcgagggg 180

gccgccgagc gcgaggtgtc ggtgcccacc ttcagctggg aggagattca gaagcataac 240

ctgcgcaccg acaggtggct ggtcattgac cgcaaggttt acaacatcac caaatggtcc 300

atccagcacc cggggggcca gcgggtcatc gggcactacg ctggagaaga tgcaacggat 360

gccttccgcg ccttccaccc tgacctggaa ttcgtgggca agttcttgaa acccctgctg 420

attggtgaac tggccccgga ggagcccagc caggaccacg gcaagaactc aaagatcact 480

gaggacttcc gggccctgag gaagacggct gaggacatga acctgttcaa gaccaaccac 540

gtgttcttcc tcctcctcct ggcccacatc atcgccctgg agagcattgc atggttcact 600

gtcttttact ttggcaatgg ctggattcct accctcatca cggcctttgt ccttgctacc 660

tctcaggccc aagctggatg gctgcaacat gattatggcc acctgtctgt ctacagaaaa 720

cccaagtgga accaccttgt ccacaaattc gtcattggcc acttaaaggg tgcctctgcc 780

aactggtgga atcatcgcca cttccagcac cacgccaagc ctaacatctt ccacaaggat 840

cccgatgtga acatgctgca cgtgtttgtt ctgggcgaat ggcagcccat cgagtacggc 900

aagaagaagc tgaaatacct gccctacaat caccagcacg aatacttctt cctgattggg 960

ccgccgctgc tcatccccat gtatttccag taccagatca tcatgaccat gatcgtccat 1020

aagaactggg tggacctggc ctgggccgtc agctactaca tccggttctt catcacctac 1080

atccctttct acggcatcct gggagccctc cttttcctca acttcatcag gttcctggag 1140

agccactggt ttgtgtgggt cacacagatg aatcacatcg tcatggagat tgaccaggag 1200

gcctaccgtg actggttcag tagccagctg acagccacct gcaacgtgga gcagtccttc 1260

ttcaacgact ggttcagtgg acaccttaac ttccagattg agcaccacct cttccccacc 1320

atgccccggc acaacttaca caagatcgcc ccgctggtga agtctctatg tgccaagcat 1380

ggcattgaat accaggagaa gccgctactg agggccctgc tggacatcat caggtccctg 1440

aagaagtctg ggaagctgtg gctggacgcc taccttcaca aatgaagcca cagcccccgg 1500

gacaccgtgg ggaaggggtg caggtggggt gatggccaga ggaatgatgg gcttttgttc 1560

tgaggggtgt ccgagaggct ggtgtatgca ctgctcacgg accccatgtt ggatctttct 1620

ccctttctcc tctccttttt ctcttcacat ctcccccata gcaccctgcc ctcatgggac 1680

ctgccctccc tcagccgtca gccatcagcc atggccctcc cagtgcctcc tagccccttc 1740

ttccaaggag cagagaggtg gccaccgggg gtggctctgt cctacctcca ctctctgccc 1800

ctaaagatgg gaggagacca gcggtccatg ggtctggcct gtgagtctcc ccttgcagcc 1860

tggtcactag gcatcacccc cgctttggtt cttcagatgc tcttggggtt cataggggca 1920

ggtcctagtc gggcagggcc cctgaccctc ccggcctggc ttcactctcc ctgacggctg 1980

ccattggtcc accctttcat agagaggcct gctttgttac aaagctcggg tctccctcct 2040

gcagctcggt taagtacccg aggcctctct taagatgtcc agggccccag gcccgcgggc 2100

acagccagcc caaaccttgg gccctggaag agtcctccac cccatcacta gagtgctctg 2160

accctgggct ttcacgggcc ccattccacc gcctccccaa cttgagcctg tgaccttggg 2220

accaaagggg gagtccctcg tctcttgtga ctcagcagag gcagtggcca cgttcaggga 2280

ggggccggct ggcctggagg ctcagcccac cctccagctt ttcctcaggg tgtcctgagg 2340

tccaagattc tggagcaatc tgacccttct ccaaaggctc tgttatcagc tgggcagtgc 2400

cagccaatcc ctggccattt ggccccaggg gacgtgggcc ctgcaggctg caggagggca 2460

ctggagctgg gaggtctcgt cccagccctc cccatctcgg ggctgctgtg tggacggcgc 2520

tgcctcaggc actctcctgt ctgaacctgc ccttactgtg tttaacctgt tgctccagga 2580

tgcattctga taggaggggg cggcagggct gggccttgtg acaatctgcc tttcaccaca 2640

tggccttgcc tcggtggccc tgactgtcag ggagggccag ggaggcagag cgggagggag 2700

tctcaggagg aggctgccct gaggggctgg ggagggggta cctcatgagg accagggtgg 2760

agctgagaag aggaggaggt gggggctgga ggtgctggta gctgagggga cgggcaagtg 2820

agaggggagg gagggaagtc ctgggaggat cctgagctgc tgttgcagtc taacccacta 2880

atcagttctt agattcaggg gaagggcagg caccaacaac tcagaatggg ggctttcggg 2940

gagggcgcct agtcccccca gctctaagca gccaggaggg acctgcatct aagcatctgg 3000

gttgccatgg caatggcatg ccccccagct actgtatgcc cccgaccccc gcagaggcag 3060

aatgaaccca tagggagctg atcgtaatgt ttatcatgtt acttccccac ccctacattt 3120

tttgaaataa aataaggaat tttattctc 3149

<210> 30

<211> 1996

<212> DNA

<213> Homo sapiens

<220>

<221> RAR-related orphan receptor A variant 3

<222> (1)..(1996)

<223> LocusID: 6095

NM_002943

<400> 30

gcagattcac agggcctctg agcattatcc cccatactcc tccccatcat tctccaccca 60

gctgttggag ccatctgtct gatcaccttg gactccatag tacactgggg caaagcacag 120

ccccagtttc tggaggcaga tgggtaacca ggaaaaggca tgaatgaggg ggccccagga 180

gacagtgact tagagactga ggcaagagtg ccgtggtcaa tcatgggtca ttgtcttcga 240

actggacagg ccagaatgtc tgccacaccc acacctgcag gtgaaggagc cagaagctct 300

tcaacctgta gctccctgag caggctgttc tggtctcaac ttgagcacat aaactgggat 360

ggagccacag ccaagaactt tattaattta agggagttct tctcttttct gctccctgca 420

ttgagaaaag ctcaaattga aattattcca tgcaagatct gtggagacaa atcatcagga 480

atccattatg gtgtcattac atgtgaaggc tgcaagggct ttttcaggag aagtcagcaa 540

agcaatgcca cctactcctg tcctcgtcag aagaactgtt tgattgatcg aaccagtaga 600

aaccgctgcc aacactgtcg attacagaaa tgccttgccg tagggatgtc tcgagatgct 660

gtaaaatttg gccgaatgtc aaaaaagcag agagacagct tgtatgcaga agtacagaaa 720

caccggatgc agcagcagca gcgcgaccac cagcagcagc ctggagaggc tgagccgctg 780

acgcccacct acaacatctc ggccaacggg ctgacggaac ttcacgacga cctcagtaac 840

tacattgacg ggcacacccc tgaggggagt aaggcagact ccgccgtcag cagcttctac 900

ctggacatac agccttcccc agaccagtca ggtcttgata tcaatggaat caaaccagaa 960

ccaatatgtg actacacacc agcatcaggc ttctttccct actgttcgtt caccaacggc 1020

gagacttccc caactgtgtc catggcagaa ttagaacacc ttgcacagaa tatatctaaa 1080

tcgcatctgg aaacctgcca atacttgaga gaagagctcc agcagataac gtggcagacc 1140

tttttacagg aagaaattga gaactatcaa aacaagcagc gggaggtgat gtggcaattg 1200

tgtgccatca aaattacaga agctatacag tatgtggtgg agtttgccaa acgcattgat 1260

ggatttatgg aactgtgtca aaatgatcaa attgtgcttc taaaagcagg ttctctagag 1320

gtggtgttta tcagaatgtg ccgtgccttt gactctcaga acaacaccgt gtactttgat 1380

gggaagtatg ccagccccga cgtcttcaaa tccttaggtt gtgaagactt tattagcttt 1440

gtgtttgaat ttggaaagag tttatgttct atgcacctga ctgaagatga aattgcatta 1500

ttttctgcat ttgtactgat gtcagcagat cgctcatggc tgcaagaaaa ggtaaaaatt 1560

gaaaaactgc aacagaaaat tcagctagct cttcaacacg tcctacagaa gaatcaccga 1620

gaagatggaa tactaacaaa gttaatatgc aaggtgtcta cattaagagc cttatgtgga 1680

cgacatacag aaaagctaat ggcatttaaa gcaatatacc cagacattgt gcgacttcat 1740

tttcctccat tatacaagga gttgttcact tcagaatttg agccagcaat gcaaattgat 1800

gggtaaatgt tatcacctaa gcacttctag aatgtctgaa gtacaaacat gaaaaacaaa 1860

caaaaaaatt aaccgagaca ctttatatgg ccctgcacag acctggagcg ccacacactg 1920

cacatctttt ggtgatcggg gtcaggcaaa ggaggggaaa caatgaaaac aaataaagtt 1980

gaacttgttt ttctca 1996

<210> 31

<211> 2020

<212> DNA

<213> Homo sapiens

<220>

<221> RAR-related orphan receptor A variant 2

<222> (1)..(2020)

<223> LocusID: 6095

NM_134260

<400> 31

gcagattcac agggcctctg agcattatcc cccatactcc tccccatcat tctccaccca 60

gctgttggag ccatctgtct gatcaccttg gactccatag tacactgggg caaagcacag 120

ccccagtttc tggaggcaga tgggtaacca ggaaaaggca tgaatgaggg ggccccagga 180

gacagtgact tagagactga ggcaagagtg ccgtggtcaa tcatgggtca ttgtcttcga 240

actggacagg ccagaatgtc tgccacaccc acacctgcag gtgaaggagc cagaagggat 300

gaactttttg ggattctcca aatactccat cagtgtatcc tgtcttcagg tgatgctttt 360

gttcttactg gcgtctgttg ttcctggagg cagaatggca agccaccata ttcacaaaag 420

gaagataagg aagtacaaac tggatacatg aatgctcaaa ttgaaattat tccatgcaag 480

atctgtggag acaaatcatc aggaatccat tatggtgtca ttacatgtga aggctgcaag 540

ggctttttca ggagaagtca gcaaagcaat gccacctact cctgtcctcg tcagaagaac 600

tgtttgattg atcgaaccag tagaaaccgc tgccaacact gtcgattaca gaaatgcctt 660

gccgtaggga tgtctcgaga tgctgtaaaa tttggccgaa tgtcaaaaaa gcagagagac 720

agcttgtatg cagaagtaca gaaacaccgg atgcagcagc agcagcgcga ccaccagcag 780

cagcctggag aggctgagcc gctgacgccc acctacaaca tctcggccaa cgggctgacg 840

gaacttcacg acgacctcag taactacatt gacgggcaca cccctgaggg gagtaaggca 900

gactccgccg tcagcagctt ctacctggac atacagcctt ccccagacca gtcaggtctt 960

gatatcaatg gaatcaaacc agaaccaata tgtgactaca caccagcatc aggcttcttt 1020

ccctactgtt cgttcaccaa cggcgagact tccccaactg tgtccatggc agaattagaa 1080

caccttgcac agaatatatc taaatcgcat ctggaaacct gccaatactt gagagaagag 1140

ctccagcaga taacgtggca gaccttttta caggaagaaa ttgagaacta tcaaaacaag 1200

cagcgggagg tgatgtggca attgtgtgcc atcaaaatta cagaagctat acagtatgtg 1260

gtggagtttg ccaaacgcat tgatggattt atggaactgt gtcaaaatga tcaaattgtg 1320

cttctaaaag caggttctct agaggtggtg tttatcagaa tgtgccgtgc ctttgactct 1380

cagaacaaca ccgtgtactt tgatgggaag tatgccagcc ccgacgtctt caaatcctta 1440

ggttgtgaag actttattag ctttgtgttt gaatttggaa agagtttatg ttctatgcac 1500

ctgactgaag atgaaattgc attattttct gcatttgtac tgatgtcagc agatcgctca 1560

tggctgcaag aaaaggtaaa aattgaaaaa ctgcaacaga aaattcagct agctcttcaa 1620

cacgtcctac agaagaatca ccgagaagat ggaatactaa caaagttaat atgcaaggtg 1680

tctacattaa gagccttatg tggacgacat acagaaaagc taatggcatt taaagcaata 1740

tacccagaca ttgtgcgact tcattttcct ccattataca aggagttgtt cacttcagaa 1800

tttgagccag caatgcaaat tgatgggtaa atgttatcac ctaagcactt ctagaatgtc 1860

tgaagtacaa acatgaaaaa caaacaaaaa aattaaccga gacactttat atggccctgc 1920

acagacctgg agcgccacac actgcacatc ttttggtgat cggggtcagg caaaggaggg 1980

gaaacaatga aaacaaataa agttgaactt gtttttctca 2020

<210> 32

<211> 1847

<212> DNA

<213> Homo sapiens

<220>

<221> RAR-related orphan receptor A variant 1

<222> (1)..(1847)

<223> LocusID: 6095

NM_134261

<400> 32

ggtaccatag agttgctctg aaaacagaag atagagggag tctcggagct cgccatctcc 60

agcgatctct acattgggaa aaaacatgga gtcagctccg gcagcccccg accccgccgc 120

cagcgagcca ggcagcagcg gcgcggacgc ggccgccggc tccagggaga ccccgctgaa 180

ccaggaatcc gcccgcaaga gcgagccgcc tgccccggtg cgcagacaga gctattccag 240

caccagcaga ggtatctcag taacgaagaa gacacataca tctcaaattg aaattattcc 300

atgcaagatc tgtggagaca aatcatcagg aatccattat ggtgtcatta catgtgaagg 360

ctgcaagggc tttttcagga gaagtcagca aagcaatgcc acctactcct gtcctcgtca 420

gaagaactgt ttgattgatc gaaccagtag aaaccgctgc caacactgtc gattacagaa 480

atgccttgcc gtagggatgt ctcgagatgc tgtaaaattt ggccgaatgt caaaaaagca 540

gagagacagc ttgtatgcag aagtacagaa acaccggatg cagcagcagc agcgcgacca 600

ccagcagcag cctggagagg ctgagccgct gacgcccacc tacaacatct cggccaacgg 660

gctgacggaa cttcacgacg acctcagtaa ctacattgac gggcacaccc ctgaggggag 720

taaggcagac tccgccgtca gcagcttcta cctggacata cagccttccc cagaccagtc 780

aggtcttgat atcaatggaa tcaaaccaga accaatatgt gactacacac cagcatcagg 840

cttctttccc tactgttcgt tcaccaacgg cgagacttcc ccaactgtgt ccatggcaga 900

attagaacac cttgcacaga atatatctaa atcgcatctg gaaacctgcc aatacttgag 960

agaagagctc cagcagataa cgtggcagac ctttttacag gaagaaattg agaactatca 1020

aaacaagcag cgggaggtga tgtggcaatt gtgtgccatc aaaattacag aagctataca 1080

gtatgtggtg gagtttgcca aacgcattga tggatttatg gaactgtgtc aaaatgatca 1140

aattgtgctt ctaaaagcag gttctctaga ggtggtgttt atcagaatgt gccgtgcctt 1200

tgactctcag aacaacaccg tgtactttga tgggaagtat gccagccccg acgtcttcaa 1260

atccttaggt tgtgaagact ttattagctt tgtgtttgaa tttggaaaga gtttatgttc 1320

tatgcacctg actgaagatg aaattgcatt attttctgca tttgtactga tgtcagcaga 1380

tcgctcatgg ctgcaagaaa aggtaaaaat tgaaaaactg caacagaaaa ttcagctagc 1440

tcttcaacac gtcctacaga agaatcaccg agaagatgga atactaacaa agttaatatg 1500

caaggtgtct acattaagag ccttatgtgg acgacataca gaaaagctaa tggcatttaa 1560

agcaatatac ccagacattg tgcgacttca ttttcctcca ttatacaagg agttgttcac 1620

ttcagaattt gagccagcaa tgcaaattga tgggtaaatg ttatcaccta agcacttcta 1680

gaatgtctga agtacaaaca tgaaaaacaa acaaaaaaat taaccgagac actttatatg 1740

gccctgcaca gacctggagc gccacacact gcacatcttt tggtgatcgg ggtcaggcaa 1800

aggaggggaa acaatgaaaa caaataaagt tgaacttgtt tttctca 1847

<210> 33

<211> 1687

<212> DNA

<213> Homo sapiens

<220>

<221> RAR-related orphan receptor A variant 4

<222> (1)..(1687)

<223> LocusID:6095

NM_134262

<400> 33

tgtggctcgg gcggcggcgg cgcggcggcg gcagaggggg ctccggggtc ggaccatccg 60

ctctccctgc gctctccgca ccgcgcttaa atgatgtatt ttgtgatcgc agcgatgaaa 120

gctcaaattg aaattattcc atgcaagatc tgtggagaca aatcatcagg aatccattat 180

ggtgtcatta catgtgaagg ctgcaagggc tttttcagga gaagtcagca aagcaatgcc 240

acctactcct gtcctcgtca gaagaactgt ttgattgatc gaaccagtag aaaccgctgc 300

caacactgtc gattacagaa atgccttgcc gtagggatgt ctcgagatgc tgtaaaattt 360

ggccgaatgt caaaaaagca gagagacagc ttgtatgcag aagtacagaa acaccggatg 420

cagcagcagc agcgcgacca ccagcagcag cctggagagg ctgagccgct gacgcccacc 480

tacaacatct cggccaacgg gctgacggaa cttcacgacg acctcagtaa ctacattgac 540

gggcacaccc ctgaggggag taaggcagac tccgccgtca gcagcttcta cctggacata 600

cagccttccc cagaccagtc aggtcttgat atcaatggaa tcaaaccaga accaatatgt 660

gactacacac cagcatcagg cttctttccc tactgttcgt tcaccaacgg cgagacttcc 720

ccaactgtgt ccatggcaga attagaacac cttgcacaga atatatctaa atcgcatctg 780

gaaacctgcc aatacttgag agaagagctc cagcagataa cgtggcagac ctttttacag 840

gaagaaattg agaactatca aaacaagcag cgggaggtga tgtggcaatt gtgtgccatc 900

aaaattacag aagctataca gtatgtggtg gagtttgcca aacgcattga tggatttatg 960

gaactgtgtc aaaatgatca aattgtgctt ctaaaagcag gttctctaga ggtggtgttt 1020

atcagaatgt gccgtgcctt tgactctcag aacaacaccg tgtactttga tgggaagtat 1080

gccagccccg acgtcttcaa atccttaggt tgtgaagact ttattagctt tgtgtttgaa 1140

tttggaaaga gtttatgttc tatgcacctg actgaagatg aaattgcatt attttctgca 1200

tttgtactga tgtcagcaga tcgctcatgg ctgcaagaaa aggtaaaaat tgaaaaactg 1260

caacagaaaa ttcagctagc tcttcaacac gtcctacaga agaatcaccg agaagatgga 1320

atactaacaa agttaatatg caaggtgtct acattaagag ccttatgtgg acgacataca 1380

gaaaagctaa tggcatttaa agcaatatac ccagacattg tgcgacttca ttttcctcca 1440

ttatacaagg agttgttcac ttcagaattt gagccagcaa tgcaaattga tgggtaaatg 1500

ttatcaccta agcacttcta gaatgtctga agtacaaaca tgaaaaacaa acaaaaaaat 1560

taaccgagac actttatatg gccctgcaca gacctggagc gccacacact gcacatcttt 1620

tggtgatcgg ggtcaggcaa aggaggggaa acaatgaaaa caaataaagt tgaacttgtt 1680

tttctca 1687

<210> 34

<211> 2760

<212> DNA

<213> Homo sapiens

<220>

<221> phosphofructokinase

<222> (1)..(2760)

<223> LocusID: 5213

NM_000289

<400> 34

ctaaaagagt ggatcatgac ccatgaagag caccatgcag ccaaaaccct ggggattggc 60

aaagccattg ctgtcttaac ctctggtgga gatgcccaag gtatgaatgc tgctgtcagg 120

gctgtggttc gagttggtat cttcaccggt gcccgtgtct tctttgtcca tgagggttat 180

caaggcctgg tggatggtgg agatcacatc aaggaagcca cctgggagag cgtttcgatg 240

atgcttcagc tgggaggcac ggtgattgga agtgcccggt gcaaggactt tcgggaacga 300

gaaggacgac tccgagctgc ctacaacctg gtgaagcgtg ggatcaccaa tctctgtgtc 360

attgggggtg atggcagcct cactggggct gacaccttcc gttctgagtg gagtgacttg 420

ttgagtgacc tccagaaagc aggtaagatc acagatgagg aggctacgaa gtccagctac 480

ctgaacattg tgggcctggt tgggtcaatt gacaatgact tctgtggcac cgatatgacc 540

attggcactg actctgccct gcatcggatc atggaaattg tagatgccat cactaccact 600

gcccagagcc accagaggac atttgtgtta gaagtaatgg gccgccactg tggatacctg 660

gcccttgtca cctctctgtc ctgtggggcc gactgggttt ttattcctga atgtccacca 720

gatgacgact gggaggaaca cctttgtcgc cgactcagcg agacaaggac ccgtggttct 780

cgtctcaaca tcatcattgt ggctgagggt gcaattgaca agaatggaaa accaatcacc 840

tcagaagaca tcaagaatct ggtggttaag cgtctgggat atgacacccg ggttactgtc 900

ttggggcatg tgcagagggg tgggacgcca tcagcctttg acagaattct gggcagcagg 960

atgggtgtgg aagcagtgat ggcacttttg gaggggaccc cagatacccc agcctgtgta 1020

gtgagcctct ctggtaacca ggctgtgcgc ctgcccctca tggaatgtgt ccaggtgacc 1080

aaagatgtga ccaaggccat ggatgagaag aaatttgacg aagccctgaa gctgagaggc 1140

cggagcttca tgaacaactg ggaggtgtac aagcttctag ctcatgtcag acccccggta 1200

tctaagagtg gttcgcacac agtggctgtg atgaacgtgg gggctccggc tgcaggcatg 1260

aatgctgctg ttcgctccac tgtgaggatt ggccttatcc agggcaaccg agtgctcgtt 1320

gtccatgatg gtttcgaggg cctggccaag gggcagatag aggaagctgg ctggagctat 1380

gttgggggct ggactggcca aggtggctct aaacttggga ctaaaaggac tctacccaag 1440

aagagctttg aacagatcag tgccaatata actaagttta acattcaggg ccttgtcatc 1500

attgggggct ttgaggctta cacagggggc ctggaactga tggagggcag gaagcagttt 1560

gatgagctct gcatcccatt tgtggtcatt cctgctacag tctccaacaa tgtccctggc 1620

tcagacttca gcgttggggc tgacacagca ctcaatacta tctgcacaac ctgtgaccgc 1680

atcaagcagt cagcagctgg caccaagcgt cgggtgttta tcattgagac tatgggtggc 1740

tactgtggct acctggctac catggctgga ctggcagctg gggccgatgc tgcctacatt 1800

tttgaggagc ccttcaccat tcgagacctg caggcaaatg ttgaacatct ggtgcaaaag 1860

atgaaaacaa ctgtgaaaag gggcttggtg ttaaggaatg aaaagtgcaa tgagaactat 1920

accactgact tcattttcaa cctgtactct gaggagggga agggcatctt cgacagcagg 1980

aagaatgtgc ttggtcacat gcagcagggt gggagcccaa ccccatttga taggaatttt 2040

gccactaaga tgggcgccaa ggctatgaac tggatgtctg ggaaaatcaa agagagttac 2100

cgtaatgggc ggatctttgc caatactcca gattcgggct gtgttctggg gatgcgtaag 2160

agggctctgg tcttccaacc agtggctgag ctgaaggacc agacagattt tgagcatcga 2220

atccccaagg aacagtggtg gctgaaactg aggcccatcc tcaaaatcct agccaagtac 2280

gagattgact tggacacttc agaccatgcc cacctggagc acatcacccg gaagcggtcc 2340

ggggaagctg ccgtctaaac ctctctggag tgaggggaat agattacctg atcatggtca 2400

gctcacaccc taataagtcc acatcttctc agtgttttag ctgttttttt cattaggttt 2460

ccttttattc tgtaccttgc agccatgacc agttctggcc aggagctgga ggagcaggca 2520

gtgggtggga gctcctttta ggtagaattt aacatgactt ctgccccagc tttatctgtc 2580

acacaaggct gggcacctct agtgctactg ctagatatca cttactcagt tagaattttc 2640

ctaaaaataa gctttattta tttctttgtg ataacaaaga gtcttggttc ctctactact 2700

tttactacag tgacaaattg taactacact aataaatgcc aactggtcac tgtgaaaaaa 2760

<210> 35

<211> 3316

<212> DNA

<213> Homo sapiens

<220>

<221> calpain 3, (p94) (CAPN3), transcript variant 1

<222> (1)..(3316)

<223> LocusID:825

NM_000070

<400> 35

cactctcttt ctctctccct ctggcatgca tgctgctggt aggagacccc caagtcaaca 60

ttgcttcaga aatcctttag cactcatttc tcaggagaac ttatggcttc agaatcacag 120

ctcggttttt aagatggaca taacctgtac gaccttctga tgggctttca actttgaact 180

ggatgtggac acttttctct cagatgacag aattactcca acttcccctt tgcagttgct 240

tcctttcctt gaaggtagct gtatcttatt ttctttaaaa agctttttct tccaaagcca 300

cttgccatgc cgaccgtcat tagcgcatct gtggctccaa ggacagcggc tgagccccgg 360

tccccagggc cagttcctca cccggcccag agcaaggcca ctgaggctgg gggtggaaac 420

ccaagtggca tctattcagc catcatcagc cgcaattttc ctattatcgg agtgaaagag 480

aagacattcg agcaacttca caagaaatgt ctagaaaaga aagttcttta tgtggaccct 540

gagttcccac cggatgagac ctctctcttt tatagccaga agttccccat ccagttcgtc 600

tggaagagac ctccggaaat ttgcgagaat ccccgattta tcattgatgg agccaacaga 660

actgacatct gtcaaggaga gctaggggac tgctggtttc tcgcagccat tgcctgcctg 720

accctgaacc agcaccttct tttccgagtc ataccccatg atcaaagttt catcgaaaac 780

tacgcaggga tcttccactt ccagttctgg cgctatggag agtgggtgga cgtggttata 840

gatgactgcc tgccaacgta caacaatcaa ctggttttca ccaagtccaa ccaccgcaat 900

gagttctgga gtgctctgct ggagaaggct tatgctaagc tccatggttc ctacgaagct 960

ctgaaaggtg ggaacaccac agaggccatg gaggacttca caggaggggt ggcagagttt 1020

tttgagatca gggatgctcc tagtgacatg tacaagatca tgaagaaagc catcgagaga 1080

ggctccctca tgggctgctc cattgatgat ggcacgaaca tgacctatgg aacctctcct 1140

tctggtctga acatggggga gttgattgca cggatggtaa ggaatatgga taactcactg 1200

ctccaggact cagacctcga ccccagaggc tcagatgaaa gaccgacccg gacaatcatt 1260

ccggttcagt atgagacaag aatggcctgc gggctggtca gaggtcacgc ctactctgtc 1320

acggggctgg atgaggtccc gttcaaaggt gagaaagtga agctggtgcg gctgcggaat 1380

ccgtggggcc aggtggagtg gaacggttct tggagtgata gatggaagga ctggagcttt 1440

gtggacaaag atgagaaggc ccgtctgcag caccaggtca ctgaggatgg agagttctgg 1500

atgtcctatg aggatttcat ctaccatttc acaaagttgg agatctgcaa cctcacggcc 1560

gatgctctgc agtctgacaa gcttcagacc tggacagtgt ctgtgaacga gggccgctgg 1620

gtacggggtt gctctgccgg aggctgccgc aacttcccag atactttctg gaccaaccct 1680

cagtaccgtc tgaagctcct ggaggaggac gatgaccctg atgactcgga ggtgatttgc 1740

agcttcctgg tggccctgat gcagaagaac cggcggaagg accggaagct aggggccagt 1800

ctcttcacca ttggcttcgc catctacgag gttcccaaag agatgcacgg gaacaagcag 1860

cacctgcaga aggacttctt cctgtacaac gcctccaagg ccaggagcaa aacctacatc 1920

aacatgcggg aggtgtccca gcgcttccgc ctgcctccca gcgagtacgt catcgtgccc 1980

tccacctacg agccccacca ggagggggaa ttcatcctcc gggtcttctc tgaaaagagg 2040

aacctctctg aggaagttga aaataccatc tccgtggatc ggccagtgaa aaagaaaaaa 2100

accaagccca tcatcttcgt ttcggacaga gcaaacagca acaaggagct gggtgtggac 2160

caggagtcag aggagggcaa aggcaaaaca agccctgata agcaaaagca gtccccacag 2220

ccacagcctg gcagctctga tcaggaaagt gaggaacagc aacaattccg gaacattttc 2280

aagcagatag caggagatga catggagatc tgtgcagatg agctcaagaa ggtccttaac 2340

acagtcgtga acaaacacaa ggacctgaag acacacgggt tcacactgga gtcctgccgt 2400

agcatgattg cgctcatgga tacagatggc tctggaaagc tcaacctgca ggagttccac 2460

cacctctgga acaagattaa ggcctggcag aaaattttca aacactatga cacagaccag 2520

tccggcacca tcaacagcta cgagatgcga aatgcagtca acgacgcagg attccacctc 2580

aacaaccagc tctatgacat cattaccatg cggtacgcag acaaacacat gaacatcgac 2640

tttgacagtt tcatctgctg cttcgttagg ctggagggca tgttcagagc ttttcatgca 2700

tttgacaagg atggagatgg tatcatcaag ctcaacgttc tggagtggct gcagctcacc 2760

atgtatgcct gaaccaggct ggcctcatcc aaagccatgc aggatcactc aggatttcag 2820

tttcaccctc tatttccaaa gccatttacc tcaaaggacc cagcagctac acccctacag 2880

gcttccaggc acctcatcag tcatgctcct cctccatttt accccctacc catccttgat 2940

cggtcatgcc tagcctgacc ctttagtaaa gcaatgaggt aggaagaaca aacccttgtc 3000

cctttgccat gtggaggaaa gtgcctgcct ctggtccgag ccgcctcggt tctgaagcga 3060

gtgctcctgc ttaccttgct ctaggctgtc tgcagaagca cctgccggtg gcactcagca 3120

cctccttgtg ctagagccct ccatcacctt cacgctgtcc caccatgggc caggaaccaa 3180

accagcactg ggttctactg ctgtggggta aactaactca gtggaatagg gctggttact 3240

ttgggctgtc caactcataa gtttggctgc attttgaaaa aagctgatct aaataaaggc 3300

atgtgtatgg ctggtc 3316

<210> 36

<211> 3298

<212> DNA

<213> Homo sapiens

<220>

<221> calpain 3, (p94) (CAPN3), transcript variant 2

<222> (1)..(3298)

<223> LocusID:825

NM_024344

<400> 36

cactctcttt ctctctccct ctggcatgca tgctgctggt aggagacccc caagtcaaca 60

ttgcttcaga aatcctttag cactcatttc tcaggagaac ttatggcttc agaatcacag 120

ctcggttttt aagatggaca taacctgtac gaccttctga tgggctttca actttgaact 180

ggatgtggac acttttctct cagatgacag aattactcca acttcccctt tgcagttgct 240

tcctttcctt gaaggtagct gtatcttatt ttctttaaaa agctttttct tccaaagcca 300

cttgccatgc cgaccgtcat tagcgcatct gtggctccaa ggacagcggc tgagccccgg 360

tccccagggc cagttcctca cccggcccag agcaaggcca ctgaggctgg gggtggaaac 420

ccaagtggca tctattcagc catcatcagc cgcaattttc ctattatcgg agtgaaagag 480

aagacattcg agcaacttca caagaaatgt ctagaaaaga aagttcttta tgtggaccct 540

gagttcccac cggatgagac ctctctcttt tatagccaga agttccccat ccagttcgtc 600

tggaagagac ctccggaaat ttgcgagaat ccccgattta tcattgatgg agccaacaga 660

actgacatct gtcaaggaga gctaggggac tgctggtttc tcgcagccat tgcctgcctg 720

accctgaacc agcaccttct tttccgagtc ataccccatg atcaaagttt catcgaaaac 780

tacgcaggga tcttccactt ccagttctgg cgctatggag agtgggtgga cgtggttata 840

gatgactgcc tgccaacgta caacaatcaa ctggttttca ccaagtccaa ccaccgcaat 900

gagttctgga gtgctctgct ggagaaggct tatgctaagc tccatggttc ctacgaagct 960

ctgaaaggtg ggaacaccac agaggccatg gaggacttca caggaggggt ggcagagttt 1020

tttgagatca gggatgctcc tagtgacatg tacaagatca tgaagaaagc catcgagaga 1080

ggctccctca tgggctgctc cattgatgat ggcacgaaca tgacctatgg aacctctcct 1140

tctggtctga acatggggga gttgattgca cggatggtaa ggaatatgga taactcactg 1200

ctccaggact cagacctcga ccccagaggc tcagatgaaa gaccgacccg gacaatcatt 1260

ccggttcagt atgagacaag aatggcctgc gggctggtca gaggtcacgc ctactctgtc 1320

acggggctgg atgaggtccc gttcaaaggt gagaaagtga agctggtgcg gctgcggaat 1380

ccgtggggcc aggtggagtg gaacggttct tggagtgata gatggaagga ctggagcttt 1440

gtggacaaag atgagaaggc ccgtctgcag caccaggtca ctgaggatgg agagttctgg 1500

atgtcctatg aggatttcat ctaccatttc acaaagttgg agatctgcaa cctcacggcc 1560

gatgctctgc agtctgacaa gcttcagacc tggacagtgt ctgtgaacga gggccgctgg 1620

gtacggggtt gctctgccgg aggctgccgc aacttcccag atactttctg gaccaaccct 1680

cagtaccgtc tgaagctcct ggaggaggac gatgaccctg atgactcgga ggtgatttgc 1740

agcttcctgg tggccctgat gcagaagaac cggcggaagg accggaagct aggggccagt 1800

ctcttcacca ttggcttcgc catctacgag gttcccaaag agatgcacgg gaacaagcag 1860

cacctgcaga aggacttctt cctgtacaac gcctccaagg ccaggagcaa aacctacatc 1920

aacatgcggg aggtgtccca gcgcttccgc ctgcctccca gcgagtacgt catcgtgccc 1980

tccacctacg agccccacca ggagggggaa ttcatcctcc gggtcttctc tgaaaagagg 2040

aacctctctg aggaagttga aaataccatc tccgtggatc ggccagtgcc catcatcttc 2100

gtttcggaca gagcaaacag caacaaggag ctgggtgtgg accaggagtc agaggagggc 2160

aaaggcaaaa caagccctga taagcaaaag cagtccccac agccacagcc tggcagctct 2220

gatcaggaaa gtgaggaaca gcaacaattc cggaacattt tcaagcagat agcaggagat 2280

gacatggaga tctgtgcaga tgagctcaag aaggtcctta acacagtcgt gaacaaacac 2340

aaggacctga agacacacgg gttcacactg gagtcctgcc gtagcatgat tgcgctcatg 2400

gatacagatg gctctggaaa gctcaacctg caggagttcc accacctctg gaacaagatt 2460

aaggcctggc agaaaatttt caaacactat gacacagacc agtccggcac catcaacagc 2520

tacgagatgc gaaatgcagt caacgacgca ggattccacc tcaacaacca gctctatgac 2580

atcattacca tgcggtacgc agacaaacac atgaacatcg actttgacag tttcatctgc 2640

tgcttcgtta ggctggaggg catgttcaga gcttttcatg catttgacaa ggatggagat 2700

ggtatcatca agctcaacgt tctggagtgg ctgcagctca ccatgtatgc ctgaaccagg 2760

ctggcctcat ccaaagccat gcaggatcac tcaggatttc agtttcaccc tctatttcca 2820

aagccattta cctcaaagga cccagcagct acacccctac aggcttccag gcacctcatc 2880

agtcatgctc ctcctccatt ttacccccta cccatccttg atcggtcatg cctagcctga 2940

ccctttagta aagcaatgag gtaggaagaa caaacccttg tccctttgcc atgtggagga 3000

aagtgcctgc ctctggtccg agccgcctcg gttctgaagc gagtgctcct gcttaccttg 3060

ctctaggctg tctgcagaag cacctgccgg tggcactcag cacctccttg tgctagagcc 3120

ctccatcacc ttcacgctgt cccaccatgg gccaggaacc aaaccagcac tgggttctac 3180

tgctgtgggg taaactaact cagtggaata gggctggtta ctttgggctg tccaactcat 3240

aagtttggct gcattttgaa aaaagctgat ctaaataaag gcatgtgtat ggctggtc 3298

<210> 37

<211> 3040

<212> DNA

<213> Homo sapiens

<220>

<221> calpain 3, (p94) (CAPN3), transcript variant 3

<222> (1)..(3040)

<223> LocusID: 825

NM_173087

<400> 37

cactctcttt ctctctccct ctggcatgca tgctgctggt aggagacccc caagtcaaca 60

ttgcttcaga aatcctttag cactcatttc tcaggagaac ttatggcttc agaatcacag 120

ctcggttttt aagatggaca taacctgtac gaccttctga tgggctttca actttgaact 180

ggatgtggac acttttctct cagatgacag aattactcca acttcccctt tgcagttgct 240

tcctttcctt gaaggtagct gtatcttatt ttctttaaaa agctttttct tccaaagcca 300

cttgccatgc cgaccgtcat tagcgcatct gtggctccaa ggacagcggc tgagccccgg 360

tccccagggc cagttcctca cccggcccag agcaaggcca ctgaggctgg gggtggaaac 420

ccaagtggca tctattcagc catcatcagc cgcaattttc ctattatcgg agtgaaagag 480

aagacattcg agcaacttca caagaaatgt ctagaaaaga aagttcttta tgtggaccct 540

gagttcccac cggatgagac ctctctcttt tatagccaga agttccccat ccagttcgtc 600

tggaagagac ctccggaaat ttgcgagaat ccccgattta tcattgatgg agccaacaga 660

actgacatct gtcaaggaga gctaggggac tgctggtttc tcgcagccat tgcctgcctg 720

accctgaacc agcaccttct tttccgagtc ataccccatg atcaaagttt catcgaaaac 780

tacgcaggga tcttccactt ccagttctgg cgctatggag agtgggtgga cgtggttata 840

gatgactgcc tgccaacgta caacaatcaa ctggttttca ccaagtccaa ccaccgcaat 900

gagttctgga gtgctctgct ggagaaggct tatgctaagc tccatggttc ctacgaagct 960

ctgaaaggtg ggaacaccac agaggccatg gaggacttca caggaggggt ggcagagttt 1020

tttgagatca gggatgctcc tagtgacatg tacaagatca tgaagaaagc catcgagaga 1080

ggctccctca tgggctgctc cattgataca atcattccgg ttcagtatga gacaagaatg 1140

gcctgcgggc tggtcagagg tcacgcctac tctgtcacgg ggctggatga ggtcccgttc 1200

aaaggtgaga aagtgaagct ggtgcggctg cggaatccgt ggggccaggt ggagtggaac 1260

ggttcttgga gtgatagatg gaaggactgg agctttgtgg acaaagatga gaaggcccgt 1320

ctgcagcacc aggtcactga ggatggagag ttctggatgt cctatgagga tttcatctac 1380

catttcacaa agttggagat ctgcaacctc acggccgatg ctctgcagtc tgacaagctt 1440

cagacctgga cagtgtctgt gaacgagggc cgctgggtac ggggttgctc tgccggaggc 1500

tgccgcaact tcccagatac tttctggacc aaccctcagt accgtctgaa gctcctggag 1560

gaggacgatg accctgatga ctcggaggtg atttgcagct tcctggtggc cctgatgcag 1620

aagaaccggc ggaaggaccg gaagctaggg gccagtctct tcaccattgg cttcgccatc 1680

tacgaggttc ccaaagagat gcacgggaac aagcagcacc tgcagaagga cttcttcctg 1740

tacaacgcct ccaaggccag gagcaaaacc tacatcaaca tgcgggaggt gtcccagcgc 1800

ttccgcctgc ctcccagcga gtacgtcatc gtgccctcca cctacgagcc ccaccaggag 1860

ggggaattca tcctccgggt cttctctgaa aagaggaacc tctctgagga agttgaaaat 1920

accatctccg tggatcggcc agtgccacag cctggcagct ctgatcagga aagtgaggaa 1980

cagcaacaat tccggaacat tttcaagcag atagcaggag atgacatgga gatctgtgca 2040

gatgagctca agaaggtcct taacacagtc gtgaacaaac acaaggacct gaagacacac 2100

gggttcacac tggagtcctg ccgtagcatg attgcgctca tggatacaga tggctctgga 2160

aagctcaacc tgcaggagtt ccaccacctc tggaacaaga ttaaggcctg gcagaaaatt 2220

ttcaaacact atgacacaga ccagtccggc accatcaaca gctacgagat gcgaaatgca 2280

gtcaacgacg caggattcca cctcaacaac cagctctatg acatcattac catgcggtac 2340

gcagacaaac acatgaacat cgactttgac agtttcatct gctgcttcgt taggctggag 2400

ggcatgttca gagcttttca tgcatttgac aaggatggag atggtatcat caagctcaac 2460

gttctggagt ggctgcagct caccatgtat gcctgaacca ggctggcctc atccaaagcc 2520

atgcaggatc actcaggatt tcagtttcac cctctatttc caaagccatt tacctcaaag 2580

gacccagcag ctacacccct acaggcttcc aggcacctca tcagtcatgc tcctcctcca 2640

ttttaccccc tacccatcct tgatcggtca tgcctagcct gaccctttag taaagcaatg 2700

aggtaggaag aacaaaccct tgtccctttg ccatgtggag gaaagtgcct gcctctggtc 2760

cgagccgcct cggttctgaa gcgagtgctc ctgcttacct tgctctaggc tgtctgcaga 2820

agcacctgcc ggtggcactc agcacctcct tgtgctagag ccctccatca ccttcacgct 2880

gtcccaccat gggccaggaa ccaaaccagc actgggttct actgctgtgg ggtaaactaa 2940

ctcagtggaa tagggctggt tactttgggc tgtccaactc ataagtttgg ctgcattttg 3000

aaaaaagctg atctaaataa aggcatgtgt atggctggtc 3040

<210> 38

<211> 1650

<212> DNA

<213> Homo sapiens

<220>

<221> calpain 3, (p94) (CAPN3), transcript variant 4

<222> (1)..(1650)

<223> LocusID:825

NM_173088

<400> 38

ccctcctcca cgcttacagc cacacacaca gtcacacaga cgcgttctga gggtggctgc 60

ccgcttggga tggaggaatc acttccctca gaacccagcc aagtcctcta ggcctccttg 120

ggggtccttc cagcctgagg ggcttcggag ctgaggagca gctgttctga tgcacgggaa 180

caagcagcac ctgcagaagg acttcttcct gtacaacgcc tccaaggcca ggagcaaaac 240

ctacatcaac atgcgggagg tgtcccagcg cttccgcctg cctcccagcg agtacgtcat 300

cgtgccctcc acctacgagc cccaccagga gggggaattc atcctccggg tcttctctga 360

aaagaggaac ctctctgagg aagttgaaaa taccatctcc gtggatcggc cagtgaaaaa 420

gaaaaaaacc aagcccatca tcttcgtttc ggacagagca aacagcaaca aggagctggg 480

tgtggaccag gagtcagagg agggcaaagg caaaacaagc cctgataagc aaaagcagtc 540

cccacagcca cagcctggca gctctgatca ggaaagtgag gaacagcaac aattccggaa 600

cattttcaag cagatagcag gagatgacat ggagatctgt gcagatgagc tcaagaaggt 660

ccttaacaca gtcgtgaaca aacacaagga cctgaagaca cacgggttca cactggagtc 720

ctgccgtagc atgattgcgc tcatggatac agatggctct ggaaagctca acctgcagga 780

gttccaccac ctctggaaca agattaaggc ctggcagaaa attttcaaac actatgacac 840

agaccagtcc ggcaccatca acagctacga gatgcgaaat gcagtcaacg acgcaggatt 900

ccacctcaac aaccagctct atgacatcat taccatgcgg tacgcagaca aacacatgaa 960

catcgacttt gacagtttca tctgctgctt cgttaggctg gagggcatgt tcagagcttt 1020

tcatgcattt gacaaggatg gagatggtat catcaagctc aacgttctgg agtggctgca 1080

gctcaccatg tatgcctgaa ccaggctggc ctcatccaaa gccatgcagg atcactcagg 1140

atttcagttt caccctctat ttccaaagcc atttacctca aaggacccag cagctacacc 1200

cctacaggct tccaggcacc tcatcagtca tgctcctcct ccattttacc ccctacccat 1260

ccttgatcgg tcatgcctag cctgaccctt tagtaaagca atgaggtagg aagaacaaac 1320

ccttgtccct ttgccatgtg gaggaaagtg cctgcctctg gtccgagccg cctcggttct 1380

gaagcgagtg ctcctgctta ccttgctcta ggctgtctgc agaagcacct gccggtggca 1440

ctcagcacct ccttgtgcta gagccctcca tcaccttcac gctgtcccac catgggccag 1500

gaaccaaacc agcactgggt tctactgctg tggggtaaac taactcagtg gaatagggct 1560

ggttactttg ggctgtccaa ctcataagtt tggctgcatt ttgaaaaaag ctgatctaaa 1620

taaaggcatg tgtatggctg gtcaaaaaaa 1650

<210> 39

<211> 1280

<212> DNA

<213> Homo sapiens

<220>

<221> calpain 3, (p94) (CAPN3), transcript variant 5

<222>  (1)..(1280)

<223>  LocusID: 825

NM_173089

<400>  39

cataatcctg accctgagcc agtgccaggt ctccaagtgc cttctgaatg accacaggcg    60

attggtttta gtggtaggtg tgtggggatc tgttctggtc atctggatgc tggtcatcgg   120

tgtgcagtat tgatcaggac ctgcaaaccc aaaagcttat gggagctggc acgtcaccca   180

cagcctggca gctctgatca ggaaagtgag gaacagcaac aattccggaa cattttcaag   240

cagatagcag gagatgacat ggagatctgt gcagatgagc tcaagaaggt ccttaacaca   300

gtcgtgaaca aacacaagga cctgaagaca cacgggttca cactggagtc ctgccgtagc   360

atgattgcgc tcatggatac agatggctct ggaaagctca acctgcagga gttccaccac   420

ctctggaaca agattaaggc ctggcagaaa attttcaaac actatgacac agaccagtcc   480

ggcaccatca acagctacga gatgcgaaat gcagtcaacg acgcaggatt ccacctcaac   540

aaccagctct atgacatcat taccatgcgg tacgcagaca aacacatgaa catcgacttt   600

gacagtttca tctgctgctt cgttaggctg gagggcatgt tcagagcttt tcatgcattt   660

gacaaggatg gagatggtat catcaagctc aacgttctgg agtggctgca gctcaccatg   720

tatgcctgaa ccaggctggc ctcatccaaa gccatgcagg atcactcagg atttcagttt   780

caccctctat ttccaaagcc atttacctca aaggacccag cagctacacc cctacaggct   840

tccaggcacc tcatcagtca tgctcctcct ccattttacc ccctacccat ccttgatcgg   900

tcatgcctag cctgaccctt tagtaaagca atgaggtagg aagaacaaac ccttgtccct   960

ttgccatgtg gaggaaagtg cctgcctctg gtccgagccg cctcggttct gaagcgagtg  1020

ctcctgctta ccttgctcta ggctgtctgc agaagcacct gccggtggca ctcagcacct  1080

ccttgtgcta gagccctcca tcaccttcac gctgtcccac catgggccag gaaccaaacc 1140

agcactgggt tctactgctg tggggtaaac taactcagtg gaatagggct ggttactttg 1200

ggctgtccaa ctcataagtt tggctgcatt ttgaaaaaag ctgatctaaa taaaggcatg 1260

tgtatggctg gtcaaaaaaa 1280

<210> 40

<211> 1300

<212> DNA

<213> Homo sapiens

<220>

<221> calpain 3, (p94) (CAPN3), transcript variant 6

<222> (1)..(1300)

<223> LocusID: 825

NM_173090

<400> 40

cataatcctg accctgagcc agtgccaggt ctccaagtgc cttctgaatg accacaggcg 60

attggtttta gtggtaggtg tgtggggatc tgttctggtc atctggatgc tggtcatcgg 120

tgtgcagtat tgatcaggac ctgcaaaccc aaaagcttat gggagctggc acgtcacaaa 180

aagaaaaaaa ccaagccaca gcctggcagc tctgatcagg aaagtgagga acagcaacaa 240

ttccggaaca ttttcaagca gatagcagga gatgacatgg agatctgtgc agatgagctc 300

aagaaggtcc ttaacacagt cgtgaacaaa cacaaggacc tgaagacaca cgggttcaca 360

ctggagtcct gccgtagcat gattgcgctc atggatacag atggctctgg aaagctcaac 420

ctgcaggagt tccaccacct ctggaacaag attaaggcct ggcagaaaat tttcaaacac 480

tatgacacag accagtccgg caccatcaac agctacgaga tgcgaaatgc agtcaacgac 540

gcaggattcc acctcaacaa ccagctctat gacatcatta ccatgcggta cgcagacaaa 600

cacatgaaca tcgactttga cagtttcatc tgctgcttcg ttaggctgga gggcatgttc 660

agagcttttc atgcatttga caaggatgga gatggtatca tcaagctcaa cgttctggag 720

tggctgcagc tcaccatgta tgcctgaacc aggctggcct catccaaagc catgcaggat 780

cactcaggat ttcagtttca ccctctattt ccaaagccat ttacctcaaa ggacccagca 840

gctacacccc tacaggcttc caggcacctc atcagtcatg ctcctcctcc attttacccc 900

ctacccatcc ttgatcggtc atgcctagcc tgacccttta gtaaagcaat gaggtaggaa 960

gaacaaaccc ttgtcccttt gccatgtgga ggaaagtgcc tgcctctggt ccgagccgcc 1020

tcggttctga agcgagtgct cctgcttacc ttgctctagg ctgtctgcag aagcacctgc 1080

cggtggcact cagcacctcc ttgtgctaga gccctccatc accttcacgc tgtcccacca 1140

tgggccagga accaaaccag cactgggttc tactgctgtg gggtaaacta actcagtgga 1200

atagggctgg ttactttggg ctgtccaact cataagtttg gctgcatttt gaaaaaagct 1260

gatctaaata aaggcatgtg tatggctggt caaaaaaaaa 1300

<210> 41

<211> 3890

<212> DNA

<213> Homo sapiens

<220>

<221> calpain 3, (p94) (CAPN3), transcript variant 8

<222>  (1)..(3890)

<223>  LocusID: 825

NM_212464

<400>  41

ctattccagt gtttcagcga ggtggaagtg tgataccaat aaagacaact gtaggaaaat    60

ccacaggctg gatgactgaa tcctcctatg gactccgggt tgctctaagc actaagggtt   120

cttcagtggg tgagttatat cttgatgatg gccattcatt ccaatacctc caccagaagc   180

aatttttgca caggaagttt tcattctgtt ccagtgttct gatcaatagt tttgctgacc   240

agaggggtca ttatcccagc aagtgtgtgg tggagaagat cttggtctta ggcttcagga   300

aggagccatc ttctgtgact acccactcat ctgatggtaa agatcagcct gtggctttta   360

cgtattgtgc caaaacatcc atcctgagcc tggagaagct ctcactcaac attgccactg   420

actgggaggg ttcctaaaaa agctctaaaa tgaatgcata gtgtgtcttt ccttcaagat   480

gtaaatgtca aggaatgagt tggcaaatca gtttaaaaac acaaactacc caacagaaaa   540

atgaaatttg cgagaatccc cgatttatca ttgatggagc caacagaact gacatctgtc   600

aaggagagct aggggactgc tggtttctcg cagccattgc ctgcctgacc ctgaaccagc   660

accttctttt ccgagtcata ccccatgatc aaagtttcat cgaaaactac gcagggatct   720

tccacttcca gttctggcgc tatggagagt gggtggacgt ggttatagat gactgcctgc   780

caacgtacaa caatcaactg gttttcacca agtccaacca ccgcaatgag ttctggagtg   840

ctctgctgga gaaggcttat gctaagctcc atggttccta cgaagctctg aaaggtggga   900

acaccacaga ggccatggag gacttcacag gaggggtggc agagtttttt gagatcaggg   960

atgctcctag tgacatgtac aagatcatga agaaagccat cgagagaggc tccctcatgg  1020

gctgctccat tgatgatggc acgaacatga cctatggaac ctctccttct ggtctgaaca  1080

tgggggagtt gattgcacgg atggtaagga atatggataa ctcactgctc caggactcag 1140

acctcgaccc cagaggctca gatgaaagac cgacccggac aatcattccg gttcagtatg 1200

agacaagaat ggcctgcggg ctggtcagag gtcacgccta ctctgtcacg gggctggatg 1260

aggtcccgtt caaaggtgag aaagtgaagc tggtgcggct gcggaatccg tggggccagg 1320

tggagtggaa cggttcttgg agtgatagat ggaaggactg gagctttgtg gacaaagatg 1380

agaaggcccg tctgcagcac caggtcactg aggatggaga gttctggatg tcctatgagg 1440

atttcatcta ccatttcaca aagttggaga tctgcaacct cacggccgat gctctgcagt 1500

ctgacaagct tcagacctgg acagtgtctg tgaacgaggg ccgctgggta cggggttgct 1560

ctgccggagg ctgccgcaac ttcccagata ctttctggac caaccctcag taccgtctga 1620

agctcctgga ggaggacgat gaccctgatg actcggaggt gatttgcagc ttcctggtgg 1680

ccctgatgca gaagaaccgg cggaaggacc ggaagctagg ggccagtctc ttcaccattg 1740

gcttcgccat ctacgaggtt cccaaagagg tatagcagca gcagcggcca gcagttgtgt 1800

gcagcactac ccagggggcc ccgagtctgt ctgtggctcg tcgagaagct tcctggtggg 1860

gtttgtgggc aggactgtga taggagaggg ccttgcctgt gttatttcca ctgcagagca 1920

ggtgcctcag ggcattgcat gacccatgac taccaccccc aggatgtgca ctttctccct 1980

cgcaccagac actgcacgtc acacacatgc ctttgcacac tcaccctcct ccacgcttac 2040

agccacacac acagtcacac agacgcgttc tgagggtggc tgcccgcttg ggatggagga 2100

atcacttccc tcagaaccca gccaagtcct ctaggcctcc ttgggggtcc ttccagcctg 2160

aggggcttcg gagctgagga gcagctgttc tggtaagtgt ccctgagtgt ggggatgaca 2220

catttgccat tcactctgaa tcacaacaga aaaggaagag gaagtgaggt agggaggcta 2280

tttaagcctt gggagtcgga agtagggagg ttgaaactgt gacatgggtg accagggagt 2340

tgggaaggga cccttggagg tggctgtggc aggacaggac gttcctcccg aggggctcat 2400

gtgccctggg ctctccccat ctctcagatg cacgggaaca agcagcacct gcagaaggac 2460

ttcttcctgt acaacgcctc caaggccagg agcaaaacct acatcaacat gcgggaggtg 2520

tcccagcgct tccgcctgcc tcccagcgag tacgtcatcg tgccctccac ctacgagccc 2580

caccaggagg gggaattcat cctccgggtc ttctctgaaa agaggaacct ctctgaggaa 2640

gttgaaaata ccatctccgt ggatcggcca gtgcccatca tcttcgtttc ggacagagca 2700

aacagcaaca aggagctggg tgtggaccag gagtcagagg agggcaaagg caaaacaagc 2760

cctgataagc aaaagcagtc cccacagcca cagcctggca gctctgatca ggaaagtgag 2820

gaacagcaac aattccggaa cattttcaag cagatagcag gagatgacat ggagatctgt 2880

gcagatgagc tcaagaaggt ccttaacaca gtcgtgaaca aacacaagga cctgaagaca 2940

cacgggttca cactggagtc ctgccgtagc atgattgcgc tcatggatac agatggctct 3000

ggaaagctca acctgcagga gttccaccac ctctggaaca agattaaggc ctggcagaaa 3060

attttcaaac actatgacac agaccagtcc ggcaccatca acagctacga gatgcgaaat 3120

gcagtcaacg acgcaggatt ccacctcaac aaccagctct atgacatcat taccatgcgg 3180

tacgcagaca aacacatgaa catcgacttt gacagtttca tctgctgctt cgttaggctg 3240

gagggcatgt tcagagcttt tcatgcattt gacaaggatg gagatggtat catcaagctc 3300

aacgttctgg agtggctgca gctcaccatg tatgcctgaa ccaggctggc ctcatccaaa 3360

gccatgcagg atcactcagg atttcagttt caccctctat ttccaaagcc atttacctca 3420

aaggacccag cagctacacc cctacaggct tccaggcacc tcatcagtca tgttcctcct 3480

ccattttacc ccctacccat ccttgatcgg tcatgcctag cctgaccctt tagtaaagca 3540

atgaggtagg aagaacaaac ccttgtccct ttgccatgtg gaggaaagtg cctgcctctg 3600

gtccgagccg cctcggttct gaagcgagtg ctcctgctta ccttgctcta ggctgtctgc 3660

agaagcacct gccggtggca ctcagcacct ccttgtgcta gagccctcca tcaccttcac 3720

gctgtcccac catgggccag gaaccaaacc agcactgggt tctactgctg tggggtaaac 3780

taactcagtg gaatagggct ggttactttg ggctgtccaa ctcataagtt tggctgcatt 3840

ttgaaaaaag ctgatctaaa taaaggcatg tgtatggctg gtcaaaaaaa 3890

<210> 42

<211> 3230

<212> DNA

<213> Homo sapiens

<220>

<221> calpain 3, (p94) (CAPN3), transcript variant 7

<222> (1)..(3230)

<223> LocusID: 825

NM_212465

<400> 42

ctattccagt gtttcagcga ggtggaagtg tgataccaat aaagacaact gtaggaaaat 60

ccacaggctg gatgactgaa tcctcctatg gactccgggt tgctctaagc actaagggtt 120

cttcagtggg tgagttatat cttgatgatg gccattcatt ccaatacctc caccagaagc 180

aatttttgca caggaagttt tcattctgtt ccagtgttct gatcaatagt tttgctgacc 240

agaggggtca ttatcccagc aagtgtgtgg tggagaagat cttggtctta ggcttcagga 300

aggagccatc ttctgtgact acccactcat ctgatggtaa agatcagcct gtggctttta 360

cgtattgtgc caaaacatcc atcctgagcc tggagaagct ctcactcaac attgccactg 420

actgggaggg ttcctaaaaa agctctaaaa tgaatgcata gtgtgtcttt ccttcaagat 480

gtaaatgtca aggaatgagt tggcaaatca gtttaaaaac acaaactacc caacagaaaa 540

atgaaatttg cgagaatccc cgatttatca ttgatggagc caacagaact gacatctgtc 600

aaggagagct aggggactgc tggtttctcg cagccattgc ctgcctgacc ctgaaccagc 660

accttctttt ccgagtcata ccccatgatc aaagtttcat cgaaaactac gcagggatct 720

tccacttcca gttctggcgc tatggagagt gggtggacgt ggttatagat gactgcctgc 780

caacgtacaa caatcaactg gttttcacca agtccaacca ccgcaatgag ttctggagtg 840

ctctgctgga gaaggcttat gctaagctcc atggttccta cgaagctctg aaaggtggga 900

acaccacaga ggccatggag gacttcacag gaggggtggc agagtttttt gagatcaggg 960

atgctcctag tgacatgtac aagatcatga agaaagccat cgagagaggc tccctcatgg 1020

gctgctccat tgatgatggc acgaacatga cctatggaac ctctccttct ggtctgaaca 1080

tgggggagtt gattgcacgg atggtaagga atatggataa ctcactgctc caggactcag 1140

acctcgaccc cagaggctca gatgaaagac cgacccggac aatcattccg gttcagtatg 1200

agacaagaat ggcctgcggg ctggtcagag gtcacgccta ctctgtcacg gggctggatg 1260

aggtcccgtt caaaggtgag aaagtgaagc tggtgcggct gcggaatccg tggggccagg 1320

tggagtggaa cggttcttgg agtgatagat ggaaggactg gagctttgtg gacaaagatg 1380

agaaggcccg tctgcagcac caggtcactg aggatggaga gttctggatg tcctatgagg 1440

atttcatcta ccatttcaca aagttggaga tctgcaacct cacggccgat gctctgcagt 1500

ctgacaagct tcagacctgg acagtgtctg tgaacgaggg ccgctgggta cggggttgct 1560

ctgccggagg ctgccgcaac ttcccagata ctttctggac caaccctcag taccgtctga 1620

agctcctgga ggaggacgat gaccctgatg actcggaggt gatttgcagc ttcctggtgg 1680

ccctgatgca gaagaaccgg cggaaggacc ggaagctagg ggccagtctc ttcaccattg 1740

gcttcgccat ctacgaggtt cccaaagaga tgcacgggaa caagcagcac ctgcagaagg 1800

acttcttcct gtacaacgcc tccaaggcca ggagcaaaac ctacatcaac atgcgggagg 1860

tgtcccagcg cttccgcctg cctcccagcg agtacgtcat cgtgccctcc acctacgagc 1920

cccaccagga gggggaattc atcctccggg tcttctctga aaagaggaac ctctctgagg 1980

aagttgaaaa taccatctcc gtggatcggc cagtgcccat catcttcgtt tcggacagag 2040

caaacagcaa caaggagctg ggtgtggacc aggagtcaga ggagggcaaa ggcaaaacaa 2100

gccctgataa gcaaaagcag tccccacagc cacagcctgg cagctctgat caggaaagtg 2160

aggaacagca acaattccgg aacattttca agcagatagc aggagatgac atggagatct 2220

gtgcagatga gctcaagaag gtccttaaca cagtcgtgaa caaacacaag gacctgaaga 2280

cacacgggtt cacactggag tcctgccgta gcatgattgc gctcatggat acagatggct 2340

ctggaaagct caacctgcag gagttccacc acctctggaa caagattaag gcctggcaga 2400

aaattttcaa acactatgac acagaccagt ccggcaccat caacagctac gagatgcgaa 2460

atgcagtcaa cgacgcagga ttccacctca acaaccagct ctatgacatc attaccatgc 2520

ggtacgcaga caaacacatg aacatcgact ttgacagttt catctgctgc ttcgttaggc 2580

tggagggcat gttcagagct tttcatgcat ttgacaagga tggagatggt atcatcaagc 2640

tcaacgttct ggagtggctg cagctcacca tgtatgcctg aaccaggctg gcctcatcca 2700

aagccatgca ggatcactca ggatttcagt ttcaccctct atttccaaag ccatttacct 2760

caaaggaccc agcagctaca cccctacagg cttccaggca cctcatcagt catgttcctc 2820

ctccatttta ccccctaccc atccttgatc ggtcatgcct agcctgaccc tttagtaaag 2880

caatgaggta ggaagaacaa acccttgtcc ctttgccatg tggaggaaag tgcctgcctc 2940

tggtccgagc cgcctcggtt ctgaagcgag tgctcctgct taccttgctc taggctgtct 3000

gcagaagcac ctgccggtgg cactcagcac ctccttgtgc tagagccctc catcaccttc 3060

acgctgtccc accatgggcc aggaaccaaa ccagcactgg gttctactgc tgtggggtaa 3120

actaactcag tggaataggg ctggttactt tgggctgtcc aactcataag tttggctgca 3180

ttttgaaaaa agctgatcta aataaaggca tgtgtatggc tggtcaaaaa 3230

<210> 43

<211> 3510

<212> DNA

<213> Homo sapiens

<220>

<221>  calpain 3, (p94) (CAPN3), transcript variant 9

<222>  (1)..(3510)

<223>  LocusID: 825

NM_212467

<400>  43

ctattccagt gtttcagcga ggtggaagtg tgataccaat aaagacaact gtaggaaaat    60

ccacaggctg gatgactgaa tcctcctatg gactccgggt tgctctaagc actaagggtt   120

cttcagtggg tgagttatat cttgatgatg gccattcatt ccaatacctc caccagaagc   180

aatttttgca caggaagttt tcattctgtt ccagtgttct gatcaatagt tttgctgacc   240

agaggggtca ttatcccagc aagtgtgtgg tggagaagat cttggtctta ggcttcagga   300

aggagccatc ttctgtgact acccactcat ctgatggtaa agatcagcct gtggctttta   360

cgtattgtgc caaaacatcc atcctgagcc tggagaagct ctcactcaac attgccactg   420

actgggaggg ttcctaaaaa agctctaaaa tgaatgcata gtgtgtcttt ccttcaagat   480

gtaaatgtca aggaatgagt tggcaaatca gtttaaaaac acaaactacc caacagaaaa   540

atgaaatttg cgagaatccc cgatttatca ttgatggagc caacagaact gacatctgtc   600

aaggagagct agacgggttc cagggcagct gcctacctgg ccttccttcc aatacaaatc   660

atcttggtgg atggttctct gaggctcagt cttcgctgaa gtcagaagag gaattggact   720

cacattgcaa aggcacaggg cagggcagat ttcctacagg tgttaggaag aacaacccag   780

ttatgatcac ctactgctct gtctccattg aggcctaaaa aggaagtgag tttatactgc   840

agttggagga actgcctgca gccttgagga aaatgtctag tcacaaggga gggactgctg   900

gtttctcgca gccattgcct gcctgaccct gaaccagcac cttcttttcc gagtcatacc   960

ccatgatcaa agtttcatcg aaaactacgc agggatcttc cacttccagt tctggcgcta 1020

tggagagtgg gtggacgtgg ttatagatga ctgcctgcca acgtacaaca atcaactggt 1080

tttcaccaag tccaaccacc gcaatgagtt ctggagtgct ctgctggaga aggcttatgc 1140

taagctccat ggttcctacg aagctctgaa aggtgggaac accacagagg ccatggagga 1200

cttcacagga ggggtggcag agttttttga gatcagggat gctcctagtg acatgtacaa 1260

gatcatgaag aaagccatcg agagaggctc cctcatgggc tgctccattg atgatggcac 1320

gaacatgacc tatggaacct ctccttctgg tctgaacatg ggggagttga ttgcacggat 1380

ggtaaggaat atggataact cactgctcca ggactcagac ctcgacccca gaggctcaga 1440

tgaaagaccg acccggacaa tcattccggt tcagtatgag acaagaatgg cctgcgggct 1500

ggtcagaggt cacgcctact ctgtcacggg gctggatgag gtcccgttca aaggtgagaa 1560

agtgaagctg gtgcggctgc ggaatccgtg gggccaggtg gagtggaacg gttcttggag 1620

tgatagatgg aaggactgga gctttgtgga caaagatgag aaggcccgtc tgcagcacca 1680

ggtcactgag gatggagagt tctggatgtc ctatgaggat ttcatctacc atttcacaaa 1740

gttggagatc tgcaacctca cggccgatgc tctgcagtct gacaagcttc agacctggac 1800

agtgtctgtg aacgagggcc gctgggtacg gggttgctct gccggaggct gccgcaactt 1860

cccagatact ttctggacca accctcagta ccgtctgaag ctcctggagg aggacgatga 1920

ccctgatgac tcggaggtga tttgcagctt cctggtggcc ctgatgcaga agaaccggcg 1980

gaaggaccgg aagctagggg ccagtctctt caccattggc ttcgccatct acgaggttcc 2040

caaagagatg cacgggaaca agcagcacct gcagaaggac ttcttcctgt acaacgcctc 2100

caaggccagg agcaaaacct acatcaacat gcgggaggtg tcccagcgct tccgcctgcc 2160

tcccagcgag tacgtcatcg tgccctccac ctacgagccc caccaggagg gggaattcat 2220

cctccgggtc ttctctgaaa agaggaacct ctctgaggaa gttgaaaata ccatctccgt 2280

ggatcggcca gtgcccatca tcttcgtttc ggacagagca aacagcaaca aggagctggg 2340

tgtggaccag gagtcagagg agggcaaagg caaaacaagc cctgataagc aaaagcagtc 2400

cccacagcca cagcctggca gctctgatca ggaaagtgag gaacagcaac aattccggaa 2460

cattttcaag cagatagcag gagatgacat ggagatctgt gcagatgagc tcaagaaggt 2520

ccttaacaca gtcgtgaaca aacacaagga cctgaagaca cacgggttca cactggagtc 2580

ctgccgtagc atgattgcgc tcatggatac agatggctct ggaaagctca acctgcagga 2640

gttccaccac ctctggaaca agattaaggc ctggcagaaa attttcaaac actatgacac 2700

agaccagtcc ggcaccatca acagctacga gatgcgaaat gcagtcaacg acgcaggatt 2760

ccacctcaac aaccagctct atgacatcat taccatgcgg tacgcagaca aacacatgaa 2820

catcgacttt gacagtttca tctgctgctt cgttaggctg gagggcatgt tcagagcttt 2880

tcatgcattt gacaaggatg gagatggtat catcaagctc aacgttctgg agtggctgca 2940

gctcaccatg tatgcctgaa ccaggctggc ctcatccaaa gccatgcagg atcactcagg 3000

atttcagttt caccctctat ttccaaagcc atttacctca aaggacccag cagctacacc 3060

cctacaggct tccaggcacc tcatcagtca tgttcctcct ccattttacc ccctacccat 3120

ccttgatcgg tcatgcctag cctgaccctt tagtaaagca atgaggtagg aagaacaaac 3180

ccttgtccct ttgccatgtg gaggaaagtg cctgcctctg gtccgagccg cctcggttct 3240

gaagcgagtg ctcctgctta ccttgctcta ggctgtctgc agaagcacct gccggtggca 3300

ctcagcacct ccttgtgcta gagccctcca tcaccttcac gctgtcccac catgggccag 3360

gaaccaaacc agcactgggt tctactgctg tggggtaaac taactcagtg gaatagggct 3420

ggttactttg ggctgtccaa ctcataagtt tggctgcatt ttgaaaaaag ctgatctaaa 3480

taaaggcatg tgtatggctg gtcaaaaaaa 3510

<210> 44

<211> 720

<212> PRT

<213>  Homo sapiens

<220>

<221>  acyl-CoA synthetase long-chain family member 3 transcript
variant

       1

<222>  (1)..(720)

<223>  LocusID: 2181


NM_004457



<400>  44



Met Asn Asn His Val Ser Ser Lys Pro Ser Thr Met Lys Leu Lys His

1               5                   10                  15

Thr Ile Asn Pro Ile Leu Leu Tyr Phe Ile His Phe Leu Ile Ser Leu

            20                  25                  30

Tyr Thr Ile Leu Thr Tyr Ile Pro Phe Tyr Phe Phe Ser Glu Ser Arg

        35                  40                  45

Gln Glu Lys Ser Asn Arg Ile Lys Ala Lys Pro Val Asn Ser Lys Pro

    50                  55                  60

Asp Ser Ala Tyr Arg Ser Val Asn Ser Leu Asp Gly Leu Ala Ser Val

65                  70                  75                  80

Leu Tyr Pro Gly Cys Asp Thr Leu Asp Lys Val Phe Thr Tyr Ala Lys

                85                  90                  95

Asn Lys Phe Lys Asn Lys Arg Leu Leu Gly Thr Arg Glu Val Leu Asn
            100                 105                 110
Glu Glu Asp Glu Val Gln Pro Asn Gly Lys Ile Phe Lys Lys Val Ile
        115                 120                 125
Leu Gly Gln Tyr Asn Trp Leu Ser Tyr Glu Asp Val Phe Val Arg Ala
    130                 135                 140
Phe Asn Phe Gly Asn Gly Leu Gln Met Leu Gly Gln Lys Pro Lys Thr
145                 150                 155                 160
Asn Ile Ala Ile Phe Cys Glu Thr Arg Ala Glu Trp Met Ile Ala Ala
                165                 170                 175
Gln Ala Cys Phe Met Tyr Asn Phe Gln Leu Val Thr Leu Tyr Ala Thr
            180                 185                 190
Leu Gly Gly Pro Ala Ile Val His Ala Leu Asn Glu Thr Glu Val Thr
        195                 200                 205
Asn Ile Ile Thr Ser Lys Glu Leu Leu Gln Thr Lys Leu Lys Asp Ile
    210                 215                 220
Val Ser Leu Val Pro Arg Leu Arg His Ile Ile Thr Val Asp Gly Lys
225                 230                 235                 240
Pro Pro Thr Trp Ser Glu Phe Pro Lys Gly Ile Ile Val His Thr Met
                245                 250                 255
Ala Ala Val Glu Ala Leu Gly Ala Lys Ala Ser Met Glu Asn Gln Pro
            260                 265                 270
His Ser Lys Pro Leu Pro Ser Asp Ile Ala Val Ile Met Tyr Thr Ser
        275                 280                 285

Gly Ser Thr Gly Leu Pro Lys Gly Val Met Ile Ser His Ser Asn Ile
    290                 295                 300
Ile Ala Gly Ile Thr Gly Met Ala Glu Arg Ile Pro Glu Leu Gly Glu
305                 310                 315                 320
Glu Asp Val Tyr Ile Gly Tyr Leu Pro Leu Ala His Val Leu Glu Leu
                325                 330                 335
Ser Ala Glu Leu Val Cys Leu Ser His Gly Cys Arg Ile Gly Tyr Ser
            340                 345                 350
Ser Pro Gln Thr Leu Ala Asp Gln Ser Ser Lys Ile Lys Lys Gly Ser
        355                 360                 365
Lys Gly Asp Thr Ser Met Leu Lys Pro Thr Leu Met Ala Ala Val Pro
    370                 375                 380
Glu Ile Met Asp Arg Ile Tyr Lys Asn Val Met Asn Lys Val Ser Glu
385                 390                 395                 400
Met Ser Ser Phe Gln Arg Asn Leu Phe Ile Leu Ala Tyr Asn Tyr Lys
                405                 410                 415
Met Glu Gln Ile Ser Lys Gly Arg Asn Thr Pro Leu Cys Asp Ser Phe
            420                 425                 430
Val Phe Arg Lys Val Arg Ser Leu Leu Gly Gly Asn Ile Arg Leu Leu
        435                 440                 445
Leu Cys Gly Gly Ala Pro Leu Ser Ala Thr Thr Gln Arg Phe Met Asn
    450                 455                 460
Ile Cys Phe Cys Cys Pro Val Gly Gln Gly Tyr Gly Leu Thr Glu Ser
465                 470                 475                 480

Ala Gly Ala Gly Thr Ile Ser Glu Val Trp Asp Tyr Asn Thr Gly Arg

                485                 490                 495

Val Gly Ala Pro Leu Val Cys Cys Glu Ile Lys Leu Lys Asn Trp Glu

            500                 505                 510

Glu Gly Gly Tyr Phe Asn Thr Asp Lys Pro His Pro Arg Gly Glu Ile

        515                 520                 525

Leu Ile Gly Gly Gln Ser Val Thr Met Gly Tyr Tyr Lys Asn Glu Ala

    530                 535                 540

Lys Thr Lys Ala Asp Phe Phe Glu Asp Glu Asn Gly Gln Arg Trp Leu

545                 550                 555                 560

Cys Thr Gly Asp Ile Gly Glu Phe Glu Pro Asp Gly Cys Leu Lys Ile

                565                 570                 575

Ile Asp Arg Lys Lys Asp Leu Val Lys Leu Gln Ala Gly Glu Tyr Val

            580                 585                 590

Ser Leu Gly Lys Val Glu Ala Ala Leu Lys Asn Leu Pro Leu Val Asp

        595                 600                 605

Asn Ile Cys Ala Tyr Ala Asn Ser Tyr His Ser Tyr Val Ile Gly Phe

    610                 615                 620

Val Val Pro Asn Gln Lys Glu Leu Thr Glu Leu Ala Arg Lys Lys Gly

625                 630                 635                 640

Leu Lys Gly Thr Trp Glu Glu Leu Cys Asn Ser Cys Glu Met Glu Asn

                645                 650                 655

Glu Val Leu Lys Val Leu Ser Glu Ala Ala Ile Ser Ala Ser Leu Glu

            660                 665                 670

Lys Phe Glu Ile Pro Val Lys Ile Arg Leu Ser Pro Glu Pro Trp Thr

        675                 680                 685

Pro Glu Thr Gly Leu Val Thr Asp Ala Phe Lys Leu Lys Arg Lys Glu

    690                 695                 700

Leu Lys Thr His Tyr Gln Ala Asp Ile Glu Arg Met Tyr Gly Arg Lys

705                 710                 715                 720

<210> 45

<211> 720

<212> PRT

<213> Homo sapiens

<220>

<221> acyl-CoA synthetase long-chain family member 3 transcript
variant

2

<222> (1)..(720)

<223> LocusID: 2181

NM_203372

<400> 45

Met Asn Asn His Val Ser Ser Lys Pro Ser Thr Met Lys Leu Lys His

1               5                   10                  15

Thr Ile Asn Pro Ile Leu Leu Tyr Phe Ile His Phe Leu Ile Ser Leu

            20                  25                  30

Tyr Thr Ile Leu Thr Tyr Ile Pro Phe Tyr Phe Phe Ser Glu Ser Arg

        35                  40                  45

Gln Glu Lys Ser Asn Arg Ile Lys Ala Lys Pro Val Asn Ser Lys Pro

    50                  55                  60

Asp Ser Ala Tyr Arg Ser Val Asn Ser Leu Asp Gly Leu Ala Ser Val

65                  70                  75                  80

Leu Tyr Pro Gly Cys Asp Thr Leu Asp Lys Val Phe Thr Tyr Ala Lys

                85                  90                  95

Asn Lys Phe Lys Asn Lys Arg Leu Leu Gly Thr Arg Glu Val Leu Asn

            100                 105                 110

Glu Glu Asp Glu Val Gln Pro Asn Gly Lys Ile Phe Lys Lys Val Ile

        115                 120                 125

Leu Gly Gln Tyr Asn Trp Leu Ser Tyr Glu Asp Val Phe Val Arg Ala

    130                 135                 140

Phe Asn Phe Gly Asn Gly Leu Gln Met Leu Gly Gln Lys Pro Lys Thr

145                 150                 155                 160

Asn Ile Ala Ile Phe Cys Glu Thr Arg Ala Glu Trp Met Ile Ala Ala

                165                 170                 175

Gln Ala Cys Phe Met Tyr Asn Phe Gln Leu Val Thr Leu Tyr Ala Thr

            180                 185                 190

Leu Gly Gly Pro Ala Ile Val His Ala Leu Asn Glu Thr Glu Val Thr

        195                 200                 205
Asn Ile Ile Thr Ser Lys Glu Leu Leu Gln Thr Lys Leu Lys Asp Ile
    210                 215                 220
Val Ser Leu Val Pro Arg Leu Arg His Ile Ile Thr Val Asp Gly Lys
225                 230                 235                 240
Pro Pro Thr Trp Ser Glu Phe Pro Lys Gly Ile Ile Val His Thr Met
                245                 250                 255
Ala Ala Val Glu Ala Leu Gly Ala Lys Ala Ser Met Glu Asn Gln Pro
            260                 265                 270
His Ser Lys Pro Leu Pro Ser Asp Ile Ala Val Ile Met Tyr Thr Ser
        275                 280                 285
Gly Ser Thr Gly Leu Pro Lys Gly Val Met Ile Ser His Ser Asn Ile
    290                 295                 300
Ile Ala Gly Ile Thr Gly Met Ala Glu Arg Ile Pro Glu Leu Gly Glu
305                 310                 315                 320
Glu Asp Val Tyr Ile Gly Tyr Leu Pro Leu Ala His Val Leu Glu Leu
                325                 330                 335
Ser Ala Glu Leu Val Cys Leu Ser His Gly Cys Arg Ile Gly Tyr Ser
            340                 345                 350
Ser Pro Gln Thr Leu Ala Asp Gln Ser Ser Lys Ile Lys Lys Gly Ser
        355                 360                 365
Lys Gly Asp Thr Ser Met Leu Lys Pro Thr Leu Met Ala Ala Val Pro
    370                 375                 380
Glu Ile Met Asp Arg Ile Tyr Lys Asn Val Met Asn Lys Val Ser Glu

385                 390                 395                 400

Met Ser Ser Phe Gln Arg Asn Leu Phe Ile Leu Ala Tyr Asn Tyr Lys

                405                 410                 415

Met Glu Gln Ile Ser Lys Gly Arg Asn Thr Pro Leu Cys Asp Ser Phe

            420                 425                 430

Val Phe Arg Lys Val Arg Ser Leu Leu Gly Gly Asn Ile Arg Leu Leu

        435                 440                 445

Leu Cys Gly Gly Ala Pro Leu Ser Ala Thr Thr Gln Arg Phe Met Asn

    450                 455                 460

Ile Cys Phe Cys Cys Pro Val Gly Gln Gly Tyr Gly Leu Thr Glu Ser

465                 470                 475                 480

Ala Gly Ala Gly Thr Ile Ser Glu Val Trp Asp Tyr Asn Thr Gly Arg

                485                 490                 495

Val Gly Ala Pro Leu Val Cys Cys Glu Ile Lys Leu Lys Asn Trp Glu

            500                 505                 510

Glu Gly Gly Tyr Phe Asn Thr Asp Lys Pro His Pro Arg Gly Glu Ile

        515                 520                 525

Leu Ile Gly Gly Gln Ser Val Thr Met Gly Tyr Tyr Lys Asn Glu Ala

    530                 535                 540

Lys Thr Lys Ala Asp Phe Phe Glu Asp Glu Asn Gly Gln Arg Trp Leu

545                 550                 555                 560

Cys Thr Gly Asp Ile Gly Glu Phe Glu Pro Asp Gly Cys Leu Lys Ile

                565                 570                 575

Ile Asp Arg Lys Lys Asp Leu Val Lys Leu Gln Ala Gly Glu Tyr Val

            580                 585                 590

Ser Leu Gly Lys Val Glu Ala Ala Leu Lys Asn Leu Pro Leu Val Asp

        595                 600                 605

Asn Ile Cys Ala Tyr Ala Asn Ser Tyr His Ser Tyr Val Ile Gly Phe

    610                 615                 620

Val Val Pro Asn Gln Lys Glu Leu Thr Glu Leu Ala Arg Lys Lys Gly

625                 630                 635                 640

Leu Lys Gly Thr Trp Glu Glu Leu Cys Asn Ser Cys Glu Met Glu Asn

                645                 650                 655

Glu Val Leu Lys Val Leu Ser Glu Ala Ala Ile Ser Ala Ser Leu Glu

            660                 665                 670

Lys Phe Glu Ile Pro Val Lys Ile Arg Leu Ser Pro Glu Pro Trp Thr

        675                 680                 685

Pro Glu Thr Gly Leu Val Thr Asp Ala Phe Lys Leu Lys Arg Lys Glu

    690                 695                 700

Leu Lys Thr His Tyr Gln Ala Asp Ile Glu Arg Met Tyr Gly Arg Lys

705                 710                 715                 720

<210> 46

<211> 297

<212> PRT

<213> Homo sapiens

<220>

<221>  solute carrier family 25 member 4

<222>  (1)..(297)

<223>  LocusID: 291

NM_001151

<400>  46

Met Gly Asp His Ala Trp Ser Phe Leu Lys Asp Phe Leu Ala Gly Ala
1               5                   10                  15

Val Ala Ala Ala Val Ser Lys Thr Ala Val Ala Pro Ile Glu Arg Val
            20                  25                  30

Lys Leu Leu Leu Gln Val Gln His Ala Ser Lys Gln Ile Ser Ala Glu
        35                  40                  45

Lys Gln Tyr Lys Gly Ile Ile Asp Cys Val Val Arg Ile Pro Lys Glu
    50                  55                  60

Gln Gly Phe Leu Ser Phe Trp Arg Gly Asn Leu Ala Asn Val Ile Arg
65                  70                  75                  80

Tyr Phe Pro Thr Gln Ala Leu Asn Phe Ala Phe Lys Asp Lys Tyr Lys
                85                  90                  95

Gln Leu Phe Leu Gly Gly Val Asp Arg His Lys Gln Phe Trp Arg Tyr
            100                 105                 110

Phe Ala Gly Asn Leu Ala Ser Gly Gly Ala Ala Gly Ala Thr Ser Leu

115                 120                 125

Cys Phe Val Tyr Pro Leu Asp Phe Ala Arg Thr Arg Leu Ala Ala Asp

    130                 135                 140

Val Gly Arg Arg Ala Gln Arg Glu Phe His Gly Leu Gly Asp Cys Ile

145                 150                 155                 160

Ile Lys Ile Phe Lys Ser Asp Gly Leu Arg Gly Leu Tyr Gln Gly Phe

                165                 170                 175

Asn Val Ser Val Gln Gly Ile Ile Ile Tyr Arg Ala Ala Tyr Phe Gly

            180                 185                 190

Val Tyr Asp Thr Ala Lys Gly Met Leu Pro Asp Pro Lys Asn Val His

        195                 200                 205

Ile Phe Val Ser Trp Met Ile Ala Gln Ser Val Thr Ala Val Ala Gly

    210                 215                 220

Leu Leu Ser Tyr Pro Phe Asp Thr Val Arg Arg Arg Met Met Met Gln

225                 230                 235                 240

Ser Gly Arg Lys Gly Ala Asp Ile Met Tyr Thr Gly Thr Val Asp Cys

                245                 250                 255

Trp Arg Lys Ile Ala Lys Asp Glu Gly Ala Lys Ala Phe Phe Lys Gly

            260                 265                 270

Ala Trp Ser Asn Val Leu Arg Gly Met Gly Gly Ala Phe Val Leu Val

        275                 280                 285

Leu Tyr Asp Glu Ile Lys Lys Tyr Val

    290                 295

<210> 47

<211> 1124

<212> PRT

<213> Homo sapiens

<220>

<221> TEK tyrosine kinase

<222> (1)..(1124)

<223> LocusID: 7010

NM_000459

<400> 47

Met Asp Ser Leu Ala Ser Leu Val Leu Cys Gly Val Ser Leu Leu Leu

1               5                   10                  15

Ser Gly Thr Val Glu Gly Ala Met Asp Leu Ile Leu Ile Asn Ser Leu

            20                  25                  30

Pro Leu Val Ser Asp Ala Glu Thr Ser Leu Thr Cys Ile Ala Ser Gly

        35                  40                  45

Trp Arg Pro His Glu Pro Ile Thr Ile Gly Arg Asp Phe Glu Ala Leu

    50                  55                  60

Met Asn Gln His Gln Asp Pro Leu Glu Val Thr Gln Asp Val Thr Arg

65                  70                  75                  80

Glu Trp Ala Lys Lys Val Val Trp Lys Arg Glu Lys Ala Ser Lys Ile
                85                  90                  95
Asn Gly Ala Tyr Phe Cys Glu Gly Arg Val Arg Gly Glu Ala Ile Arg
            100                 105                 110
Ile Arg Thr Met Lys Met Arg Gln Gln Ala Ser Phe Leu Pro Ala Thr
        115                 120                 125
Leu Thr Met Thr Val Asp Lys Gly Asp Asn Val Asn Ile Ser Phe Lys
    130                 135                 140
Lys Val Leu Ile Lys Glu Glu Asp Ala Val Ile Tyr Lys Asn Gly Ser
145                 150                 155                 160
Phe Ile His Ser Val Pro Arg His Glu Val Pro Asp Ile Leu Glu Val
                165                 170                 175
His Leu Pro His Ala Gln Pro Gln Asp Ala Gly Val Tyr Ser Ala Arg
            180                 185                 190
Tyr Ile Gly Gly Asn Leu Phe Thr Ser Ala Phe Thr Arg Leu Ile Val
        195                 200                 205
Arg Arg Cys Glu Ala Gln Lys Trp Gly Pro Glu Cys Asn His Leu Cys
    210                 215                 220
Thr Ala Cys Met Asn Asn Gly Val Cys His Glu Asp Thr Gly Glu Cys
225                 230                 235                 240
Ile Cys Pro Pro Gly Phe Met Gly Arg Thr Cys Glu Lys Ala Cys Glu
                245                 250                 255
Leu His Thr Phe Gly Arg Thr Cys Lys Glu Arg Cys Ser Gly Gln Glu
            260                 265                 270

Gly Cys Lys Ser Tyr Val Phe Cys Leu Pro Asp Pro Tyr Gly Cys Ser

        275                 280                 285

Cys Ala Thr Gly Trp Lys Gly Leu Gln Cys Asn Glu Ala Cys His Pro

    290                 295                 300

Gly Phe Tyr Gly Pro Asp Cys Lys Leu Arg Cys Ser Cys Asn Asn Gly

305                 310                 315                 320

Glu Met Cys Asp Arg Phe Gln Gly Cys Leu Cys Ser Pro Gly Trp Gln

                325                 330                 335

Gly Leu Gln Cys Glu Arg Glu Gly Ile Pro Arg Met Thr Pro Lys Ile

            340                 345                 350

Val Asp Leu Pro Asp His Ile Glu Val Asn Ser Gly Lys Phe Asn Pro

        355                 360                 365

Ile Cys Lys Ala Ser Gly Trp Pro Leu Pro Thr Asn Glu Glu Met Thr

    370                 375                 380

Leu Val Lys Pro Asp Gly Thr Val Leu His Pro Lys Asp Phe Asn His

385                 390                 395                 400

Thr Asp His Phe Ser Val Ala Ile Phe Thr Ile His Arg Ile Leu Pro

                405                 410                 415

Pro Asp Ser Gly Val Trp Val Cys Ser Val Asn Thr Val Ala Gly Met

            420                 425                 430

Val Glu Lys Pro Phe Asn Ile Ser Val Lys Val Leu Pro Lys Pro Leu

        435                 440                 445

Asn Ala Pro Asn Val Ile Asp Thr Gly His Asn Phe Ala Val Ile Asn

    450                 455                 460

Ile Ser Ser Glu Pro Tyr Phe Gly Asp Gly Pro Ile Lys Ser Lys Lys
465                 470                 475                 480
Leu Leu Tyr Lys Pro Val Asn His Tyr Glu Ala Trp Gln His Ile Gln
                485                 490                 495
Val Thr Asn Glu Ile Val Thr Leu Asn Tyr Leu Glu Pro Arg Thr Glu
            500                 505                 510
Tyr Glu Leu Cys Val Gln Leu Val Arg Arg Gly Glu Gly Gly Glu Gly
        515                 520                 525
His Pro Gly Pro Val Arg Arg Phe Thr Thr Ala Ser Ile Gly Leu Pro
    530                 535                 540
Pro Pro Arg Gly Leu Asn Leu Leu Pro Lys Ser Gln Thr Thr Leu Asn
545                 550                 555                 560
Leu Thr Trp Gln Pro Ile Phe Pro Ser Ser Glu Asp Asp Phe Tyr Val
                565                 570                 575
Glu Val Glu Arg Arg Ser Val Gln Lys Ser Asp Gln Gln Asn Ile Lys
            580                 585                 590
Val Pro Gly Asn Leu Thr Ser Val Leu Leu Asn Asn Leu His Pro Arg
        595                 600                 605
Glu Gln Tyr Val Val Arg Ala Arg Val Asn Thr Lys Ala Gln Gly Glu
    610                 615                 620
Trp Ser Glu Asp Leu Thr Ala Trp Thr Leu Ser Asp Ile Leu Pro Pro
625                 630                 635                 640
Gln Pro Glu Asn Ile Lys Ile Ser Asn Ile Thr His Ser Ser Ala Val
                645                 650                 655

Ile Ser Trp Thr Ile Leu Asp Gly Tyr Ser Ile Ser Ser Ile Thr Ile

            660                 665                 670

Arg Tyr Lys Val Gln Gly Lys Asn Glu Asp Gln His Val Asp Val Lys

        675                 680                 685

Ile Lys Asn Ala Thr Ile Ile Gln Tyr Gln Leu Lys Gly Leu Glu Pro

    690                 695                 700

Glu Thr Ala Tyr Gln Val Asp Ile Phe Ala Glu Asn Asn Ile Gly Ser

705                 710                 715                 720

Ser Asn Pro Ala Phe Ser His Glu Leu Val Thr Leu Pro Glu Ser Gln

                725                 730                 735

Ala Pro Ala Asp Leu Gly Gly Gly Lys Met Leu Leu Ile Ala Ile Leu

            740                 745                 750

Gly Ser Ala Gly Met Thr Cys Leu Thr Val Leu Leu Ala Phe Leu Ile

        755                 760                 765

Ile Leu Gln Leu Lys Arg Ala Asn Val Gln Arg Arg Met Ala Gln Ala

    770                 775                 780

Phe Gln Asn Val Arg Glu Glu Pro Ala Val Gln Phe Asn Ser Gly Thr

785                 790                 795                 800

Leu Ala Leu Asn Arg Lys Val Lys Asn Asn Pro Asp Pro Thr Ile Tyr

                805                 810                 815

Pro Val Leu Asp Trp Asn Asp Ile Lys Phe Gln Asp Val Ile Gly Glu

            820                 825                 830

Gly Asn Phe Gly Gln Val Leu Lys Ala Arg Ile Lys Lys Asp Gly Leu

        835                 840                 845

Arg Met Asp Ala Ala Ile Lys Arg Met Lys Glu Tyr Ala Ser Lys Asp

    850                 855                 860

Asp His Arg Asp Phe Ala Gly Glu Leu Glu Val Leu Cys Lys Leu Gly

865                 870                 875                 880

His His Pro Asn Ile Ile Asn Leu Leu Gly Ala Cys Glu His Arg Gly

                885                 890                 895

Tyr Leu Tyr Leu Ala Ile Glu Tyr Ala Pro His Gly Asn Leu Leu Asp

            900                 905                 910

Phe Leu Arg Lys Ser Arg Val Leu Glu Thr Asp Pro Ala Phe Ala Ile

        915                 920                 925

Ala Asn Ser Thr Ala Ser Thr Leu Ser Ser Gln Gln Leu Leu His Phe

    930                 935                 940

Ala Ala Asp Val Ala Arg Gly Met Asp Tyr Leu Ser Gln Lys Gln Phe

945                 950                 955                 960

Ile His Arg Asp Leu Ala Ala Arg Asn Ile Leu Val Gly Glu Asn Tyr

                965                 970                 975

Val Ala Lys Ile Ala Asp Phe Gly Leu Ser Arg Gly Gln Glu Val Tyr

            980                 985                 990

Val Lys Lys Thr Met Gly Arg Leu Pro Val Arg Trp Met Ala Ile Glu

        995                 1000                1005

Ser Leu Asn Tyr Ser Val Tyr Thr Thr Asn Ser Asp Val Trp Ser

    1010                1015                1020

Tyr Gly Val Leu Leu Trp Glu Ile Val Ser Leu Gly Gly Thr Pro

    1025                1030                1035

Tyr Cys Gly Met Thr Cys Ala Glu Leu Tyr Glu Lys Leu Pro Gln

    1040                1045                1050

Gly Tyr Arg Leu Glu Lys Pro Leu Asn Cys Asp Asp Glu Val Tyr

    1055                1060                1065

Asp Leu Met Arg Gln Cys Trp Arg Glu Lys Pro Tyr Glu Arg Pro

    1070                1075                1080

Ser Phe Ala Gln Ile Leu Val Ser Leu Asn Arg Met Leu Glu Glu

    1085                1090                1095

Arg Lys Thr Tyr Val Asn Thr Thr Leu Tyr Glu Lys Phe Thr Tyr

    1100                1105                1110

Ala Gly Ile Asp Cys Ser Ala Glu Glu Ala Ala

    1115                1120

<210> 48

<211> 103

<212> PRT

<213> Homo sapiens

<220>

<221> ATP synthase, H+ transporting, mitochondrial F0 complex, subunit

g

<222> (1)..(103)

<223> LocusID: 10632

NM_006476

<400> 48

Met Ala Gln Phe Val Arg Asn Leu Val Glu Lys Thr Pro Ala Leu Val
1               5                   10                  15

Asn Ala Ala Val Thr Tyr Ser Lys Pro Arg Leu Ala Thr Phe Trp Tyr
            20                  25                  30

Tyr Ala Lys Val Glu Leu Val Pro Pro Thr Pro Ala Glu Ile Pro Arg
        35                  40                  45

Ala Ile Gln Ser Leu Lys Lys Ile Val Asn Ser Ala Gln Thr Gly Ser
    50                  55                  60

Phe Lys Gln Leu Thr Val Lys Glu Ala Val Leu Asn Gly Leu Val Ala
65                  70                  75                  80

Thr Glu Val Leu Met Trp Phe Tyr Val Gly Glu Ile Ile Gly Lys Arg
                85                  90                  95

Gly Ile Ile Gly Tyr Asp Val
            100

<210> 49

<211> 148

<212> PRT

<213> Homo sapiens

<220>

<221>  ATP synthase, H+ transporting, mitochondrial F0 complex, subunit

       b, isoform 1

<222>  (1)..(148)

<223>  LocusID: 515

transcript variant 2

NM_001002014

<400>  49

Met Val Tyr Gly Ile Lys Lys Tyr Gly Pro Phe Val Ala Asp Phe Ala

1               5                   10                  15

Asp Lys Leu Asn Glu Gln Lys Leu Ala Gln Leu Glu Glu Ala Lys Gln

            20                  25                  30

Ala Ser Ile Gln His Ile Gln Asn Ala Ile Asp Thr Glu Lys Ser Gln

        35                  40                  45

Gln Ala Leu Val Gln Lys Arg His Tyr Leu Phe Asp Val Gln Arg Asn

    50                  55                  60

Asn Ile Ala Met Ala Leu Glu Val Thr Tyr Arg Glu Arg Leu Tyr Arg

65                  70                  75                  80

Val Tyr Lys Glu Val Lys Asn Arg Leu Asp Tyr His Ile Ser Val Gln

                85                  90                  95

Asn Met Met Arg Arg Lys Glu Gln Glu His Met Ile Asn Trp Val Glu

            100                 105                 110

Lys His Val Val Gln Ser Ile Ser Thr Gln Gln Glu Lys Glu Thr Ile

        115                 120                 125

Ala Lys Cys Ile Ala Asp Leu Lys Leu Leu Ala Lys Lys Ala Gln Ala

    130                 135                 140

Gln Pro Val Met

145

<210> 50

<211> 195

<212> PRT

<213> Homo sapiens

<220>

<221> ATP synthase, H+ transporting, mitochondrial F0 complex, subunit

b, isoform 1

<222> (1)..(195)

<223> LocusID: 515

transcript variant 3

NM_001002015

<400> 50

Met Leu Ser Arg Val Val Leu Ser Ala Ala Ala Thr Ala Gly Pro Tyr
1               5                   10                  15

Val Leu Gly Thr Gly Leu Ile Leu Tyr Ala Leu Ser Lys Glu Ile Tyr
            20                  25                  30

Val Ile Ser Ala Glu Thr Phe Thr Ala Leu Ser Val Leu Gly Val Met
        35                  40                  45

Val Tyr Gly Ile Lys Lys Tyr Gly Pro Phe Val Ala Asp Phe Ala Asp
    50                  55                  60

Lys Leu Asn Glu Gln Lys Leu Ala Gln Leu Glu Glu Ala Lys Gln Ala
65                  70                  75                  80

Ser Ile Gln His Ile Gln Asn Ala Ile Asp Thr Glu Lys Ser Gln Gln
                85                  90                  95

Ala Leu Val Gln Lys Arg His Tyr Leu Phe Asp Val Gln Arg Asn Asn
            100                 105                 110

Ile Ala Met Ala Leu Glu Val Thr Tyr Arg Glu Arg Leu Tyr Arg Val
        115                 120                 125

Tyr Lys Glu Val Lys Asn Arg Leu Asp Tyr His Ile Ser Val Gln Asn
    130                 135                 140

Met Met Arg Arg Lys Glu Gln Glu His Met Ile Asn Trp Val Glu Lys
145                 150                 155                 160

His Val Val Gln Ser Ile Ser Thr Gln Gln Glu Lys Glu Thr Ile Ala
                165                 170                 175

Lys Cys Ile Ala Asp Leu Lys Leu Leu Ala Lys Lys Ala Gln Ala Gln

            180                 185                 190

Pro Val Met

        195

<210> 51

<211> 256

<212> PRT

<213> Homo sapiens

<220>

<221> ATP synthase, H+ transporting, mitochondrial F0 complex, subunit

b, isoform 1

<222> (1)..(256)

<223> LocusID: 515

transcript variant 1

NM_001688

<400> 51

Met Leu Ser Arg Val Val Leu Ser Ala Ala Ala Thr Ala Ala Pro Ser

1               5                   10                  15

Leu Lys Asn Ala Ala Phe Leu Gly Pro Gly Val Leu Gln Ala Thr Arg
            20                  25                  30
Thr Phe His Thr Gly Gln Pro His Leu Val Pro Val Pro Pro Leu Pro
        35                  40                  45
Glu Tyr Gly Gly Lys Val Arg Tyr Gly Leu Ile Pro Glu Glu Phe Phe
    50                  55                  60
Gln Phe Leu Tyr Pro Lys Thr Gly Val Thr Gly Pro Tyr Val Leu Gly
65                  70                  75                  80
Thr Gly Leu Ile Leu Tyr Ala Leu Ser Lys Glu Ile Tyr Val Ile Ser
                85                  90                  95
Ala Glu Thr Phe Thr Ala Leu Ser Val Leu Gly Val Met Val Tyr Gly
            100                 105                 110
Ile Lys Lys Tyr Gly Pro Phe Val Ala Asp Phe Ala Asp Lys Leu Asn
        115                 120                 125
Glu Gln Lys Leu Ala Gln Leu Glu Glu Ala Lys Gln Ala Ser Ile Gln
    130                 135                 140
His Ile Gln Asn Ala Ile Asp Thr Glu Lys Ser Gln Gln Ala Leu Val
145                 150                 155                 160
Gln Lys Arg His Tyr Leu Phe Asp Val Gln Arg Asn Asn Ile Ala Met
                165                 170                 175
Ala Leu Glu Val Thr Tyr Arg Glu Arg Leu Tyr Arg Val Tyr Lys Glu
            180                 185                 190
Val Lys Asn Arg Leu Asp Tyr His Ile Ser Val Gln Asn Met Met Arg
        195                 200                 205

Arg Lys Glu Gln Glu His Met Ile Asn Trp Val Glu Lys His Val Val

    210                 215                 220

Gln Ser Ile Ser Thr Gln Gln Glu Lys Glu Thr Ile Ala Lys Cys Ile

225                 230                 235                 240

Ala Asp Leu Lys Leu Leu Ala Lys Lys Ala Gln Ala Gln Pro Val Met

                245                 250                 255

<210>  52

<211>  155

<212>  PRT

<213>  Homo sapiens

<220>

<221>  thyroid hormone receptor interactor 3

<222>  (1)..(155)

<223>  LocusID: 9326

NM_004773

<400>  52

<210> 53

<211> 334

<212> PRT

<213> Homo sapiens

<220>

<221>  lactate dehydrogenase B

<222>  (1)..(334)

<223>  LocusID: 3945

NM_002300

<400>  53

Met Ala Thr Leu Lys Glu Lys Leu Ile Ala Pro Val Ala Glu Glu Glu
1               5                   10                  15

Ala Thr Val Pro Asn Asn Lys Ile Thr Val Val Gly Val Gly Gln Val
            20                  25                  30

Gly Met Ala Cys Ala Ile Ser Ile Leu Gly Lys Ser Leu Ala Asp Glu
        35                  40                  45

Leu Ala Leu Val Asp Val Leu Glu Asp Lys Leu Lys Gly Glu Met Met
    50                  55                  60

Asp Leu Gln His Gly Ser Leu Phe Leu Gln Thr Pro Lys Ile Val Ala
65                  70                  75                  80

Asp Lys Asp Tyr Ser Val Thr Ala Asn Ser Lys Ile Val Val Val Thr
                85                  90                  95

Ala Gly Val Arg Gln Gln Glu Gly Glu Ser Arg Leu Asn Leu Val Gln
            100                 105                 110

Arg Asn Val Asn Val Phe Lys Phe Ile Ile Pro Gln Ile Val Lys Tyr

        115                 120                 125

Ser Pro Asp Cys Ile Ile Ile Val Val Ser Asn Pro Val Asp Ile Leu

    130                 135                 140

Thr Tyr Val Thr Trp Lys Leu Ser Gly Leu Pro Lys His Arg Val Ile

145                 150                 155                 160

Gly Ser Gly Cys Asn Leu Asp Ser Ala Arg Phe Arg Tyr Leu Met Ala

                165                 170                 175

Glu Lys Leu Gly Ile His Pro Ser Ser Cys His Gly Trp Ile Leu Gly

            180                 185                 190

Glu His Gly Asp Ser Ser Val Ala Val Trp Ser Gly Val Asn Val Ala

        195                 200                 205

Gly Val Ser Leu Gln Glu Leu Asn Pro Glu Met Gly Thr Asp Asn Asp

    210                 215                 220

Ser Glu Asn Trp Lys Glu Val His Lys Met Val Val Glu Ser Ala Tyr

225                 230                 235                 240

Glu Val Ile Lys Leu Lys Gly Tyr Thr Asn Trp Ala Ile Gly Leu Ser

                245                 250                 255

Val Ala Asp Leu Ile Glu Ser Met Leu Lys Asn Leu Ser Arg Ile His

            260                 265                 270

Pro Val Ser Thr Met Val Lys Gly Met Tyr Gly Ile Glu Asn Glu Val

        275                 280                 285

Phe Leu Ser Leu Pro Cys Ile Leu Asn Ala Arg Gly Leu Thr Ser Val

    290                 295                 300

Ile Asn Gln Lys Leu Lys Asp Asp Glu Val Ala Gln Leu Lys Lys Ser

305                 310                 315                 320

Ala Asp Thr Leu Trp Asp Ile Gln Lys Asp Leu Lys Asp Leu

                325                 330

<210>  54

<211>  133

<212>  PRT

<213>  Homo sapiens

<220>

<221>  fatty acid binding protein 3

<222>  (1)..(133)

<223>  LocusID: 2170

NM_004102

<400>  54

<210>  55

<211>  350

<212>  PRT

<213>  Homo sapiens

<220>

<221>  glycogenin

<222>  (1)..(350)

<223>  LocusID: 2992

NM_004130

<400> 55

Met Thr Asp Gln Ala Phe Val Thr Leu Thr Thr Asn Asp Ala Tyr Ala
1               5                   10                  15

Lys Gly Ala Leu Val Leu Gly Ser Ser Leu Lys Gln His Arg Thr Thr
            20                  25                  30

Arg Arg Leu Val Val Leu Ala Thr Pro Gln Val Ser Asp Ser Met Arg
        35                  40                  45

Lys Val Leu Glu Thr Val Phe Asp Glu Val Ile Met Val Asp Val Leu
    50                  55                  60

Asp Ser Gly Asp Ser Ala His Leu Thr Leu Met Lys Arg Pro Glu Leu
65                  70                  75                  80

Gly Val Thr Leu Thr Lys Leu His Cys Trp Ser Leu Thr Gln Tyr Ser
                85                  90                  95

Lys Cys Val Phe Met Asp Ala Asp Thr Leu Val Leu Ala Asn Ile Asp
            100                 105                 110

Asp Leu Phe Asp Arg Glu Glu Leu Ser Ala Ala Pro Asp Pro Gly Trp
        115                 120                 125

Pro Asp Cys Phe Asn Ser Gly Val Phe Val Tyr Gln Pro Ser Val Glu
    130                 135                 140

Thr Tyr Asn Gln Leu Leu His Leu Ala Ser Glu Gln Gly Ser Phe Asp
145                 150                 155                 160

Gly Gly Asp Gln Gly Ile Leu Asn Thr Phe Phe Ser Ser Trp Ala Thr

                165                 170                 175
Thr Asp Ile Arg Lys His Leu Pro Phe Ile Tyr Asn Leu Ser Ser Ile
            180                 185                 190
Ser Ile Tyr Ser Tyr Leu Pro Ala Phe Lys Val Phe Gly Ala Ser Ala
        195                 200                 205
Lys Val Val His Phe Leu Gly Arg Val Lys Pro Trp Asn Tyr Thr Tyr
    210                 215                 220
Asp Pro Lys Thr Lys Ser Val Lys Ser Glu Ala His Asp Pro Asn Met
225                 230                 235                 240
Thr His Pro Glu Phe Leu Ile Leu Trp Trp Asn Ile Phe Thr Thr Asn
                245                 250                 255
Val Leu Pro Leu Leu Gln Gln Phe Gly Leu Val Lys Asp Thr Cys Ser
            260                 265                 270
Tyr Val Asn Val Leu Ser Asp Leu Val Tyr Thr Leu Ala Phe Ser Cys
        275                 280                 285
Gly Phe Cys Arg Lys Glu Asp Val Ser Gly Ala Ile Ser His Leu Ser
    290                 295                 300
Leu Gly Glu Ile Pro Ala Met Ala Gln Pro Phe Val Ser Ser Glu Glu
305                 310                 315                 320
Arg Lys Glu Arg Trp Glu Gln Gly Gln Ala Asp Tyr Met Gly Ala Asp
                325                 330                 335
Ser Phe Asp Asn Ile Lys Arg Lys Leu Asp Thr Tyr Leu Gln
            340                 345                 350

<210>  56

<211>  444

<212>  PRT

<213>  Homo sapiens

<220>

<221>  fatty acid desaturase 2

<222>  (1)..(444)

<223>  LocusID: 9415

NM_004265

<400>  56

Met Gly Lys Gly Gly Asn Gln Gly Glu Gly Ala Ala Glu Arg Glu Val
1               5                   10                  15

Ser Val Pro Thr Phe Ser Trp Glu Glu Ile Gln Lys His Asn Leu Arg
            20                  25                  30

Thr Asp Arg Trp Leu Val Ile Asp Arg Lys Val Tyr Asn Ile Thr Lys
        35                  40                  45

Trp Ser Ile Gln His Pro Gly Gly Gln Arg Val Ile Gly His Tyr Ala
    50                  55                  60

Gly Glu Asp Ala Thr Asp Ala Phe Arg Ala Phe His Pro Asp Leu Glu
65                  70                  75                  80

Phe Val Gly Lys Phe Leu Lys Pro Leu Leu Ile Gly Glu Leu Ala Pro
                85                  90                  95
Glu Glu Pro Ser Gln Asp His Gly Lys Asn Ser Lys Ile Thr Glu Asp
            100                 105                 110
Phe Arg Ala Leu Arg Lys Thr Ala Glu Asp Met Asn Leu Phe Lys Thr
        115                 120                 125
Asn His Val Phe Phe Leu Leu Leu Leu Ala His Ile Ile Ala Leu Glu
    130                 135                 140
Ser Ile Ala Trp Phe Thr Val Phe Tyr Phe Gly Asn Gly Trp Ile Pro
145                 150                 155                 160
Thr Leu Ile Thr Ala Phe Val Leu Ala Thr Ser Gln Ala Gln Ala Gly
                165                 170                 175
Trp Leu Gln His Asp Tyr Gly His Leu Ser Val Tyr Arg Lys Pro Lys
            180                 185                 190
Trp Asn His Leu Val His Lys Phe Val Ile Gly His Leu Lys Gly Ala
        195                 200                 205
Ser Ala Asn Trp Trp Asn His Arg His Phe Gln His His Ala Lys Pro
    210                 215                 220
Asn Ile Phe His Lys Asp Pro Asp Val Asn Met Leu His Val Phe Val
225                 230                 235                 240
Leu Gly Glu Trp Gln Pro Ile Glu Tyr Gly Lys Lys Lys Leu Lys Tyr
                245                 250                 255
Leu Pro Tyr Asn His Gln His Glu Tyr Phe Phe Leu Ile Gly Pro Pro
            260                 265                 270

Leu Leu Ile Pro Met Tyr Phe Gln Tyr Gln Ile Ile Met Thr Met Ile

        275                 280                 285

Val His Lys Asn Trp Val Asp Leu Ala Trp Ala Val Ser Tyr Tyr Ile

    290                 295                 300

Arg Phe Phe Ile Thr Tyr Ile Pro Phe Tyr Gly Ile Leu Gly Ala Leu

305                 310                 315                 320

Leu Phe Leu Asn Phe Ile Arg Phe Leu Glu Ser His Trp Phe Val Trp

                325                 330                 335

Val Thr Gln Met Asn His Ile Val Met Glu Ile Asp Gln Glu Ala Tyr

            340                 345                 350

Arg Asp Trp Phe Ser Ser Gln Leu Thr Ala Thr Cys Asn Val Glu Gln

        355                 360                 365

Ser Phe Phe Asn Asp Trp Phe Ser Gly His Leu Asn Phe Gln Ile Glu

    370                 375                 380

His His Leu Phe Pro Thr Met Pro Arg His Asn Leu His Lys Ile Ala

385                 390                 395                 400

Pro Leu Val Lys Ser Leu Cys Ala Lys His Gly Ile Glu Tyr Gln Glu

                405                 410                 415

Lys Pro Leu Leu Arg Ala Leu Leu Asp Ile Ile Arg Ser Leu Lys Lys

            420                 425                 430

Ser Gly Lys Leu Trp Leu Asp Ala Tyr Leu His Lys

        435                 440

<210>  57

<211>  548

<212>  PRT

<213>  Homo sapiens

<220>

<221>  RAR-related orphan receptor A variant 3

<222>  (1)..(548)

<223>  LocusID: 6095

NM_002943

<400>  57

Met Asn Glu Gly Ala Pro Gly Asp Ser Asp Leu Glu Thr Glu Ala Arg

1               5                   10                  15

Val Pro Trp Ser Ile Met Gly His Cys Leu Arg Thr Gly Gln Ala Arg

            20                  25                  30

Met Ser Ala Thr Pro Thr Pro Ala Gly Glu Gly Ala Arg Ser Ser Ser

        35                  40                  45

Thr Cys Ser Ser Leu Ser Arg Leu Phe Trp Ser Gln Leu Glu His Ile

    50                  55                  60

Asn Trp Asp Gly Ala Thr Ala Lys Asn Phe Ile Asn Leu Arg Glu Phe

65                  70                  75                  80

Phe Ser Phe Leu Leu Pro Ala Leu Arg Lys Ala Gln Ile Glu Ile Ile

                85                  90                  95

Pro Cys Lys Ile Cys Gly Asp Lys Ser Ser Gly Ile His Tyr Gly Val

            100                 105                 110

Ile Thr Cys Glu Gly Cys Lys Gly Phe Phe Arg Arg Ser Gln Gln Ser

        115                 120                 125

Asn Ala Thr Tyr Ser Cys Pro Arg Gln Lys Asn Cys Leu Ile Asp Arg

    130                 135                 140

Thr Ser Arg Asn Arg Cys Gln His Cys Arg Leu Gln Lys Cys Leu Ala

145                 150                 155                 160

Val Gly Met Ser Arg Asp Ala Val Lys Phe Gly Arg Met Ser Lys Lys

                165                 170                 175

Gln Arg Asp Ser Leu Tyr Ala Glu Val Gln Lys His Arg Met Gln Gln

            180                 185                 190

Gln Gln Arg Asp His Gln Gln Gln Pro Gly Glu Ala Glu Pro Leu Thr

        195                 200                 205

Pro Thr Tyr Asn Ile Ser Ala Asn Gly Leu Thr Glu Leu His Asp Asp

    210                 215                 220

Leu Ser Asn Tyr Ile Asp Gly His Thr Pro Glu Gly Ser Lys Ala Asp

225                 230                 235                 240

Ser Ala Val Ser Ser Phe Tyr Leu Asp Ile Gln Pro Ser Pro Asp Gln

                245                 250                 255

Ser Gly Leu Asp Ile Asn Gly Ile Lys Pro Glu Pro Ile Cys Asp Tyr

            260                 265                 270

Thr Pro Ala Ser Gly Phe Phe Pro Tyr Cys Ser Phe Thr Asn Gly Glu

        275                 280                 285

Thr Ser Pro Thr Val Ser Met Ala Glu Leu Glu His Leu Ala Gln Asn

    290                 295                 300

Ile Ser Lys Ser His Leu Glu Thr Cys Gln Tyr Leu Arg Glu Glu Leu

305                 310                 315                 320

Gln Gln Ile Thr Trp Gln Thr Phe Leu Gln Glu Glu Ile Glu Asn Tyr

                325                 330                 335

Gln Asn Lys Gln Arg Glu Val Met Trp Gln Leu Cys Ala Ile Lys Ile

            340                 345                 350

Thr Glu Ala Ile Gln Tyr Val Val Glu Phe Ala Lys Arg Ile Asp Gly

        355                 360                 365

Phe Met Glu Leu Cys Gln Asn Asp Gln Ile Val Leu Leu Lys Ala Gly

    370                 375                 380

Ser Leu Glu Val Val Phe Ile Arg Met Cys Arg Ala Phe Asp Ser Gln

385                 390                 395                 400

Asn Asn Thr Val Tyr Phe Asp Gly Lys Tyr Ala Ser Pro Asp Val Phe

                405                 410                 415

Lys Ser Leu Gly Cys Glu Asp Phe Ile Ser Phe Val Phe Glu Phe Gly

            420                 425                 430

Lys Ser Leu Cys Ser Met His Leu Thr Glu Asp Glu Ile Ala Leu Phe

        435                 440                 445

Ser Ala Phe Val Leu Met Ser Ala Asp Arg Ser Trp Leu Gln Glu Lys

    450                 455                 460

Val Lys Ile Glu Lys Leu Gln Gln Lys Ile Gln Leu Ala Leu Gln His

465                 470                 475                 480

Val Leu Gln Lys Asn His Arg Glu Asp Gly Ile Leu Thr Lys Leu Ile

                485                 490                 495

Cys Lys Val Ser Thr Leu Arg Ala Leu Cys Gly Arg His Thr Glu Lys

            500                 505                 510

Leu Met Ala Phe Lys Ala Ile Tyr Pro Asp Ile Val Arg Leu His Phe

        515                 520                 525

Pro Pro Leu Tyr Lys Glu Leu Phe Thr Ser Glu Phe Glu Pro Ala Met

    530                 535                 540

Gln Ile Asp Gly

545

<210>  58

<211>  556

<212>  PRT

<213>  Homo sapiens

<220>

<221>  RAR-related orphan receptor A variant 2

<222>  (1)..(556)

<223>  LocusID: 825

NM_134260

<400> 58

Met Asn Glu Gly Ala Pro Gly Asp Ser Asp Leu Glu Thr Glu Ala Arg
1               5                   10                  15
Val Pro Trp Ser Ile Met Gly His Cys Leu Arg Thr Gly Gln Ala Arg
            20                  25                  30
Met Ser Ala Thr Pro Thr Pro Ala Gly Glu Gly Ala Arg Arg Asp Glu
        35                  40                  45
Leu Phe Gly Ile Leu Gln Ile Leu His Gln Cys Ile Leu Ser Ser Gly
    50                  55                  60
Asp Ala Phe Val Leu Thr Gly Val Cys Cys Ser Trp Arg Gln Asn Gly
65                  70                  75                  80
Lys Pro Pro Tyr Ser Gln Lys Glu Asp Lys Glu Val Gln Thr Gly Tyr
                85                  90                  95
Met Asn Ala Gln Ile Glu Ile Ile Pro Cys Lys Ile Cys Gly Asp Lys
            100                 105                 110
Ser Ser Gly Ile His Tyr Gly Val Ile Thr Cys Glu Gly Cys Lys Gly
        115                 120                 125
Phe Phe Arg Arg Ser Gln Gln Ser Asn Ala Thr Tyr Ser Cys Pro Arg
    130                 135                 140
Gln Lys Asn Cys Leu Ile Asp Arg Thr Ser Arg Asn Arg Cys Gln His
145                 150                 155                 160
Cys Arg Leu Gln Lys Cys Leu Ala Val Gly Met Ser Arg Asp Ala Val
                165                 170                 175

Lys Phe Gly Arg Met Ser Lys Lys Gln Arg Asp Ser Leu Tyr Ala Glu
            180                 185                 190
Val Gln Lys His Arg Met Gln Gln Gln Gln Arg Asp His Gln Gln Gln
        195                 200                 205
Pro Gly Glu Ala Glu Pro Leu Thr Pro Thr Tyr Asn Ile Ser Ala Asn
    210                 215                 220
Gly Leu Thr Glu Leu His Asp Asp Leu Ser Asn Tyr Ile Asp Gly His
225                 230                 235                 240
Thr Pro Glu Gly Ser Lys Ala Asp Ser Ala Val Ser Ser Phe Tyr Leu
                245                 250                 255
Asp Ile Gln Pro Ser Pro Asp Gln Ser Gly Leu Asp Ile Asn Gly Ile
            260                 265                 270
Lys Pro Glu Pro Ile Cys Asp Tyr Thr Pro Ala Ser Gly Phe Phe Pro
        275                 280                 285
Tyr Cys Ser Phe Thr Asn Gly Glu Thr Ser Pro Thr Val Ser Met Ala
    290                 295                 300
Glu Leu Glu His Leu Ala Gln Asn Ile Ser Lys Ser His Leu Glu Thr
305                 310                 315                 320
Cys Gln Tyr Leu Arg Glu Glu Leu Gln Gln Ile Thr Trp Gln Thr Phe
                325                 330                 335
Leu Gln Glu Glu Ile Glu Asn Tyr Gln Asn Lys Gln Arg Glu Val Met
            340                 345                 350
Trp Gln Leu Cys Ala Ile Lys Ile Thr Glu Ala Ile Gln Tyr Val Val
        355                 360                 365

Glu Phe Ala Lys Arg Ile Asp Gly Phe Met Glu Leu Cys Gln Asn Asp

    370                 375                 380

Gln Ile Val Leu Leu Lys Ala Gly Ser Leu Glu Val Val Phe Ile Arg

385                 390                 395                 400

Met Cys Arg Ala Phe Asp Ser Gln Asn Asn Thr Val Tyr Phe Asp Gly

                405                 410                 415

Lys Tyr Ala Ser Pro Asp Val Phe Lys Ser Leu Gly Cys Glu Asp Phe

            420                 425                 430

Ile Ser Phe Val Phe Glu Phe Gly Lys Ser Leu Cys Ser Met His Leu

        435                 440                 445

Thr Glu Asp Glu Ile Ala Leu Phe Ser Ala Phe Val Leu Met Ser Ala

    450                 455                 460

Asp Arg Ser Trp Leu Gln Glu Lys Val Lys Ile Glu Lys Leu Gln Gln

465                 470                 475                 480

Lys Ile Gln Leu Ala Leu Gln His Val Leu Gln Lys Asn His Arg Glu

                485                 490                 495

Asp Gly Ile Leu Thr Lys Leu Ile Cys Lys Val Ser Thr Leu Arg Ala

            500                 505                 510

Leu Cys Gly Arg His Thr Glu Lys Leu Met Ala Phe Lys Ala Ile Tyr

        515                 520                 525

Pro Asp Ile Val Arg Leu His Phe Pro Pro Leu Tyr Lys Glu Leu Phe

    530                 535                 540

Thr Ser Glu Phe Glu Pro Ala Met Gln Ile Asp Gly

545                 550                 555

<210>  59

<211>  523

<212>  PRT

<213>  Homo sapiens

<220>

<221>  RAR-related orphan receptor A variant 1

<222>  (1)..(523)

<223>  LocusID: 6095

NM_134261

<400>  59

<210>  60
<211>  468
<212>  PRT
<213>  Homo sapiens
<220>
<221>  RAR-related orphan receptor A variant 4
<222>  (1)..(468)
<223>  LocusID: 6095

NM_134262

<400>  60

Met Met Tyr Phe Val Ile Ala Ala Met Lys Ala Gln Ile Glu Ile Ile

1               5                   10                  15

Pro Cys Lys Ile Cys Gly Asp Lys Ser Ser Gly Ile His Tyr Gly Val

            20                  25                  30

Ile Thr Cys Glu Gly Cys Lys Gly Phe Phe Arg Arg Ser Gln Gln Ser

        35                  40                  45

Asn Ala Thr Tyr Ser Cys Pro Arg Gln Lys Asn Cys Leu Ile Asp Arg

    50                  55                  60

Thr Ser Arg Asn Arg Cys Gln His Cys Arg Leu Gln Lys Cys Leu Ala

65                  70                  75                  80

Val Gly Met Ser Arg Asp Ala Val Lys Phe Gly Arg Met Ser Lys Lys

                85                  90                  95

Gln Arg Asp Ser Leu Tyr Ala Glu Val Gln Lys His Arg Met Gln Gln

            100                 105                 110

Gln Gln Arg Asp His Gln Gln Gln Pro Gly Glu Ala Glu Pro Leu Thr

        115                 120                 125

Pro Thr Tyr Asn Ile Ser Ala Asn Gly Leu Thr Glu Leu His Asp Asp

    130                 135                 140

Leu Ser Asn Tyr Ile Asp Gly His Thr Pro Glu Gly Ser Lys Ala Asp

145                 150                 155                 160

Ser Ala Val Ser Ser Phe Tyr Leu Asp Ile Gln Pro Ser Pro Asp Gln

                165                 170                 175

Ser Gly Leu Asp Ile Asn Gly Ile Lys Pro Glu Pro Ile Cys Asp Tyr

            180                 185                 190

Thr Pro Ala Ser Gly Phe Phe Pro Tyr Cys Ser Phe Thr Asn Gly Glu

195                 200                 205

Thr Ser Pro Thr Val Ser Met Ala Glu Leu Glu His Leu Ala Gln Asn

    210                 215                 220

Ile Ser Lys Ser His Leu Glu Thr Cys Gln Tyr Leu Arg Glu Glu Leu

225                 230                 235                 240

Gln Gln Ile Thr Trp Gln Thr Phe Leu Gln Glu Glu Ile Glu Asn Tyr

                245                 250                 255

Gln Asn Lys Gln Arg Glu Val Met Trp Gln Leu Cys Ala Ile Lys Ile

            260                 265                 270

Thr Glu Ala Ile Gln Tyr Val Val Glu Phe Ala Lys Arg Ile Asp Gly

        275                 280                 285

Phe Met Glu Leu Cys Gln Asn Asp Gln Ile Val Leu Leu Lys Ala Gly

    290                 295                 300

Ser Leu Glu Val Val Phe Ile Arg Met Cys Arg Ala Phe Asp Ser Gln

305                 310                 315                 320

Asn Asn Thr Val Tyr Phe Asp Gly Lys Tyr Ala Ser Pro Asp Val Phe

                325                 330                 335

Lys Ser Leu Gly Cys Glu Asp Phe Ile Ser Phe Val Phe Glu Phe Gly

            340                 345                 350

Lys Ser Leu Cys Ser Met His Leu Thr Glu Asp Glu Ile Ala Leu Phe

        355                 360                 365

Ser Ala Phe Val Leu Met Ser Ala Asp Arg Ser Trp Leu Gln Glu Lys

    370                 375                 380

Val Lys Ile Glu Lys Leu Gln Gln Lys Ile Gln Leu Ala Leu Gln His

385                 390                 395                 400

Val Leu Gln Lys Asn His Arg Glu Asp Gly Ile Leu Thr Lys Leu Ile

                405                 410                 415

Cys Lys Val Ser Thr Leu Arg Ala Leu Cys Gly Arg His Thr Glu Lys

            420                 425                 430

Leu Met Ala Phe Lys Ala Ile Tyr Pro Asp Ile Val Arg Leu His Phe

        435                 440                 445

Pro Pro Leu Tyr Lys Glu Leu Phe Thr Ser Glu Phe Glu Pro Ala Met

    450                 455                 460

Gln Ile Asp Gly

465

<210>  61

<211>  780

<212>  PRT

<213>  Homo sapiens

<220>

<221>  phosphofructokinase, muscle

<222>  (1)..(780)

<223>  LocusID: 5213

NM_000289

<400> 61

<210> 62

<211> 821

<212> PRT

<213> Homo sapiens

<220>

<221> calpain 3, (p94) (CAPN3), transcript variant 1

<222> (1)..(821)

<223> LocusID: 825

NM_000070

<400> 62

<210> 63

<211> 815

<212> PRT

<213> Homo sapiens

<220>

<221> calpain 3, (p94) (CAPN3), transcript variant 2

<222> (1)..(815)

<223> LocusID: 825

NM_024344

<400> 63

Met Pro Thr Val Ile Ser Ala Ser Val Ala Pro Arg Thr Ala Ala Glu

1               5                   10                  15

Pro Arg Ser Pro Gly Pro Val Pro His Pro Ala Gln Ser Lys Ala Thr

            20                  25                  30

Glu Ala Gly Gly Gly Asn Pro Ser Gly Ile Tyr Ser Ala Ile Ile Ser

        35                  40                  45

Arg Asn Phe Pro Ile Ile Gly Val Lys Glu Lys Thr Phe Glu Gln Leu
    50                  55                  60
His Lys Lys Cys Leu Glu Lys Lys Val Leu Tyr Val Asp Pro Glu Phe
65                  70                  75                  80
Pro Pro Asp Glu Thr Ser Leu Phe Tyr Ser Gln Lys Phe Pro Ile Gln
                85                  90                  95
Phe Val Trp Lys Arg Pro Pro Glu Ile Cys Glu Asn Pro Arg Phe Ile
            100                 105                 110
Ile Asp Gly Ala Asn Arg Thr Asp Ile Cys Gln Gly Glu Leu Gly Asp
        115                 120                 125
Cys Trp Phe Leu Ala Ala Ile Ala Cys Leu Thr Leu Asn Gln His Leu
    130                 135                 140
Leu Phe Arg Val Ile Pro His Asp Gln Ser Phe Ile Glu Asn Tyr Ala
145                 150                 155                 160
Gly Ile Phe His Phe Gln Phe Trp Arg Tyr Gly Glu Trp Val Asp Val
                165                 170                 175
Val Ile Asp Asp Cys Leu Pro Thr Tyr Asn Asn Gln Leu Val Phe Thr
            180                 185                 190
Lys Ser Asn His Arg Asn Glu Phe Trp Ser Ala Leu Leu Glu Lys Ala
        195                 200                 205
Tyr Ala Lys Leu His Gly Ser Tyr Glu Ala Leu Lys Gly Gly Asn Thr
    210                 215                 220
Thr Glu Ala Met Glu Asp Phe Thr Gly Gly Val Ala Glu Phe Phe Glu
225                 230                 235                 240

Ile Arg Asp Ala Pro Ser Asp Met Tyr Lys Ile Met Lys Lys Ala Ile
                245                 250                 255
Glu Arg Gly Ser Leu Met Gly Cys Ser Ile Asp Asp Gly Thr Asn Met
            260                 265                 270
Thr Tyr Gly Thr Ser Pro Ser Gly Leu Asn Met Gly Glu Leu Ile Ala
        275                 280                 285
Arg Met Val Arg Asn Met Asp Asn Ser Leu Leu Gln Asp Ser Asp Leu
    290                 295                 300
Asp Pro Arg Gly Ser Asp Glu Arg Pro Thr Arg Thr Ile Ile Pro Val
305                 310                 315                 320
Gln Tyr Glu Thr Arg Met Ala Cys Gly Leu Val Arg Gly His Ala Tyr
                325                 330                 335
Ser Val Thr Gly Leu Asp Glu Val Pro Phe Lys Gly Glu Lys Val Lys
            340                 345                 350
Leu Val Arg Leu Arg Asn Pro Trp Gly Gln Val Glu Trp Asn Gly Ser
        355                 360                 365
Trp Ser Asp Arg Trp Lys Asp Trp Ser Phe Val Asp Lys Asp Glu Lys
    370                 375                 380
Ala Arg Leu Gln His Gln Val Thr Glu Asp Gly Glu Phe Trp Met Ser
385                 390                 395                 400
Tyr Glu Asp Phe Ile Tyr His Phe Thr Lys Leu Glu Ile Cys Asn Leu
                405                 410                 415
Thr Ala Asp Ala Leu Gln Ser Asp Lys Leu Gln Thr Trp Thr Val Ser
            420                 425                 430

Val Asn Glu Gly Arg Trp Val Arg Gly Cys Ser Ala Gly Gly Cys Arg

        435                 440                 445

Asn Phe Pro Asp Thr Phe Trp Thr Asn Pro Gln Tyr Arg Leu Lys Leu

    450                 455                 460

Leu Glu Glu Asp Asp Asp Pro Asp Asp Ser Glu Val Ile Cys Ser Phe

465                 470                 475                 480

Leu Val Ala Leu Met Gln Lys Asn Arg Arg Lys Asp Arg Lys Leu Gly

                485                 490                 495

Ala Ser Leu Phe Thr Ile Gly Phe Ala Ile Tyr Glu Val Pro Lys Glu

            500                 505                 510

Met His Gly Asn Lys Gln His Leu Gln Lys Asp Phe Phe Leu Tyr Asn

        515                 520                 525

Ala Ser Lys Ala Arg Ser Lys Thr Tyr Ile Asn Met Arg Glu Val Ser

    530                 535                 540

Gln Arg Phe Arg Leu Pro Pro Ser Glu Tyr Val Ile Val Pro Ser Thr

545                 550                 555                 560

Tyr Glu Pro His Gln Glu Gly Glu Phe Ile Leu Arg Val Phe Ser Glu

                565                 570                 575

Lys Arg Asn Leu Ser Glu Glu Val Glu Asn Thr Ile Ser Val Asp Arg

            580                 585                 590

Pro Val Pro Ile Ile Phe Val Ser Asp Arg Ala Asn Ser Asn Lys Glu

        595                 600                 605

Leu Gly Val Asp Gln Glu Ser Glu Glu Gly Lys Gly Lys Thr Ser Pro

    610                 615                 620

Asp Lys Gln Lys Gln Ser Pro Gln Pro Gln Pro Gly Ser Ser Asp Gln

625                 630                 635                 640

Glu Ser Glu Glu Gln Gln Gln Phe Arg Asn Ile Phe Lys Gln Ile Ala

                645                 650                 655

Gly Asp Asp Met Glu Ile Cys Ala Asp Glu Leu Lys Lys Val Leu Asn

            660                 665                 670

Thr Val Val Asn Lys His Lys Asp Leu Lys Thr His Gly Phe Thr Leu

        675                 680                 685

Glu Ser Cys Arg Ser Met Ile Ala Leu Met Asp Thr Asp Gly Ser Gly

    690                 695                 700

Lys Leu Asn Leu Gln Glu Phe His His Leu Trp Asn Lys Ile Lys Ala

705                 710                 715                 720

Trp Gln Lys Ile Phe Lys His Tyr Asp Thr Asp Gln Ser Gly Thr Ile

                725                 730                 735

Asn Ser Tyr Glu Met Arg Asn Ala Val Asn Asp Ala Gly Phe His Leu

            740                 745                 750

Asn Asn Gln Leu Tyr Asp Ile Ile Thr Met Arg Tyr Ala Asp Lys His

        755                 760                 765

Met Asn Ile Asp Phe Asp Ser Phe Ile Cys Cys Phe Val Arg Leu Glu

    770                 775                 780

Gly Met Phe Arg Ala Phe His Ala Phe Asp Lys Asp Gly Asp Gly Ile

785                 790                 795                 800

Ile Lys Leu Asn Val Leu Glu Trp Leu Gln Leu Thr Met Tyr Ala

                805                 810                 815

<210>  64

<211>  729

<212>  PRT

<213>  Homo sapiens

<220>

<221>  calpain 3, (p94) (CAPN3), transcript variant 3

<222>  (1)..(729)

<223>  LocusID: 825

NM_173087

<400>  64

Met Pro Thr Val Ile Ser Ala Ser Val Ala Pro Arg Thr Ala Ala Glu

1               5                   10                  15

Pro Arg Ser Pro Gly Pro Val Pro His Pro Ala Gln Ser Lys Ala Thr

            20                  25                  30

Glu Ala Gly Gly Gly Asn Pro Ser Gly Ile Tyr Ser Ala Ile Ile Ser

        35                  40                  45

Arg Asn Phe Pro Ile Ile Gly Val Lys Glu Lys Thr Phe Glu Gln Leu

    50                  55                  60

His Lys Lys Cys Leu Glu Lys Lys Val Leu Tyr Val Asp Pro Glu Phe

65                  70                  75                  80

Pro Pro Asp Glu Thr Ser Leu Phe Tyr Ser Gln Lys Phe Pro Ile Gln

                85                  90                  95

Phe Val Trp Lys Arg Pro Pro Glu Ile Cys Glu Asn Pro Arg Phe Ile

            100                 105                 110

Ile Asp Gly Ala Asn Arg Thr Asp Ile Cys Gln Gly Glu Leu Gly Asp

        115                 120                 125

Cys Trp Phe Leu Ala Ala Ile Ala Cys Leu Thr Leu Asn Gln His Leu

    130                 135                 140

Leu Phe Arg Val Ile Pro His Asp Gln Ser Phe Ile Glu Asn Tyr Ala

145                 150                 155                 160

Gly Ile Phe His Phe Gln Phe Trp Arg Tyr Gly Glu Trp Val Asp Val

                165                 170                 175

Val Ile Asp Asp Cys Leu Pro Thr Tyr Asn Asn Gln Leu Val Phe Thr

            180                 185                 190

Lys Ser Asn His Arg Asn Glu Phe Trp Ser Ala Leu Leu Glu Lys Ala

        195                 200                 205

Tyr Ala Lys Leu His Gly Ser Tyr Glu Ala Leu Lys Gly Gly Asn Thr

    210                 215                 220

Thr Glu Ala Met Glu Asp Phe Thr Gly Gly Val Ala Glu Phe Phe Glu

225                 230                 235                 240

Ile Arg Asp Ala Pro Ser Asp Met Tyr Lys Ile Met Lys Lys Ala Ile

                245                 250                 255

Glu Arg Gly Ser Leu Met Gly Cys Ser Ile Asp Thr Ile Ile Pro Val

            260                 265                 270

Gln Tyr Glu Thr Arg Met Ala Cys Gly Leu Val Arg Gly His Ala Tyr

        275                 280                 285

Ser Val Thr Gly Leu Asp Glu Val Pro Phe Lys Gly Glu Lys Val Lys

    290                 295                 300

Leu Val Arg Leu Arg Asn Pro Trp Gly Gln Val Glu Trp Asn Gly Ser

305                 310                 315                 320

Trp Ser Asp Arg Trp Lys Asp Trp Ser Phe Val Asp Lys Asp Glu Lys

                325                 330                 335

Ala Arg Leu Gln His Gln Val Thr Glu Asp Gly Glu Phe Trp Met Ser

            340                 345                 350

Tyr Glu Asp Phe Ile Tyr His Phe Thr Lys Leu Glu Ile Cys Asn Leu

        355                 360                 365

Thr Ala Asp Ala Leu Gln Ser Asp Lys Leu Gln Thr Trp Thr Val Ser

    370                 375                 380

Val Asn Glu Gly Arg Trp Val Arg Gly Cys Ser Ala Gly Gly Cys Arg

385                 390                 395                 400

Asn Phe Pro Asp Thr Phe Trp Thr Asn Pro Gln Tyr Arg Leu Lys Leu

                405                 410                 415

Leu Glu Glu Asp Asp Asp Pro Asp Asp Ser Glu Val Ile Cys Ser Phe

            420                 425                 430

Leu Val Ala Leu Met Gln Lys Asn Arg Arg Lys Asp Arg Lys Leu Gly

        435                 440                 445

Ala Ser Leu Phe Thr Ile Gly Phe Ala Ile Tyr Glu Val Pro Lys Glu

450                 455                 460

Met His Gly Asn Lys Gln His Leu Gln Lys Asp Phe Phe Leu Tyr Asn

465                 470                 475                 480

Ala Ser Lys Ala Arg Ser Lys Thr Tyr Ile Asn Met Arg Glu Val Ser

                485                 490                 495

Gln Arg Phe Arg Leu Pro Pro Ser Glu Tyr Val Ile Val Pro Ser Thr

            500                 505                 510

Tyr Glu Pro His Gln Glu Gly Glu Phe Ile Leu Arg Val Phe Ser Glu

        515                 520                 525

Lys Arg Asn Leu Ser Glu Glu Val Glu Asn Thr Ile Ser Val Asp Arg

    530                 535                 540

Pro Val Pro Gln Pro Gly Ser Ser Asp Gln Glu Ser Glu Glu Gln Gln

545                 550                 555                 560

Gln Phe Arg Asn Ile Phe Lys Gln Ile Ala Gly Asp Asp Met Glu Ile

                565                 570                 575

Cys Ala Asp Glu Leu Lys Lys Val Leu Asn Thr Val Val Asn Lys His

            580                 585                 590

Lys Asp Leu Lys Thr His Gly Phe Thr Leu Glu Ser Cys Arg Ser Met

        595                 600                 605

Ile Ala Leu Met Asp Thr Asp Gly Ser Gly Lys Leu Asn Leu Gln Glu

    610                 615                 620

Phe His His Leu Trp Asn Lys Ile Lys Ala Trp Gln Lys Ile Phe Lys

625                 630                 635                 640

His Tyr Asp Thr Asp Gln Ser Gly Thr Ile Asn Ser Tyr Glu Met Arg

                645                 650                 655

Asn Ala Val Asn Asp Ala Gly Phe His Leu Asn Asn Gln Leu Tyr Asp

            660                 665                 670

Ile Ile Thr Met Arg Tyr Ala Asp Lys His Met Asn Ile Asp Phe Asp

        675                 680                 685

Ser Phe Ile Cys Cys Phe Val Arg Leu Glu Gly Met Phe Arg Ala Phe

    690                 695                 700

His Ala Phe Asp Lys Asp Gly Asp Gly Ile Ile Lys Leu Asn Val Leu

705                 710                 715                 720

Glu Trp Leu Gln Leu Thr Met Tyr Ala

                725

<210> 65

<211> 309

<212> PRT

<213> Homo sapiens

<220>

<221> calpain 3, (p94) (CAPN3), transcript variant 4

<222> (1)..(309)

<223> LocusID: 825

NM_173088

<400> 65

Met His Gly Asn Lys Gln His Leu Gln Lys Asp Phe Phe Leu Tyr Asn
1               5                   10                  15
Ala Ser Lys Ala Arg Ser Lys Thr Tyr Ile Asn Met Arg Glu Val Ser
            20                  25                  30
Gln Arg Phe Arg Leu Pro Pro Ser Glu Tyr Val Ile Val Pro Ser Thr
        35                  40                  45
Tyr Glu Pro His Gln Glu Gly Glu Phe Ile Leu Arg Val Phe Ser Glu
    50                  55                  60
Lys Arg Asn Leu Ser Glu Glu Val Glu Asn Thr Ile Ser Val Asp Arg
65                  70                  75                  80
Pro Val Lys Lys Lys Lys Thr Lys Pro Ile Ile Phe Val Ser Asp Arg
                85                  90                  95
Ala Asn Ser Asn Lys Glu Leu Gly Val Asp Gln Glu Ser Glu Glu Gly
            100                 105                 110
Lys Gly Lys Thr Ser Pro Asp Lys Gln Lys Gln Ser Pro Gln Pro Gln
        115                 120                 125
Pro Gly Ser Ser Asp Gln Glu Ser Glu Glu Gln Gln Gln Phe Arg Asn
    130                 135                 140
Ile Phe Lys Gln Ile Ala Gly Asp Asp Met Glu Ile Cys Ala Asp Glu
145                 150                 155                 160
Leu Lys Lys Val Leu Asn Thr Val Val Asn Lys His Lys Asp Leu Lys
                165                 170                 175

Thr His Gly Phe Thr Leu Glu Ser Cys Arg Ser Met Ile Ala Leu Met

            180                 185                 190

Asp Thr Asp Gly Ser Gly Lys Leu Asn Leu Gln Glu Phe His His Leu

        195                 200                 205

Trp Asn Lys Ile Lys Ala Trp Gln Lys Ile Phe Lys His Tyr Asp Thr

    210                 215                 220

Asp Gln Ser Gly Thr Ile Asn Ser Tyr Glu Met Arg Asn Ala Val Asn

225                 230                 235                 240

Asp Ala Gly Phe His Leu Asn Asn Gln Leu Tyr Asp Ile Ile Thr Met

                245                 250                 255

Arg Tyr Ala Asp Lys His Met Asn Ile Asp Phe Asp Ser Phe Ile Cys

            260                 265                 270

Cys Phe Val Arg Leu Glu Gly Met Phe Arg Ala Phe His Ala Phe Asp

        275                 280                 285

Lys Asp Gly Asp Gly Ile Ile Lys Leu Asn Val Leu Glu Trp Leu Gln

    290                 295                 300

Leu Thr Met Tyr Ala

305

<210> 66

<211> 156

<212> PRT

<213> Homo sapiens

<220>

<221>  calpain 3, (p94) (CAPN3), transcript variant 5

<222>  (1)..(156)

<223>  LocusID: 825

NM_173089

<400>  66

Met Glu Ile Cys Ala Asp Glu Leu Lys Lys Val Leu Asn Thr Val Val
1               5                   10                  15

Asn Lys His Lys Asp Leu Lys Thr His Gly Phe Thr Leu Glu Ser Cys
            20                  25                  30

Arg Ser Met Ile Ala Leu Met Asp Thr Asp Gly Ser Gly Lys Leu Asn
        35                  40                  45

Leu Gln Glu Phe His His Leu Trp Asn Lys Ile Lys Ala Trp Gln Lys
    50                  55                  60

Ile Phe Lys His Tyr Asp Thr Asp Gln Ser Gly Thr Ile Asn Ser Tyr
65                  70                  75                  80

Glu Met Arg Asn Ala Val Asn Asp Ala Gly Phe His Leu Asn Asn Gln
                85                  90                  95

Leu Tyr Asp Ile Ile Thr Met Arg Tyr Ala Asp Lys His Met Asn Ile
            100                 105                 110

Asp Phe Asp Ser Phe Ile Cys Cys Phe Val Arg Leu Glu Gly Met Phe

        115                 120                 125

Arg Ala Phe His Ala Phe Asp Lys Asp Gly Asp Gly Ile Ile Lys Leu

    130                 135                 140

Asn Val Leu Glu Trp Leu Gln Leu Thr Met Tyr Ala

145                 150                 155

<210> 67

<211> 156

<212> PRT

<213> Homo sapiens

<220>

<221> calpain 3, (p94) (CAPN3), transcript variant 6

<222> (1)..(156)

<223> LocusID: 825

NM_173090

<400> 67

Met Glu Ile Cys Ala Asp Glu Leu Lys Lys Val Leu Asn Thr Val Val

1               5                   10                  15

Asn Lys His Lys Asp Leu Lys Thr His Gly Phe Thr Leu Glu Ser Cys

            20                  25                  30

Arg Ser Met Ile Ala Leu Met Asp Thr Asp Gly Ser Gly Lys Leu Asn

        35                  40                  45

Leu Gln Glu Phe His His Leu Trp Asn Lys Ile Lys Ala Trp Gln Lys

    50                  55                  60

Ile Phe Lys His Tyr Asp Thr Asp Gln Ser Gly Thr Ile Asn Ser Tyr

65                  70                  75                  80

Glu Met Arg Asn Ala Val Asn Asp Ala Gly Phe His Leu Asn Asn Gln

                85                  90                  95

Leu Tyr Asp Ile Ile Thr Met Arg Tyr Ala Asp Lys His Met Asn Ile

            100                 105                 110

Asp Phe Asp Ser Phe Ile Cys Cys Phe Val Arg Leu Glu Gly Met Phe

        115                 120                 125

Arg Ala Phe His Ala Phe Asp Lys Asp Gly Asp Gly Ile Ile Lys Leu

    130                 135                 140

Asn Val Leu Glu Trp Leu Gln Leu Thr Met Tyr Ala

145                 150                 155

<210>  68

<211>  426

<212>  PRT

<213>  Homo sapiens

<220>

<221>  calpain 3, (p94) (CAPN3), transcript variant 8

<222>  (1)..(426)

<223>  LocusID: 825

NM_212464

<400>  68

Met Ser Trp Gln Ile Ser Leu Lys Thr Gln Thr Thr Gln Gln Lys Asn
1               5                   10                  15

Glu Ile Cys Glu Asn Pro Arg Phe Ile Ile Asp Gly Ala Asn Arg Thr
            20                  25                  30

Asp Ile Cys Gln Gly Glu Leu Gly Asp Cys Trp Phe Leu Ala Ala Ile
        35                  40                  45

Ala Cys Leu Thr Leu Asn Gln His Leu Leu Phe Arg Val Ile Pro His
    50                  55                  60

Asp Gln Ser Phe Ile Glu Asn Tyr Ala Gly Ile Phe His Phe Gln Phe
65                  70                  75                  80

Trp Arg Tyr Gly Glu Trp Val Asp Val Val Ile Asp Asp Cys Leu Pro
                85                  90                  95

Thr Tyr Asn Asn Gln Leu Val Phe Thr Lys Ser Asn His Arg Asn Glu
            100                 105                 110

Phe Trp Ser Ala Leu Leu Glu Lys Ala Tyr Ala Lys Leu His Gly Ser
        115                 120                 125

Tyr Glu Ala Leu Lys Gly Gly Asn Thr Thr Glu Ala Met Glu Asp Phe

    130                 135                 140

Thr Gly Gly Val Ala Glu Phe Phe Glu Ile Arg Asp Ala Pro Ser Asp

145                 150                 155                 160

Met Tyr Lys Ile Met Lys Lys Ala Ile Glu Arg Gly Ser Leu Met Gly

                165                 170                 175

Cys Ser Ile Asp Asp Gly Thr Asn Met Thr Tyr Gly Thr Ser Pro Ser

            180                 185                 190

Gly Leu Asn Met Gly Glu Leu Ile Ala Arg Met Val Arg Asn Met Asp

        195                 200                 205

Asn Ser Leu Leu Gln Asp Ser Asp Leu Asp Pro Arg Gly Ser Asp Glu

    210                 215                 220

Arg Pro Thr Arg Thr Ile Ile Pro Val Gln Tyr Glu Thr Arg Met Ala

225                 230                 235                 240

Cys Gly Leu Val Arg Gly His Ala Tyr Ser Val Thr Gly Leu Asp Glu

                245                 250                 255

Val Pro Phe Lys Gly Glu Lys Val Lys Leu Val Arg Leu Arg Asn Pro

            260                 265                 270

Trp Gly Gln Val Glu Trp Asn Gly Ser Trp Ser Asp Arg Trp Lys Asp

        275                 280                 285

Trp Ser Phe Val Asp Lys Asp Glu Lys Ala Arg Leu Gln His Gln Val

    290                 295                 300

Thr Glu Asp Gly Glu Phe Trp Met Ser Tyr Glu Asp Phe Ile Tyr His

305                 310                 315                 320

Phe Thr Lys Leu Glu Ile Cys Asn Leu Thr Ala Asp Ala Leu Gln Ser

325                 330                 335

Asp Lys Leu Gln Thr Trp Thr Val Ser Val Asn Glu Gly Arg Trp Val

            340                 345                 350

Arg Gly Cys Ser Ala Gly Gly Cys Arg Asn Phe Pro Asp Thr Phe Trp

        355                 360                 365

Thr Asn Pro Gln Tyr Arg Leu Lys Leu Leu Glu Glu Asp Asp Asp Pro

    370                 375                 380

Asp Asp Ser Glu Val Ile Cys Ser Phe Leu Val Ala Leu Met Gln Lys

385                 390                 395                 400

Asn Arg Arg Lys Asp Arg Lys Leu Gly Ala Ser Leu Phe Thr Ile Gly

                405                 410                 415

Phe Ala Ile Tyr Glu Val Pro Lys Glu Val

            420                 425

<210>  69

<211>  728

<212>  PRT

<213>  Homo sapiens

<220>

<221>  calpain 3, (p94) (CAPN3), transcript variant 7

<222>  (1)..(728)

<223>  LocusID: 825

NM_212465

<400> 69

Met Ser Trp Gln Ile Ser Leu Lys Thr Gln Thr Thr Gln Gln Lys Asn
1               5                   10                  15

Glu Ile Cys Glu Asn Pro Arg Phe Ile Ile Asp Gly Ala Asn Arg Thr
            20                  25                  30

Asp Ile Cys Gln Gly Glu Leu Gly Asp Cys Trp Phe Leu Ala Ala Ile
        35                  40                  45

Ala Cys Leu Thr Leu Asn Gln His Leu Leu Phe Arg Val Ile Pro His
    50                  55                  60

Asp Gln Ser Phe Ile Glu Asn Tyr Ala Gly Ile Phe His Phe Gln Phe
65                  70                  75                  80

Trp Arg Tyr Gly Glu Trp Val Asp Val Val Ile Asp Asp Cys Leu Pro
                85                  90                  95

Thr Tyr Asn Asn Gln Leu Val Phe Thr Lys Ser Asn His Arg Asn Glu
            100                 105                 110

Phe Trp Ser Ala Leu Leu Glu Lys Ala Tyr Ala Lys Leu His Gly Ser
        115                 120                 125

Tyr Glu Ala Leu Lys Gly Gly Asn Thr Thr Glu Ala Met Glu Asp Phe
    130                 135                 140

Thr Gly Gly Val Ala Glu Phe Phe Glu Ile Arg Asp Ala Pro Ser Asp
145                 150                 155                 160

Met Tyr Lys Ile Met Lys Lys Ala Ile Glu Arg Gly Ser Leu Met Gly
                165                 170                 175
Cys Ser Ile Asp Asp Gly Thr Asn Met Thr Tyr Gly Thr Ser Pro Ser
            180                 185                 190
Gly Leu Asn Met Gly Glu Leu Ile Ala Arg Met Val Arg Asn Met Asp
        195                 200                 205
Asn Ser Leu Leu Gln Asp Ser Asp Leu Asp Pro Arg Gly Ser Asp Glu
    210                 215                 220
Arg Pro Thr Arg Thr Ile Ile Pro Val Gln Tyr Glu Thr Arg Met Ala
225                 230                 235                 240
Cys Gly Leu Val Arg Gly His Ala Tyr Ser Val Thr Gly Leu Asp Glu
                245                 250                 255
Val Pro Phe Lys Gly Glu Lys Val Lys Leu Val Arg Leu Arg Asn Pro
            260                 265                 270
Trp Gly Gln Val Glu Trp Asn Gly Ser Trp Ser Asp Arg Trp Lys Asp
        275                 280                 285
Trp Ser Phe Val Asp Lys Asp Glu Lys Ala Arg Leu Gln His Gln Val
    290                 295                 300
Thr Glu Asp Gly Glu Phe Trp Met Ser Tyr Glu Asp Phe Ile Tyr His
305                 310                 315                 320
Phe Thr Lys Leu Glu Ile Cys Asn Leu Thr Ala Asp Ala Leu Gln Ser
                325                 330                 335
Asp Lys Leu Gln Thr Trp Thr Val Ser Val Asn Glu Gly Arg Trp Val
            340                 345                 350

Arg Gly Cys Ser Ala Gly Gly Cys Arg Asn Phe Pro Asp Thr Phe Trp
        355                 360                 365
Thr Asn Pro Gln Tyr Arg Leu Lys Leu Leu Glu Glu Asp Asp Asp Pro
    370                 375                 380
Asp Asp Ser Glu Val Ile Cys Ser Phe Leu Val Ala Leu Met Gln Lys
385                 390                 395                 400
Asn Arg Arg Lys Asp Arg Lys Leu Gly Ala Ser Leu Phe Thr Ile Gly
                405                 410                 415
Phe Ala Ile Tyr Glu Val Pro Lys Glu Met His Gly Asn Lys Gln His
            420                 425                 430
Leu Gln Lys Asp Phe Phe Leu Tyr Asn Ala Ser Lys Ala Arg Ser Lys
        435                 440                 445
Thr Tyr Ile Asn Met Arg Glu Val Ser Gln Arg Phe Arg Leu Pro Pro
    450                 455                 460
Ser Glu Tyr Val Ile Val Pro Ser Thr Tyr Glu Pro His Gln Glu Gly
465                 470                 475                 480
Glu Phe Ile Leu Arg Val Phe Ser Glu Lys Arg Asn Leu Ser Glu Glu
                485                 490                 495
Val Glu Asn Thr Ile Ser Val Asp Arg Pro Val Pro Ile Ile Phe Val
            500                 505                 510
Ser Asp Arg Ala Asn Ser Asn Lys Glu Leu Gly Val Asp Gln Glu Ser
        515                 520                 525
Glu Glu Gly Lys Gly Lys Thr Ser Pro Asp Lys Gln Lys Gln Ser Pro
    530                 535                 540

Gln Pro Gln Pro Gly Ser Ser Asp Gln Glu Ser Glu Glu Gln Gln Gln
545                 550                 555                 560
Phe Arg Asn Ile Phe Lys Gln Ile Ala Gly Asp Asp Met Glu Ile Cys
                565                 570                 575
Ala Asp Glu Leu Lys Lys Val Leu Asn Thr Val Val Asn Lys His Lys
            580                 585                 590
Asp Leu Lys Thr His Gly Phe Thr Leu Glu Ser Cys Arg Ser Met Ile
        595                 600                 605
Ala Leu Met Asp Thr Asp Gly Ser Gly Lys Leu Asn Leu Gln Glu Phe
    610                 615                 620
His His Leu Trp Asn Lys Ile Lys Ala Trp Gln Lys Ile Phe Lys His
625                 630                 635                 640
Tyr Asp Thr Asp Gln Ser Gly Thr Ile Asn Ser Tyr Glu Met Arg Asn
                645                 650                 655
Ala Val Asn Asp Ala Gly Phe His Leu Asn Asn Gln Leu Tyr Asp Ile
            660                 665                 670
Ile Thr Met Arg Tyr Ala Asp Lys His Met Asn Ile Asp Phe Asp Ser
        675                 680                 685
Phe Ile Cys Cys Phe Val Arg Leu Glu Gly Met Phe Arg Ala Phe His
    690                 695                 700
Ala Phe Asp Lys Asp Gly Asp Gly Ile Ile Lys Leu Asn Val Leu Glu
705                 710                 715                 720
Trp Leu Gln Leu Thr Met Tyr Ala
                725

<210>  70

<211>  107

<212>  PRT

<213>  Homo sapiens

<220>

<221>  calpain 3, (p94) (CAPN3), transcript variant 9

<222>  (1)..(107)

<223>  LocusID: 825

NM_212467

<400>  70

Met Ser Trp Gln Ile Ser Leu Lys Thr Gln Thr Thr Gln Gln Lys Asn

1               5                   10                  15

Glu Ile Cys Glu Asn Pro Arg Phe Ile Ile Asp Gly Ala Asn Arg Thr

            20                  25                  30

Asp Ile Cys Gln Gly Glu Leu Asp Gly Phe Gln Gly Ser Cys Leu Pro

        35                  40                  45

Gly Leu Pro Ser Asn Thr Asn His Leu Gly Gly Trp Phe Ser Glu Ala

    50                  55                  60

Gln Ser Ser Leu Lys Ser Glu Glu Glu Leu Asp Ser His Cys Lys Gly

65                  70                  75                  80

Thr Gly Gln Gly Arg Phe Pro Thr Gly Val Arg Lys Asn Asn Pro Val

                85                  90                  95

Met Ile Thr Tyr Cys Ser Val Ser Ile Glu Ala

            100                 105